# EUROPEAN PATENT SPECIFICATION

(11) **EP 3 976 617 B1**
(45) Date of publication and mention of the grant of the patent: **23.04.2025**
(21) Application number: 20744199.9
(22) Date of filing: 27.05.2020
(51) Int. Cl.: C07D 471/14, C07D 498/14, A61K 31/551, A61P 31/12

(54) **FUSED HETEROCYCLIC DERIVATIVES**
ANNELIERTE HETEROZYKLISCHE DERIVATE
DÉRIVÉS HÉTÉROCYCLIQUES FUSIONÉS

(30) Priority: 28.05.2019 EP 19176933; 28.05.2019 US 201962853533 P; 20.04.2020 WO PCT/CN2020/085720
(43) Date of publication of application: 06.04.2022
(73) Proprietor: Janssen Sciences Ireland Unlimited Company, Ringaskiddy, Co Cork (IE)
(72) Inventor: GROSSE, Sandrine Céline, 2340 Beerse (BE); DERATT, Lindsey Graham, Raritan, New Jersey 08869 (US); VANDYCK, Koen, 2340 Beerse (BE); RABOISSON, Pierre Jean-Marie Bernard, 2340 Beerse (BE); PIETERS, Serge Maria Aloysius, 2340 Beerse (BE); KESTELEYN, Bart Rudolf Romanie, 2340 Beerse (BE); VERSCHUEREN, Wim Gaston, 2340 Beerse (BE); BERKE, Jan Martin, 2340 Beerse (BE); LECOMTE, Morgan Charles R., 2340 Beerse (BE); MARTINEZ LAMENCA, Carolina, 2340 Beerse (BE); JONCKERS, Tim Hugo Maria, 2340 Beerse (BE); DENG, Gang, Shanghai (CN); JIANG, Yimin, Shanghai (CN); XU, Yanping, Shanghai (CN); CHENG, Zhanling, Shanghai (CN); HU, Lili, 2340 Beerse (BE); KUDUK, Scott D., Raritan, New Jersey 08869 (US)
(74) Representative: Garcia Prieto, Maria
(86) International application number: PCT/US2020/034643
(87) International publication number: WO 2020/243135

(56) References cited:
- WO-A1-2016/038045
- WO-A1-2018/005881
- WO-A1-2018/005883
- US-A1- 2016 185 777

## Description

### FIELD

The application relates to fused heterocyclic derivative compounds, pharmaceutical compositions comprising these compounds, chemical processes for preparing these compounds and their use in the treatment of diseases associated with HBV infection.

### RELATED APPLICATIONS

This application claims priority to European Application No. 19176933.0 filed May 28, 2019, PCT/CN2020/085720 filed April 20, 2020, and U.S. Provisional Application No. 62/853,533 filed on May 28, 2019, the contents of which are hereby incorporated in their entirety.

### BACKGROUND

Chronic hepatitis B virus (HBV) infection is a significant global health problem, affecting over 5% of the world population (over 350 million people worldwide and 1.25 million individuals in the U.S.).

Despite the availability of a prophylactic HBV vaccine, the burden of chronic HBV infection continues to be a significant unmet worldwide medical problem, due to suboptimal treatment options and sustained rates of new infections in most parts of the developing world. Current treatments do not provide a cure and are limited to only two classes of agents (interferon alpha and nucleoside analogues/inhibitors of the viral polymerase); drug resistance, low efficacy, and tolerability issues limit their impact. The low cure rates of HBV are attributed at least in part to the fact that complete suppression of virus production is difficult to achieve with a single antiviral agent. However, persistent suppression of HBV DNA slows liver disease progression and helps to prevent hepatocellular carcinoma. Current therapy goals for HBV-infected patients are directed to reducing serum HBV DNA to low or undetectable levels, and to ultimately reducing or preventing the development of cirrhosis and hepatocellular carcinoma.

The HBV capsid protein plays essential functions during the viral life cycle. HBV capsid/core proteins form metastable viral particles or protein shells that protect the viral genome during intercellular passage, and also play a central role in viral replication processes, including genome encapsidation, genome replication, and virion morphogenesis and egress. Capsid structures also respond to environmental cues to allow un-coating after viral entry. Consistently, the appropriate timing of capsid assembly and dis-assembly, the appropriate capsid stability and the function of core protein have been found to be critical for viral infectivity.

The crucial function of HBV capsid proteins imposes stringent evolutionary constraints on the viral capsid protein sequence, leading to the observed low sequence variability and high conservation. Consistently, mutations in HBV capsid that disrupt its assembly are lethal, and mutations that perturb capsid stability severely attenuate viral replication. The high functional constraints on the multi-functional HBV core/capsid protein is consistent with a high sequence conservation, as many mutations are deleterious to function. Indeed, the core/capsid protein sequences are >90% identical across HBV genotypes and show only a small number of polymorphic residues. Resistance selection to HBV core/capsid protein binding compounds may therefore be difficult to select without large impacts on virus replication fitness.

Reports describing compounds that bind viral capsids and inhibit replication of HIV, rhinovirus and HBV provide strong pharmacological proof of concept for viral capsid proteins as antiviral drug targets.

There is a need in the art for therapeutic agents that can increase the suppression of virus production and that can treat, ameliorate, and/or prevent HBV infection. Administration of such therapeutic agents to an HBV infected patient, either as monotherapy or in combination with other HBV treatments or ancillary treatments, will lead to significantly reduced virus burden, improved prognosis, diminished progression of the disease and enhanced seroconversion rates.

In view of the clinical importance of HBV, the identification of compounds that can increase the suppression of virus production and that can treat, ameliorate, and/or prevent HBV infection represents an attractive avenue into the development of new therapeutic agents. Such compounds are provided herein.

Background art includes WO2018/005881. Herein oxadiazepinone derivatives are disclosed for use in the treatment of hetaptitis-B infections. The main teaching in WO2018/005881 is to provide compounds that have an aminocarbonyl substituent on a central ring nitrogen, resulting in a urea moiety present. The background art further includes WO 2018/005883, disclosing diazepinone derivatives for use in the treatment of hetaptitis-B infections.

### SUMMARY

The present disclosure is directed to the general and preferred embodiments defined, respectively, by the independent and dependent claims appended hereto, which are incorporated by reference herein. The present invention is directed to compounds capable of capsid assembly modulation. The compounds of the present invention may provide a beneficial balance of properties with respect to prior art compounds, e.g. they may display a different profile, display improved solubility, etc. Thus, in particular, the present disclosure is directed to a compound of Formula (I): 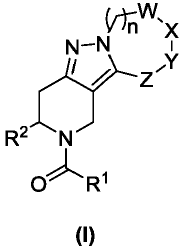
or a stereoisomer or tautomer thereof,
wherein
   R¹ is a 5- to 10-membered monocyclic or bicyclic ring, more particularly a 5- to 9-membered monocyclic or bicyclic ring, wherein the 5- to 10-membered monocyclic or bicyclic ring, more particularly the 5- to 9-membered monocyclic or bicyclic ring :
      - optionally contains 1 to 3 heteroatoms, the heteroatoms each independently being selected from N, O and S; and/or
      - is optionally substituted with one or more substituents each independently selected from hydrogen, halogens, CN, CF₃, CF₂H, CFH₂, CF₂CH₃, C₁₋₆alkyl, OC₁₋₆alkyl, OCF₃, OCF₂H and C₃₋₄cycloalkyl;
   more particularly R¹ is phenyl substituted with one or more substituents each independently selected from the group consisting of Cl, F, CN, CH₃, CF₃, and CF₂H;
   R² is selected from the group consisting of H, C₁₋₄alkyl and C₁₋₄alkyl substituted with one or more F;
   n is an integer of 0 or 1;
   W is CHR³ or C=CH₂;
   R³ is selected from the group consisting of H, F, OH, C₁₋₄alkyl optionally substituted with OH or F, CONHC₁₋₄alkyl, CONHC₃₋₆cycloalkyl, NHSO₂C₁₋₄alkyl and NHSO₂C₃₋₆cycloalkyl;
   X is CH₂;
   Y is NR⁵;
   R⁵ is selected from the group consisting of
      H,
      C₁₋₄alkyl,
      C₁₋₄alkyl substituted with one of more F,
      C₁₋₄alkyl substituted with a 5- to 10-membered monocyclic or bicyclic aromatic ring, wherein the 5- to 10-membered monocyclic or bicyclic aromatic ring optionally contains one, two, three or four heteroatoms, the heteroatoms independently being selected from N, O and S, and
      wherein the said 5- to 10-membered monocyclic or bicyclic aromatic ring is optionally substituted with one or more substituents each independently selected from the group consisting of hydrogen, halogens, CN, CF₃, CF₂H, CFH₂, CF₂CH₃, CH₂CF₃, C₁₋₄alkyl, OH, OC₁₋₄alkyl, OCF₃, OCF₂H, SO₂N(CH₃)₂ and C₃₋₄cycloalkyl; and
   Z is selected from the group consisting of C(=O) and C(=S),
   or a pharmaceutically acceptable salt thereof.

Further embodiments include pharmaceutically acceptable salts of compounds of Formula (I), pharmaceutically acceptable prodrugs of compounds of Formula (I), pharmaceutically active metabolites of compounds of Formula (I), and enantiomers and diastereomers of the compounds of Formula (I), as well as pharmaceutically acceptable salts thereof.

In embodiments, the compounds of Formula (I) are compounds selected from those species described or exemplified in the detailed description below.

The present disclosure is also directed to pharmaceutical compositions comprising one or more compounds of Formula (I), pharmaceutically acceptable salts of compounds of Formula (I), pharmaceutically acceptable prodrugs of compounds of Formula (I), and pharmaceutically active metabolites of Formula (I). Pharmaceutical compositions may further comprise one or more pharmaceutically acceptable excipients or one or more other agents or therapeutics.

The present disclosure is also directed to methods of using or uses of compounds of Formula (I). In embodiments, compounds of Formula (I) are used to treat or ameliorate hepatitis B viral (HBV) infection, increase the suppression of HBV production, interfere with HBV capsid assembly or other HBV viral replication steps or products thereof. The methods comprise administering to a subject in need of such method an effective amount of at least one compound of Formula (I), pharmaceutically acceptable salts of compounds of Formula (I), pharmaceutically acceptable prodrugs of compounds of Formula (I), and pharmaceutically active metabolites of compounds of Formula (I). Additional embodiments of methods of treatment are set forth in the detailed description.

The present disclosure is also directed to compounds of Formula (Ia): 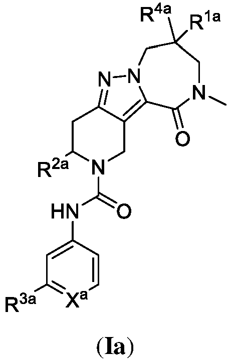
and pharmaceutically acceptable salts, stereoisomers, isotopic variants, N-oxides, or solvates of compounds of Formula (Ia);
wherein
   R^{1a} is C₆₋₁₀aryl or a 5- or 6-membered heteroaryl, wherein R^{1a} is optionally substituted with a substituent selected from: methyl or fluoro;
   R^{2a} is independently selected from the group consisting of: hydrogen and C₁₋₆alkyl;
   R^{3a} is selected from the group consisting of: Cl, CN, and C₁₋₄haloalkyl;
   R^{4a} is selected from the group consisting of: hydrogen, hydroxy, fluoro, and methyl; and
   X^{a} is CF or N.

Further embodiments include pharmaceutically acceptable salts of compounds of Formula (Ia), pharmaceutically acceptable prodrugs of compounds of Formula (Ia), pharmaceutically active metabolites of compounds of Formula (Ia), and enantiomers and diastereomers of the compounds of Formula (Ia), as well as pharmaceutically acceptable salts thereof.

In embodiments, the compounds of Formula (Ia) are compounds selected from those species described or exemplified in the detailed description below.

The present disclosure is also directed to pharmaceutical compositions comprising one or more compounds of Formula (Ia), pharmaceutically acceptable salts of compounds of Formula (Ia), pharmaceutically acceptable prodrugs of compounds of Formula (Ia), and pharmaceutically active metabolites of Formula (Ia). Pharmaceutical compositions may further comprise one or more pharmaceutically acceptable excipients or one or more other agents or therapeutics.

The present disclosure is also directed to methods of using or uses of compounds of Formula (Ia). In embodiments, compounds of Formula (Ia) are used to treat or ameliorate hepatitis B viral (HBV) infection, increase the suppression of HBV production, interfere with HBV capsid assembly or other HBV viral replication steps or products thereof. The methods comprise administering to a subject in need of such method an effective amount of at least one compound of Formula (Ia), pharmaceutically acceptable salts of compounds of Formula (Ia), pharmaceutically acceptable prodrugs of compounds of Formula (Ia), and pharmaceutically active metabolites of compounds of Formula (Ia). Additional embodiments of methods of treatment are set forth in the detailed description.

An object of the present disclosure is to overcome or ameliorate at least one of the disadvantages of the conventional methodologies and/or prior art, or to provide a useful alternative thereto. Additional embodiments, features, and advantages of the present disclosure will be apparent from the following detailed description and through practice of the disclosed subject matter.

### DETAILED DESCRIPTION

Additional embodiments, features, and advantages of the subject matter of the present disclosure will be apparent from the following detailed description of such disclosure and through its practice. For the sake of brevity, the publications, including patents, cited in this specification are herein incorporated by reference.

Provided herein are compounds of Formula (I), and their pharmaceutically acceptable salts, pharmaceutically acceptable prodrugs, and pharmaceutically active metabolites of the disclosed compounds.

In one embodiment, provided herein are compounds of Formula (I), 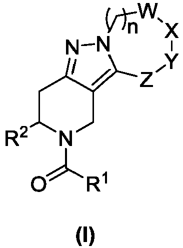
or a stereoisomer or tautomer thereof, or a pharmaceutically acceptable salt thereof,
wherein
   R¹ is phenyl substituted with one or more substituents, in particular one to three, each independently selected from the group consisting of halogens, CN, CF₃, CF₂H, CFH₂, CF₂CH₃, C₁₋₄alkyl, OC₁₋₆alkyl, OCF₃, OCF₂H and C₃₋₄cycloalkyl; or a 9-membered bicyclic ring containing 1 or 2 N atoms, and optionally substituted with one or more substituents, in particular one to three substituents, each independently selected from the group consisting of halogens, CN, CF₃, CF₂H, CFH₂, CF₂CH₃, C₁₋₆alkyl, OC₁₋₆alkyl, OCF₃, OCF₂H and C₃₋₄cycloalkyl;
   R² is selected from the group consisting of H, C₁₋₄alkyl and C₁₋₄alkyl substituted with one or more F;
   n is an integer of 0 or 1;
   W is CHR³ or C=CH₂;
   R³ is selected from the group consisting of H, F, C₁₋₄alkyl optionally substituted with OH or F, CONHC₁₋₄alkyl, CONHC₃₋₆cycloalkyl, NHSO₂C₁₋₄alkyl and NHSO₂C₃₋₆cycloalkyl;
   X is CH₂;
   Y is NR⁵;
   R⁵ is selected from the group consisting of
      H,
      C₁₋₄alkyl,
      C₁₋₄alkyl substituted with one of more F,
      C₁₋₄alkyl substituted with a 5- to 10-membered monocyclic or bicyclic aromatic ring, wherein the 5- to 10-membered monocyclic or bicyclic aromatic ring optionally contains one, two, three or four heteroatoms, the heteroatoms independently being selected from N, O and S, and
      wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, CH₂CF₃, C₁₋₄alkyl, OH, OC₁₋₄alkyl, OCF₃, OCF₂H, SO₂N(CH₃)₂ and C₃₋₄cycloalkyl; and
   Z is selected from the group consisting of C(=O) and C(=S).

In an additional embodiment, the invention provides compounds of Formula (I), and the stereoisomers or tautomers thereof, and the pharmaceutically acceptable salts thereof, as described herein, wherein
R¹ is a 5- to 10-membered monocyclic or bicyclic ring, more particularly a 5- to 9-membered monocyclic or bicyclic ring, wherein the 5- to 10-membered monocyclic or bicyclic ring, more particularly the 5- to 9-membered monocyclic or bicyclic ring :
   - optionally contains 1 to 3 heteroatoms, the heteroatoms independently being selected from N, O and S; and/or
   - is optionally substituted with one or more substituents selected from hydrogen, halogens, CN, CF₃, CF₂H, CFH₂, CF₂CH₃, C₁₋₆alkyl, OC₁₋₆alkyl, OCF₃, OCF₂H and C₃₋₄cycloalkyl;
more particularly R¹ is phenyl substituted with one or more substituents selected from the group consisting of Cl, F, CN, CH₃, CF₃, and CF₂H;
R² is selected from the group consisting of H, C₁₋₄alkyl and C₁₋₄alkyl substituted with one or more F;
n is an integer of 0 or 1;
W is CHR³ or C=CH₂;
R³ is selected from the group consisting of H, F, OH, C₁₋₄alkyl optionally substituted with OH or F, CONHC₁₋₄alkyl, CONHC₃₋₆cycloalkyl, NHSO₂C₁₋₄alkyl and NHSO₂C₃₋₆cycloalkyl;
X is CH₂;
Y is NR⁵;
R⁵ is selected from the group consisting of
   H,
   C₁₋₄alkyl,
   C₁₋₄alkyl substituted with one of more F,
   C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and
   wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H and C₃₋₄cycloalkyl; and
Z is selected from the group consisting of C(=O) and C(=S).

In a further embodiment, the invention provides a compound of Formula (I), or a stereoisomer or tautomer thereof, or a pharmaceutically acceptable salt thereof,
wherein
R¹ is phenyl substituted with one or more substituents selected from the group consisting of Cl, F, CN, CH₃, CF₃, and CF₂H;
R² is selected from the group consisting of H, C₁₋₄alkyl and C₁₋₄alkyl substituted with one or more F;
n is an integer of 0 or 1;
W is CHR³ or C=CH₂;
R³ is selected from the group consisting of H, F, C₁₋₄alkyl optionally substituted by OH or F, CONHC₁₋₄alkyl, and CONHC₃₋₆cycloalkyl, NHSO₂C₁₋₄alkyl; NHSO₂C₃₋₆cycloalkyl
X is CH₂;
Y is NR⁵;
R⁵ is selected from the group consisting of
   H,
   C₁₋₄alkyl,
   C₁₋₄alkyl substituted with one or more F,
   C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and
   wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H and C₃₋₄cycloalkyl; and
Z is selected from the group consisting of C(=O) and C(=S).

In a further embodiment, the invention provides a compound of Formula (I), or a stereoisomer or tautomer thereof, or a pharmaceutically acceptable salt thereof,
wherein
R¹ is phenyl substituted with one or more substituents, in particular one to three substituents, each independently selected from the group consisting of halo, in particular Cl or F, CN, C₁₋₆alkyl, in particular CH₃, CH₂F, CHF₂, and CF₃H;
R² is selected from the group consisting of H, C₁₋₄alkyl and C₁₋₄alkyl substituted with one or more F;
n is 0;
W is CHR³;
R³ is selected from the group consisting of H, F, C₁₋₄alkyl optionally substituted by OH or F, and NHSO₂C₁₋₄alkyl;
X is CH₂;
Y is NR⁵;
R⁵ is selected from the group consisting of
   C₁₋₄alkyl, in particular C₁₋₂alkyl, substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, oxadiazolyl, indolyl, imidazo[1,2-a]pyridinyl, and quinolinyl;
   wherein the said 5- to 10-membered monocyclic or bicyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl; and
Z is selected from the group consisting of C(=O) and C(=S).

In a further embodiment, the invention provides a compound of Formula (I), or a stereoisomer or tautomer thereof, or a pharmaceutically acceptable salt thereof,
wherein
R¹ is phenyl substituted with one or more substituents, in particular one to three substituents, each independently selected from the group consisting of halo, in particular Cl or F, CN, C₁₋₆alkyl, in particular CH₃, CH₂F, CHF₂, CF₃ and CF₂CH₃;
R² is selected from the group consisting of H, C₁₋₄alkyl and C₁₋₄alkyl substituted with one or more F;
n is 0;
W is CHR³;
R³ is selected from the group consisting of H, F, C₁₋₄alkyl optionally substituted by OH or F, and NHSO₂C₁₋₄alkyl;
X is CH₂;
Y is NR⁵;
R⁵ is selected from the group consisting of
   C₁₋₄alkyl, in particular C₁₋₂alkyl, substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, oxadiazolyl, indolyl, imidazo[1,2-a]pyridinyl, and quinolinyl;
   wherein the said 5- to 10-membered monocyclic or bicyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl; and
Z is selected from the group consisting of C(=O) and C(=S).

In yet a further embodiment, the invention provides a compound of Formula (I), or a stereoisomer or tautomer thereof, or a pharmaceutically acceptable salt thereof,
wherein
R¹ is phenyl substituted with one or more substituents, in particular one to three substituents, each independently selected from the group consisting of halo, in particular Cl or F, CN, C₁₋₆alkyl, in particular CH₃, CH₂F, CHF₂, CF₃ and CF₂CH₃;
R² is H or methyl, in particular methyl;
n is 0;
W is CHR³;
R³ is H;
X is CH₂;
Y is NR⁵;
R⁵ is selected from the group consisting of
   C₁₋₄alkyl, in particular C₁₋₂alkyl, substituted with a 5- to 6-membered monocyclic aromatic ring, optionally containing one, two or three heteroatoms, each independently selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, and oxadiazolyl;
   wherein the said 5- to 6-membered monocyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl; and
Z is C(=O).

In embodiments, the compound of Formula (I) is a compound wherein R¹ is phenyl substituted with one or more substituents, in particular one to three substituents, each independently selected from the group consisting of fluoro, chlorine and cyano substituents, more particularly wherein R¹ is dichlorophenyl, 3-cyano-4-chlorophenyl; or 3-cyano-4-fluorophenyl.

In embodiments, the compound of Formula (I) is a compound wherein R¹ is phenyl substituted with one or more chlorine substituents, more particularly wherein R¹ is dichlorophenyl.

In embodiments, the compound of Formula (I) is a compound wherein R² is H or methyl.

In embodiments, the compound of Formula (I) is a compound wherein Z is C=O.

In embodiments, the compound of Formula (I) is a compound wherein X is CH₂.

In embodiments, the compound of Formula (I) is a compound wherein W is CH₂ or CHF.

In embodiments, the compound of Formula (I) is a compound wherein R⁵ is selected from the group consisting of C₁₋₄alkyl and C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and wherein the said 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound wherein R⁵ is C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms being N, and wherein the said 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound wherein R⁵ is C₁₋₄alkyl substituted with a 5- to 10-membered monocyclic or bicyclic aromatic ring, wherein the 5- to 10-membered monocyclic or bicyclic aromatic ring is selected from the group consisting of phenyl, pyridyl, pyrazolyl or indolyl, and wherein the 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound wherein R⁵ is C₁₋₄alkyl substituted with a 5- to 10-membered monocyclic or bicyclic aromatic ring, wherein the 5- to 10-membered monocyclic or bicyclic aromatic ring is selected from the group consisting of phenyl, pyridyl, pyrazolyl or indolyl, and wherein the 5- to 10-membered monocyclic or bicyclic aromatic ring is optionally substituted with one or more substituents selected from the group consisting of halogens, C₁₋₄alkyl, OC₁₋₄alkyl, OCF₃ and OCF₂H.

In embodiments, the compound of Formula (I) is a compound as described herein wherein Y is NR⁵; wherein
R⁵ is selected from the group consisting of
C₁₋₄alkyl, in particular C₁₋₂alkyl, substituted with a 5- to 10-membered monocyclic or bicyclic aromatic ring, wherein the 5- to 10-membered monocyclic or bicyclic aromatic ring optionally contains one, two, three or four heteroatoms, the heteroatoms each independently being selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, oxadiazolyl, tetrazolyl, indolyl, imidazo[1,2-a]pyridinyl, quinolinyl and [1,2,4]triazolo[1,5-a]pyrimidinyl;
wherein the said 5- to 10-membered monocyclic or bicyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound as described herein wherein Y is NR⁵; wherein
R⁵ is selected from the group consisting of
C₁₋₄alkyl, in particular C₁₋₂alkyl, substituted with a 5- to 6-membered monocyclic aromatic ring, optionally containing one, two, three or four heteroatoms, each independently selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, oxadiazolyl, and tetrazolyl;
wherein the said 5- to 6-membered monocyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound as described herein wherein Y is NR⁵; wherein
R⁵ is selected from the group consisting of
C₁₋₄alkyl, in particular C₁₋₂alkyl, substituted with a 5- to 6-membered monocyclic aromatic ring, optionally containing one, two or three heteroatoms, each independently selected from N, O and S, and selected from the group consisting of phenyl, pyridyl, pyrimidinyl, pyridazinyl, pyrazinyl, pyrazolyl, imidazolyl, isoxazolyl, oxazolyl, thiazolyl, triazolyl, and oxadiazolyl;
wherein the said 5- to 6-membered monocyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound as described herein wherein Y is NR⁵; wherein
R⁵ is selected from the group consisting of
C₁₋₄alkyl, in particular C₁₋₂alkyl, substituted with a 5- to 6-membered monocyclic aromatic ring, optionally containing one, two or three N atoms;
wherein the said 5- to 6-membered monocyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound as described herein wherein Y is NR⁵; wherein
R⁵ is selected from the group consisting of
C₁₋₄alkyl, in particular C₁₋₂alkyl, substituted with a 5- to 6-membered monocyclic aromatic ring, optionally containing one, two or three N atoms, selected from the group consisting of phenyl, pyridyl, pyrimidinyl, pyridazinyl, pyrazinyl, pyrazolyl, imidazolyl, and triazolyl;
wherein the said 5- to 6-membered monocyclic aromatic ring is optionally substituted with one or more substituents, in particular one to three substituents, each independently selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

In embodiments, the compound of Formula (I) is a compound which shows an EC₅₀ of less than 0.10 µM for the inhibition of HBV DNA in the hepG2.117 cell line.

A further embodiment of the present disclosure is a compound selected from the group consisting of the compounds described below (cf. Table 1), a stereoisomer or tautomer thereof, or a pharmaceutically acceptable salt thereof.

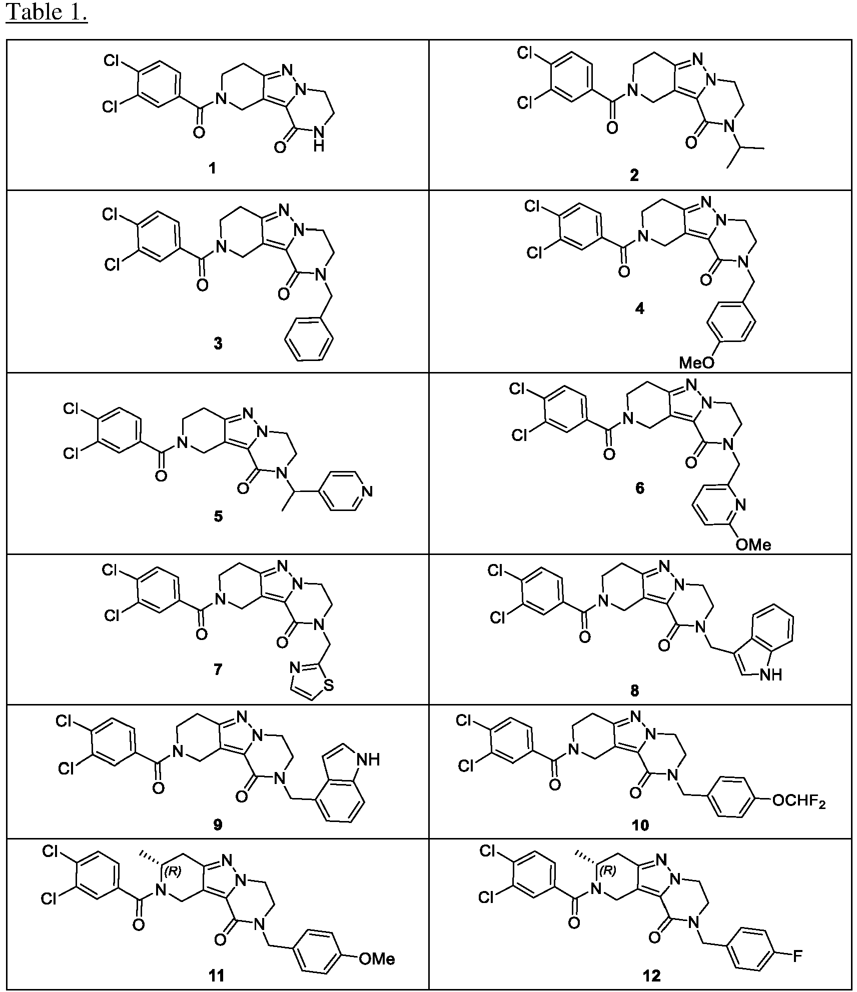

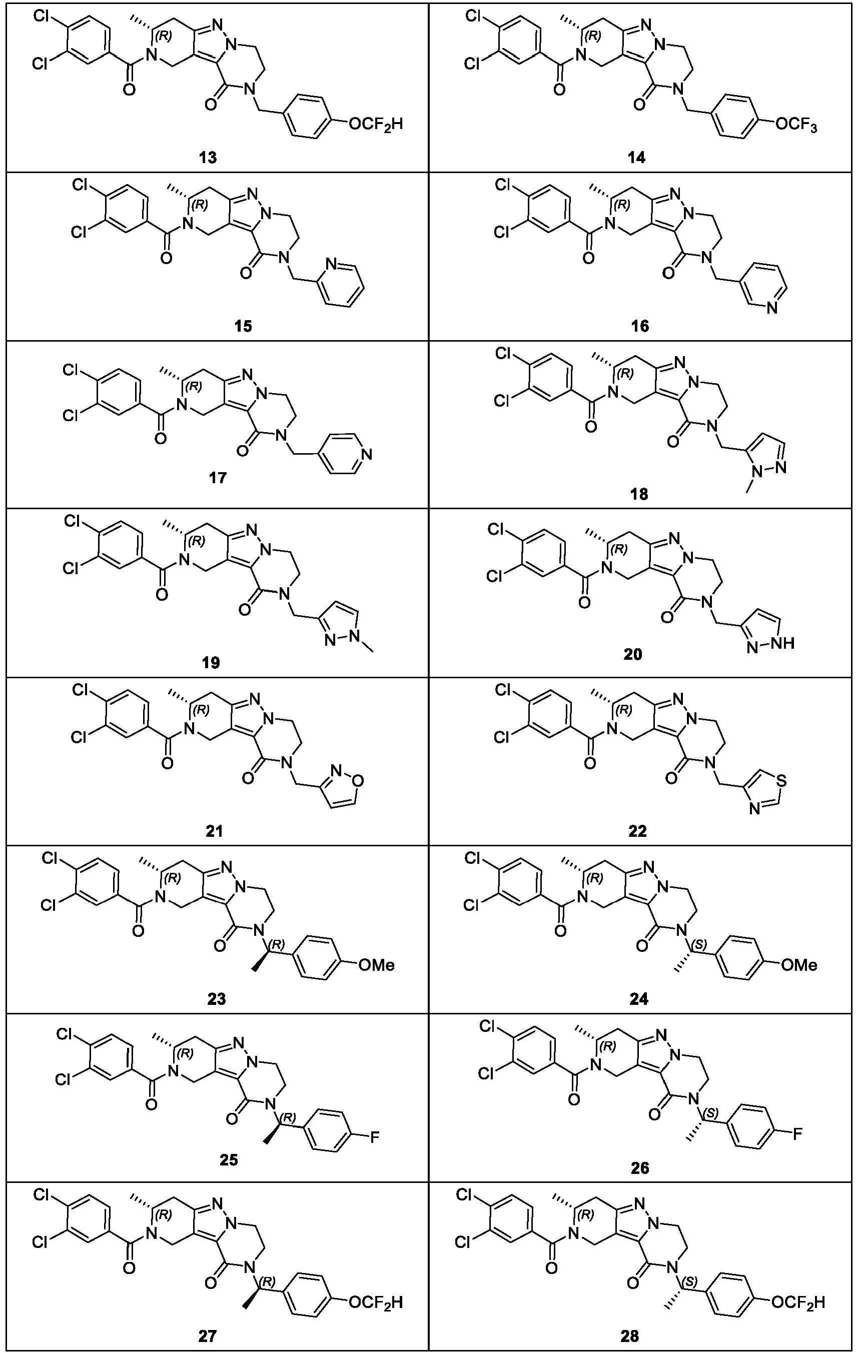

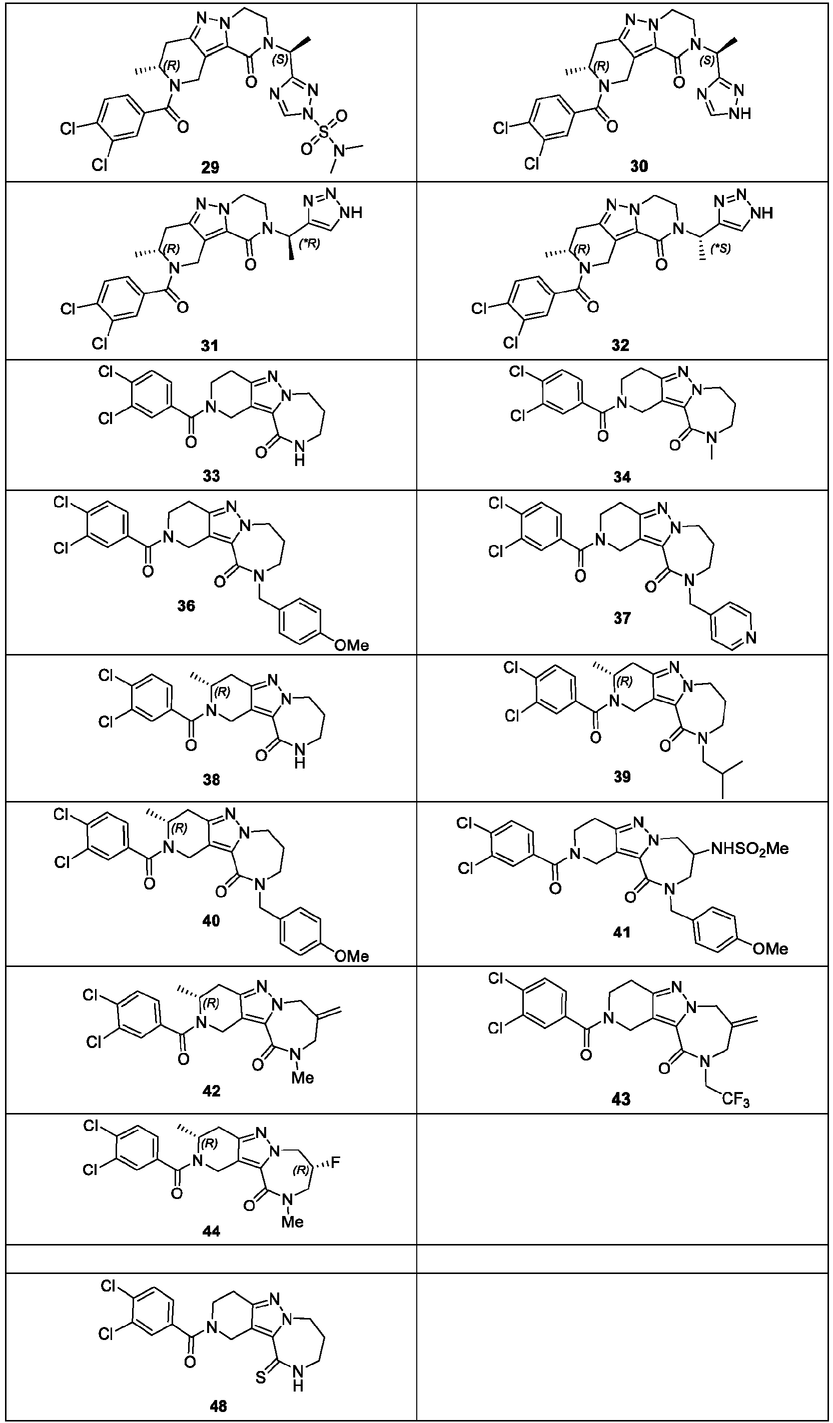

In one aspect, provided herein are compounds of Formula (Ia), and pharmaceutically acceptable salts, stereoisomers, isotopic variants, N-oxides, or solvates thereof, 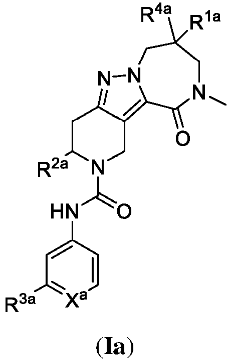 wherein
R^{1a} is C₆₋₁₀aryl or a 5- or 6-membered heteroaryl, wherein R^{1a} is optionally substituted with a substituent selected from methyl or fluoro;
R^{2a} is independently selected from the group consisting of: hydrogen and C₁₋₆alkyl;
R^{3a} is selected from the group consisting of: Cl, CN, and C₁₋₄haloalkyl;
R^{4a} is selected from the group consisting of: hydrogen, hydroxy, fluoro, and methyl; and
X^{a} is CF or N.

In embodiments, the compound of Formula (Ia) is a compound wherein R^{1a} is independently selected from: 1H-1,2,4-triazol-3-yl, 1,2,4-oxadiazol-5-yl, 1H-tetrazol-5-yl, 1H-pyrazol-3-yl, 1-methyl-1H-pyrazol-3-yl, 1-methyl-1H-pyrazol-5-yl, isoxazol-3-yl, isoxazol-5-yl, or phenyl.

In embodiments, the compound of Formula (Ia) is a compound wherein R^{2a} is selected from hydrogen or methyl.

In embodiments, the compound of Formula (Ia) is a compound wherein R^{3a} is Cl, CN, or C₁₋₄haloalkyl.

In embodiments, the compound of Formula (Ia) is a compound wherein X^{a} is CF.

In embodiments, the compound of Formula (Ia) is a compound wherein X^{a} is N.

In embodiments, the compound of Formula (Ia) is a compound wherein 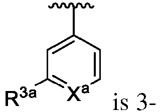 cyano-4-fluorophenyl, 3-chloro-4-fluorophenyl, or 4-fluoro-3-trifluoromethylphenyl.

In embodiments, the compound of Formula (Ia) is a compound wherein R^{4a} is hydrogen, hydroxy, or fluoro.

In embodiments, the compound of Formula (Ia) is a compound wherein R^{4a} is hydrogen.

In embodiments, the compound of Formula (Ia) is a compound wherein R^{4a} is hydroxy.

In embodiments, the compound of Formula (Ia) is a compound wherein R^{4a} is fluoro.

A further embodiment of the compound of Formula (Ia) is a compound as shown below:

**Table 2.**

| **Ex #** | **Structure** | **Compound_Name** |
|---|---|---|
| 1a | 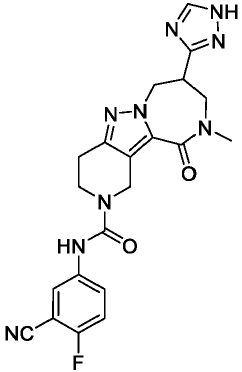 | N-(3-Cyano-4-fluorophenyl)-10-methyl-11-oxo-8-(1H-1,2,4-triazol-3-yl)-3,4,8,9, 10,11-hexahydro-1H-pyrido[4',3':3,4]-pyrazolo[1,5-a][1,4]diazepine-2(7H)-carboxamide; |
| 2a | 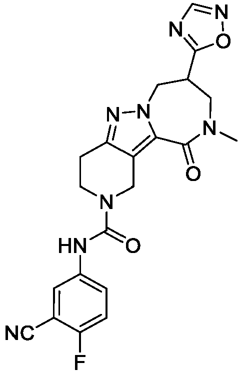 | N-(3-Cyano-4-fluorophenyl)-10-methyl-8-(1,2,4-oxadiazol-5-yl)-11-oxo-3,4,8,9,10,11-hexahydro-1H-pyrido[4',3':3,4]pyrazolo[1,5-a][1,4]diazepine-2(7H)-carboxamide; |
| 3a | 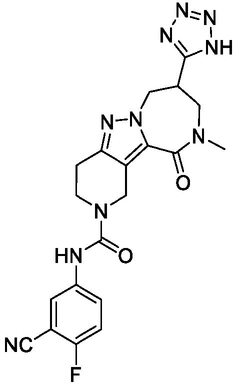 | N-(3-Cyano-4-fluorophenyl)-10-methyl-11-oxo-8-(1H-tetrazol-5-yl)-3,4,8,9,10,11-hexahydro-1H-pyrido[4',3':3,4]pyrazolo[1,5-a][1,4]diazepine-2(7H)-carboxamide; |
| 4a | 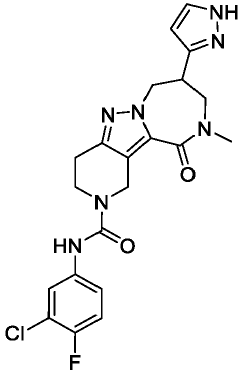 | N-(3-Chloro-4-fluorophenyl)-10-methyl-11-oxo-8-(1H-pyrazol-3-yl)-3,4,8, 9,10,11-hexahydro-1H-pyrido[4',3':3,4]pyrazolo[1,5-a][1,4]diazepine-2(7H)-carboxamide; |
| 5a | 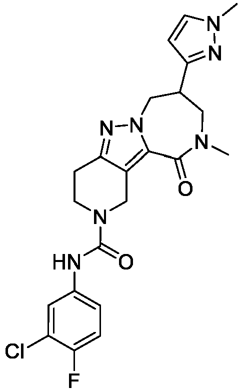 | N-(3-Cyano-4-fluorophenyl)-10-methyl-8-(1-methyl-1H-pyrazol-3-yl)-11-oxo-3,4,8,9,10,11-hexahydro-1H-pyrido[4',3':3,4]pyrazolo[1,5-a][1,4]diazepine-2(7H)-carboxamide; |
| 6a | 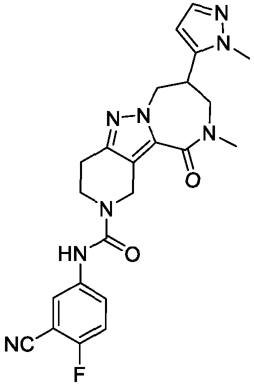 | N-(3-Cyano-4-fluorophenyl)-10-methyl-8-(1-methyl-1H-pyrazol-5-yl)-11-oxo-3,4,8,9,10,11-hexahydro-1H-pyrido[4',3':3,4]pyrazolo[1,5-a][1,4]diazepine-2(7H)-carboxamide; |
| 7a | 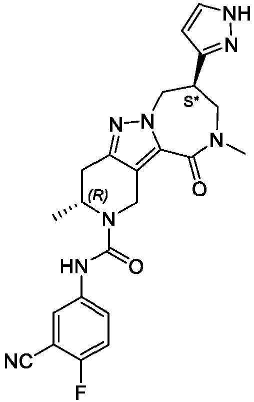 | (3R,8S*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; |
| 8a | 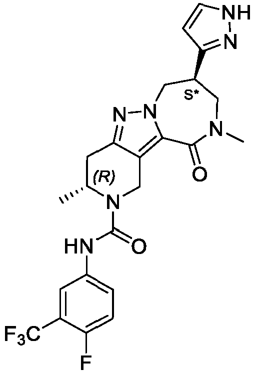 | (3R,8S*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; |
| 9a | 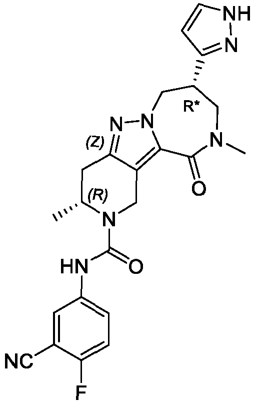 | (3R,8R*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; |
| 10a | 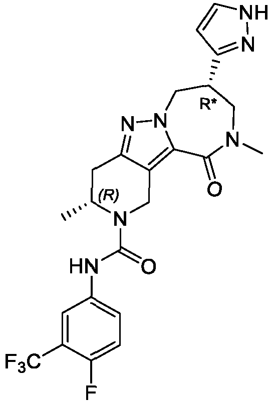 | (3R,8R*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide |
| 11a | 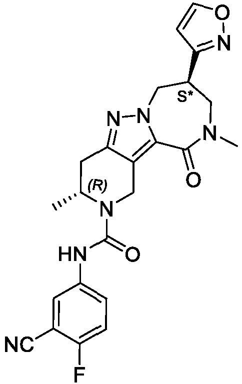 | (3R,8S*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide; |
| 12a | 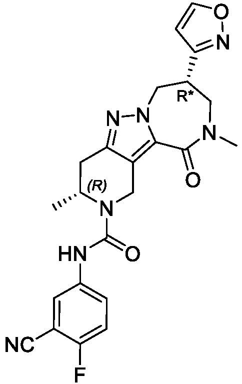 | (3R,8R*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide; |
| 13a | 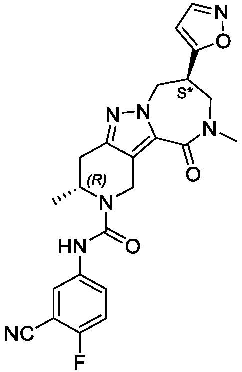 | (3R,8S*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide; |
| 14a | 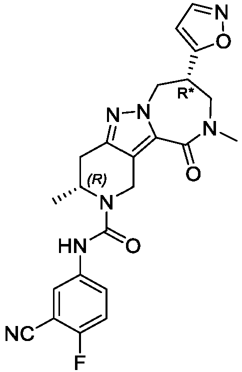 | (3R,8R*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide; |
| 15a | 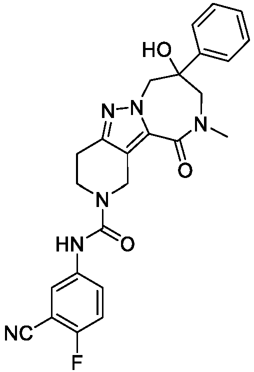 | N-(3-Cyano-4-fluoro-phenyl)-11-hydroxy-13-methyl-14-oxo-11-phenyl-4,8,9,13-tetrazatricyclo[7.5.0.02,7]tetradeca-1,7-diene-4-carboxamide; |
| 16a | 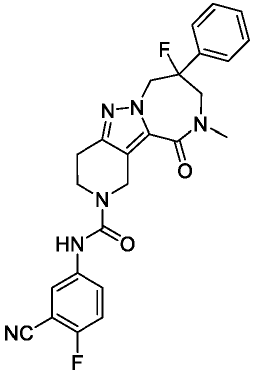 | N-(3-Cyano-4-fluorophenyl)-8-fluoro-10-methyl-11-oxo-8-phenyl-3,4,8,9,10,11-hexahydro-1H-pyrido[4',3':3,4]pyrazolo[1,5-a][1,4]diazepine-2(7H)-carboxamide; |

and pharmaceutically acceptable salts, N-oxides, or solvates thereof.

### Pharmaceutical Compositions

Also disclosed herein are pharmaceutical compositions comprising
(A) at least one compound of Formula (I): 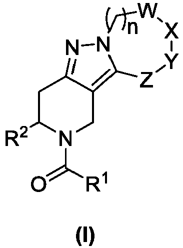
   or a stereoisomer or tautomer thereof,
   wherein
      R¹ is a 5- to 10-membered monocyclic or bicyclic ring, more particularly a 5- to 9-membered monocyclic or bicyclic ring, wherein the 5- to 10-membered monocyclic or bicyclic ring, more particularly the 5- to 9-membered monocyclic or bicyclic ring :
         - optionally contains 1 to 3 heteroatoms, the heteroatoms each independently being selected from N, O and S; and/or
         - is optionally substituted with one or more substituents each independently selected from hydrogen, halogens, CN, CF₃, CF₂H, CFH₂, CF₂CH₃, C₁₋₆alkyl, OC₁₋₆alkyl, OCF₃, OCF₂H and C₃₋₄cycloalkyl;
      more particularly R¹ is phenyl substituted with one or more substituents each independently selected from the group consisting of Cl, F, CN, CH₃, CF₃, and CF₂H;
      R² is selected from the group consisting of H, C₁₋₄alkyl and C₁₋₄alkyl substituted with one or more F;
      n is an integer of 0 or 1;
      W is CHR³ or C=CH₂;
      R³ is selected from the group consisting of H, F, OH, C₁₋₄alkyl optionally substituted with OH or F, CONHC₁₋₄alkyl, CONHC₃₋₆cycloalkyl, NHSO₂C₁₋₄alkyl and NHSO₂C₃₋₆cycloalkyl;
      X is selected from the group consisting of CH₂ and O;
      Y is NR⁵;
      R⁵ is selected from the group consisting of
         H,
         C₁₋₄alkyl,
         C₁₋₄alkyl substituted with one of more F,
         C₁₋₄alkyl substituted with a 5- to 10-membered monocyclic or bicyclic aromatic ring, wherein the 5- to 10-membered monocyclic or bicyclic aromatic ring optionally contains one, two, three or four heteroatoms, the heteroatoms independently being selected from N, O and S, and
         wherein the said 5- to 10-membered monocyclic or bicyclic aromatic ring is optionally substituted with one or more substituents each independently selected from the group consisting of hydrogen, halogens, CN, CF₃, CF₂H, CFH₂, CF₂CH₃, CH₂CF₃, C₁₋₄alkyl, OH, OC₁₋₄alkyl, OCF₃, OCF₂H, SO₂N(CH₃)₂ and C₃₋₄cycloalkyl; and
      Z is selected from the group consisting of C(=O) and C(=S),
      or a pharmaceutically acceptable salt thereof, and
(B) at least one pharmaceutically acceptable excipient.

Also disclosed herein are pharmaceutical compositions comprising
(A) at least one compound of Formula (I): 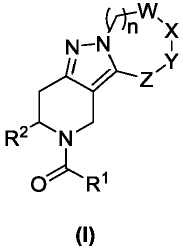 wherein
   R¹ is phenyl substituted with one or more substituents selected from the group consisting of Cl, F, CN, CH₃, CF₃, and CF₂H;
   R² is selected from the group consisting of H, C₁₋₄alkyl and C₁₋₄alkyl substituted with one or more F;
   n is an integer of 0 or 1;
   W is CHR³ or C=CH₂;
   R³ is selected from the group consisting of H, F, CONHC₁₋₄alkyl, and CONHC₃₋₆cycloalkyl;
   X is selected from the group consisting of CH₂ and O;
   Y is NR⁵;
   R⁵ is selected from the group consisting of
      H,
      C₁₋₄alkyl,
      C₁₋₄alkyl substituted with one or more F,
      C₁₋₄alkyl substituted with a 5- to 10-membered monocyclic or bicyclic aromatic ring, wherein the 5- to 10-membered monocyclic or bicyclic ring optionally contains one, two or three heteroatoms, the heteroatoms independently being selected from N, O and S, and
      wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H and C₃₋₄cycloalkyl; and
   Z is selected from the group consisting of C(=O) and C(=S);
   or a stereoisomer or tautomer thereof, or a pharmaceutically acceptable salt thereof, and
(B) at least one pharmaceutically acceptable excipient.

An embodiment of the present disclosure is a pharmaceutical composition comprising at least one pharmaceutically acceptable excipient and at least one compound as described herein, eg. selected from the group consisting of the compounds described below (cf. Table 3), or a stereoisomer or tautomer thereof, or a pharmaceutically acceptable salt thereof.

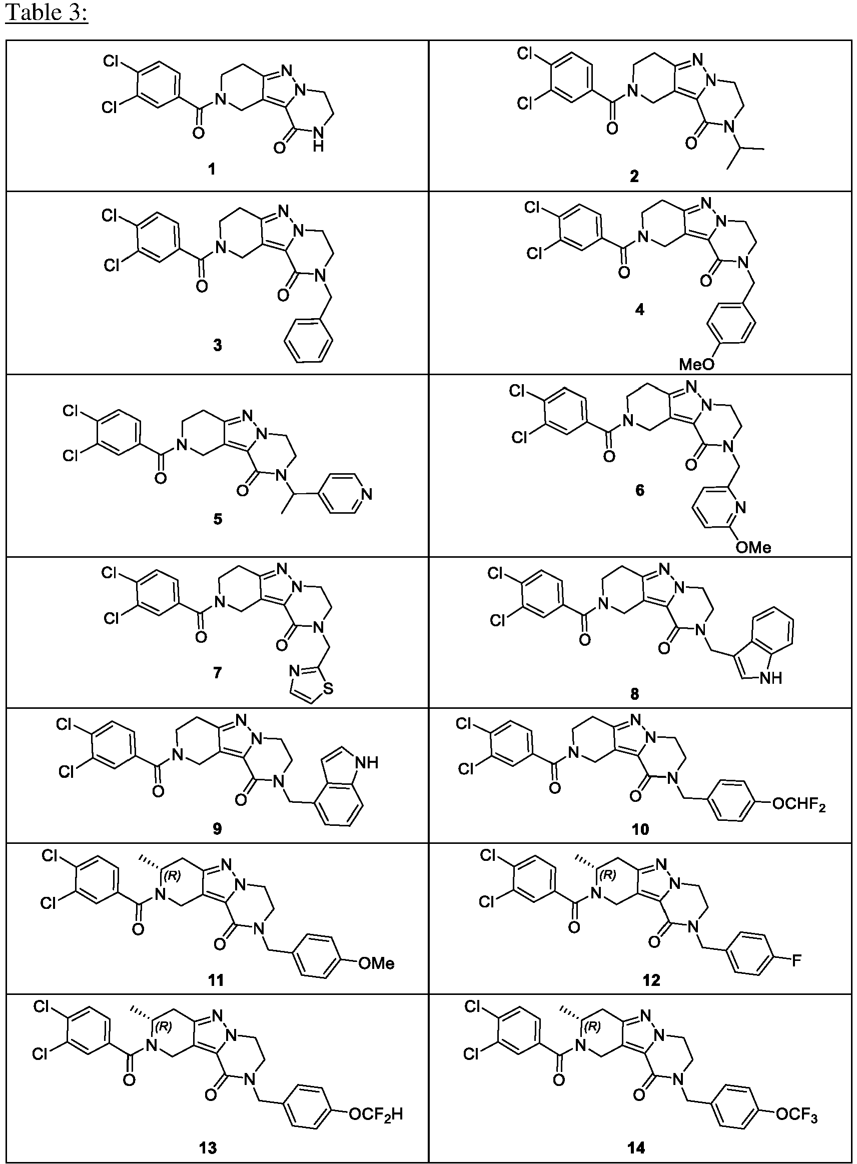

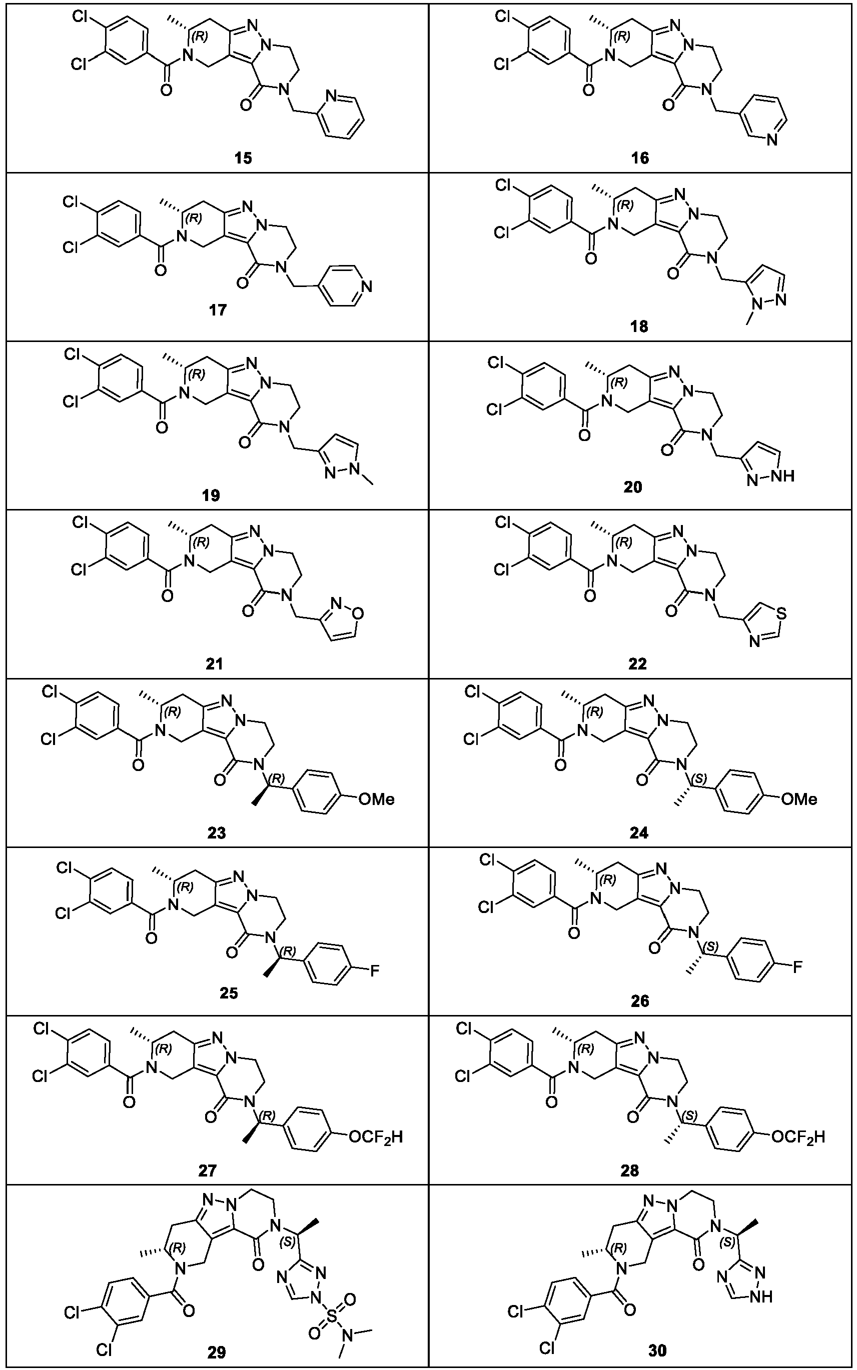

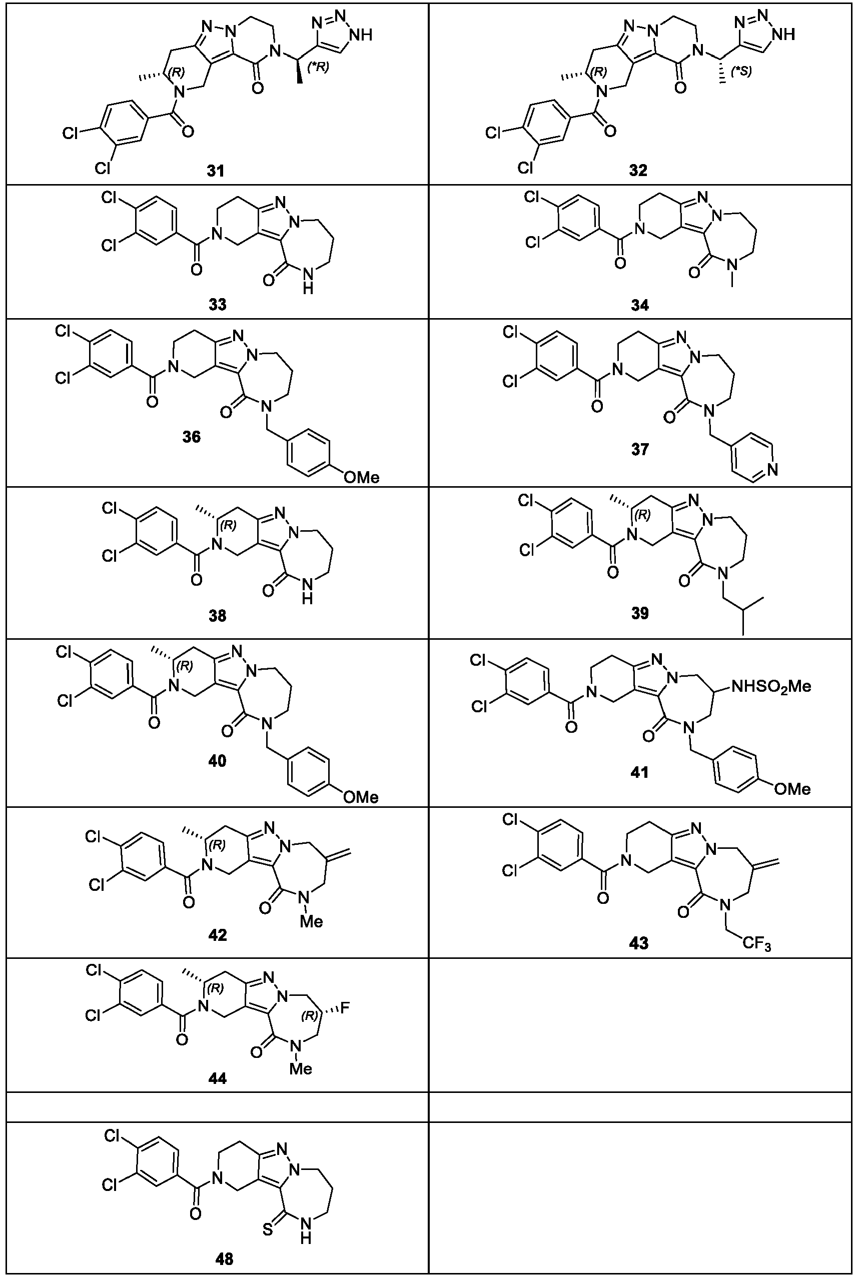

Also disclosed herein are pharmaceutical compositions comprising
(A) at least one compound of Formula (Ia): 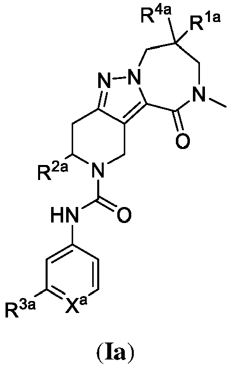 wherein
   R^{1a} is C₆₋₁₀aryl or a 5- or 6-membered heteroaryl, wherein R^{1a} is optionally substituted with a substituent selected from methyl or fluoro;
   R^{2a} is independently selected from the group consisting of: hydrogen and C₁₋₆alkyl;
   R^{3a} is selected from the group consisting of: Cl, CN, and C₁₋₄haloalkyl;
   R^{4a} is selected from the group consisting of: hydrogen, hydroxy, fluoro, and methyl; and
   X^{a} is CF or N;
   and pharmaceutically acceptable salts, stereoisomers, isotopic variants, N-oxides or solvates of compounds of Formula (I); and
(B) at least one pharmaceutically acceptable excipient.

An embodiment of the present disclosure is a pharmaceutical composition comprising at least one pharmaceutically acceptable excipient and at least one compound listed in Table 4 as well as any pharmaceutically acceptable salt, N-oxide or solvate of such compound, or any pharmaceutically acceptable prodrugs of such compound, or any pharmaceutically active metabolite of such compound.

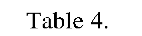

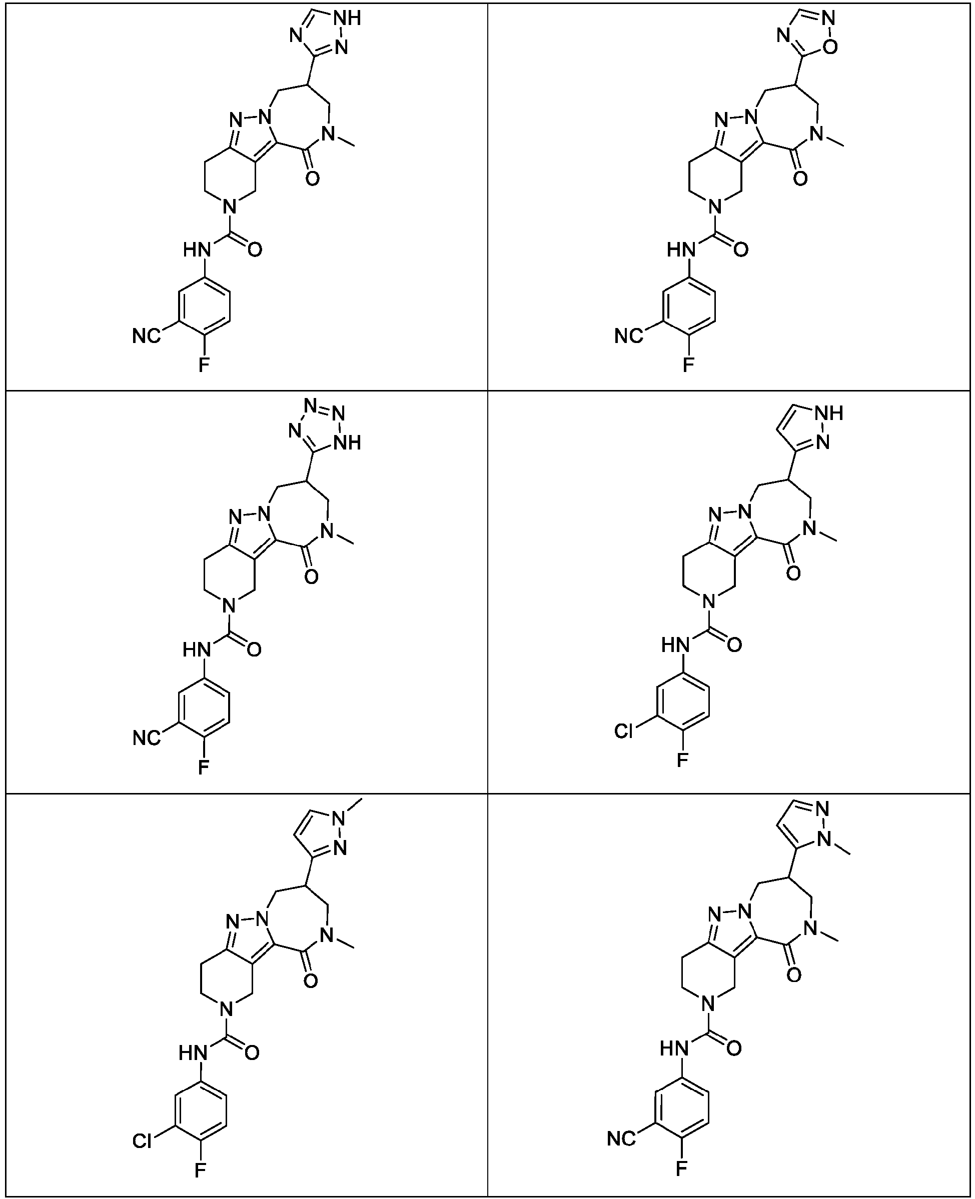

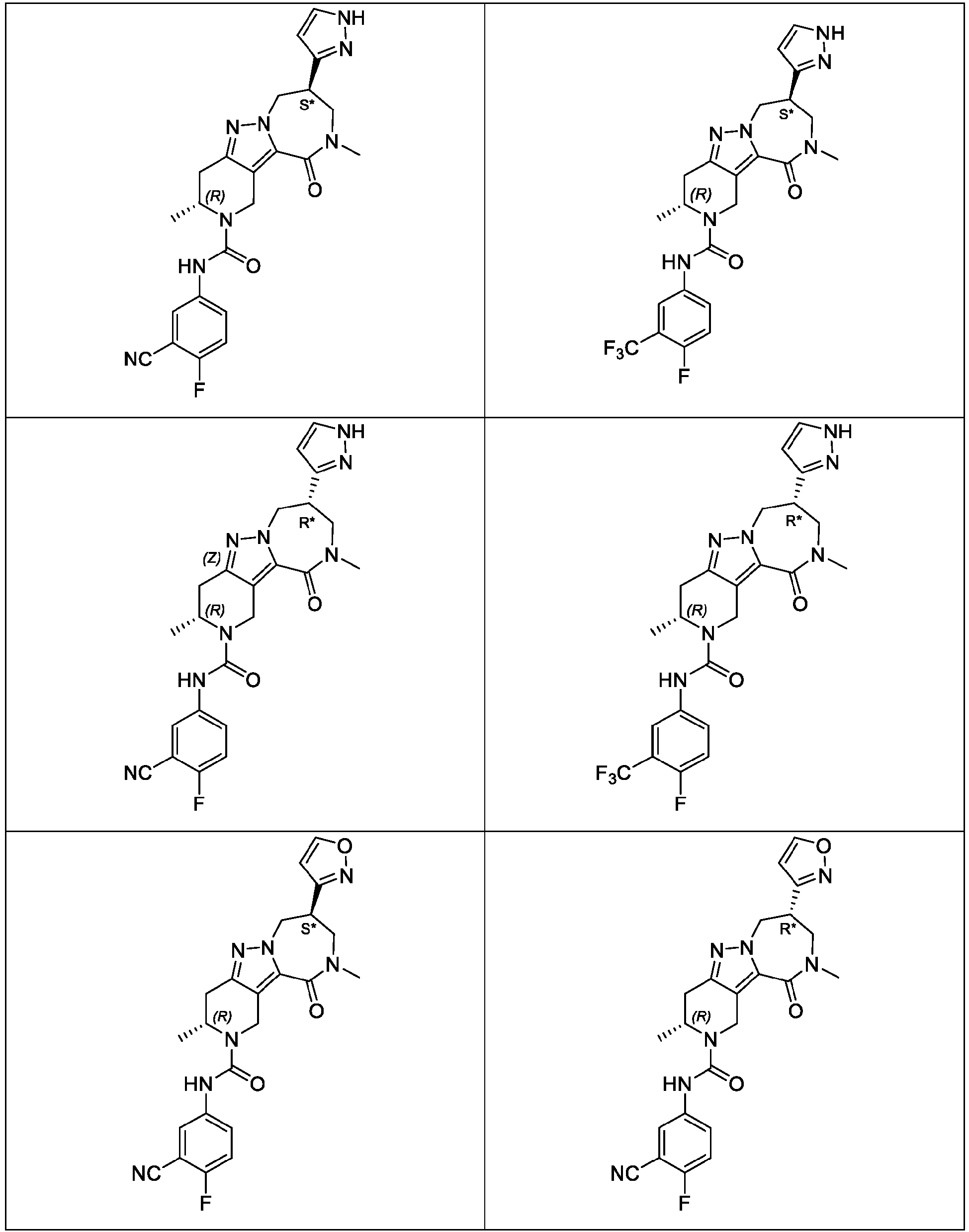

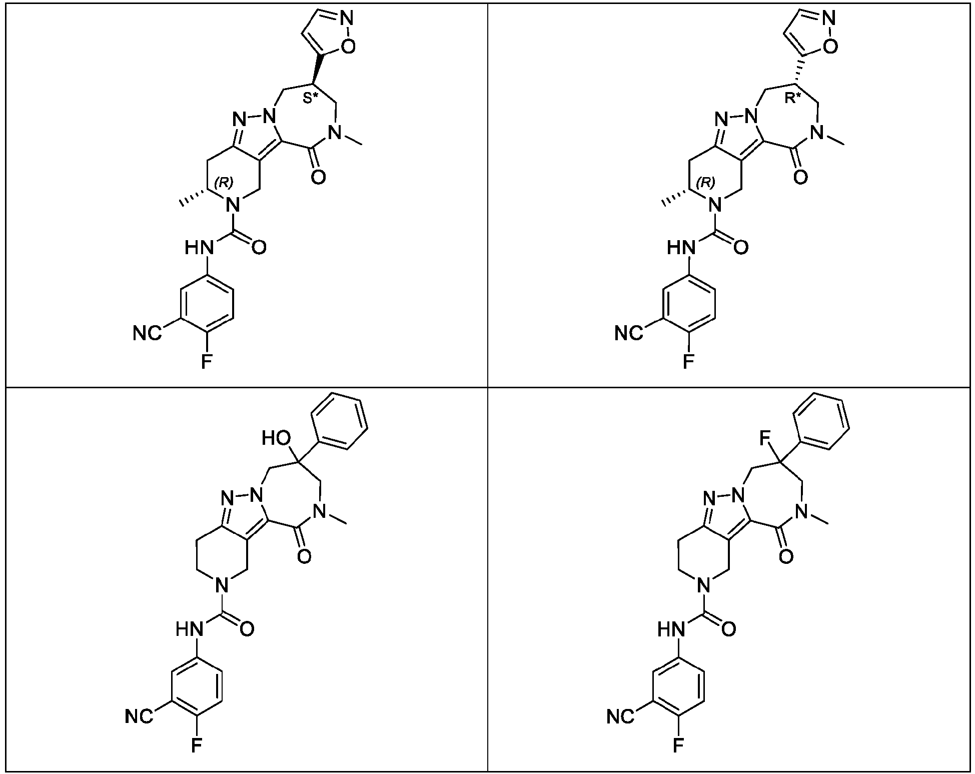

In embodiments, the pharmaceutical composition comprises at least one additional active or therapeutic agent. Additional active therapeutic agents may include, for example, an anti-HBV agent such as an HBV polymerase inhibitor, interferon, viral entry inhibitor, viral maturation inhibitor, capsid assembly modulator, reverse transcriptase inhibitor, immunomodulatory agent such as a TLR-agonist, or any other agents that affect the HBV life cycle and/or the consequences of HBV infection. The active agents of the present disclosure are used, alone or in combination with one or more additional active agents, to formulate pharmaceutical compositions of the present disclosure.

As used herein, the term "composition" or "pharmaceutical composition" refers to a mixture of at least one compound useful within the present disclosure with a pharmaceutically acceptable carrier. The pharmaceutical composition facilitates administration of the compound to a patient or subject. Multiple techniques of administering a compound exist in the art including, but not limited to, intravenous, oral, aerosol, parenteral, ophthalmic, pulmonary and topical administration.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the present disclosure within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the present disclosure, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogenfree water; isotonic saline; Ringer's solution; ethyl alcohol; phosphate buffer solutions; and other non-toxic compatible substances employed in pharmaceutical formulations.

As used herein, "pharmaceutically acceptable carrier" also includes any and all coatings, antibacterial and antifungal agents, and absorption delaying agents, and the like that are compatible with the activity of the compound useful within the present disclosure and are physiologically acceptable to the patient. Supplementary active compounds may also be incorporated into the compositions. The "pharmaceutically acceptable carrier" may further include a pharmaceutically acceptable salt of the compound useful within the present disclosure. Other additional ingredients that may be included in the pharmaceutical compositions used in the practice of the present disclosure are known in the art and described, for example in Remington's Pharmaceutical Sciences (Genaro, Ed., Mack Publishing Co., 1985, Easton, PA), which is incorporated herein by reference.

A "pharmaceutically acceptable excipient" refers to a substance that is non-toxic, biologically tolerable, and otherwise biologically suitable for administration to a subject, such as an inert substance, added to a pharmacological composition or otherwise used as a vehicle, carrier, or diluent to facilitate administration of an agent and that is compatible therewith. Examples of excipients include calcium carbonate, calcium phosphate, various sugars and types of starch, cellulose derivatives, gelatin, vegetable oils, and polyethylene glycols.

Delivery forms of the pharmaceutical compositions containing one or more dosage units of the active agents may be prepared using suitable pharmaceutical excipients and compounding techniques known or that become available to those skilled in the art. The compositions may be administered in the inventive methods by a suitable route of delivery, e.g., oral, parenteral, rectal, topical, or ocular routes, or by inhalation.

The preparation may be in the form of tablets, capsules, sachets, dragees, powders, granules, lozenges, powders for reconstitution, liquid preparations, or suppositories. Preferably, the compositions are formulated for intravenous infusion, topical administration, or oral administration.

For oral administration, the compounds of the present disclosure can be provided in the form of tablets or capsules, or as a solution, emulsion, or suspension. To prepare the oral compositions, the compounds may be formulated to yield a dosage of, e.g., from about 0.05 to about 100 mg/kg daily, or from about 0.05 to about 35 mg/kg daily, or from about 0.1 to about 10 mg/kg daily. For example, a total daily dosage of about 5 mg to 5 g daily may be accomplished by dosing once, twice, three, or four times per day.

Oral tablets may include a compound according to the present disclosure mixed with pharmaceutically acceptable excipients such as inert diluents, disintegrating agents, binding agents, lubricating agents, sweetening agents, flavoring agents, coloring agents and preservative agents. Suitable inert fillers include sodium and calcium carbonate, sodium and calcium phosphate, lactose, starch, sugar, glucose, methyl cellulose, magnesium stearate, mannitol, sorbitol, and the like. Exemplary liquid oral excipients include ethanol, glycerol, water, and the like. Starch, polyvinyl-pyrrolidone (PVP), sodium starch glycolate, microcrystalline cellulose, and alginic acid are suitable disintegrating agents. Binding agents may include starch and gelatin. The lubricating agent, if present, may be magnesium stearate, stearic acid or talc. If desired, the tablets may be coated with a material such as glyceryl monostearate or glyceryl distearate to delay absorption in the gastrointestinal tract or may be coated with an enteric coating.

Capsules for oral administration include hard and soft gelatin capsules. To prepare hard gelatin capsules, compounds of the present disclosure may be mixed with a solid, semi-solid, or liquid diluent. Soft gelatin capsules may be prepared by mixing the compound of the present disclosure with water, an oil such as peanut oil or olive oil, liquid paraffin, a mixture of mono and di-glycerides of short chain fatty acids, polyethylene glycol 400, or propylene glycol.

Liquids for oral administration may be in the form of suspensions, solutions, emulsions or syrups or may be lyophilized or presented as a dry product for reconstitution with water or other suitable vehicle before use. Such liquid compositions may optionally contain: pharmaceuticallyacceptable excipients such as suspending agents (for example, sorbitol, methyl cellulose, sodium alginate, gelatin, hydroxyethylcellulose, carboxymethylcellulose, aluminum stearate gel and the like); non-aqueous vehicles, e.g., oil (for example, almond oil or fractionated coconut oil), propylene glycol, ethyl alcohol, or water; preservatives (for example, methyl or propyl p-hydroxybenzoate or sorbic acid); wetting agents such as lecithin; and, if desired, flavoring or coloring agents.

The active agents of this present disclosure may also be administered by non-oral routes. For example, the compositions may be formulated for rectal administration as a suppository. For parenteral use, including intravenous, intramuscular, intraperitoneal, or subcutaneous routes, the compounds of the present disclosure may be provided in sterile aqueous solutions or suspensions, buffered to an appropriate pH and isotonicity or in parenterally acceptable oil. Suitable aqueous vehicles include Ringer's solution and isotonic sodium chloride. Such forms will be presented in unit-dose form such as ampules or disposable injection devices, in multi-dose forms such as vials from which the appropriate dose may be withdrawn, or in a solid form or pre-concentrate that can be used to prepare an injectable formulation. Illustrative infusion doses may range from about 1 to 1000 µg/kg/minute of compound, admixed with a pharmaceutical carrier over a period ranging from several minutes to several days.

For topical administration, the compounds may be mixed with a pharmaceutical carrier at a concentration of about 0.1% to about 10% of drug to vehicle. Another mode of administering the compounds of the present disclosure may utilize a patch formulation to affect transdermal delivery.

Compounds of the present disclosure may alternatively be administered in methods of this present disclosure by inhalation, via the nasal or oral routes, e.g., in a spray formulation also containing a suitable carrier.

### Methods of Use

The disclosed compounds are useful in the prevention or treatment of an HBV infection or of an HBV-induced disease in mammal in need thereof, more particularly in a human in need thereof.

In a non-limiting aspect, these compounds may (i) modulate or disrupt HBV assembly and other HBV core protein functions necessary for HBV replication or the generation of infectious particles, (ii) inhibit the production of infectious virus particles or infection, or (iii) interact with HBV capsid to effect defective viral particles with reduced infectivity or replication capacity acting as capsid assembly modulators. In particular, and without being bound to any particular mechanism of action, it is believed that the disclosed compounds are useful in HBV treatment by disrupting, accelerating, reducing, delaying and/or inhibiting normal viral capsid assembly and/or disassembly of immature or mature particles, thereby inducing aberrant capsid morphology leading to antiviral effects such as disruption of virion assembly and/or disassembly, virion maturation, virus egress and/or infection of target cells. The disclosed compounds may act as a disruptor of capsid assembly interacting with mature or immature viral capsid to perturb the stability of the capsid, thus affecting its assembly and/or disassembly. The disclosed compounds may perturb protein folding and/or salt bridges required for stability, function and/or normal morphology of the viral capsid, thereby disrupting and/or accelerating capsid assembly and/or disassembly. The disclosed compounds may bind capsid and alter metabolism of cellular polyproteins and precursors, leading to abnormal accumulation of protein monomers and/or oligomers and/or abnormal particles, which causes cellular toxicity and death of infected cells. The disclosed compounds may cause failure of the formation of capsids of optimal stability, affecting efficient uncoating and/or disassembly of viruses (e.g., during infectivity). The disclosed compounds may disrupt and/or accelerate capsid assembly and/or disassembly when the capsid protein is immature. The disclosed compounds may disrupt and/or accelerate capsid assembly and/or disassembly when the capsid protein is mature. The disclosed compounds may disrupt and/or accelerate capsid assembly and/or disassembly during viral infectivity which may further attenuate HBV viral infectivity and/or reduce viral load. The disruption, acceleration, inhibition, delay and/or reduction of capsid assembly and/or disassembly by the disclosed compounds may eradicate the virus from the host organism. Eradication of HBV from a subject by the disclosed compounds advantageously obviates the need for chronic long-term therapy and/or reduces the duration of long-term therapy.

An additional embodiment of the present disclosure is a method of treating a subject suffering from an HBV infection, comprising administering to a subject in need of such treatment an effective amount of at least one compound of Formula (I).

In another aspect, provided herein is a method of reducing the viral load associated with an HBV infection in an individual in need thereof, comprising administering to the individual a therapeutically effective amount of a compound of Formula (I), or a pharmaceutically acceptable salt thereof.

In another aspect, provided herein is a method of reducing reoccurrence of an HBV infection in an individual in need thereof, comprising administering to the individual a therapeutically effective amount of a compound of Formula (I), or a pharmaceutically acceptable salt thereof.

An additional embodiment of the present disclosure is a method of treating a subject suffering from an HBV infection, comprising administering to a subject in need of such treatment an effective amount of at least one compound of Formula (Ia).

In another aspect, provided herein is a method of reducing the viral load associated with an HBV infection in an individual in need thereof, comprising administering to the individual a therapeutically effective amount of a compound of Formula (Ia), or a pharmaceutically acceptable salt thereof.

In another aspect, provided herein is a method of reducing reoccurrence of an HBV infection in an individual in need thereof, comprising administering to the individual a therapeutically effective amount of a compound of Formula (Ia), or a pharmaceutically acceptable salt thereof.

Additionally, HBV acts as a helper virus to hepatitis delta virus (HDV), and it is estimated that more than 15 million people may be HBV/HDV co-infected worldwide, with an increased risk of rapid progression to cirrhosis and increased hepatic decompensation, than patients suffering from HBV alone (Hughes, S.A. et al. Lancet 2011, 378, 73-85). HDV, infects therefore subjects suffering from HBV infection. In a particular embodiment, the compounds of the invention may be used in the treatment and/or prophylaxis of HBV/HDV co-infection, or diseases associated with HBV/HDV co infection. Therefore, in a particular embodiment, the HBV infection is in particular HBV/HDV co-infection, and the mammal, in particular the human, may be HBV/HDV co-infected, or be at risk of HBV/HDV co infection.

In another aspect, provided herein is a method of inhibiting or reducing the formation or presence of HBV DNA-containing particles or HBV RNA-containing particles in an individual in need thereof, comprising administering to the individual a therapeutically effective amount of a compound of Formula (I), or a pharmaceutically acceptable salt thereof.

In another aspect, provided herein is a method of reducing an adverse physiological impact of an HBV infection in an individual in need thereof, comprising administering to the individual a therapeutically effective amount of a compound of Formula (I), or a pharmaceutically acceptable salt thereof.

In another aspect, provided herein is a method of inducing remission of hepatic injury from an HBV infection in an individual in need thereof, comprising administering to the individual a therapeutically effective amount of a compound of Formula (I), or a pharmaceutically acceptable salt thereof.

In another aspect, provided herein is a method of reducing the physiological impact of long-term antiviral therapy for HBV infection in an individual in need thereof, comprising administering to the individual a therapeutically effective amount of a compound of Formula (I), or a pharmaceutically acceptable salt thereof.

In another aspect, provided herein is a method of prophylactically treating an HBV infection in an individual in need thereof, wherein the individual is afflicted with a latent HBV infection, comprising administering to the individual a therapeutically effective amount of a compound of Formula (I), or a pharmaceutically acceptable salt thereof.

In another aspect, provided herein is a method of inhibiting or reducing the formation or presence of HBV DNA-containing particles or HBV RNA-containing particles in an individual in need thereof, comprising administering to the individual a therapeutically effective amount of a compound of Formula (Ia), or a pharmaceutically acceptable salt thereof.

In another aspect, provided herein is a method of reducing an adverse physiological impact of an HBV infection in an individual in need thereof, comprising administering to the individual a therapeutically effective amount of a compound of Formula (Ia), or a pharmaceutically acceptable salt thereof.

In another aspect, provided herein is a method of inducing remission of hepatic injury from an HBV infection in an individual in need thereof, comprising administering to the individual a therapeutically effective amount of a compound of Formula (Ia), or a pharmaceutically acceptable salt thereof.

In another aspect, provided herein is a method of reducing the physiological impact of long-term antiviral therapy for HBV infection in an individual in need thereof, comprising administering to the individual a therapeutically effective amount of a compound of Formula (Ia), or a pharmaceutically acceptable salt thereof.

In another aspect, provided herein is a method of prophylactically treating an HBV infection in an individual in need thereof, wherein the individual is afflicted with a latent HBV infection, comprising administering to the individual a therapeutically effective amount of a compound of Formula (Ia), or a pharmaceutically acceptable salt thereof.

In embodiments, the disclosed compounds are suitable for monotherapy. In embodiments, the disclosed compounds are effective against natural or native HBV strains. In embodiments, the disclosed compounds are effective against HBV strains resistant to currently known drugs.

In another embodiment, the compounds provided herein can be used in methods of modulating (e.g., inhibiting or disrupting) the activity, stability, function, and viral replication properties of HBV cccDNA.

In yet another embodiment, the compounds of the present disclosure can be used in methods of diminishing or preventing the formation of HBV cccDNA.

In another embodiment, the compounds provided herein can be used in methods of modulating (e.g., inhibiting or disrupting) the activity of HBV cccDNA.

In yet another embodiment, the compounds of the present disclosure can be used in methods of diminishing the formation of HBV cccDNA.

In another embodiment, the disclosed compounds can be used in methods of modulating, inhibiting, or disrupting the generation or release of HBV RNA particles from within the infected cell.

In a further embodiment, the total burden (or concentration) of HBV RNA particles is modulated. In a preferred embodiment, the total burden of HBV RNA is diminished.

In another embodiment, the methods provided herein reduce the viral load in the individual to a greater extent or at a faster rate compared to the administering of a compound selected from the group consisting of an HBV polymerase inhibitor, interferon, viral entry inhibitor, viral maturation inhibitor, distinct capsid assembly modulator, antiviral compounds of distinct or unknown mechanism, and any combination thereof.

In another embodiment, the methods provided herein cause a lower incidence of viral mutation and/or viral resistance than the administering of a compound selected from the group consisting of an HBV polymerase inhibitor, interferon, viral entry inhibitor, viral maturation inhibitor, distinct capsid assembly modulator, antiviral compounds of distinct or unknown mechanism, and combination thereof.

In another embodiment, the methods provided herein further comprise administering to the individual at least one HBV vaccine, a nucleoside HBV inhibitor, an interferon or any combination thereof.

In an aspect, provided herein is a method of treating an HBV infection in an individual in need thereof, comprising reducing the HBV viral load by administering to the individual a therapeutically effective amount of a compound of Formula (I), or a pharmaceutically acceptable salt thereof, alone or in combination with a reverse transcriptase inhibitor; and further administering to the individual a therapeutically effective amount of HBV vaccine.

An additional embodiment of the present disclosure is a method of treating a subject suffering from an HBV infection, comprising administering to a subject in need of such treatment an effective amount of at least one compound of Formula (I).

In another aspect, provided herein is a method of reducing the viral load associated with an HBV infection in an individual in need thereof, comprising administering to the individual a therapeutically effective amount of a compound of Formula (I), or a pharmaceutically acceptable salt thereof.

In another aspect, provided herein is a method of reducing reoccurrence of an HBV infection in an individual in need thereof, comprising administering to the individual a therapeutically effective amount of a compound of Formula (I), or a pharmaceutically acceptable salt thereof.

In another aspect, provided herein is a method of inhibiting or reducing the formation or presence of HBV DNA-containing particles or HBV RNA-containing particles in an individual in need thereof, comprising administering to the individual a therapeutically effective amount of a compound of Formula (I), or a pharmaceutically acceptable salt thereof.

In another aspect, provided herein is a method of reducing an adverse physiological impact of an HBV infection in an individual in need thereof, comprising administering to the individual a therapeutically effective amount of a compound of Formula (I), or a pharmaceutically acceptable salt thereof.

In another aspect, provided herein is a method of inducing remission of hepatic injury from an HBV infection in an individual in need thereof, comprising administering to the individual a therapeutically effective amount of a compound of Formula (I), or a pharmaceutically acceptable salt thereof.

In another aspect, provided herein is a method of reducing the physiological impact of long-term antiviral therapy for HBV infection in an individual in need thereof, comprising administering to the individual a therapeutically effective amount of a compound of Formula (I), or a pharmaceutically acceptable salt thereof.

In another aspect, provided herein is a method of prophylactically treating an HBV infection in an individual in need thereof, wherein the individual is afflicted with a latent HBV infection, comprising administering to the individual a therapeutically effective amount of a compound of Formula (I), or a pharmaceutically acceptable salt thereof.

In an aspect, provided herein is a method of treating an HBV infection in an individual in need thereof, comprising reducing the HBV viral load by administering to the individual a therapeutically effective amount of a compound of Formula (Ia), or a pharmaceutically acceptable salt thereof, alone or in combination with a reverse transcriptase inhibitor; and further administering to the individual a therapeutically effective amount of HBV vaccine.

An additional embodiment of the present disclosure is a method of treating a subject suffering from an HBV infection, comprising administering to a subject in need of such treatment an effective amount of at least one compound of Formula (Ia).

In another aspect, provided herein is a method of reducing the viral load associated with an HBV infection in an individual in need thereof, comprising administering to the individual a therapeutically effective amount of a compound of Formula (Ia), or a pharmaceutically acceptable salt thereof.

In another aspect, provided herein is a method of reducing reoccurrence of an HBV infection in an individual in need thereof, comprising administering to the individual a therapeutically effective amount of a compound of Formula (Ia), or a pharmaceutically acceptable salt thereof.

In another aspect, provided herein is a method of inhibiting or reducing the formation or presence of HBV DNA-containing particles or HBV RNA-containing particles in an individual in need thereof, comprising administering to the individual a therapeutically effective amount of a compound of Formula (Ia), or a pharmaceutically acceptable salt thereof.

In another aspect, provided herein is a method of reducing an adverse physiological impact of an HBV infection in an individual in need thereof, comprising administering to the individual a therapeutically effective amount of a compound of Formula (Ia), or a pharmaceutically acceptable salt thereof.

In another aspect, provided herein is a method of inducing remission of hepatic injury from an HBV infection in an individual in need thereof, comprising administering to the individual a therapeutically effective amount of a compound of Formula (Ia), or a pharmaceutically acceptable salt thereof.

In another aspect, provided herein is a method of reducing the physiological impact of long-term antiviral therapy for HBV infection in an individual in need thereof, comprising administering to the individual a therapeutically effective amount of a compound of Formula (Ia), or a pharmaceutically acceptable salt thereof.

In another aspect, provided herein is a method of prophylactically treating an HBV infection in an individual in need thereof, wherein the individual is afflicted with a latent HBV infection, comprising administering to the individual a therapeutically effective amount of a compound of Formula (Ia), or a pharmaceutically acceptable salt thereof.

In an embodiment, the methods provided herein further comprise monitoring the HBV viral load of the subject, wherein the method is carried out for a period of time such that the HBV virus is undetectable.

### Combinations

Provided herein are combinations of one or more of the disclosed compounds with at least one additional therapeutic agent. In embodiments, the methods provided herein can further comprise administering to the individual at least one additional therapeutic agent. In embodiments, the disclosed compounds are suitable for use in combination therapy. The compounds of the present disclosure may be useful in combination with one or more additional compounds useful for treating HBV infection. These additional compounds may comprise compounds of the present disclosure or compounds known to treat, prevent, or reduce the symptoms or effects of HBV infection.

In an exemplary embodiment, additional active ingredients are those that are known or discovered to be effective in the treatment of conditions or disorders involved in HBV infection, such as another HBV capsid assembly modulator or a compound active against another target associated with the particular condition or disorder involved in HBV infection, or the HBV infection itself. The combination may serve to increase efficacy (e.g., by including in the combination a compound potentiating the potency or effectiveness of an active agent according to the present disclosure), decrease one or more side effects, or decrease the required dose of the active agent according to the present disclosure. In a further embodiment, the methods provided herein allow for administering of the at least one additional therapeutic agent at a lower dose or frequency as compared to the administering of the at least one additional therapeutic agent alone that is required to achieve similar results in prophylactically treating an HBV infection in an individual in need thereof.

Such compounds include but are not limited to HBV combination drugs, HBV vaccines, HBV DNA polymerase inhibitors, immunomodulatory agents, toll-like receptor (TLR) modulators, interferon alpha receptor ligands, hyaluronidase inhibitors, hepatitis b surface antigen (HBsAg) inhibitors, cytotoxic T-lymphocyte-associated protein 4 (ipi4) inhibitors, cyclophilin inhibitors, HBV viral entry inhibitors, antisense oligonucleotide targeting viral mRNA, short interfering RNAs (siRNA) and ddRNAi endonuclease modulators, ribonucleotide reductase inhibitors, HBV E antigen inhibitors, covalently closed circular DNA (cccDNA) inhibitors, famesoid X receptor agonists, HBV antibodies, CCR2 chemokine antagonists, thymosin agonists, cytokines, nucleoprotein modulators, retinoic acid-inducible gene 1 simulators, NOD2 stimulators, phosphatidylinositol 3-kinase (PI3K) inhibitors, indoleamine-2, 3-dioxygenase (IDO) pathway inhibitors, PD-1 inhibitors, PD-L1 inhibitors, recombinant thymosin alpha-1, bruton's tyrosine kinase (BTK) inhibitors, KDM inhibitors, HBV replication inhibitors, arginase inhibitors, and any other agent that affects the HBV life cycle and/or affect the consequences of HBV infection or combinations thereof.

In embodiments, the compounds of the present disclosure may be used in combination with an HBV polymerase inhibitor, immunomodulatory agents, interferon such as pegylated interferon, viral entry inhibitor, viral maturation inhibitor, capsid assembly modulator, reverse transcriptase inhibitor, a cyclophilin/TNF inhibitor, immunomodulatory agent such as a TLR-agonist, an HBV vaccine, and any other agent that affects the HBV life cycle and/or affect the consequences of HBV infection or combinations thereof.In particular, the compounds of the present disclosure may be used in combination with one or more agents (or a salt thereof) selected from the group consisting of
HBV reverse transcriptase inhibitors, and DNA and RNA polymerase inhibitors, including but not limited to: lamivudine (3TC, Zeffix, Heptovir, Epivir, and Epivir-HBV), entecavir (Baraclude, Entavir), adefovir dipivoxil (Hepsara, Preveon, bis-POM PMEA), tenofovir disoproxil fumarate (Viread, TDF or PMPA);
interferons, including but not limited to interferon alpha (IFN-α), interferon beta (IFN-β), interferon lambda (IFN-λ), and interferon gamma (IFN-γ);
viral entry inhibitors;
viral maturation inhibitors;
literature-described capsid assembly modulators, such as, but not limited to BAY 41-4109;
reverse transcriptase inhibitor;
an immunomodulatory agent such as a TLR-agonist; and
agents of distinct or unknown mechanism, such as but not limited to AT-61 ((E)-N-(1-chloro-3-oxo-1-phenyl-3-(piperidin-1-yl)prop-1-en-2-yl)benzamide), AT-130 ((E)-N-(1-bromo-1-(2-methoxyphenyl)-3-oxo-3-(piperidin-1-yl)prop-1-en-2-yl)-4-nitrobenzamide), and similar analogs.

In embodiments, the additional therapeutic agent is an interferon. The term "interferon" or "IFN" refers to any member the family of highly homologous species-specific proteins that inhibit viral replication and cellular proliferation and modulate immune response. Human interferons are grouped into three classes; Type I, which include interferon-alpha (IFN-α), interferon-beta (IFN-β), and interferon-omega (IFN-ω), Type II, which includes interferon-gamma (IFN-γ), and Type III, which includes interferon-lambda (IFN-λ). Recombinant forms of interferons that have been developed and are commercially available are encompassed by the term "interferon" as used herein. Subtypes of interferons, such as chemically modified or mutated interferons, are also encompassed by the term "interferon" as used herein. Chemically modified interferons include pegylated interferons and glycosylated interferons. Examples of interferons also include, but are not limited to, interferon-alpha-2a, interferon-alpha-2b, interferon-alpha-n1, interferon-beta-1a, interferon-beta-1b, interferon-lamda-1, interferon-lamda-2, and interferon-lamda-3. Examples of pegylated interferons include pegylated interferon-alpha-2a and pegylated interferon alpha-2b.

Accordingly, in one embodiment, the compounds of Formula I, can be administered in combination with an interferon selected from the group consisting of interferon alpha (IFN-α), interferon beta (IFN-β), interferon lambda (IFN-λ), and interferon gamma (IFN-γ). In one specific embodiment, the interferon is interferon-alpha-2a, interferon-alpha-2b, or interferon-alpha-n1. In another specific embodiment, the interferon-alpha-2a or interferon-alpha-2b is pegylated. In a preferred embodiment, the interferon-alpha-2a is pegylated interferon-alpha-2a (PEGASYS).

In another embodiment, the additional therapeutic agent is selected from immune modulator or immune stimulator therapies, which includes biological agents belonging to the interferon class.

Further, the additional therapeutic agent may be an agent that disrupts the function of other essential viral protein(s) or host proteins required for HBV replication or persistence.

In another embodiment, the additional therapeutic agent is an antiviral agent that blocks viral entry or maturation or targets the HBV polymerase such as nucleoside or nucleotide or nonnucleos(t)ide polymerase inhibitors. In a further embodiment of the combination therapy, the reverse transcriptase inhibitor and/or DNA and/or RNA polymerase inhibitor is Zidovudine, Didanosine, Zalcitabine, ddA, Stavudine, Lamivudine, Abacavir, Emtricitabine, Entecavir, Apricitabine, Atevirapine, ribavirin, acyclovir, famciclovir, valacyclovir, ganciclovir, valganciclovir, Tenofovir, Adefovir, PMPA, cidofovir, Efavirenz, Nevirapine, Delavirdine, or Etravirine.

In an embodiment, the additional therapeutic agent is an immunomodulatory agent that induces a natural, limited immune response leading to induction of immune responses against unrelated viruses. In other words, the immunomodulatory agent can affect maturation of antigen presenting cells, proliferation of T-cells and cytokine release (e.g., IL-12, IL-18, IFN-alpha, -beta, and -gamma and TNF-alpha among others).

In a further embodiment, the additional therapeutic agent is a TLR modulator or a TLR agonist, such as a TLR-7 agonist or TLR-9 agonist. In further embodiment of the combination therapy, the TLR-7 agonist is selected from the group consisting of SM360320 (9-benzyl-8-hydroxy-2-(2-methoxy-ethoxy)adenine) and AZD 8848 (methyl [3-({[3-(6-amino-2-butoxy-8-oxo-7,8-dihydro-9H-purin-9-yl)propyl][3-(4-morpholinyl)propyl]amino}methyl)phenyl]acetate).

In any of the methods provided herein, the method may further comprise administering to the individual at least one HBV vaccine, a nucleoside HBV inhibitor, an interferon or any combination thereof. In an embodiment, the HBV vaccine is at least one of RECOMBIVAX HB, ENGERIX-B, ELOVAC B, GENEVAC-B, or SHANVAC B.

In another aspect, provided herein is method of treating an HBV infection in an individual in need thereof, comprising reducing the HBV viral load by administering to the individual a therapeutically effective amount of a compound of the present disclosure alone or in combination with a reverse transcriptase inhibitor; and further administering to the individual a therapeutically effective amount of HBV vaccine. The reverse transcriptase inhibitor may be one of Zidovudine, Didanosine, Zalcitabine, ddA, Stavudine, Lamivudine, Abacavir, Emtricitabine, Entecavir, Apricitabine, Atevirapine, ribavirin, acyclovir, famciclovir, valacyclovir, ganciclovir, valganciclovir, Tenofovir, Adefovir, PMPA, cidofovir, Efavirenz, Nevirapine, Delavirdine, or Etravirine.

For any combination therapy described herein, synergistic effect may be calculated, for example, using suitable methods such as the Sigmoid-Eₘₐₓ equation (Holford & Scheiner, 19981, Clin. Pharmacokinet. 6: 429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22: 27-55). Each equation referred to above may be applied to experimental data to generate a corresponding graph to aid in assessing the effects of the drug combination. The corresponding graphs associated with the equations referred to above are the concentration-effect curve, isobologram curve and combination index curve, respectively.

### Methods

The application relates to a method for the preparation of a compound of Formula (I) as described herein.

The method comprises at least one step among steps a), b), c), d), e) and f):
a) reacting a compound of Formula (II), 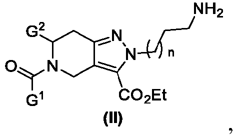 with a compound of Formula (III), 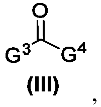 in the presence of NaBH₃CN, to form a compound of Formula (IV), 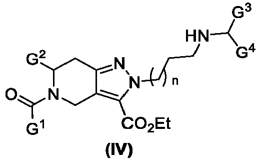 wherein n is an integer of 0 or 1;
   G¹ is dichlorophenyl;
   G² is hydrogen or C₁₋₄alkyl;
   G³ is hydrogen or C₁₋₄alkyl
   G⁴ is 5- to 10-membered monocyclic or bicyclic aromatic ring optionally containing one, two or three heteroatoms, the heteroatoms independently being selected from N, O and S, and wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H and C₃₋₄cycloalkyl;
b) reacting a compound of Formula (V), 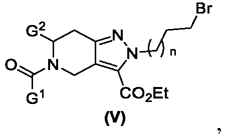 with a compound of Formula (VI), 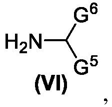 in the presence of potassium iodide (KI), to form a compound of Formula (VII), 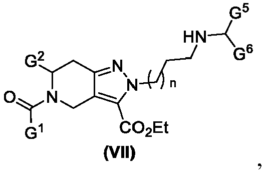 wherein n is 0,
   G¹ is dichlorophenyl;
   G² is hydrogen or C₁₋₄alkyl;
   G⁵ is hydrogen or C₁₋₄alkyl;
   G⁶ is C₂₋₅alkynyl,
      5- to 10-membered monocyclic or bicyclic aromatic ring optionally containing one, two or three heteroatoms, the heteroatoms independently being selected from N, O and S, and wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H, C₃₋₄cycloalkyl and SO₂N(CH₃)₂;
c) reacting a compound of Formula (VIII), 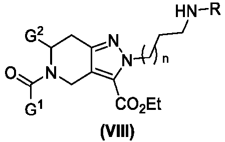 with 1,5,7-triazabicyclo[4.4.0]dec-5-en (TBD) or trimethylaluminium (Al(CH₃)₃), optionally in the presence of 1,8-diazabicyclo[5.4.0]undec-7-ene (DBU), to form a compound of Formula (IX), 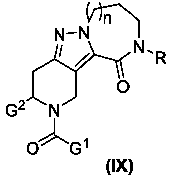 wherein
   R is hydrogen or 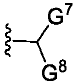
   n is an integer of 0 or 1;
   G¹ is dichlorophenyl or tert-butoxide;
   G² is hydrogen or C₁₋₄alkyl;
   G⁷ is C₁₋₄alkyl,
      C₁₋₄alkyl substituted with one or more F,
      5- to 10-membered monocyclic or bicyclic aromatic ring optionally containing one, two or three heteroatoms, the heteroatoms independently being selected from N, O and S, and wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H, C₃₋₄cycloalkyl and SO₂N(CH₃)₂,
      C₂₋₅alkynyl;
   G⁸ is hydrogen or C₁₋₄alkyl;
d) reacting a compound of Formula (X), 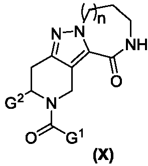 with a compound of Formula (XI), 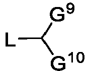 **(XI)** in the presence of a non-nucleophilic base, more particularly cesium carbonate (Cs₂CO₃) or sodium hydride (NaH) to form a compound or Formula (XII), 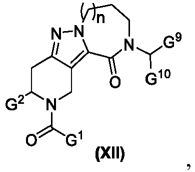 wherein n is an integer of 0 or 1;
   G¹ is dichlorophenyl;
   G² is hydrogen or C₁₋₄alkyl;
   G⁹ is hydrogen or C₁₋₄alkyl,
   G¹⁰ is 5- to 10-membered monocyclic or bicyclic aromatic ring optionally containing one, two or three heteroatoms, the heteroatoms independently being selected from N, O and S, and wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H, C₃₋₄cycloalkyl, C(=O)Ot-Bu and SO₂N(CH₃)₂;
   L is bromide, chloride or iodide;
e) reacting a compound of Formula (XIII), 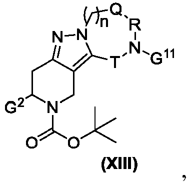 with a strong acid, more particularly hydrochloric acid (HCl) or trifluoroacetic acid (TFA), to form a compound of Formula (XIV), 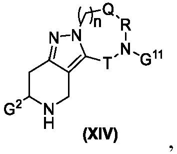 wherein
   n is an integer of 0 or 1;
   G² is hydrogen or methyl;
   G¹¹ is H,
      C₁₋₄alkyl,
      C₁₋₄alkyl substituted with one or more F,
      C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and
   wherein the said 5- to 10-membered monocyclic or bicyclic aromatic ring is optionally substituted with one or more substituents selected from the group consisting of hydrogen, halogens, CN, CF₃, CF₂H, CFH₂, CF₂CH₃, C₁₋₄alkyl, OC₁₋₄alkyl, OCF₃, OCF₂H and C₃₋₄cycloalkyl;
   Q is CH₂, CHF, CH(CH₂OH), CH(CH₂F), C=CH₂, or CHNHSO₂CH₃;
   R is CH₂;
   T is C=O or C=S;
f) reacting a compound of Formula (XIV), 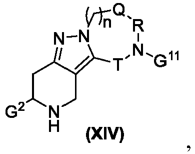 with a compound of Formula (XV), 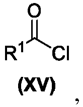 in the presence of a non-nucleophilic base, more particularly sodium carbonate (Na₂CO₃), triethylamine (Et₃N) or diisopropylethylamine (DIPEA), to form a compound of Formula (XVI), 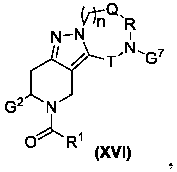
   wherein R₁ is dichlorophenyl and wherein G², n, Q, R, T and G¹¹ have been defined in step e),
      provided that
   when the process comprises step a, the process further comprises step c,
   when the process comprises step b, the process further comprises step c, and
   when the process comprises step e, the process further comprises step f.

### Definitions

Listed below are definitions of various terms used to describe this present disclosure. These definitions apply to the terms as they are used throughout this specification and claims, unless otherwise limited in specific instances, either individually or as part of a larger group.

Unless defined otherwise, all technical and scientific terms used herein generally have the same meaning as commonly understood by one of ordinary skill in the applicable art. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e. to at least one) of the grammatical object of the article. By way of example, "an element" means one element or more than one element. Furthermore, use of the term "including" as well as other forms, such as "include," "includes," and "included," is not limiting.

As used in the specification and in the claims, the term "comprising" can include the embodiments "consisting of" and "consisting essentially of." The terms "comprise(s)," "include(s)," "having," "has," "can," "contain(s)," and variants thereof, as used herein, are intended to be open-ended transitional phrases, terms, or words that require the presence of the named ingredients/steps and permit the presence of other ingredients/steps. However, such description should be construed as also describing compositions or processes as "consisting of" and "consisting essentially of" the enumerated compounds, which allows the presence of only the named compounds, along with any pharmaceutically acceptable carriers, and excludes other compounds. All ranges disclosed herein are inclusive of the recited endpoint and independently combinable (for example, the range of "from 50 mg to 300 mg" is inclusive of the endpoints, 50 mg and 300 mg, and all the intermediate values). The endpoints of the ranges and any values disclosed herein are not limited to the precise range or value; they are sufficiently imprecise to include values approximating these ranges and/or values.

As used herein, approximating language can be applied to modify any quantitative representation that can vary without resulting in a change in the basic function to which it is related. Accordingly, a value modified by a term or terms, such as "substantially," cannot be limited to the precise value specified, in some cases. In at least some instances, the approximating language can correspond to the precision of an instrument for measuring the value.

The term "alkyl" refers to a straight- or branched-chain alkyl group having from 1 to 12 carbon atoms in the chain. Examples of alkyl groups include methyl (Me, which also may be structurally depicted by the symbol, "/"), ethyl (Et), n-propyl, isopropyl, butyl, isobutyl, sec-butyl, tert-butyl (tBu), pentyl, isopentyl, tert-pentyl, hexyl, isohexyl, and groups that in light of the ordinary skill in the art and the teachings provided herein would be considered equivalent to any one of the foregoing examples. The term C₁₋₄alkyl as used here refers to a straight- or branched-chain alkyl group having from 1 to 4 carbon atoms in the chain. The term C₁-₆alkyl as used here refers to a straight- or branched-chain alkyl group having from 1 to 6 carbon atoms in the chain.

The term "cycloalkyl" refers to a saturated or partially saturated, monocyclic, fused polycyclic, or spiro polycyclic carbocycle having from 3 to 12 ring atoms per carbocycle. Illustrative examples of cycloalkyl groups include the following entities, in the form of properly bonded moieties:

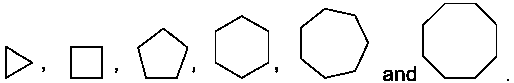

A monocyclic, bicyclic or tricyclic aromatic carbocycle represents an aromatic ring system consisting of 1, 2 or 3 rings, said ring system being composed of only carbon atoms; the term aromatic is well known to a person skilled in the art and designates cyclically conjugated systems of 4n + 2 electrons, that is with 6, 10, 14 etc. π-electrons (rule of Hückel).

Particular examples of monocyclic, bicyclic or tricyclic aromatic carbocycles are phenyl, naphthalenyl, anthracenyl.

The term "phenyl" represents the following moiety:

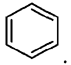

The term "heteroaryl" refers to an aromatic monocyclic or bicyclic aromatic ring system having 5 to 10 ring members and which contains carbon atoms and from 1 to 4 heteroatoms independently selected from the group consisting of N, O, and S. Included within the term heteroaryl are aromatic rings of 5 or 6 members wherein the ring consists of carbon atoms and has at least one heteroatom member. Suitable heteroatoms include nitrogen, oxygen, and sulfur. In the case of 5 membered rings, the heteroaryl ring preferably contains one member of nitrogen, oxygen or sulfur and, in addition, up to 3 additional nitrogens. In the case of 6 membered rings, the heteroaryl ring preferably contains from 1 to 4, e.g. tetrazolyl (e.g. 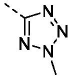 )more in particular from 1 to 3 nitrogen atoms. For the case wherein the 6 membered ring has 3 nitrogens, at most 2 nitrogen atoms are adjacent. Examples of heteroaryl groups include furyl, thienyl, pyrrolyl, oxazolyl, thiazolyl, imidazolyl, pyrazolyl, oxazolyl, thiazolyl, oxadiazolyl, triazolyl, thiadiazolyl, pyridinyl, pyridazinyl, pyrimidinyl, pyrazinyl, indolyl, isoindolyl, benzofuryl, benzothienyl, indazolyl, benzimidazolyl, benzothiazolyl, benzoxazolyl, benzisoxazolyl, benzothiadiazolyl, benzotriazolyl, quinolinyl, isoquinolinyl and quinazolinyl. Unless otherwise noted, the heteroaryl is attached to its pendant group at any heteroatom or carbon atom that results in a stable structure.

Those skilled in the art will recognize that the species of heteroaryl groups listed or illustrated above are not exhaustive, and that additional species within the scope of these defined terms may also be selected.

The term "cyano" refers to the group -CN.

The terms "halo" or "halogen" represent chloro, fluoro, bromo or iodo.

The term "substituted" means that the specified group or moiety bears one or more substituents. The term "unsubstituted" means that the specified group bears no substituents. The term "optionally substituted" means that the specified group is unsubstituted or substituted by one or more substituents. Where the term "substituted" is used to describe a structural system, the substitution is meant to occur at any valency-allowed position on the system. In cases where a specified moiety or group is not expressly noted as being optionally substituted or substituted with any specified substituent, it is understood that such a moiety or group is intended to be unsubstituted.

The terms "para", "meta", and "ortho" have the meanings as understood in the art. Thus, for example, a fully substituted phenyl group has substituents at both "ortho"(*o*) positions adjacent to the point of attachment of the phenyl ring, both "meta" (*m*) positions, and the one "para" (*p*) position across from the point of attachment. To further clarify the position of substituents on the phenyl ring, the 2 different ortho positions will be designated as ortho and ortho' and the 2 different meta positions as meta and meta' as illustrated below.

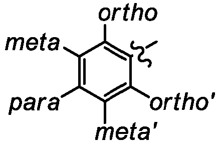

When referring to substituents on a pyridyl group, the terms "para", "meta", and "ortho" refer to the placement of a substituent relative to the point of attachment of the pyridyl ring. For example, the structure below is described as 3-pyridyl with the X¹ substituent in the ortho position, the X² substituent in the meta position, and X³ substituent in the para position:

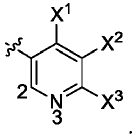

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value. Whenever a yield is given as a percentage, such yield refers to a mass of the entity for which the yield is given with respect to the maximum amount of the same entity that could be obtained under the particular stoichiometric conditions. Concentrations that are given as percentages refer to mass ratios, unless indicated differently.

The terms "buffered" solution or "buffer" solution are used herein interchangeably according to their standard meaning. Buffered solutions are used to control the pH of a medium, and their choice, use, and function is known to those of ordinary skill in the art. See, for example, G.D. Considine, ed., Van Nostrand's Encyclopedia of Chemistry, p. 261, 5th ed. (2005), describing, inter alia, buffer solutions and how the concentrations of the buffer constituents relate to the pH of the buffer. For example, a buffered solution is obtained by adding MgSO₄ and NaHCO₃ to a solution in a 10:1 w/w ratio to maintain the pH of the solution at about 7.5.

Any formula given herein is intended to represent compounds having structures depicted by the structural formula as well as certain variations or forms. In particular, compounds of any formula given herein may have asymmetric centers and therefore exist in different enantiomeric forms. All optical isomers of the compounds of the general formula, and mixtures thereof, are considered within the scope of the formula. Thus, any formula given herein is intended to represent a racemate, one or more enantiomeric forms, one or more diastereomeric forms, one or more atropisomeric forms, and mixtures thereof. Furthermore, certain structures may exist as geometric isomers (i.e., *cis* and *trans* isomers), as tautomers, or as atropisomers.

It is also to be understood that compounds that have the same molecular formula but differ in the nature or sequence of bonding of their atoms or the arrangement of their atoms in space are termed "isomers."

Stereoisomers that are not mirror images of one another are termed "diastereomers" and those that are non-superimposable mirror images of each other are termed "enantiomers." When a compound has an asymmetric center, for example, it is bonded to four different groups, and a pair of enantiomers is possible. An enantiomer can be characterized by the absolute configuration of its asymmetric center and is described by the R-and S-sequencing rules of Cahn and Prelog, or by the manner in which the molecule rotates the plane of polarized light and designated as dextrorotatory or levorotatory (*i.e.,* as (+)- or (-)-isomers respectively). A chiral compound can exist as either an individual enantiomer or as a mixture thereof. A mixture containing equal proportions of the enantiomers is called a "racemic mixture."

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci-and nitro-forms of phenyl nitromethane, that are likewise formed by treatment with acid or base.

Tautomeric forms may be relevant to the attainment of the optimal chemical reactivity and biological activity of a compound of interest.

The compounds of this present disclosure may possess one or more asymmetric centers; such compounds can therefore be produced as individual (R)- or (S)-stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

Certain examples contain chemical structures that are depicted as an absolute enantiomer but are intended to indicate enantiopure material that is of unknown configuration. In these cases (R*) or (S*) or (*R) or (*S) is used in the name to indicate that the absolute stereochemistry of the corresponding stereocenter is unknown. Thus, a compound designated as (R*) or (*R) refers to an enantiopure compound with an absolute configuration of either (R) or (S). In cases where the absolute stereochemistry has been confirmed, the structures are named using (R) and (S).

The symbols 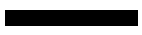 and 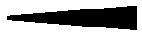 are used as meaning the same spatial arrangement in chemical structures shown herein. Analogously, the symbols 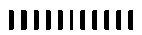 and 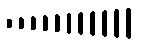 are used as meaning the same spatial arrangement in chemical structures shown herein.

Additionally, any formula given herein is intended to refer also to hydrates, solvates, and polymorphs of such compounds, and mixtures thereof, even if such forms are not listed explicitly. Certain compounds of Formula (I), or pharmaceutically acceptable salts of compounds of Formula (I), may be obtained as solvates. Solvates include those formed from the interaction or complexation of compounds of the present disclosure with one or more solvents, either in solution or as a solid or crystalline form. In some embodiments, the solvent is water and the solvates are hydrates. In addition, certain crystalline forms of compounds of Formula (I), or pharmaceutically acceptable salts of compounds of Formula (I) may be obtained as co-crystals. In certain embodiments of the present disclosure, compounds of Formula (I) were obtained in a crystalline form. In other embodiments, crystalline forms of compounds of Formula (I) were cubic in nature. In other embodiments, pharmaceutically acceptable salts of compounds of Formula (I) were obtained in a crystalline form. In still other embodiments, compounds of Formula (I) were obtained in one of several polymorphic forms, as a mixture of crystalline forms, as a polymorphic form, or as an amorphous form. In other embodiments, compounds of Formula (I) convert in solution between one or more crystalline forms and/or polymorphic forms.

Additionally, any formula given herein is intended to refer also to hydrates, solvates, and polymorphs of such compounds, and mixtures thereof, even if such forms are not listed explicitly. Certain compounds of Formula (Ia), or pharmaceutically acceptable salts of compounds of Formula (Ia), may be obtained as solvates. Solvates include those formed from the interaction or complexation of compounds of the present disclosure with one or more solvents, either in solution or as a solid or crystalline form. In some embodiments, the solvent is water and the solvates are hydrates. In addition, certain crystalline forms of compounds of Formula (Ia), or pharmaceutically acceptable salts of compounds of Formula (Ia) may be obtained as co-crystals. In certain embodiments of the present disclosure, compounds of Formula (Ia) were obtained in a crystalline form. In other embodiments, crystalline forms of compounds of Formula (Ia) were cubic in nature. In other embodiments, pharmaceutically acceptable salts of compounds of Formula (Ia) were obtained in a crystalline form. In still other embodiments, compounds of Formula (Ia) were obtained in one of several polymorphic forms, as a mixture of crystalline forms, as a polymorphic form, or as an amorphous form. In other embodiments, compounds of Formula (Ia) convert in solution between one or more crystalline forms and/or polymorphic forms.

Reference to a compound herein stands for a reference to any one of: (a) the actually recited form of such compound, and (b) any of the forms of such compound in the medium in which the compound is being considered when named. For example, reference herein to a compound such as R-COOH, encompasses reference to any one of, for example, R-COOH₍ₛ₎, R-COOH₍ₛₒₗ₎, and R-COO⁻₍ₛₒₗ₎. In this example, R-COOH₍ₛ₎ refers to the solid compound, as it could be for example in a tablet or some other solid pharmaceutical composition or preparation; R-COOH₍ₛₒₗ₎ refers to the undissociated form of the compound in a solvent; and R-COO⁻₍ₛₒₗ₎ refers to the dissociated form of the compound in a solvent, such as the dissociated form of the compound in an aqueous environment, whether such dissociated form derives from R-COOH, from a salt thereof, or from any other entity that yields R-COO⁻ upon dissociation in the medium being considered. In another example, an expression such as "exposing an entity to compound of formula R-COOH" refers to the exposure of such entity to the form, or forms, of the compound R-COOH that exists, or exist, in the medium in which such exposure takes place. In still another example, an expression such as "reacting an entity with a compound of formula R-COOH" refers to the reacting of (a) such entity in the chemically relevant form, or forms, of such entity that exists, or exist, in the medium in which such reacting takes place, with (b) the chemically relevant form, or forms, of the compound R-COOH that exists, or exist, in the medium in which such reacting takes place. In this regard, if such entity is for example in an aqueous environment, it is understood that the compound R-COOH is in such same medium, and therefore the entity is being exposed to species such as R-COOH_{(aq)} and/or R-COO⁻_{(aq)}, where the subscript "(aq)" stands for "aqueous" according to its conventional meaning in chemistry and biochemistry. A carboxylic acid functional group has been chosen in these nomenclature examples; this choice is not intended, however, as a limitation but it is merely an illustration. It is understood that analogous examples can be provided in terms of other functional groups, including but not limited to hydroxyl, basic nitrogen members, such as those in amines, and any other group that interacts or transforms according to known manners in the medium that contains the compound. Such interactions and transformations include, but are not limited to, dissociation, association, tautomerism, solvolysis, including hydrolysis, solvation, including hydration, protonation, and deprotonation. No further examples in this regard are provided herein because these interactions and transformations in a given medium are known by any one of ordinary skill in the art.

In another example, a zwitterionic compound is encompassed herein by referring to a compound that is known to form a zwitterion, even if it is not explicitly named in its zwitterionic form. Terms such as zwitterion, zwitterions, and their synonyms zwitterionic compound(s) are standard IUPAC-endorsed names that are well known and part of standard sets of defined scientific names. In this regard, the name zwitterion is assigned the name identification CHEBI:27369 by the Chemical Entities of Biological Interest (ChEBI) dictionary of molecular entities. As generally well known, a zwitterion or zwitterionic compound is a neutral compound that has formal unit charges of opposite sign. Sometimes these compounds are referred to by the term "inner salts". Other sources refer to these compounds as "dipolar ions", although the latter term is regarded by still other sources as a misnomer. As a specific example, aminoethanoic acid (the amino acid glycine) has the formula H₂NCH₂COOH, and it exists in some media (in this case in neutral media) in the form of the zwitterion ⁺H₃NCH₂COO⁻. Zwitterions, zwitterionic compounds, inner salts and dipolar ions in the known and well established meanings of these terms are within the scope of this present disclosure, as would in any case be so appreciated by those of ordinary skill in the art. Because there is no need to name each and every embodiment that would be recognized by those of ordinary skill in the art, no structures of the zwitterionic compounds that are associated with the compounds of this present disclosure are given explicitly herein. They are, however, part of the embodiments of this present disclosure. No further examples in this regard are provided herein because the interactions and transformations in a given medium that lead to the various forms of a given compound are known by any one of ordinary skill in the art.

Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the present disclosure include isotopes of hydrogen, carbon, nitrogen, oxygen, phosphorus, sulfur, fluorine, chlorine, and iodine such as ²H, ³H, ¹¹C, ¹³C, ¹⁴C, ¹⁵N, ¹⁸O, ¹⁷O, ³¹P, ³²P, ³⁵S, ¹⁸F, ³⁶Cl, ¹²⁵I, respectively. Such isotopically labeled compounds are useful in metabolic studies (preferably with ¹⁴C), reaction kinetic studies (with, for example deuterium (i.e., D or ²H); or tritium (i.e., T or ³H)), detection or imaging techniques such as positron emission tomography (PET) or single-photon emission computed tomography (SPECT) including drug or substrate tissue distribution assays, or in radioactive treatment of patients. In particular, an ¹⁸F or ¹¹C labeled compound may be particularly preferred for PET or SPECT studies. Further, substitution with heavier isotopes such as deuterium (i.e., ²H) may afford certain therapeutic advantages resulting from greater metabolic stability, for example increased *in vivo* half-life or reduced dosage requirements. Isotopically labeled compounds of this present disclosure and prodrugs thereof can generally be prepared by carrying out the procedures disclosed in the schemes or in the examples and preparations described below by substituting a readily available isotopically labeled reagent for a non-isotopically labeled reagent.

When referring to any formula given herein, the selection of a particular moiety from a list of possible species for a specified variable is not intended to define the same choice of the species for the variable appearing elsewhere. In other words, where a variable appears more than once, the choice of the species from a specified list is independent of the choice of the species for the same variable elsewhere in the formula, unless stated otherwise.

According to the foregoing interpretive considerations on assignments and nomenclature, it is understood that explicit reference herein to a set implies, where chemically meaningful and unless indicated otherwise, independent reference to embodiments of such set, and reference to each and every one of the possible embodiments of subsets of the set referred to explicitly.

By way of a first example on substituent terminology, if substituent S¹ₑₓₐₘₚₗₑ is one of S₁ and S₂, and substituent S²ₑₓₐₘₚₗₑ is one of S₃ and S₄, then these assignments refer to embodiments of this present disclosure given according to the choices S¹ₑₓₐₘₚₗₑ is S₁ and S²ₑₓₐₘₚₗₑ is S₃; S¹ₑₓₐₘₚₗₑ is S₁ and S²ₑₓₐₘₚₗₑ is S₄; S¹ₑₓₐₘₚₗₑ is S₂ and S²ₑₓₐₘₚₗₑ is S₃; S¹ₑₓₐₘₚₗₑ is S₂ and S²ₑₓₐₘₚₗₑ is S₄; and equivalents of each one of such choices. The shorter terminology "S¹ₑₓₐₘₚₗₑ is one of S₁ and S₂, and S²ₑₓₐₘₚₗₑ is one of S₃ and S₄" is accordingly used herein for the sake of brevity, but not by way of limitation. The foregoing first example on substituent terminology, which is stated in generic terms, is meant to illustrate the various substituent assignments described herein. The foregoing convention given herein for substituents extends, when applicable, to members such as R¹, R², R³, R⁴, R⁵, G¹, G², G³, G⁴, G⁵, G⁶, G⁷, G⁸, G⁹, G¹⁰, G¹¹, n, L, R, T, Q, W, X, Y, and Z and any other generic substituent symbol used herein.

Furthermore, when more than one assignment is given for any member or substituent, embodiments of this present disclosure comprise the various groupings that can be made from the listed assignments, taken independently, and equivalents thereof. By way of a second example on substituent terminology, if it is herein described that substituent Sₑₓₐₘₚₗₑ is one of S₁, S₂, and S₃, this listing refers to embodiments of this present disclosure for which Sₑₓₐₘₚₗₑ is S₁; Sₑₓₐₘₚₗₑ is S₂; Sₑₓₐₘₚₗₑ is S₃; Sₑₓₐₘₚₗₑ is one of S₁ and S₂; Sₑₓₐₘₚₗₑ is one of S₁ and S₃; Sₑₓₐₘₚₗₑ is one of S₂ and S₃; Sₑₓₐₘₚₗₑ is one of S₁, S₂ and S₃; and Sₑₓₐₘₚₗₑ is any equivalent of each one of these choices. The shorter terminology "Sₑₓₐₘₚₗₑ is one of S₁, S₂, and S₃" is accordingly used herein for the sake of brevity, but not by way of limitation. The foregoing second example on substituent terminology, which is stated in generic terms, is meant to illustrate the various substituent assignments described herein. The foregoing convention given herein for substituents extends, when applicable, to members such as R¹, R², R³, R⁴, R⁵, G¹, G², G³, G⁴, G⁵, G⁶, G⁷, G⁸, G⁹, G¹⁰, G¹¹, n, L, R, T, Q, W, X, Y, and Z and any other generic substituent symbol used herein.

The nomenclature "Cᵢ₋ⱼ" with j > i, when applied herein to a class of substituents, is meant to refer to embodiments of this present disclosure for which each and every one of the number of carbon members, from i to j including i and j, is independently realized. By way of example, the term C₁₋₄ refers independently to embodiments that have one carbon member (C₁), embodiments that have two carbon members (C₂), embodiments that have three carbon members (C₃), and embodiments that have four carbon members (C₄).

The term Cₙ₋ₘalkyl refers to an aliphatic chain, whether straight or branched, with a total number N of carbon members in the chain that satisfies n ≤ N ≤ m, with m > n. Any disubstituent referred to herein is meant to encompass the various attachment possibilities when more than one of such possibilities are allowed. For example, reference to disubstituent -A-B-, where A ≠ B, refers herein to such disubstituent with A attached to a first substituted member and B attached to a second substituted member, and it also refers to such disubstituent with A attached to the second substituted member and B attached to the first substituted member.

The present disclosure includes also pharmaceutically acceptable salts of the compounds of Formula (I), preferably of those described above and of the specific compounds exemplified herein, and methods of treatment using such salts.

The term "pharmaceutically acceptable" means approved or approvable by a regulatory agency of Federal or a state government or the corresponding agency in countries other than the United States, or that is listed in the U. S. Pharmacopoeia or other generally recognized pharmacopoeia for use in animals, and more particularly, in humans.

A "pharmaceutically acceptable salt" is intended to mean a salt of a free acid or base of compounds represented by Formula (I) that are non-toxic, biologically tolerable, or otherwise biologically suitable for administration to the subject. It should possess the desired pharmacological activity of the parent compound. See, generally, G.S. Paulekuhn, et al., "Trends in Active Pharmaceutical Ingredient Salt Selection based on Analysis of the Orange Book Database", J. Med. Chem., 2007, 50:6665-72, S.M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002. Examples of pharmaceutically acceptable salts are those that are pharmacologically effective and suitable for contact with the tissues of patients without undue toxicity, irritation, or allergic response. A compound of Formula (I) may possess a sufficiently acidic group, a sufficiently basic group, or both types of functional groups, and accordingly react with a number of inorganic or organic bases, and inorganic and organic acids, to form a pharmaceutically acceptable salt.

The present disclosure also relates to pharmaceutically acceptable prodrugs of the compounds of Formula (I) and compounds of Formula (Ia), and treatment methods employing such pharmaceutically acceptable prodrugs. The term "prodrug" means a precursor of a designated compound that, following administration to a subject, yields the compound *in vivo* via a chemical or physiological process such as solvolysis or enzymatic cleavage, or under physiological conditions (e.g., a prodrug on being brought to physiological pH is converted to the compound of Formula (I) or the compound of Formula (Ia)). A "pharmaceutically acceptable prodrug" is a prodrug that is non-toxic, biologically tolerable, and otherwise biologically suitable for administration to the subject. Illustrative procedures for the selection and preparation of suitable prodrug derivatives are described, for example, in *"*Design of Prodrugs ", ed. H. Bundgaard, Elsevier, 1985.

The present disclosure also relates to pharmaceutically active metabolites of the compounds of Formula (I) and compounds of Formula (Ia), which may also be used in the methods of the present disclosure. A "pharmaceutically active metabolite" means a pharmacologically active product of metabolism in the body of a compound of Formula (I) or salt thereof or a compound of Formula (Ia) or salt thereof. Prodrugs and active metabolites of a compound may be determined using routine techniques known or available in the art. See, e.g., Bertolini, et al., J Med Chem. 1997, 40, 2011-2016; Shan, et al., J Pharm Sci. 1997, 86 (7), 765-767; Bagshawe, Drug Dev Res. 1995, 34, 220-230; Bodor, Adv Drug Res. 1984, 13, 224-331; Bundgaard, Design of Prodrugs (Elsevier Press, 1985); and Larsen, Design and Application of Prodrugs, Drug Design and Development (Krogsgaard-Larsen, et al., eds., Harwood Academic Publishers, 1991).

As used herein, the term "composition" or "pharmaceutical composition" refers to a mixture of at least one compound provided herein with a pharmaceutically acceptable carrier. The pharmaceutical composition facilitates administration of the compound to a patient or subject. Multiple techniques of administering a compound exist in the art including, but not limited to, intravenous, oral, aerosol, parenteral, ophthalmic, pulmonary and topical administration.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound provided herein within or to the patient such that it can perform its intended function. Typically, such constructs are carried or transported from one organ, or portion of the body, to another organ, or portion of the body. Each carrier must be "acceptable" in the sense of being compatible with the other ingredients of the formulation, including the compound provided herein, and not injurious to the patient. Some examples of materials that can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound provided herein, and are physiologically acceptable to the patient. Supplementary active compounds can also be incorporated into the compositions. The "pharmaceutically acceptable carrier" can further include a pharmaceutically acceptable salt of the compound provided herein. Other additional ingredients that can be included in the pharmaceutical compositions provided herein are known in the art and described, for example in Remington's Pharmaceutical Sciences (Genaro, Ed., Mack Publishing Co., 1985, Easton, PA), which is incorporated herein by reference.

The term "stabilizer," as used herein, refers to polymers capable of chemically inhibiting or preventing degradation of a compound disclosed herein. Stabilizers are added to formulations of compounds to improve chemical and physical stability of the compound.

The term "tablet," as used herein, denotes an orally administrable, single-dose, solid dosage form that can be produced by compressing a drug substance or a pharmaceutically acceptable salt thereof, with suitable excipients (e.g., fillers, disintegrants, lubricants, glidants, and/or surfactants) by conventional tableting processes. The tablet can be produced using conventional granulation methods, for example, wet or dry granulation, with optional comminution of the granules with subsequent compression and optional coating. The tablet can also be produced by spray-drying.

As used herein, the term "capsule" refers to a solid dosage form in which the drug is enclosed within either a hard or soft soluble container or "shell." The container or shell can be formed from gelatin, starch and/or other suitable substances.

As used herein, the terms "effective amount," "pharmaceutically effective amount," and "therapeutically effective amount" refer to a nontoxic but sufficient amount of an agent to provide the desired biological result. That result may be reduction or alleviation of the signs, symptoms, or causes of a disease, or any other desired alteration of a biological system. An appropriate therapeutic amount in any individual case may be determined by one of ordinary skill in the art using routine experimentation.

The term "combination," "therapeutic combination," "pharmaceutical combination," or "combination product" as used herein refer to a non-fixed combination or a kit of parts for the combined administration where two or more therapeutic agents can be administered independently, at the same time or separately within time intervals, especially where these time intervals allow that the combination partners show a cooperative, e.g., synergistic, effect.

The term "modulators" include both inhibitors and activators, where "inhibitors" refer to compounds that decrease, prevent, inactivate, desensitize, or down-regulate HBV assembly and other HBV core protein functions necessary for HBV replication or the generation of infectious particles.

As used herein, the term "capsid assembly modulator" refers to a compound that disrupts or accelerates or inhibits or hinders or delays or reduces or modifies normal capsid assembly (e.g., during maturation) or normal capsid disassembly (e.g., during infectivity) or perturbs capsid stability, thereby inducing aberrant capsid morphology and function. In one embodiment, a capsid assembly modulator accelerates capsid assembly or disassembly, thereby inducing aberrant capsid morphology. In another embodiment, a capsid assembly modulator interacts (e.g. binds at an active site, binds at an allosteric site, modifies and/or hinders folding and the like) with the major capsid assembly protein (CA), thereby disrupting capsid assembly or disassembly. In yet another embodiment, a capsid assembly modulator causes a perturbation in structure or function of CA (e.g., ability of CA to assemble, disassemble, bind to a substrate, fold into a suitable conformation, or the like), which attenuates viral infectivity and/or is lethal to the virus.

As used herein, the term "treatment" or "treating," is defined as the application or administration of a therapeutic agent, i.e., a compound of the present disclosure (alone or in combination with another pharmaceutical agent), to a patient, or application or administration of a therapeutic agent to an isolated tissue or cell line from a patient (e.g., for diagnosis or ex vivo applications), who has an HBV infection, a symptom of HBV infection or the potential to develop an HBV infection, with the purpose to cure, heal, alleviate, relieve, alter, remedy, ameliorate, improve or affect the HBV infection, the symptoms of HBV infection or the potential to develop an HBV infection. Such treatments may be specifically tailored or modified, based on knowledge obtained from the field of pharmacogenomics.

As used herein, the term "prevent" or "prevention" means no disorder or disease development if none had occurred, or no further disorder or disease development if there had already been development of the disorder or disease. Also considered is the ability of one to prevent some or all of the symptoms associated with the disorder or disease.

As used herein, the term "patient," "individual" or "subject" refers to a human or a non-human mammal. Non-human mammals include, for example, livestock and pets, such as ovine, bovine, porcine, canine, feline and murine mammals. Preferably, the patient, subject or individual is human.

In treatment methods according to the present disclosure, an effective amount of a pharmaceutical agent according to the present disclosure is administered to a subject suffering from or diagnosed as having such a disease, disorder, or condition. An "effective amount" means an amount or dose sufficient to generally bring about the desired therapeutic or prophylactic benefit in patients in need of such treatment for the designated disease, disorder, or condition. Effective amounts or doses of the compounds of the present disclosure may be ascertained by routine methods such as modeling, dose escalation studies or clinical trials, and by taking into consideration routine factors, e.g., the mode or route of administration or drug delivery, the pharmacokinetics of the compound, the severity and course of the disease, disorder, or condition, the subject's previous or ongoing therapy, the subject's health status and response to drugs, and the judgment of the treating physician. An example of a dose is in the range of from about 0.001 to about 200 mg of compound per kg of subject's body weight per day, preferably about 0.05 to 100 mg/kg/day, or about 1 to 35 mg/kg/day, in single or divided dosage units (e.g., BID, TID, QID). For a 70-kg human, an illustrative range for a suitable dosage amount is from about 0.05 to about 7 g/day, or about 0.2 to about 2.5 g/day.

An example of a dose of a compound is from about 1 mg to about 2,500 mg. In some embodiments, a dose of a compound of the present disclosure used in compositions described herein is less than about 10,000 mg, or less than about 8,000 mg, or less than about 6,000 mg, or less than about 5,000 mg, or less than about 3,000 mg, or less than about 2,000 mg, or less than about 1,000 mg, or less than about 500 mg, or less than about 200 mg, or less than about 50 mg. Similarly, in some embodiments, a dose of a second compound (i.e., another drug for HBV treatment) as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

Once improvement of the patient's disease, disorder, or condition has occurred, the dose may be adjusted for preventative or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Patients may, however, require intermittent treatment on a long-term basis upon any recurrence of symptoms.

HBV infections that may be treated according to the disclosed methods include HBV genotype A, B, C, and/or D infections. However, in an embodiment, the methods disclosed may treat any HBV genotype ("pan-genotypic treatment"). HBV genotyping may be performed using methods known in the art, for example, INNO-LIPA^{®} HBV Genotyping, Innogenetics N.V., Ghent, Belgium).

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated.

Each of the relevant disclosures of all references cited herein is specifically incorporated by reference. The following examples are offered by way of illustration, and not by way of limitation.

### EXAMPLES

Exemplary compounds useful in methods of the present disclosure will now be described by reference to the illustrative synthetic schemes for their general preparation below and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Unless otherwise specified, the variables are as defined above in reference to Formula (I) and Formula (Ia). Reactions may be performed between the melting point and the reflux temperature of the solvent, and preferably between 0 °C and the reflux temperature of the solvent. Reactions may be heated employing conventional heating or microwave heating. Reactions may also be conducted in sealed pressure vessels above the normal reflux temperature of the solvent.

Compounds of Formula (I) and Formula (Ia) may be converted to their corresponding salts using methods known to one of ordinary skill in the art. For example, an amine of Formula (I) is treated with trifluoroacetic acid, HCl, or citric acid in a solvent such as Et₂O, CH₂Cl₂, THF, MeOH, chloroform, or isopropanol to provide the corresponding salt form. Alternately, trifluoroacetic acid or formic acid salts are obtained as a result of reverse phase HPLC purification conditions. Crystalline forms of pharmaceutically acceptable salts of compounds of Formula (I) and Formula (Ia) may be obtained in crystalline form by recrystallization from polar solvents (including mixtures of polar solvents and aqueous mixtures of polar solvents) or from non-polar solvents (including mixtures of non-polar solvents).

Where the compounds according to this present disclosure have at least one chiral center, they may accordingly exist as enantiomers. Where the compounds possess two or more chiral centers, they may additionally exist as diastereomers. It is to be understood that all such isomers and mixtures thereof are encompassed within the scope of the present disclosure.

Compounds represented as "stereomeric mixture" (means a mixture of two or more stereoisomers and includes enantiomers, diastereomers and combinations thereof) are separated by SFC resolution.

Compounds may be obtained as single forms, such as single enantiomers, by form-specific synthesis, or by resolution. Compounds may alternately be obtained as mixtures of various forms, such as racemic (1:1) or non-racemic (not 1:1) mixtures. Where racemic and non-racemic mixtures of enantiomers are obtained, single enantiomers may be isolated using conventional separation methods known to one of ordinary skill in the art, such as chiral chromatography, recrystallization, diastereomeric salt formation, derivatization into diastereomeric adducts, biotransformation, or enzymatic transformation. Where regioisomeric or diastereomeric mixtures are obtained, as applicable, single isomers may be separated using conventional methods such as chromatography or crystallization.

### 1. GENERAL INFORMATION

### CHEMICAL NAMES

Chemical names were generated using the chemistry software: ACD/ChemSketch, and may follow preferably the IUPAC rules.

### GENERAL PROCEDURE FOR LCMS METHODS

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc.). All results were obtained with experimental uncertainties that are commonly associated with the method used. Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica., "Q-Tof" Quadrupole Time-of-flight mass spectrometers, "CLND", ChemiLuminescent Nitrogen Detector, "ELSD" Evaporative Light Scanning Detector.

### LCMS Methods

**Table 5**

| (Flow expressed in mL/min; column temperature (T) in °C; Run time in minutes). | | | | | | |
|---|---|---|---|---|---|---|
| Method code | Instrument | Column | Mobile phase | Gradient | $\frac{Flow}{Col T}$ | Run time |
| A | Thermoscient ific Ultimate 3000 DAD and Brucker HCT ultra | Agilent: Poroshell EC-C18 (4 µm, 4.6x100 mm) | A: HCO₂H 0.1% in water/ | 98% A for 2 min, to 0% A in 10 min, held for 3.4 min, back to 98% A in 1.3 min, held for 1.7 min | $\frac{1}{30}$ | 18.4 |
| | | | B: HCOOH 0.05% in CH₃CN | | | |
| B | Waters: Acquity^{®} UPLC^{®} - DAD, SQD and ELSD | Waters: HSS T3 (1.8µm, 2.1*100mm) | A: 10mM CH₃COONH₄ in 95% H₂O + 5% CH₃CN | From 100% A to 5% A in 2.10 min, to 0% A in 0.90 min, to 5% A in 0.5 min | $\frac{0.6}{55}$ | 3.5 |
| | | | B: CH₃CN | | | |
| C | Waters: Acquity^{®} UPLC^{®} - DAD and SQD | Waters: BEH C18 (1.7µm, 2.1*50mm) | A: 10mM CH₃COONH₄ in 95% H₂O + 5% CH₃CN | From 95% A to 5% A in 1.3 min, held for 0.7 min. | $\frac{0.8}{55}$ | 2 |
| | | | B: CH₃CN | | | |
| D | Waters: Acquity^{®} UPLC^{®} - DAD and SQD | Waters: BEH (1.8µm, 2.1*100mm) | A: 10mM CH₃COONH₄ in 95% H₂O + 5% CH₃CN | From 100% to 5% A in 2.10 min, to 0% A in 0.90 min, to 5% A in 0.5 min | $\frac{0.6}{55}$ | 3.5 |
| | | | B: CH₃CN | | | |
| E | Waters: Acquity^{®} UPLC^{®} - DAD and SQD | Waters : BEH (1.8µm, 2.1*100mm) | A: 10mM CH₃COONH₄ in 95% H₂O + 5% CH₃CN B: CH₃CN | From 100% A to 5% A in 2.10min, to 0% A in 0.90min, to 5% A in 0.5min | $\frac{0.6}{55}$ | 3.5 |
| F | Agilent Poro shell 120 | SB-C18 4.6x30mm 2. 7 µm with UHPLC Guard Infinity Lab Poroshell 120 SB-C18 4.6x 5mm 2.7 µm | A - acetonitrile : water (99:1%), 0.1% formic acid | 0.01 min -99% B | $\frac{3}{60}$ | 5,92 |
| | | | | 1.5 min - 0% B | | |
| | | | | 2.2 min - 0% B | | |
| | | | B - water (0.1% formic acid) | 2.21 min - 99% B | | |
| G | Agilent 1200 with DAD | Sunfire C18 column (5 µm, 4.6 x 50 mm) | A1: 0.02% ammoniumacet ate; A2: 0.1% TFA; mobile phase B1: acetonitrile | From 95 % A1 or A2 and 5% B to 5 % A1 or A2 and 95% B | 1.5 | 6.5 |
| H | Agilent 1260 with VWD | Dikma plus C18 column (5 µm, 4.6 x 30 mm) | A1: 0.02% ammoniumacet ate; A2: 0.1% TFA; B: acetonitrile | 95 % A1 or A2 and 5% B to 5 % A1 or A2 and 95% B | 1.5 | 4 |
| I | Waters: Acquity^{®} UPLC^{®} - DAD and SQD | Waters :BEH (1.8µm, 2.1*100mm) | A: 0.1% NH₄HCO₃ in 95% H₂O + 5% CH₃CN | From 100% A to 5% A in 2.10min, to 0% A in 0.9min, to 5% A in 0.5min | $\frac{0.6}{55}$ | 3.5 |
| | | | B: CH₃CN | | | |
| J | Waters: Acquity^{®} UPLC^{®} - DAD and SQD | Waters :BEH (1.8µm, 2.1*100mm) | A: 10mM CH₃COONH₄ in 95% H₂O + 5% CH₃CN | From 100% A to 5% A in 2.10min, to 0% A in 0.9min, to 5% A in 0.4min | $\frac{0.6}{55}$ | 3.5 |
| | | | B: CH₃CN | | | |

### GENERAL PROCEDURE FOR SFCMS METHODS

The SFC measurement was performed using an Analytical Supercritical fluid chromatography (SFC) system composed by a binary pump for delivering carbon dioxide (CO2) and modifier, an autosampler, a column oven, a diode array detector equipped with a high-pressure flow cell standing up to 400 bars. If configured with a Mass Spectrometer (MS) the flow from the column was brought to the (MS). It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Analytical SFC-MS Methods (Flow expressed in mL/min; column temperature (T) in °C; Run time in minutes, Backpressure (BPR) in bars.

### SFC Methods

**Table 6**

| (Flow expressed in mL/min; column temperature (T) in °C; Run time in minutes). | | | | | |
|---|---|---|---|---|---|
| Method code | Column | Mobile phase | Gradient | $\frac{Flow}{Col T}$ | $\frac{Run time}{BPR}$ |
| SFC_A | Daicel Chiralpak^{®} IC3 column (3.0 µm, 150 x 4.6 mm) | A:CO₂ | 10%-50% B in 6 min, hold 3.5 min | $\frac{2.5}{40}$ | $\frac{9.5}{130}$ |
| | | B: EtOH+0.2% iPrNH₂ | | | |
| SFC_B | Daicel Chiralpak^{®} AD3 column (3.0 µm, 150 x 4.6 mm) | A:CO₂ | 10%-50% B in 6 min, hold 3.5 min | $\frac{2.5}{40}$ | $\frac{9.5}{130}$ |
| | | B: EtOH-iPrOH (50-50)+0.2% iPrNH₂ | | | |

| Method code | Column | Mobile phase | Gradient | $\frac{Flow}{Col T}$ | $\frac{Run time}{BPR}$ |
|---|---|---|---|---|---|
| SFC_C | Daicel Chiralpak^{®} OJ3 column (3.0 µm, 150 x 4.6 mm) | A:CO₂ | 10%-50% B in 6 min, hold 3.5 min | $\frac{2.5}{40}$ | $\frac{9.5}{130}$ |
| | | B: EtOH+0.2% iPrNH₂ | | | |
| | | | | | |
| SFC_D | Daicel Chiralpak^{®} AD-H column (3.0 µm, 150 x 4.6 mm) | A:CO₂ | 10%-50% B in 6 min, hold 3.5 min | $\frac{2.5}{40}$ | $\frac{9.5}{110}$ |
| | | B: iPrOH+0.2% iPrNH₂ | | | |
| | | | | | |
| SFC_E | Daicel Chiralpak^{®} AD-H column (3.0 µm, 150 x 4.6 mm) | A:CO₂ | 10%-50% B in 6 min, hold 3.5 min | $\frac{2.5}{40}$ | $\frac{9.5}{110}$ |
| | | B: EtOH+0.2% iPrNH₂ | | | |

### NMR ANALYSIS

¹H NMR spectra were recorded on 1) a Bruker DPX 400 MHz spectrometer or 2) a Bruker Avance 400 MHz spectrometer or c) Bruker Avance III 400 MHz spectrometer or d) Bruker Avance 600 MHz spectrometer or e) a Bruker Avance NEO 400 MHz spectrometer or f) Bruker model AVIII 400 spectrometer g) ZKNJ BIXI-1 300 MHz, Bruker Avance III 400 MHz or h) Bruker AVANCE Neo 400 MHz.

NMR spectra were recorded at ambient temperature unless otherwise stated. Data are reported as follow: chemical shift in parts per million (ppm) relative to TMS (δ = 0 ppm) on the scale, integration, multiplicity (s = singulet, d = doublet, t = triplet, q = quartet, quin = quintet, sext = sextet, sept = septet, m = multiplet, b = broad, or a combination of these), coupling constant(s) *J* in Hertz (Hz).

### MS ANALYSIS

Mass spectra were obtained on a Shimadzu LCMS-2020 MSD or Agilent 1200/G6110A MSD using electrospray ionization (ESI) in positive mode unless otherwise indicated.

### 2. ABBREVIATIONS

**Table 7**

| | |
|---|---|
| µw | Microwave |
| ADDP | 1,1'-(Azodicarbonyl)dipiperidine |
| AlMe₃ | Trimethylaluminium |
| aq. | Aqueous |
| atm | Atmosphere |
| Boc₂O | Di-tert-butyl dicarbonate |
| BOC | tert-Butyloxycarbonyl |
| BODIPY | Boron-dipyrromethene |
| br | broad |
| CA | Capsid assembly |
| DAST | (Diethylamino)sulfur trifluoride |
| DBU | 1,8-Diazabicyclo[5.4.0]undec-7-ene |
| DCE | 1,2-Dichloroethane |
| DCM | Dichloromethane |
| dd | Doublet of doublets |
| DEA | Diethylamine |
| DIPE | Diisopropyl ether |
| DIPEA/DIEA | *N*,*N*-diisopropylethylamine |
| DMF | Dimethylformamide |
| DMF-DMA | Dimethylformamide-dimethylacetal |
| DMSO | Dimethylsulfoxide |
| DNA | Deoxyribonucleic Acid |
| EDCI | *N*-(3-Dimethylaminopropyl)-*N*'-ethylcarbodiimide hydrochloride |
| ESI | Electrospray ionization |
| Et₃N | Triethylamine |
| Et₂O | Ether, diethyl ether |
| EtOAc/EA | Ethyl acetate |
| EtOH | Ethanol |
| FCC | Normal-phase silica gel chromatography |
| g | grams |
| h/hr | hour |
| HBV | Hepatitis B virus |
| HOAc | Acetic acid |
| HPLC | High Performance Liquid Chromatography |
| Hz | Hertz |
| *i*-PrNH₂ | Isopropylamine |
| *i*-PrOH/IPA | Isopropyl alcohol |
| KO*t*Bu | Potassium tert-butoxide |
| LAH | Lithium aluminum hydride |
| LCMS | Liquid Chromatography Mass Spectrometry |
| LHMDS, LiHMDS | Lithium bis(trimethylsilyl)amide |
| M | molar |
| m | multiplet |
| MeCN/ACN | Acetonitrile |
| MeOH | Methanol |
| mg | milligrams |
| MHz | megahertz |
| min | Minutes |
| mL | Milliliters |
| µL | microliters |
| mmol | Millimole |
| µmol | micromole |
| MS | Mass spectra |
| MsCl | Mesityl chloride |
| m/z | Mass to charge ratio |
| N | normal |
| NaOAc/AcONa | Sodium acetate |
| NMR | Nuclear Magnetic Resonance |
| o/n | Overnight |
| PCR | Polymerase chain reaction |
| PE | Petroleum ether |
| PMPA | 9-(2-Phosphonyl-methoxypropyl)adenine |
| ppm | Parts per million |
| ppt | precipitate |
| Py | pyridine |
| RNA | Ribonucleic Acid |
| Rₜ | Retention time |
| rt | Room temperature |
| s | singlet |
| sat. | Saturated |
| SFC | Supercritical Fluid Chromatography |
| t | triplet |
| T₃P | Propanephosphonic acid anhydride |
| TBAI | Tetrabutylammonium iodide |
| TBD | 1,5,7-Triazabicyclo[4.4.0]dec-5-ene |
| TEA | triethylamine |
| TFA | Trifluoroacetic acid |
| THF | Tetrahydrofuran |
| TLC | Thin Layer Chromatography |
| TLR | Toll-like receptor |
| TNF | Tumor necrosis factor |
| V or volumes | Volume in milliliters of solvent per gram of substrate |
| Δ | Heating under reflux |

### 3. PROCEDURES AND COMPOUNDS ANALYSIS

### 3.1. SYNTHESIS OF THE 6-MEMBERED RINGS

### 3.1.1. Synthesis of Intermediates

### 3.1.1.1. Synthesis of Intermediate I3

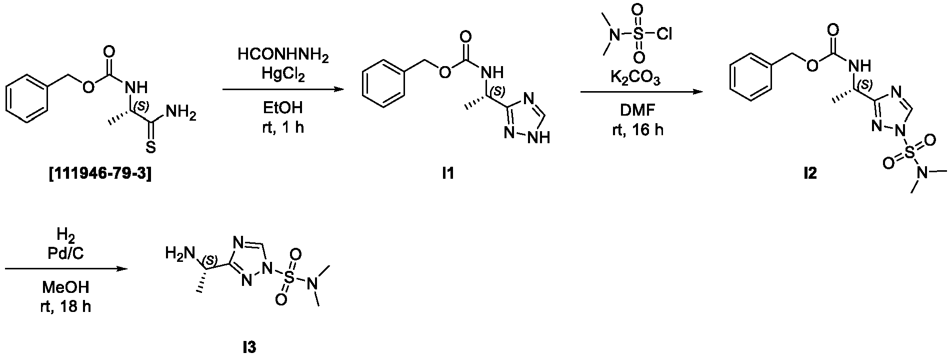

### Intermediate I1

### Benzyl [(1S)-1-(1H-1,2,4-triazol-3-yl)ethyl]carbamate

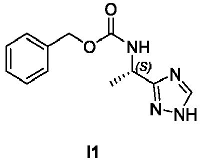

The reaction was performed under anhydrous conditions and under argon atmosphere.

Benzyl [(2*S*)-1-amino-1-sulfanylidenepropan-2-yl]carbamate (3.36 g, 12.1 mmol, 86% purity) was dissolved in EtOH (135 mL). Formic acid hydrazine (4.36 g, 72.7 mmol) and HgCl₂ (4.28 g, 15.7 mmol) were added. The reaction mixture was stirred at room temperature for 1 h. The reaction mixture was filtered and the filtrate was concentrated under reduced pressure. The residue was diluted in EtOAc (200 mL), washed with NaHCO₃ (sat., aq.), brine, dried (Na₂SO₄), filtered and concentrated under reduced pressure. The combined aqueous layers were extracted with EtOAc (5 x 200 mL). The combined organic extracts were dried (Na₂SO₄), filtered and concentrated under reduced pressure. The fraction was combined with the previously obtained residue. The residue was solubilized in EtOH (135 mL) and stirred at 80 °C for 16 h. The reaction mixture was concentrated to dryness. The crude mixture was purified by flash column chromatography (silica, DCM/MeOH, gradient from 100:0 to 90:10) to afford intermediate **I1** (1.82 g, 58%) as a white solid.

### Intermediate I2

### Benzyl {(1S)-1-[1-(dimethylsulfamoyl)-1H-1,2,4-triazol-3-yl]ethyl}carbamate

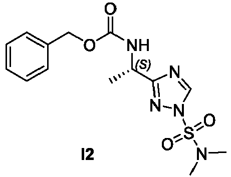

The reaction was performed under anhydrous conditions and under argon atmosphere.

Intermediate **I1** (2.10 g, 8.08 mmol, 95% purity) was dissolved in DMF (53 mL). K₂CO₃ (3.35 g, 24.3 mmol) and dimethylsulfamoyl chloride (1.04 mL, 9.70 mmol) were added. The reaction mixture was stirred at room temperature for 16 h and diluted with H₂O (50 mL). The aqueous layer was extracted with EtOAc (3 x 100 mL). The combined organic layers were washed with brine (3 x 100 mL), dried (Na₂SO₄), filtered and concentrated under reduced pressure. The crude mixture was purified by flash column chromatography (silica, DCM/MeOH, gradient from 100:0 to 90: 10) to give intermediate **I2** (1.42 g, 47%, 94% purity).

### Intermediate I3

### 3-[(1S)-1-Aminoethyl]-N,N-dimethyl-1H-1,2,4-triazole-1-sulfonamide

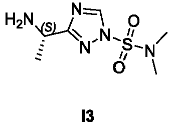

The reaction was performed under anhydrous conditions and under argon atmosphere.

Intermediate **I2** (1.42 g, 3.76 mmol, 94% purity) was dissolved in MeOH (40 mL). The mixture was purged under argon and Pd/C (10% purity, 142 mg, 0.13 mmol) was added. The reaction mixture was purged with H₂ (3 times) and stirred at room temperature for 18 h. The reaction mixture was filtered on Celite^{®} and the filtrate was concentrated under reduced pressure to give intermediate **I3** (830 mg) which was used as such in the next step.

### 3.1.1.2. Synthesis of Intermediate I5

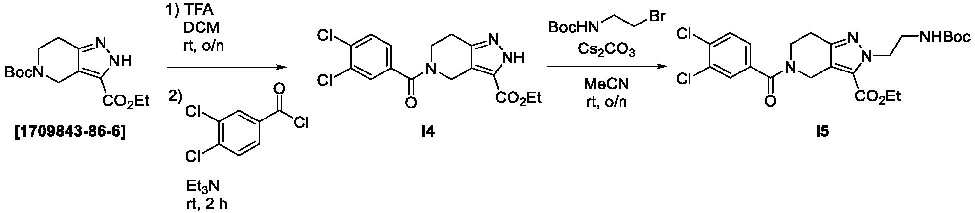

### Intermediate I4

### Ethyl 5-(3,4-dichlorobenzoyl)-4,5,6,7-tetrahydro-2H-pyrazolo[4,3-c]pyridine-3-carboxylate

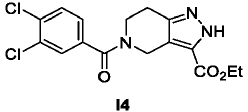

A mixture of 5-*tert*-butyl-3-ethyl 6,7-dihydro-2H-pyrazolo[3,4-c]pyridine-3,5(4H) dicarboxylate (17.8 g, 60.4 mmol) and TFA (46.2 mL, 604 mmol) in DCM (500 mL) was stirred at room temperature overnight. The reaction mixture was concentrated under reduced pressure and the residue was dissolved in DCM. The solution was cooled to 0 °C. Et₃N (33.6 mL, 242 mmol) was added followed by 3,4-dichlorobenzoyl chloride (13.3 g, 63.4 mmol) and the reaction mixture was stirred at room temperature for 2 h. The reaction was quenched with water (300 mL). The layers were separated and the organic phase was dried (MgSO₄), filtered and concentrated under reduced pressure. The crude mixture was purified by flash column chromatography (silica, heptane/(EtOAc/EtOH, 3:1), gradient from 90:10 to 0:100) to afford intermediate **I4** (16.2 g, 73%) as a white solid.

### Intermediate I5

### Ethyl 2-{2-[(tert-butoxycarbonyl)amino]ethyl}-5-(3,4-dichlorobenzoyl)-4,5,6,7-tetrahydro-2H-pyrazolo[4,3-c]pyridine-3-carboxylate

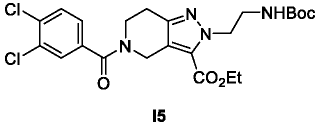

A mixture of intermediate **I4** (4.00 g, 10.9 mmol), Cs₂CO₃ (5.31 g, 16.3 mmol), and 3-(boc-amino)propyl bromide (2.56 g, 11.4 mmol) in MeCN (150 mL) was stirred at room temperature overnight. The mixture was filtered and the filtrate was concentrated under reduced pressure. The residue was purified by flash column chromatography (silica, heptane/(EtOAc/EtOH, 3:1), gradient from 90:10 to 0:100) to afford intermediate **I5** (3.0 g, 54%) as a clear oil.

### 3.1.1.3. Synthesis of Intermediate I6

### 5-tert-Butyl 3-ethyl 2-(2-bromoethyl)-2,4,6,7-tetrahydro-5H-pyrazolo[4,3-c]pyridine-3,5-dicarboxylate

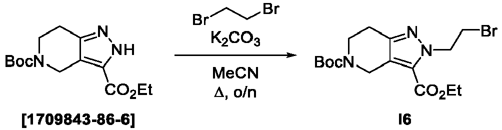

A mixture of 5-*tert*-butyl 3-ethyl 6,7-dihydro-2H-pyrazolo[4,3]pyridine-3,5(4H)-dicarboxylate (1.25 g, 4.23 mmol), 1,2-dibromoethane (4.02 g, 21.2 mmol) and K₂CO₃ (1.76 g, 12.7 mmol) in MeCN (125 mL) was stirred under reflux overnight. The reaction mixture was filtered over decalite while still hot and the filtrate was concentrated under reduced pressure. The residue was purified by flash column chromatography (silica, heptane/EtOAc, gradient from 95:5 to 0:100) to afford intermediate **I6** (1.7 g, 60%) as a clear oil.

### 3.1.1.4. Synthesis of Intermediate I8

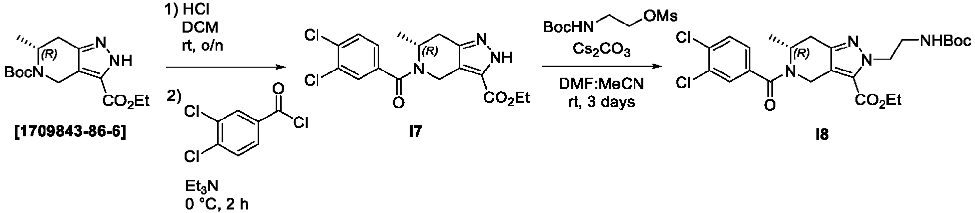

### Intermediate I7

### Ethyl (6R)-5-(3,4-dichlorobenzoyl)-6-methyl-4,5,6,7-tetrahydro-2H-pyrazolo[4,3-c]pyridine-3-carboxylate

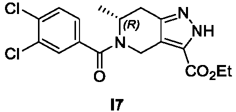

The reaction was performed under anhydrous conditions and under argon atmosphere.

To a solution of 5-tert-butyl 3-ethyl (6*R*)-6-methyl-2,4,6,7-tetrahydropyrazolo[4,3-c]pyridine-3,5-dicarboxylate (4.52 g, 14.6 mmol) in DCM (20 mL) was added HCl (4N in 1,4-dioxane, 36.5 mL, 146 mmol). The reaction mixture was stirred at room temperature for 18 h and concentrated to dryness. The residue was diluted in DCM (150 mL) and Et₃N (9.16 mL, 65.7 mmol) was added. The solution was cooled to 0 °C and a solution of 3,4-dichlorobenzoyl chloride (3.21 g, 15.3 mmol) in DCM (30 mL) was added dropwise. The reaction mixture was stirred at this temperature for 2 h and diluted with DCM (750 mL). The organic phase was washed with water (2 x 500 mL), NaHCO₃ (sat., aq., 500 mL) and brine, dried (Na₂SO₄), filtered and concentrated under reduced pressure. The crude mixture was purified by flash column chromatography (silica, DCM/MeOH, gradient from 100:0 to 95:5) to afford intermediate **I7** (4.63 g, 83%).

### Intermediate I8

### Ethyl (6R)-2-{2-[(tert-butoxycarbonyl)amino]ethyl}-5-(3,4-dichlorobenzoyl)-6-methyl-4,5,6,7-tetrahydro-2H-pyrazolo[4,3-c]pyridine-3-carboxylate

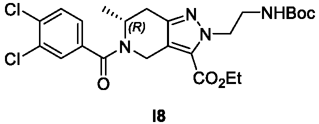

The reaction was performed under anhydrous conditions and under argon atmosphere.

To a solution of intermediate **I7** (4.85 g, 12.1 mmol) in MeCN (180 mL) and DMF (5 mL) were added Cs₂CO₃ (5.92 g, 18.2 mmol) and a solution of 2-(*tert*-butoxycarbonylamino)ethyl methanesulfonate (3.48 g, 14.5 mmol) in MeCN (20 mL). The reaction mixture was stirred at room temperature for 3 days and diluted with EtOAc (100 mL) and H₂O (100 mL). The layers were separated. The aqueous layer was extracted with EtOAc (2 x 100 mL). The combined organic extracts were washed with brine, dried (Na₂SO₄), filtered and concentrated to dryness. The crude mixture was purified by flash column chromatography (silica, cyclohexane/(EtOAc/EtOH, 3:1), gradient from 95:5 to 50:50) to afford intermediate **I8** (3.30 g, 52%).

### 3.1.1.5. Synthesis of Intermediate I9

### Ethyl (6R)-2-(2-bromoethyl)-5-(3,4-dichlorobenzoyl)-6-methyl-4,5,6,7-tetrahydro-2H-pyrazolo-[4,3-c]pyridine-3-carboxylate

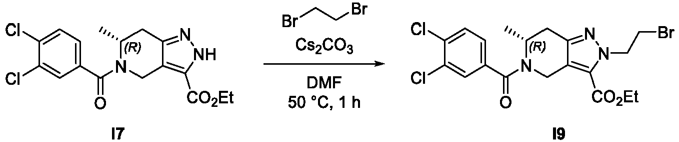

The reaction was performed under anhydrous conditions and under argon atmosphere.

To a solution of intermediate **I7** (4.00 g, 10.5 mmol) in DMF (60 mL) were added Cs₂CO₃ (6.82 g, 20.9 mmol) and 1,2-dibromoethane (4.51 mL, 52.3 mmol). The reaction mixture was stirred at 50 °C for 1 h and diluted with H₂O (50 mL). The aqueous layer was extracted with EtOAc (3 x 70 mL). The combined organic extracts were washed with brine (3 x 60 mL), dried (Na₂SO₄), filtered and concentrated to dryness. The crude mixture was purified by flash column chromatography (silica, cyclohexane/EtOAc, gradient from 9:1 to 1:1) to afford intermediate **I9** (2.90 g, 55%) as a white foam.

### 3.1.1.6. Synthesis of Intermediate I60

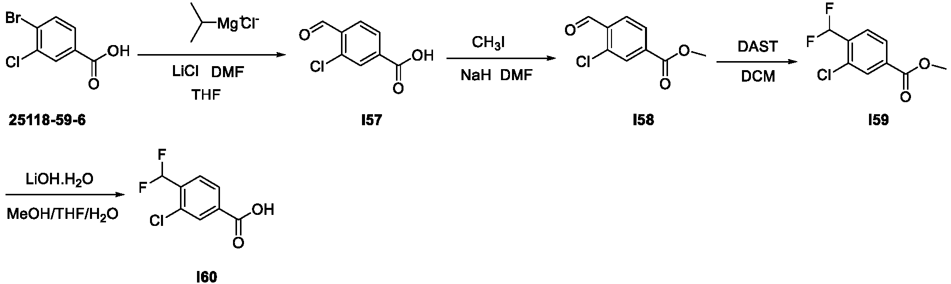

To a solution of 4-bromo-3-chlorobenzoic acid (3.00 g, 12.7 mmol) in tetrahydrofuran (40 mL) was added Lithium chloride (1.62 g, 38.2 mmol) and 2 M isopropylmagnesium chloride in tetrahydrofuran (20 mL, 40 mmol) at -78 °C under nitrogen atmosphere. After stirring at -78 °C for 10 minutes, the mixture was warmed to 0 °C and stirred at this temperature for 1 hour. Then N,N-dimethylformamide (4.69 g, 64.2 mmol) was added. The reaction mixture was warmed to room temperature and stirred for 1.5 hour. The reaction mixture was quenched with saturated aq.NH₄Cl (10 mL) and extracted with ethyl acetate (10 mL) for three times. The aqueous layer was acidified with 1 N hydrochloride aqueous solution to pH = 3. The aqueous layer was extracted with ethyl acetate (10 mL) for three times. The combined organic phases were washed with brine (10 mL), dried over anhydrous sodium sulfate and filtered. The filtrate was concentrated *in vacuo* to give intermediate **I57** (1.9 g, 73% yield, 90% purity from ¹H NMR) as yellow oil. **¹H NMR** (400 MHz, DMSO-*d6*) δ 13.68 (s, 1H), 10.38 (s, 1H), 8.04 - 7.96 (m, 3H).

To a solution of intermediate **I57** (1.00 g, 4.88 mmol, 90% purity) in N,N-dimethylformamide (10 mL) was added 60% wt. sodium hydride in mineral oil (215 mg, 5.38 mmol). The mixture was stirred at room temperature for 30 minutes. Then iodomethane (760 mg, 5.35 mmol) was added. The mixture was stirred at room temperature for 2 hours. The mixture was poured into 1 N hydrochloric acid aqueous solution (5 mL) and extracted with ethyl acetate (10 mL) for three times. The combined organic phases were washed with aq. sodium bicarbonate (10 mL) and brine (10 mL), dried over anhydrous sodium sulfate and filtered. The filtrate was concentrated *in vacuo* to give intermediate **I58** (880 mg, 82% yield, 90% purity from ¹H NMR) as a yellow oil. **¹H NMR** (400 MHz, DMSO-*d6*) δ 10.37 (s, 1H), 8.06 - 7.98 (m, 3H), 3.91 (s, 3H).

To a solution of intermediate **I58** (250 mg, 1.13 mmol, 90% purity) in dichloromethane (10 mL) was added diethylaminosulfur trifluoride (920 mg, 5.71 mmol) at -78 °C. The mixture was stirred at room temperature overnight. The mixture was poured into water (10 mL) and extracted with dichloromethane (10 mL) twice. The combined organic phases were washed with brine (5 mL), dried over anhydrous sodium sulfate and filtered. The filtrate was concentrated to give intermediate **I59** (170 mg, 61% yield, 90% purity from ¹H NMR) as yellow oil. **¹H NMR** (400 MHz,CDCl₃) δ 8.10 - 8.09 (m, 1H), 8.04 - 8.02 (m, 1H), 7.76 - 7.74 (m, 1H), 6.97 (t, *J =* 54.8 Hz, 1H), 3.96 (s, 3H).

To a solution of intermediate **I59** (170 mg, 0.694 mmol, 90% purity) in methanol (3 mL) and tetrahydrofuran (3 mL) was added lithium hydroxide hydrate (45 mg, 1.1 mmol) in water (1.5 mL). The mixture was stirred at room temperature overnight. The mixture was concentrated *in vacuo* to give a residue. The residue was diluted with water (10 mL) and the resulting mixture was acidified with 1 N hydrochloride aqueous solution to pH = 3. The mixture was extracted with ethyl acetate (10 mL) for three times. The combined organic phases were washed with brine (10 mL), dried over anhydrous sodium sulfate and filtered. The filtrate was concentrated to give intermediate **I60** (150 mg, 94% yield, 90% purity from ¹H NMR) as a white solid. **¹H NMR** (400 MHz, DMSO-d6) δ 13.62 (s, 1H), 8.04 - 8.02 (m, 2H), 7.85 - 7.83 (m, 1H), 7.28 (t, *J =* 53.6 Hz, 1H).

### 3.1.1.7. Synthesis of intermediate I64

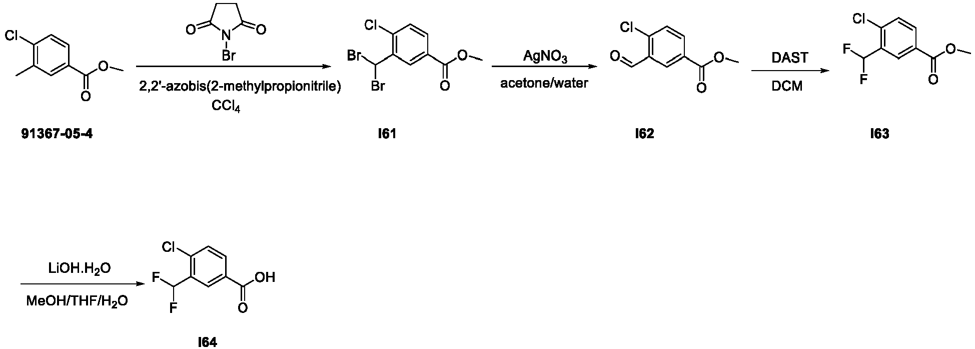

To a solution of methyl 4-chloro-3-methylbenzoate (2.00 g, 10.8 mmol) and N-bromosuccinimide (5.78 g, 32.5 mmol) in carbon tetrachloride (90 mL) was added 2,2'-azobis(2-methylpropionitrile) (450 mg, 2.74 mmol). The mixture was stirred at 80 °C overnight. Then the reaction mixture was filtered and the filtrate was washed with water (10 mL), dried over anhydrous sodium sulfate and concentrated to give a residue, which was triturated with n-hexane (10 mL) to give intermediate **I61** (3.12 g, 80% yield, 95% purity from ¹H NMR) as a white solid. **¹H NMR** (400 MHz, CDCl₃) δ 8.49 (d, *J* = 2.8 Hz, 1H), 7.96 (dd, *J* = 11.2, 2.8 Hz, 1H), 7.70 (d, *J =* 11.2 Hz, 1H), 7.54 (s, 1H), 3.93 (s, 3H).

To a solution of intermediate **I61** (3.12 g, 8.66 mmol, 95% purity) in acetone (100 mL) and water (20 mL) was added silver(I) nitrate (2.94 g, 17.3 mmol) at room temperature. The mixture was refluxed under nitrogen atmosphere for 1.5 hours. Then the reaction mixture was filtered through a pad of celite and the filtrate was concentrated to remove organic solvent. The aqueous phase was extracted with dichloromethane (10 mL) for three times. The combined organic phases were washed with brine (10 mL), dried over anhydrous sodium sulfate and filtered. The filtrate was concentrated *in vacuo* to give intermediate **I62** (1.7 g, 94% yield, 95% purity from ¹HNMR) as yellow oil. **¹H NMR** (400 MHz, CDCl₃) δ 10.49 (s, 1H), 8.57 (d, *J =* 2.4 Hz, 1H), 8.19 (dd, *J* = 8.4, 2.0 Hz, 1H), 7.56 (d, *J =* 8.4 Hz, 1H), 3.95 (s, 3H).

To a solution of intermediate **I62** (300 mg, 1.44 mmol, 95% purity) in dichloromethane (5 mL) was added diethylaminosulfur trifluoride (1.157 g, 7.175 mmol) at -78 °C. The mixture was stirred at room temperature overnight. The mixture was poured into water (10 mL) and extracted with dichloromethane (10 mL) twice. The combined organic phases were washed with brine (5 mL), dried over anhydrous sodium sulfate and filtered. The filtrate was concentrated to give intermediate **II63** (270 mg, 81% yield, 95% purity from ¹H NMR) as a yellow oil.

**¹H NMR** (400 MHz, CDCl₃) δ 8.39 (s, 1H), 8.13 (d, *J =* 11.2 Hz, 1H), 7.57 (d, *J =* 11.2 Hz, 1H), 7.01 (t, *J =* 72.8 Hz, 1H), 4.00 (s, 3H).

To a solution of intermediate **I63** (270 mg, 1.16 mmol, 95% purity) in methanol (3 mL) and tetrahydrofuran (3 mL) was added lithium hydroxide hydrate (75 mg, 1.8 mmol) in water (1.5 mL). The mixture was stirred at room temperature overnight. The mixture was concentrated *in vacuo* to give a residue. The residue was diluted with water (10 mL) and the resulting mixture was acidified with 1 N hydrochloride aqueous solution to pH = 3. The mixture was extracted with ethyl acetate (10 mL) for three times. The combined organic phases were washed with brine (10 mL), dried over anhydrous sodium sulfate and filtered. The filtrate was concentrated to give intermediate **I64** (220 mg, 87% yield, 95% purity from ¹H NMR) as a white solid. **¹H NMR** (400 MHz, DMSO-d6) δ 13.40 (s, 1H), 8.18 - 8.17 (m, 1H), 8.10 - 8.08 (m, 1H), 7.76 (d, *J =* 8.4 Hz, 1H), 7.28 (t, *J =* 54.0 Hz, 1H).

### 3.1.1.8. Synthesis of intermediate I69

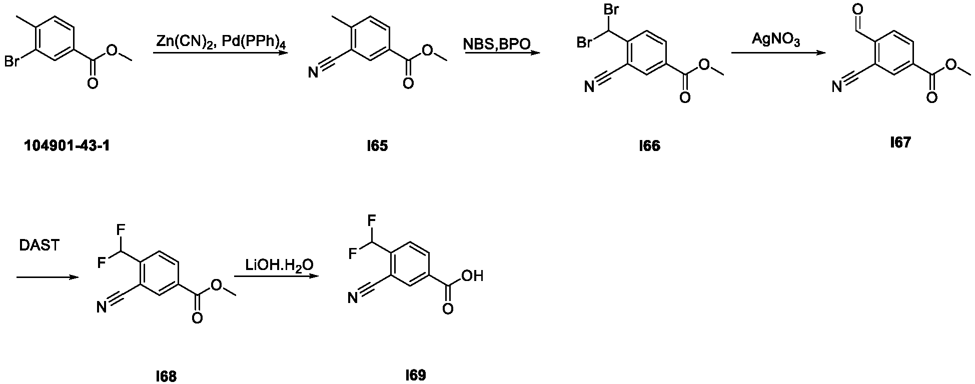

A mixture of methyl 3-bromo-4-methylbenzoate (2.00 g, 8.70 mmol), zinc cyanide (1.64 g, 14.0 mmol) and tetrakis(triphenylphosphine)palladium (1.0 g, 0.87 mmol) in N,N-dimethylformamide (30 mL) was stirred at 100°C under nitrogen for 16 hours. The mixture was filtered and the filtrate was concentrated under reduced pressure. Then the residue was purified by column chromatography on silica gel (petroleum ether: ethyl acetate = 5: 1) to give intermediate **I65** (1.4 g, 92% yield, 90% purity from ¹H NMR) as a white solid. **¹H NMR** (400 MHz, CDCl₃) δ 8.27 (d, *J =* 1.6 Hz, 1H), 8.13 (dd, *J* = 8.0, 1.6 Hz, 1H), 7.41(d, *J =* 7.6 Hz, 1H), 3.94 (s, 3H), 2.61 (s, 3H).

To a solution of intermediate **I65** (500 mg, 2.86 mmol) in perchloromethane (8 mL) was added N-bromosuccinimide (1.27 g, 7.14 mmol) and benzoyl peroxide (75 %, 28 mg, 0.086 mmol). Then the mixture was stirred at 85°C overnight. The mixture was diluted with water (20 mL) and extracted with dichloromethane (20 mL). The organic layer was washed with aqueous sodium bicarbonate (20 mL) and brine (20 mL), dried over Na₂SO₄, concentrated to get the crude intermediate **I66** (900 mg, 95% yield, 80% purity from ¹H NMR) as a yellow solid. **¹H NMR** (400 MHz, CDCl₃) δ 8.32 (dd, *J* = 8.0, 1.6 Hz, 1H), 8.27 (d, *J =* 1.2 Hz, 1H), 8.12 (d, *J =* 8.4 Hz, 1H), 7.00 (s, 1H), 3.98 (s, 3H).

To a solution of intermediate **I66** (500 mg, 1.50 mmol) in acetonitrile (6 mL) was added a solution of silver nitrate (765 mg, 4.50 mmol) in water (3 mL). Then the mixture was stirred at 80°C for 6 hours. The mixture was filtered and the filtrate was diluted with water (10 mL). The mixture was extracted with EtOAc (20 mL). The organic layer were dried over Na₂SO₄, filtered and concentrated. The residue was purified by column chromatography on silica gel (petroleum ether: ethyl acetate = 100: 1 to 5:1) to give intermediate **I67** (130 mg, 46% yield, 90% purity from ¹H NMR) as a white solid. **¹H NMR** (400 MHz, CDCl₃) δ 10.42 (s, 1H), 8.48 (d, *J* = 1.2 Hz, 1H), 8.40 (dd, *J* = 8.4, 0.8 Hz, 1H), 8.13 (d, *J =* 8.0 Hz, 1H), 4.01 (s, 3H).

To a solution of intermediate **I67** (130 mg, 0.69 mmol) in dichloromethane (4 mL) was added (diethylamino)sulfur trifluoride (222 mg, 1.38 mmol) at 0 °C. Then the mixture was stirred at 0°C for 15 min. The reaction mixture was diluted with water (10 mL) and extracted with dichloromethane (10 mL). The organic layer was washed with sat. aqueous sodium bicarbonate (10 mL) and brine (10 mL), dried over Na₂SO₄ and concentrated to get intermediate **I68** (140 mg, 97% yield, 90% purity from ¹HNMR) as yellow oil. **¹H NMR** (400 MHz, CDCl₃) δ 8.42 (s, 1H), 8.37 (d, *J* = 8.0 Hz, 1H), 7.86 (d, *J =* 8.0 Hz, 1H), 6.97 (t, *J =* 54.0 Hz, 1H), 3.99 (s, 3H).

To a solution of intermediate **I68** (140 mg, 0.66 mmol) in tetrahydrofuran/water (2 : 1, 3 mL) was added lithium hydroxide hydrate (34 mg, 0.80 mmol). The mixture was stirred at room temperature for 2 hours. Then the mixture was diluted with water (5 mL) and acidified with 1 N hydrochloric acid to pH = 2. The mixture was extracted with ethyl acetate (10 mL). The organic phase was washed with brine (10 mL), dried over Na₂SO₄ and concentrated to give intermediate **I69** (100 mg, 76.6% yield, 95% purity from ¹H NMR) as a pale yellow solid.

**¹H NMR** (400 MHz, DMSO-*d6*) δ 13.87 (brs, 1H), 8.45 (s, 1H), 8.35 (d, *J =* 8.4 Hz, 1H), 7.96 (d, *J =* 8.0 Hz, 1H), 7.35 (t, *J =* 54.0 Hz, 1H).

### 3.1.1.9. Synthesis of intermediate I71

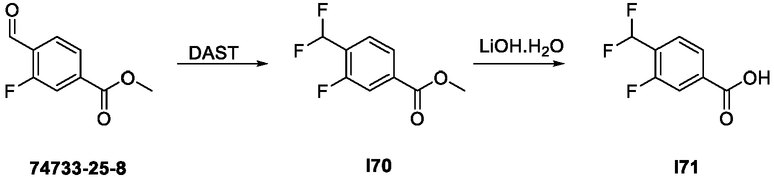

(Diethylamino)sulfur trifluoride (532 mg, 3.30 mmol) was added to an ice-cooled solution of methyl 3-fluoro-4-formylbenzoate (300 mg, 1.65 mmol) in dichloromethane (7 mL). The reaction mixture was stirred at 0 °C for 1 hour. The reaction mixture was quenched with sat. aqueous sodium bicarbonate (7 mL) and extracted with dichloromethane (10 mL). The organic layer was washed with brine (10 mL), dried over Na₂SO₄ and concentrated to get the intermediate **I70** (300 mg, 89% yield, 90% purity from ¹H NMR) as yellow oil. **¹H NMR** (400 MHz, CDCl₃) δ 7.92 (d, *J =* 8.0 Hz, 1H), 7.79 (d, *J =* 10.4 Hz, 1H), 7.68 (t, *J =* 7.6 Hz, 1H), 6.92 (t, *J =* 54.4 Hz, 1H), 3.95 (s, 3H).

To a solution of intermediate **I70** (100 mg, 0.49 mmol) in tetrahydrofuran/water (2 : 1, 3 mL) was added lithium hydroxide hydrate (23 mg, 0.54 mmol). The mixture was stirred at room temperature for 1 hour. Then the mixture was diluted with water (5 mL) and acidified with 0.5 N hydrochloric acid to pH = 4. Then the mixture was extracted with ethyl acetate (10 mL). The organic phase was washed with brine (10 mL), dried over Na₂SO₄ and concentrated to get intermediate **I71** (84 mg, 90% yield, 95% purity from ¹H NMR) as a pale yellow solid.

**¹H NMR** (400 MHz, DMSO-*d6*) δ 13.47 (brs, 1H), 7.90 (d, *J =* 8.0 Hz, 1H), 7.82 - 7.76 (m, 2H), 7.29 (t, *J =* 54.0 Hz, 1H).

### 3.1.1.10. Synthesis of Intermediate I76

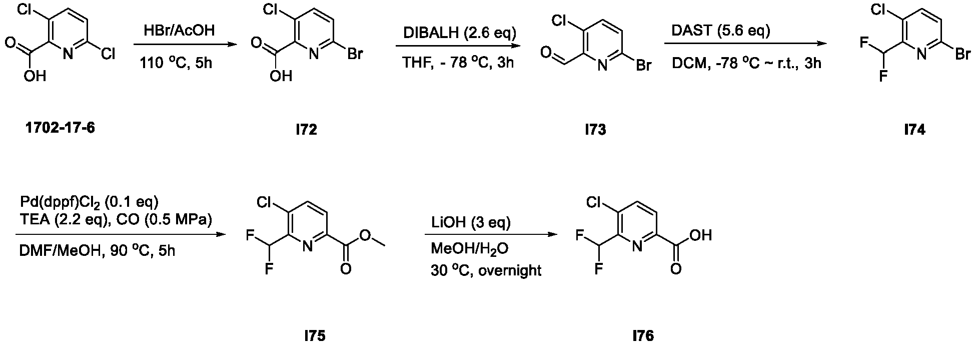

To a solution of 3,6-dichloropicolinic acid (9.5 g, 49.5 mmol) in acetic acid (19 mL) was added 33 % hydrogen bromide in acetic acid (19 mL). The mixture was heated to 110 °C and stirred at 110 °C for 1 hour. After another 33 % Hydrogen bromide in acetic acid (10 mL) was added, the mixture was stirred at 110 °C for another 2 hours. A further 33 % Hydrogen bromide in acetic acid (10 mL) was added, and then the mixture was stirred at 110 °C for a further 2 hours. After cooling down to room temperature, the mixture was poured into ice water (100 mL). The white solid was collected by filtration, washed with water (10 mL) twice and dried under reduced pressure to give intermediate **I72** (6.46 g, 90 % purity from ¹H NMR, 50 % yield). ¹H **NMR** (400 MHz, DMSO-*d6)* δ 14.15 (br s, 1H), 8.04 (d, *J =* 8.4 Hz, 1H), 7.84 (d, *J =* 8.4 Hz, 1H).

To a solution of intermediate **I72** (2 g, 90 % purity, 7.61 mmol) in tetrahydrofuran (40 mL) was slowly added 1.5 M diisobutylaluminum hydride in toluene (13 mL, 19.5 mmol) at - 60 °C under nitrogen atmosphere. After stirring at - 60 °C for 3 hours, the reaction was quenched with saturated ammonium chloride aqueous solution (10 mL), diluted with water (40 mL) and ethyl acetate (40 mL). The organic layer was separated and the aqueous layer was extracted with ethyl acetate (40 mL) twice. The combined organic layers were washed with brine (40 mL), dried over Na₂SO₄(s) and filtered. The filtrate was concentrated and purified by silica gel column chromatography (petroleum ether : ethyl acetate = 20 : 1) to give intermediate **I73** (1.52 g, 90 % purity from ¹H NMR, 82 % yield) as a yellow solid. **¹H NMR** (400 MHz, CDCl₃) δ 10.16 (s, 1H), 7.70 (d, J = 8.4 Hz, 1H), 7.63 (d, J = 8.4 Hz, 1H).

To a solution of intermediate **I73** (1.52 g, 90 % purity, 6.21 mmol) in dichloromethane (40 mL) was added diethylaminosulfur trifluoride (5.6 g, 34.7 mmol) at - 78 °C. After stirring at room temperature for 3 hours, the mixture was poured into cold saturated sodium bicarbonate aqueous solution (100 mL) and extracted with ethyl acetate (50 mL) twice. The combined organic layers were washed with brine (100 mL), dried over Na₂SO₄(s) and filtered. The filtrate was concentrated *in vacuo* to give a residue, which was purified by silica gel column chromatography (petroleum ether : ethyl acetate = 20 : 1) to give intermediate **I74** (1.45 g, 90 % purity from ¹H NMR, 87 % yield) as a yellow solid. **¹H NMR** (400 MHz, CDCl₃) δ 7.65 (d, *J* = 8.4 Hz, 1H), 7.55 (d, *J* = 8.4 Hz, 1H), 6.81 (t, *J =* 53.6 Hz, 1H).

A solution of intermediate **I74** (1.5 g, 90 % purity, 5.57 mmol), [1,1'-Bis(diphenylphosphino)ferrocene]dichloropalladium(II) (546 mg, 0.746 mmol) and triethylamine (1.26 g, 12.5 mmol) in methanol (6 mL) and N,N-dimethylformamide (6 mL) was stirred at 90 °C under carbon monoxide atmosphere (0.5 Mpa) for 5 hours. After cooling down to room temperature, the mixture was concentrated *in vacuo* to give a residue, which was purified by silica gel column chromatography (petroleum ether : ethyl acetate = 10 : 1) to give intermediate **I75** (1.1 g, 90 % purity from ¹H NMR, 80 % yield) as a light yellow solid. **¹H NMR** (400 MHz, CDCl₃) 8.19 (d, *J* = 8.0 Hz, 1H), 7.96 (d, *J =* 8.4 Hz, 1H), 6.91 (t, *J =* 53.6 Hz, 1H), 4.03 (s, 3H).

To a solution of intermediate **I75** (200 mg, 0.85 mmol) in methanol (2 mL) was added 5 M lithium hydroxide aqueous solution (0.5 mL, 2.5 mmol) at room temperature. After stirring at 30 °C overnight, the reaction mixture was diluted with water (10 mL), acidified with 1 M hydrochloride aqueous solution to pH ~ 3 and then extracted with ethyl acetate (5 mL) for three times. The combined organic layers were washed with brine (10 mL), dried over Na₂SO₄(s) and filtered. The filtrate was concentrated *in vacuo* to give intermediate **I76** (180 mg, 95 % purity from ¹H NMR, 96 % yield) as a white solid. **¹H NMR** (400 MHz, CDCl₃) δ 8.31 (d, *J =* 8.4 Hz, 1H), 8.08 (d, *J* = 8.4 Hz, 1H), 6.96 (t, *J =* 53.6 Hz, 1H).

### 3.1.1.11. Synthesis of intermediate I79

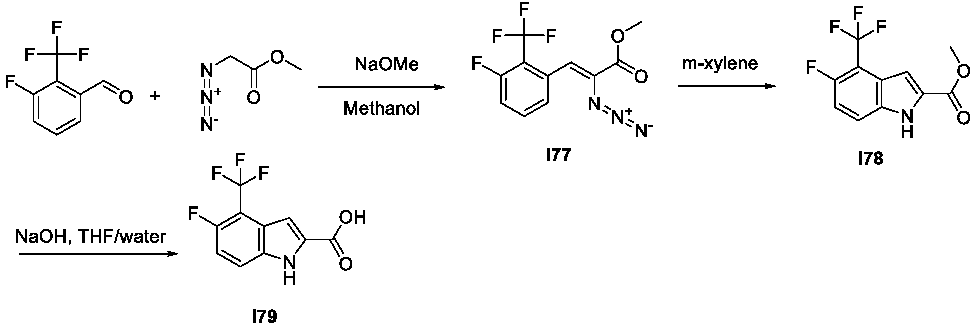

To a solution of sodium methoxide (25% in methanol) (20 mL, 87.5 mmol) in methanol (17 mL) at -10°C was added a solution of 3-fluoro-2-(trifluoromethyl)benzaldehyde (4.20 g, 21.9 mmol) and methyl 2-azidoacetate (8.5 mL, 87.5 mmol) in methanol (4.2 mL) during 1 hour (0.3 mL/min) with syringe driver. The reaction mixture was stirred 5 hours maintaining temperature below 5°C. Ice water was added to the reaction mixture and stirred for 10 minutes allowing the reaction coming to room temperature. The precipitate was filtered, washed with water and dried under vacuum to obtain methyl (Z)-2-azido-3-(3-fluoro-2-(trifluoromethyl)phenyl)acrylate **I77** (1.90 g, yield 30%) as a white solid.

The reaction was performed in 9 batches following for each one the next procedure: The solution of methyl (Z)-2-azido-3-(3-fluoro-2-(trifluoromethyl)phenyl)acrylate **I77** (210 mg, 0.726 mmol) in m-xylene (6 mL) was stirred at 160°C preheating hot plate for 1 hour. The 9 reaction mixtures were combined and co-evaporated with water (3x300 mL).The residue was purified by column (Cyclohexane/EtOAc from 90/10 to 50/50). The obtained was triturated in a mixture of EtOAc and cyclohexane. The precipitate was filtered to afford methyl 5-fluoro-4-(trifluoromethyl)-1H-indole-2-carboxylate **I78** (1.20 g, yield 70%) as a white solid.

To a solution of methyl 5-fluoro-4-(trifluoromethyl)-1H-indole-2-carboxylate **I78** (1.20 g, 4.60 mmol) in THF (10 mL) was added a solution of NaOH (551 mg, 13.8 mmol) in water (5 mL). The reaction mixture was stirred at room temperature for 46 hours. The reaction mixture was diluted with DCM (100 mL) and water (50 mL) and the layers were separated. The aqueous layer was acidified with an aqueous solution of HCl (1N) then extracted with DCM (3x100 mL) and MeOH (some drops). The combined organic layers were dried over sodium sulphate, filtered and concentrated to dryness to obtain 5-fluoro-4-(trifluoromethyl)-1H-indole-2-carboxylic acid **I79** (1.05 g yield 92%) as a white powder.

### 3.1.1.12. Synthesis of intermediate I85

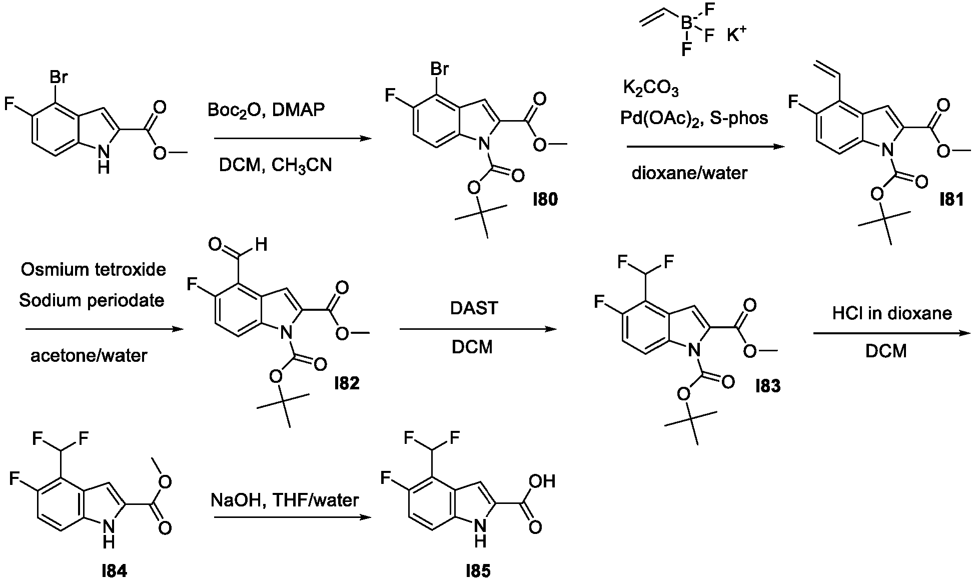

To a solution of methyl 4-bromo-5-fluoro-1H-indole-2-carboxylate (2.27 g, 8.34 mmol) in DCM (32 mL) and acetonitrile (18 mL) were added DMAP (1.33 g, 10.9 mmol) and Di-tert-butyl dicarbonate (2.58 g, 11.8 mmol). The solution was stirred at room temperature for 1 hour. The mixture was diluted with DCM (120 mL) and H₂O (50 mL). The layers were separated and the organic layer was washed with H₂O (50 mL). The organic layer was dried over sodium sulfate, filtered and concentrated in vacuo. The residue was purified by flash chromatography (cyclohexane/EtOAc, from 100/0 to 90/10) to afford 1-(tert-butyl) 2-methyl 4-bromo-5-fluoro-1H-indole-1,2-dicarboxylate **I80** (2.66 g, yield 86%) as a colourless oil.

**I80** (2.66 g, 7.15 mmol) was dissolved in a mixture of 1,4-dioxane (53 mL) and water (10 mL). The mixture was degassed with argon for 20 minutes under vigorous stirring then S-Phos ligand (293 mg, 0.715 mmol), Potassium carbonate (2.96 g, 21.4 mmol) and potassium vinyltrifluoroborate (1.92 g, 14.3 mmol). The flask was purged with vacuum/argon 3 times then palladium acetate (0.080 g, 0.357 mmol) was added. The flask was purged again with vacuum/argon 3 times then left to stir at 75°C for 1 hour. The mixture was diluted with EtOAc (350 mL) then washed with H₂O (2 x 70 mL), brine (2 x 70 mL), dried over sodium sulfate, filtered then concentrated in vacuo to afford 1-(tert-butyl) 2-methyl 5-fluoro-4-vinyl-1H-indole-1,2-dicarboxylate **I81** (2.55 g crude which was used as such in next step).

To a solution **I81** (1.00 g, 3.13 mmol) in acetone (50 mL) and water (10 mL) were added Osmium tetroxide (0.313 g, 0.094 mmol) and sodium periodate (3.35 g, 15.7 mmol). The reaction mixture was stirred at room temperature for 7 hours. The reaction mixture was diluted with water and acetone, the suspension was filtered on a sintered-glass filter and the residue was washed with acetone. The filtrate was concentrated in vacuo to remove most of the acetone. The resulting solution was diluted with EtOAc (200 mL), layers were separated and the aqueous layer was extracted with EtOAc (3 x 50 mL). The combined organic layers were washed with brine (50 mL), dried over sodium sulfate and concentrated in vacuo. The crude was purified by flash chromatography (Cyclohexane/EtOAc from 100/0 to 95/5) to afford 1-(tert-butyl) 2-methyl 5-fluoro-4-formyl-1H-indole-1,2-dicarboxylate **I82** (623 mg, yield 62%) as a white powder.

In a microwave reaction vial, **I82** (0.414 g, 1.29 mmol) was dissolved in DCM (5.8 mL). The solution was cooled down to 0°C then DAST (0.51 mL, 3.87 mmol) was added dropwise. The reaction mixture was allowed to reach room temperature over 15 minutes, the vial was sealed then stirred at 50°C for 2.5 hours and 16 hours at room temperature. The reaction mixture was cooled down to 0°C then quenched by a saturated aqueous solution of NaHCO₃ (5 mL) added slowly, vigorous bubbling appeared. The aqueous layer was extracted with DCM (3 x 15 mL). The combined organic layer was dried over Na₂SO₄, filtered and concentrated in vacuo. The residue was purified by column chromatography (cyclohexane/EtOAc from 100/0 to 95/5) to afford 1-(tert-butyl) 2-methyl 4-(difluoromethyl)-5-fluoro-1H-indole-1,2-dicarboxylate **I83** (394 mg, 84%) as a yellow oil.

To a solution of **I83** (597 mg, 1.74 mmol) in DCM (15 mL) was added HCl (6.52 mL, 1M in dioxane, 26.1 mmol). The reaction was stirred at room temperature for 20 h. LCMS incomplete conversion. The solution was heated to 40°C for 6 h. LCMS still indicated incomplete conversion. The reaction mixture was concentrated in vacuo then diluted in DCM (7 mL) and B (3.00 mL, 12 mmol) was added and the reaction was left to stir at room temperature for 16 hours. The reaction mixture was concentrated in vacuo then the residue was co-evaporated with DCM (2 x 20 mL) to afford methyl 4-(difluoromethyl)-5-fluoro-1H-indole-2-carboxylate **I84** (425 mg, yield 99%) of as a beige powder.

To a solution of **I84** (425 mg, 1.75 mmol) in THF (4 mL) was added a solution of NaOH (210 mg, 5.24 mmol) in water (2 mL). The solution was stirred at room temperature for 18 hours. The mixture was diluted with H₂O (60 mL) then washed with DCM (2 x 30 mL) and EtOAc (30 mL).

The aqueous layer was acidified until pH ~ 1 with an aqueous solution of HCl 1M then extracted with DCM (2 x 100 mL), DCM with few drops of MeOH (50 mL). The combined organic layers were dried over sodium sulfate, filtered and concentrated in vacuo. The residue was dissolved in DCM:MeOH and acidic water; the aqueous layer turned pink after agitation. The layers were separated and the organic layer was washed with water (3 x 20 mL), dried over sodium sulfate, filtered and concentrated in vacuo to afford a first fraction.

The aqueous layer was extracted with DCM/MeOH 9:1 (3 x 100 mL), the combined organic layers were dried over sodium sulfate, filtered and concentrated in vacuo to afford a second fraction. Both fractions were combined and purified by reverse phase flash chromatography (C18_IR_50_F0025, water/ACN from 98/2 to 0/100, 50 min) to afford 4-(difluoromethyl)-5-fluoro-1H-indole-2-carboxylic acid **I85** (239 mg, yield 58%) as a beige powder.

### 3.1.1.12. Synthesis of Intermediate I90

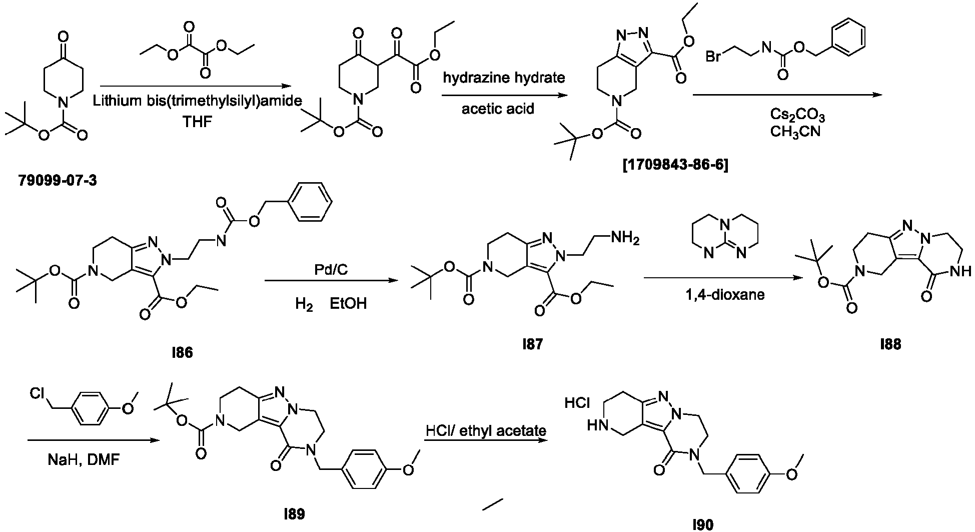

To a solution of tert-butyl 4-oxopiperidine-1-carboxylate (50 g, 98 % purity, 246 mmol) in tetrahydrofuran (500 mL) was added 1.0M Lithium bis(trimethylsilyl)amide solution in tetrahydrofuran (250 mL, 250 mmol) dropwise at - 78 °C. After stirring at - 78 °C for 30 mins, diethyl oxalate (39.5 g, 270 mmol) in tetrahydrofuran (200 mL) was added dropwise at - 78 °C. The mixture was stirred at room temperature overnight. Then the mixture was quenched with water (200 mL) and neutralized with 1M hydrochloride aqueous solution. The mixture was extracted with ethyl acetate (300 mL) twice. The combined organic phases were washed with brine (500 mL), dried over Na₂SO₄(s) and filtered. The filtrate was concentrated under reduced pressure to give tert-butyl 3-(2-ethoxy-2-oxoacetyl)-4-oxopiperidine-1-carboxylate (78.4 g, 90 % purity from ¹H NMR, 96 % yield) as yellow oil. **¹H NMR** (400 MHz, CDCl₃) 15.35 (br s, 1H), 4.45 (s, 2H), 4.39 (q, *J =* 7.2 Hz, 2H), 3.66 (t, *J =* 6.0 Hz, 2H), 2.60 (t, *J =* 6.0 Hz, 2H), 1.47 (s, 9H), 1.41 (t, *J* = 6.8 Hz, 3H).

To a solution of tert-butyl 3-(2-ethoxy-2-oxoacetyl)-4-oxopiperidine-1-carboxylate (78.4 g, 90 % purity, 236 mmol) in acetic acid (100 mL) was added 85 % hydrazine hydrate in water (34 g, 577 mmol) at 0 °C. After stirring at room temperature overnight, the mixture was poured into ice cold saturated sodium bicarbonate aqueous solution (500 mL). The mixture was extracted with ethyl acetate (500 mL) twice. The combined organic phases were washed with brine (1500 mL), dried over Na₂SO₄(s) and filtered. The filtrate was concentrated under reduced pressure to give 5-(tert-butyl) 3-ethyl 1,4,6,7-tetrahydro-5H-pyrazolo[4,3-c]pyridine-3,5-dicarboxylate (74.9 g, 90 % purity from ¹HNMR, 97 % yield) as a yellow solid. **¹H NMR** (400 MHz, DMSO-d6) 13.68 (br s, 0.3H), 13.29 (br s, 0.7H), 4.50 (s, 2H), 4.34 - 4.19 (m, 2H), 3.61 (t, *J =* 5.6 Hz, 2H), 2.68 (s, 2H), 1.42 (s, 9H), 1.31 (t, *J =* 6.4 Hz, 3H).

To a solution of intermediate 5-(tert-butyl) 3-ethyl 1,4,6,7-tetrahydro-5H-pyrazolo[4,3-c]pyridine-3,5-dicarboxylate (500 mg, 1.524 mmol, 90% purity) in acetonitrile (10 mL) was added benzyl (2-bromoethyl)carbamate (415 mg, 1.61 mmol) and cesium carbonate (745 mg, 2.29 mmol). The mixture was stirred at 30 °C overnight. The mixture was filtered and the filtrate was concentrated under reduced pressure to give a residue, which was purified by silica gel column chromatography (petroleum ether : ethyl acetate = 3 : 1) to give intermediate **I86** (320 mg, 42% yield, 95% purity from ¹H NMR) as yellow oil. **¹H NMR** (400 MHz, CDCl₃) δ 7.39 - 7.30 (m, 5H), 5.28 (s, 1H), 5.08 (s, 2H), 4.67 - 4.64 (m, 2H), 4.53 (s, 2H), 4.32 (q, *J =* 7.2 Hz, 2H), 3.75 - 3.56 (m, 4H), 2.73 - 2.71 (m, 2H), 1.49 (s, 9H), 1.37 (t, *J =* 7.2 Hz, 3H).

To a solution of intermediate **I86** (320 mg, 95% purity, 0.643 mmol) in ethanol (5 mL) was added 10% wt. palladium on charcoal (40 mg). The mixture was heated to 50 °C and stirred overnight under hydrogen balloon. After cooling to room temperature, the mixture was filtered and the filtrate was concentrated *in vacuo* to give intermediate **I87** (200 mg, 83% yield, 90 % purity from LCMS) as yellow oil. **LCMS** (ESI): Rₜ = 1.52 min, mass calcd. for C₁₆H₂₆N₄O₄ 338.2, m/z found 339.1 [M+H]⁺.

To a solution of intermediate **I87** (150 mg, 0.399 mmol, 90% purity) in 1,4-dioxane (3 mL) was added 2,3,4,6,7,8-hexahydro-1H-pyrimido[1,2-a]pyrimidine (20 mg, 0.144 mmol). The mixture was stirred at 110 °C for 2 hours. The mixture was concentrated to give a residue, which was purified by prep-TLC (dichloromethane: methanol = 10: 1) to give intermediate **I88** (100 mg, 81% yield, 95% purity from LCMS) as yellow oil. **LCMS** (ESI): Rₜ = 1.39 min, mass calcd. for C₁₄H₂₀N₄O₃ 292.2, m/z found 237.1 [M+H-56]⁺.

To a solution of intermediate **I88** (100 mg, 0.325 mmol, 95% purity) in N,N-dimethylformamide (5 mL) was added sodium hydride (60% in mineral oil, 20 mg, 0.50 mmol). The mixture was stirred at room temperature for 10 minutes. Then 4-methoxybenzylchloride (55 mg, 0.35 mmol) was added. The mixture was stirred at room temperature for 1 hour. The mixture was poured into water (10 mL) and extracted with ethyl acetate (10 mL) for three times. The combined organic phases were washed with brine (10 mL), dried over anhydrous sodium sulfate and filtered. The filtrate was concentrated *in vacuo* to give intermediate **I89** (145 mg, 97% yield, 90% purity from LCMS) as yellow oil. **LCMS** (ESI): Rₜ = 1.62 min, mass calcd. for C₂₂H₂₈N₄O₄ 412.2, m/z found 357.1 [M+H-56]⁺.

To a solution of intermediate **I89** (145 mg, 0.316 mmol, 90% purity) in ethyl acetate (2 mL) was added 4 M HCl/EtOAc (3 mL). The mixture was stirred at room temperature overnight. The mixture was concentrated *in vacuo* to give intermediate **I90** hydrochloride salt (100 mg, 86% yield, 95% purity from LCMS) as a white solid. **LCMS** (ESI): Rₜ = 1.29 min, mass calcd. for C₁₇H₂₀N₄O₂ 312.2, m/z found 313.1 [M+H]⁺.

### 3.1.1.13. Synthesis of intermediates I92*R and I92*S

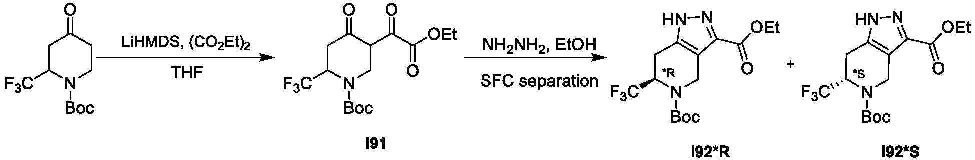

To a solution of tert-butyl 4-oxo-2-(trifluoromethyl)piperidine-1-carboxylate (4.50 g, 16.84 mmol) in THF (50 mL) was added LiHMDS (1 M, 20 mL) dropwise at -70°C. The mixture was stirred at -70°C for 0.5 h. Then diethyl oxalate (3.20 g, 21.89 mmol) was added dropwsise at -70°C. The mixture was stirred at 10°C for 1 h. LCMS showed the desired product formed mainly. The mixture was quenched by 1N HCl (500 mL) and extracted with EtOAc (5x300 mL). The combined organic layer was dried over Na₂SO₄, and filtrated. The filtrate was concentrated in vacuum to give the title compound **I91** (5.50 g, crude) as yellow oil, which was used in next step directly. MS (ESI): mass calcd. For C₁₅H₂₀F₃NO₆, 367.1; m/z found, 368.1 [M+H]⁺.

To a solution of tert-butyl 5-(2-ethoxy-2-oxoacetyl)-4-oxo-2-(trifluoromethyl)piperidine-1-carbo xylate **I91** (5.50 g, 14.97 mmol) in EtOH (30 mL) was added NH₂NH₂•H₂O (882 mg, 14.97 mm ol, 85% purity). The mixture was stirred at 10 °C for 1 h. LCMS showed the starting material w as consumed and mainly desired product was detected. The reaction mixture was concentrated i n vacuum. The residue was purified by flash chromatography (petroleum ether/EtOAc 80/20 to 60/40) to give the title racemate (4.05 g, 11.15 mmol) as yellow solid, which was resolved by SF C to afford two single entiomers: (R*)-5-tert-butyl 3-ethyl 6-(trifluoromethyl)-6,7-dihydro-1H-p yrazolo[4,3-c]pyridine-3,5(4H)-dicarboxylate **I92*R** (1.40 g, Peak 1 on SFC (OD-3S_5_5_40_3 ML_T35.M; Column: Chiralcel OD-3, 100×4.6 mm I.D., particle size 3 um; Mobile phase: 40% EtOH (0.05% DEA) in CO₂; Flow rate: 3mL/min; Wavelength: 220 nm), retention time = 1.002 min) and (S*)-5-tert-butyl 3-ethyl 6-(trifluoromethyl)-6,7-dihydro-1H-pyrazolo[4,3-c]pyridine-3, 5(4H)-dicarboxylate (1.80 g, Peak 2 on SFC (OD-3S_5_5_40_3ML_T35.M; Column: Chiralcel OD-3, 100×4.6 mm I.D., particle size 3 um; Mobile phase: 40% EtOH (0.05% DEA) in CO₂; Flo w rate: 3mL/min; Wavelength: 220 nm), retention time = 1.163 min) as yellow solid.

### 3.1.1.14. Synthesis of intermediate I99

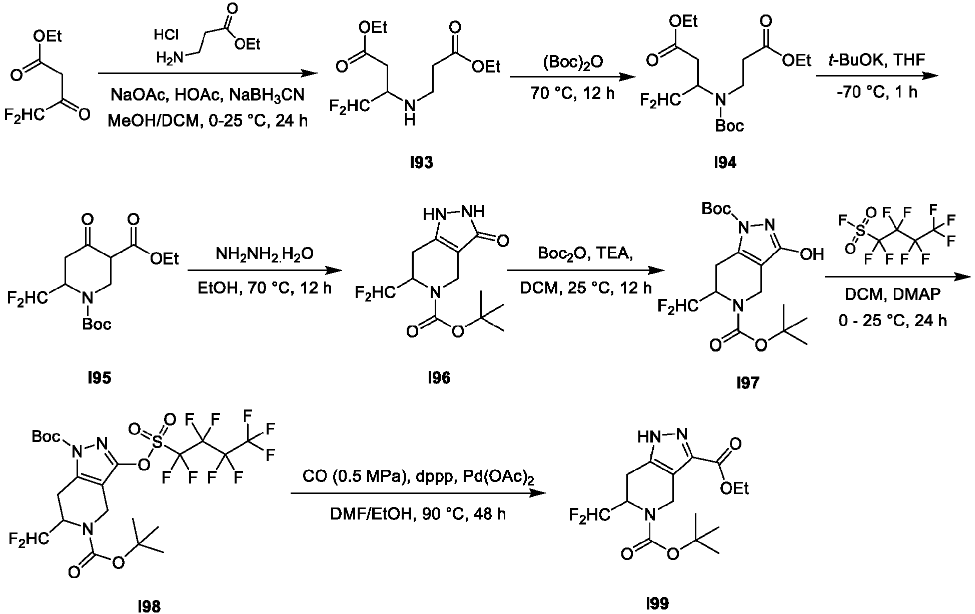

Two batches were carried out in parallel. To the solution of compound ethyl 3-aminopropanoate hydrogen chloride (332 g, 2.17 mol, 2.00 eq, HCl) in MeOH (1.50 L) was added NaOAc (177 g, 2.17 mol, 2.00 eq). The mixture was stirred at 25 °C for 1 h. Then ethyl 4,4-difluoro-3-oxobutanoate (180 g, 1.08 mol, 1.00 eq) in DCM (2.00 L) was added, followed by HOAc (130 g, 2.17 mol, 123 mL, 2.00 eq). Then mixture was stirred at 25 °C for 12 h. TLC (Petroleum ether/Ethyl acetate = 5/1) showed the reactant (R_{f} = 0.30) was consumed and new spots (R_{f} = 0.50, 0.60) formed. Then a solution of NaBH₃CN (102 g, 1.63 mol, 1.50 eq) in MeOH (1.00 L) was added in portions at 0 °C. The mixture was stirred at 20 °C for 12 h. TLC (Petroleum ether/Ethyl acetate = 5/1) showed starting material (R_{f} = 0.50, 0.60) was consumed and new spots (R_{f} = 0.40, 0.10) formed. Two batches were combined for further work-up. The mixture was quenched by saturated NaHCO₃ solution (2.00 L). The organics were separated and dried over Na₂SO₄ and concentrated. The crude was purified by SiO₂ column chromatography (Petroleum ether/Ethyl acetate = 0/1 - 10/1). **I93** (420 g, 72.5% yield) was obtained as light-yellow oil. **¹H NMR** (400 MHz, CDCl₃) δ 6.04 - 5.59 (m, 1H), 4.28 - 4.03 (m, 4H), 3.28 (tdd, *J* = 3.1, 7.9, 11.2 Hz, 1H), 2.97 (dt, *J =* 3.8, 6.3 Hz, 2H), 2.61 (dd, *J =* 4.8, 16.1 Hz, 1H), 2.52 - 2.38 (m, 3H), 1.34 - 1.17 (m, 6H)

The solution of compound **I93** (420 g, 1.57 mol, 1.00 eq) and Boc₂O (342 g, 1.57 mol, 1.00 eq) was stirred at 70 °C for 12 h. TLC (Petroleum ether/Ethyl acetate = 5/1) showed **I93** (R_{f} = 0.50) was consumed and new spots (R_{f} = 0.70, 0.90) formed. The mixture was purified by SiO₂ column chromatography (Petroleum ether/Ethyl acetate = 1/0 - 20/1). **I94** (450 g, 77.9% yield) was obtained as light-yellow oil. **¹H NMR** (400 MHz, CDCl₃) δ 6.40 - 5.68 (m, 1H), 4.38 - 4.06 (m, 5H), 3.74 - 3.36 (m, 2H), 3.09 - 2.78 (m, 1H), 2.76 - 2.48 (m, 3H), 1.47 (br s, 9H), 1.27 (dt, *J* = 4.0, 7.0 Hz, 6H)

Nine batches were carried out in parallel. To the solution of compound **I94** (50.0 g, 126 mmol, 1.00 eq) in THF (1.50 L) was added t-BuOK (1 M, 177 mL, 1.40 eq) at -70 °C in one portion. The mixture was stirred at -70 °C for 1 h. TLC (Petroleum ether/Ethyl acetate = 5/1) showed compound **3** (R_{f} = 0.50) was consumed and new spot (R_{f} = 0.60) formed. Nine batches were worked up in parallel. The mixture was poured into the 1 N HCl (500 mL) at 0 °C. The mixture was extracted with MBTE (800 mL x 2). The organics were washed with brine (500 mL) and dried over Na₂SO₄ and concentrated. Compound **I95** combined (400 g, crude 100%) was combined for next step without for further purification as light yellow oil. **¹H NMR** (400 MHz, CDCl₃) δ 11.99 (s, 1H), 5.87 - 5.47 (m, 1H), 4.75 - 4.42 (m, 1H), 4.22 - 4.07 (m, 3H), 3.69 (br s, 2H), 2.68 - 2.45 (m, 2H), 1.45 - 1.41 (m, 9H), 1.28 - 1.23 (m, 3H).

Two batches were carried out in parallel. The mixture of compound **I95** (200 g, 572 mmol, 1.00 eq), TEA (86.9 g, 858 mmol, 119 mL, 1.50 eq) and NH₂NH₂.H₂O (241 g, 4.10 mol, 234 mL, 85.0% purity, 1.50 eq) in EtOH (2.00 L) was stirred at 70 °C for 12 h. TLC (Petroleum ether/Ethyl acetate = 3/1) showed **I95** (R_{f} = 0.80) was consumed and new spot (R_{f} = 0.01) formed. Two batches were combined for work up. The reaction was concentrated to afford **I96** (330 g, crude 100%) as a white solid which was used for next step directly.

**I96** (144 g, 497 mmol, 1.00 eq), TEA (75.6 g, 746 mmol, 103 mL, 1.50 eq) and Boc₂O (130 g, 597 mmol, 137 mL, 1.20 eq) in DCM (2.00 L) was stirred at 25 °C for 12 h. TLC (Petroleum ether/Ethyl acetate = 1/1) showed compound **I96** (R_{f} = 0.01) was consumed and new spot (R_{f} = 0.40) formed. HPLC (EW15208-75-P1A) showed the reaction was complete. The mixture was concentrated to about 150 mL and the white solid was precipitated. MBTE (150 mL) was added. The mixture was stirred at 20 °C for 1 h. The white solid was filtrated to give compound **I97** (120 g, 60.6% yield) as a white solid. LCMS (EW15208-75-P1G3, R.T = 15.253 min) showed the regioisomer was separated completely. **¹H NMR** (400 MHz, CDCl₃) δ 11.17 (br s, 1H), 6.46 - 5.89 (m, 1H), 5.03 - 4.62 (m, 1H), 4.50 (br d, *J =* 15.9 Hz, 1H), 3.95 - 3.62 (m, 1H), 3.23 - 2.99 (m, 2H), 1.53 (s, 9H), 1.44 (s, 9H)

To the mixture of compound 1,1,2,2,3,3,4,4,4-nonafluorobutane-1-sulfonyl fluoride (139 g, 462 mmol, 81.9 mL, 1.50 eq) and compound **I97** (120 g, 308 mmol, 1.00 eq) and DMAP (1.88 g, 15.41 mmol, 0.05 eq) in DCM (1.00 L) was added DIPEA (119 g, 924 mmol, 161 mL, 3.00 eq) at 0 °C. The mixture was stirred at 25 °C for 24 h. TLC (Petroleum ether/Ethyl acetate = 3/1) showed compound **I97** (R_{f} = 0.20) was consumed almost and new spots (R_{f} = 0.60, 0.10) formed. The mixture was washed with water (500 mL) and concentrated. The residue was purified by SiO₂ column chromatography (Petroleum ether/Ethyl acetate = 50/1 - 10/1). Compound **I98** (105 g, 50.2% yield) was obtained as a colorless oil.

An autoclave vessel was charged with compound **I98** (105 g, 156 mmol, 1.00 eq), DPPP (6.45 g, 15.6 mmol, 0.10 eq), Pd(OAc)₂ (3.51 g, 15.64 mmol, 0.10 eq) and TEA (31.6 g, 312 mmol, 43.5 mL, 2.00 eq) in EtOH (1.20 L) and DMF (1.20 L). The mixture was then purged with nitrogen three times and carbon monoxide three times. The mixture was stirred to 90 °C under CO (0.5 MPa) for 48 h. TLC (Petroleum ether/Ethyl acetate = 1/1) **I98** (R_{f} = 0.80) was consumed and new spots (R_{f} = 0.30, 0.0) formed. The filtrate was concentrated. The residue was purified by SiO₂ chromatography (Petroleum ether/Ethyl acetate = 5/1 - 1/1) to give **I99** (26.0 g, 34.5 % yield) as a yellow solid. **¹H NMR** (400 MHz, CDCl₃) δ 12.50 - 11.55 (m, 1H), 5.94 - 5.47 (m, 1H), 5.28 - 4.72 (m, 2H), 4.51 - 4.15 (m, 3H), 3.22 - 2.99 (m, 2H), 1.52 (s, 9H), 1.45 - 1.36 (m, 3H).

### 3.1.1.15. Synthesis of intermediate I101

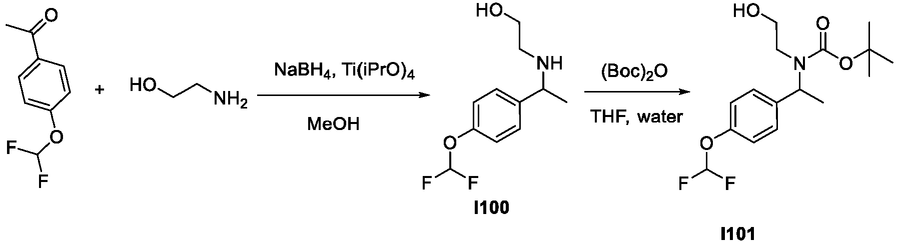

4-(difluoromethoxy)acetophenone (5 g, 26.86 mmol) and 3-5] and 2-aminoethan-1-ol (4.8 mL, 80.58 mmol) were dissolved in dry MeOH (62.5 mL) under nitrogen atmosphere. Ti(iPrO)₄ (10.2 mL, 34.92 mmol) was added and the mixture was stirred at 65°C for 16h. The mixture was cooled to RT and further cooled with an ice bath. Sodium borohydride (1.02 g, 26.86 mmol) was added in portions and the mixture was stirred at rt for 1h. The reaction was quenched by adding water and stirring was continued at rt for 20 min. The mixture was acidified with 1N HCl. The mixture was filtered over a pad of celite and washed with water and EtOAc. The filtrate was concentrated in vacuo. The residue was dissolved in water and neutralized with NaHCO₃. The mixture was extracted with Me-THF, dried over MgSO₄, filtered and concentrated in vacuo to afford 2-((1-(4-(difluoromethoxy)phenyl)ethyl)amino)ethan-1-ol **I100** (6.1 g, yield 98.214%) as an oil.

To a solution of 2-((1-(4-(difluoromethoxy)phenyl)ethyl)amino)ethan-1-ol (6.1 g, 26.38 mmol) and NaHCO₃ (8.8 g, 105.52 mmol) in THF (60 mL) and water (75 mL) was added BOC-anhydride (15.8 mL, 1M in THF, 31.65 mmol). The mixture was stirred at rt for 16 hours. THF was removed. The mixture was extracted with Me-THF twice. The combined organic layers were dried over MgSO₄, filtered and concentrated. The residue was purified by column chromatography (heptane/EtOAc from 100/5 to 40/60) to afford **I101** (6 g, yield 68%).

### 3.1.1.16. Synthesis of intermediate I103

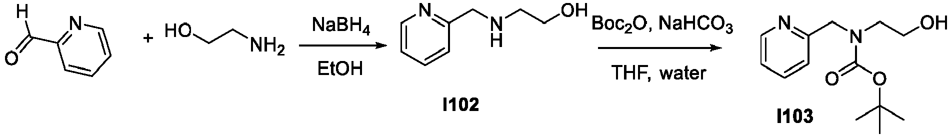

To a solution of 2-pyridinecarboxaldehyde (9 mL, 94.611mmol) in EtOH (150 mL) was added ethanolamine (5.7 mL, 94.61 mmol) and the reaction mixture was stirred at rt for 16 h. Sodium borohydride (3.6 g, 96.50 mmol) was then added by portion at 0°C and the reaction mixture was stirred at rt for 1.5 h. The reaction was quenched by a saturated solution of NaHCO₃ (100 mL) and stirred at rt for 30 min. The formed white solid was filtered off and the filtrate was evaporated to get crude 2-((pyridin-2-ylmethyl)amino)ethan-1-ol **I102** (18.31 g) as an orange oil, which was used as such in the next step.

Crude 2-((pyridin-2-ylmethyl)amino)ethan-1-ol **I102** (1.44 g, 9.46 mmol) was dissolved in THF (20 mL) and water (26 mL). Then NaHCO₃ (3.18 g, 37.84 mmol) was added followed by di-tert-butyl dicarbonate (5.7 mL, 2 M, 11.35 mmol). The reaction mixture was stirred at rt for 2 h. The formed solids/salts were filtered off and THF was separated. The water layer was extracted once more with EtOAc and the organic layers were combined and evaporated under reduced pressure. The crude was purified by column chromatography (heptane/EtOAc from 100/0 to 0/100) to afford intermediate **I103** (21.7 g, yield 85%) as a yellow oil.

### 3.1.1.17. Synthesis of intermediate I106

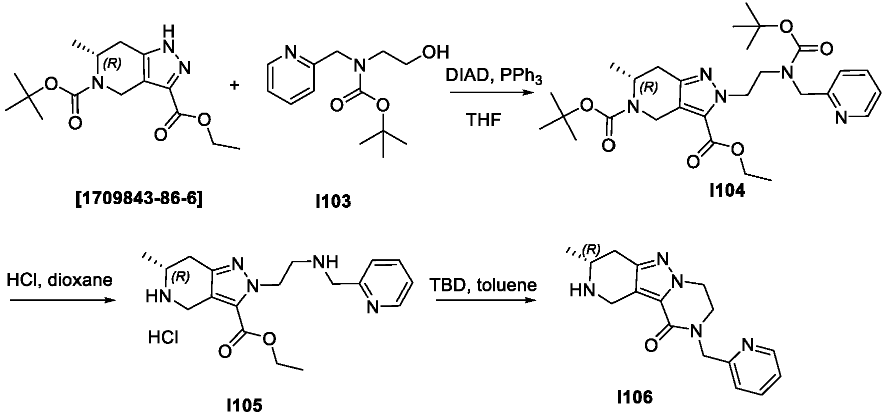

To a solution of **I103** (21.2 g, 28 mmol) in dry THF (510 mL) was added tert-butyl (1-(4-(difluoromethoxy)phenyl)ethyl)(2-hydroxyethyl)carbamate (26 g, 28 mmol) and triphenylphosphine (46.4 g, 168 mmol). The reaction mixture was sealed in a N₂-atm. Then DIAD (32.7 mL, 168 mmol) was added and the mixture was stirred at rt for 16 h. The solvents were evaporated and the crude was redissolved in DIPE and sat. NaHCO₃ was added. The mixture was stirred for 15 min. The formed solid was filtered off. The organic layer was separated and evaporated under reduced pressure. The brown oil was purified via column chromatography (heptane/ (EtOH:EtOAc 10/30) from 100/0 to 0/100). The residue was triturated in DIPE. The suspension was stirred at rt overnight. The solid was filtered off and the filtrate was evaporated to get an orange oil, 5-(tert-butyl) 3-ethyl (R)-2-(2-((tert-butoxycarbonyl)(pyridin-2-ylmethyl)amino)ethyl)-6-methyl-2,4,6,7-tetrahydro-5H-pyrazolo[4,3-c]pyridine-3,5-dicarboxylate **I104** (75 g, yield 71%) which was used as such in the next step.

A flask was charged with 5-(tert-butyl) 3-ethyl (R)-2-(2-((tert-butoxycarbonyl)(pyridin-2-ylmethyl)amino)ethyl)-6-methyl-2,4,6,7-tetrahydro-5H-pyrazolo[4,3-c]pyridine-3,5-dicarboxylate **I104** (20 g, 15.2 mmol) in dry 1,4-dioxane (94 mL). HCl (40 mL, 4M in dioxane, 158 mmol) was added and the mixture was stirred at rt for 3 days. The mixture was concentrated in vacuo. The product was co-evaporated with diethyl ether and the obtained yellowish foam was triturated in diethyl ether. The formed yellowish solid was filtered off and dried in vacuo at 45°C overnight to afford ethyl (R)-6-methyl-2-(2-((pyridin-2-ylmethyl)amino)ethyl)-4,5,6,7-tetrahydro-2H-pyrazolo[4,3-c]pyridine-3-carboxylate HCl **I105** (10.4 g, 52% pure, yield 82%). The filtrate was evaporated and the residue was triturated again in ether. The precipitate was filtered off to get a second fraction of **I105** (beige solid), (1.5 g, 52% pure, yield 11%), which was dried in vacuo overnight at 45°C. The crude fractions were used as such in the next step.

A pressure tube was charged with **I105** (10.4 g, 13 mmol) in toluene (24 mL). 1,5,7-Triazabicyclo[4.4.0]ec-5-ene (TBD) (7.22 g, 51.8 mmol) was added and the vial was closed in a N₂-atm. The mixture was stirred at 100°C for 2 h. The mixture was cooled and concentrated in vacuo. The product was purified on column chromatography (DCM/[MeOH/NH₃ 7M] from 100/0 to 90/10) to afford (R)-3-methyl-9-(pyridin-2-ylmethyl)-1,2,3,4,8,9-hexahydropyrido[4',3':3,4]pyrazolo[1,5-a]pyrazin-10(7H)-one **I106** (5 g, yield quantitative) as a yellow oil, which solidifies after standing at RT.

### 3.1.1.18. Synthesis of intermediate I109

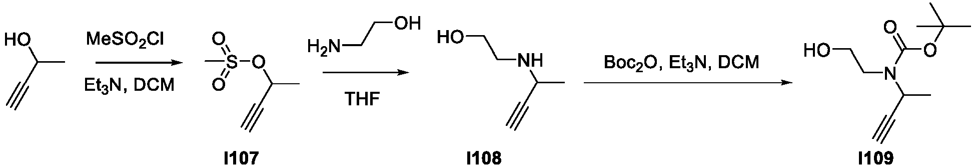

MsCl (99.8 g, 871 mmol) was added dropwise to a solution of 3-butyn-2-ol (50.4 g, 719 mmol), triethylamine (108 g, 1075 mmol), DCM (504 mL) during 30 min. in an ice bath. The reaction mixture was stirred 2 hours at room temperature before being washed with water (500mL). The organic layer was dried over sodium sulphate, filtered and concentrated yielding to 2-(but-3-yn-2-ylamino)ethan-1-ol **I107** as a pale oil used as such in the next step.

**I107** (112 g, 753 mmol), 2-aminoethan-1-ol (115 g, 1882 mmol) in THF (750 mL) was refluxed over weekend. The reaction mixture was concentrated in vacuo. and sat. aq NaHCO₃ (300mL) was added and the mixture extracted with Et₂O (3 x 300 mL). The combined organic phases were dried over Na₂SO₄ and evaporated to dryness. The residue was purified by column chromatography (heptane/ EtOAc from 90/10 to 0/100) to afford 2-(but-3-yn-2-ylamino)ethan-**1-ol I108** (37g, yield 43%) as a clear oil.

Et₃N (59 mL, 426 mmol) was added to a solution of 2-(but-3-yn-2-ylamino)ethan-1-ol **I108** (37 g, 328 mmol) and di-tert-butyl dicarbonate (86 g, 393 mmol) in DCM (500 mL) and stirred overnight. The reaction mixture was concentrated. The residue was purified by column chromatography (DCM/MeOH from 100/0 to 80/20) to afford tert-butyl but-3-yn-2-yl(2-hydroxyethyl)carbamate **I109** (53 g, 75%).

### 3.1.1.18. Synthesis of Intermediate I111

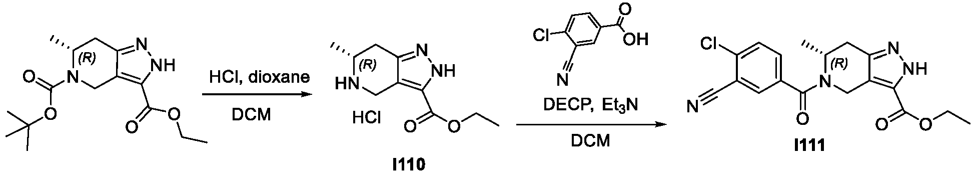

To a stirred solution of 5-(tert-butyl) 3-ethyl (R)-6-methyl-2,4,6,7-tetrahydro-5H-pyrazolo[4,3-c]pyridine-3,5-dicarboxylate (20 g, 64.7 mmol) in DCM (200 mL) was added HCl (81 mL, 4M in dioxane, 323 mmol). The reaction mixture was then stirred at rt overnight. After one night, the reaction mixture was evaporated on the residue was co-evaporated twice with diethyl ether. The residue was triturated in diethylether (200 mL) and the precipitate was filtered and washed with diethylether(2 X 50 mL) and dried in vacuum oven for 3 h to afford ethyl (R)-6-methyl-4,5,6,7-tetrahydro-2H-pyrazolo[4,3-c]pyridine-3-carboxylate **I110** (18 g, yield quantitative) as yellow powder which was used as such in next step.

A tube was charged with (R)-6-methyl-4,5,6,7-tetrahydro-2H-pyrazolo[4,3-c]pyridine-3-carboxylate **I110** (1 g, 3.95 mmol) and Et₃N (1.6 mL, 11.8 mmol) in DCM (20 mL). 4-Chloro-3-cyanobenzoic acid (788 mg, 4.34 mmol) was added and the vial was capped. Diethyl cyanophosphonate (0.85 mL, 5.13 mmol) was added dropwise and the mixture was stirred at rt for 16 h. 10 mL NaHCO₃ half sat. aq. sol. were added. After stirring vigorously for 5 minutes, the layers were separated. The organic layer was dried over MgSO₄, filtered and concentrated in vacuo. The residue was purified by column chromatography (heptane/EtOAc from 100/0 to 0/100) to afford ethyl (R)-5-(4-chloro-3-cyanobenzoyl)-6-methyl-4,5,6,7-tetrahydro-2H-pyrazolo[4,3-c]pyridine-3-carboxylate **I111** (836 mg, yield 57%) as a foam.

### 3.1.2. Synthesis of Compounds

### 3.1.2.1. Synthesis of Compound 1

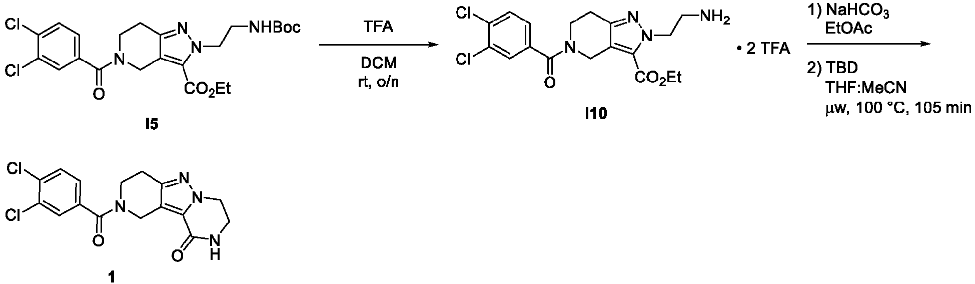

### Intermediate I10

### Ethyl 2-(2-aminoethyl)-5-(3,4-dichlorobenzoyl)-4,5,6,7-tetrahydro-2H-pyrazolo[4,3-c]pyridine-3-carboxylate • 2TFA

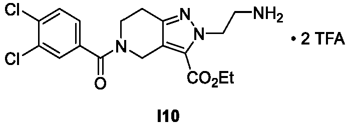

TFA (4.49 mL, 58.7 mmol) was added to a solution of intermediate **I5** (3.00 g, 5.87 mmol) in DCM (100 mL). The reaction mixture was stirred at room temperature overnight and concentrated under reduced pressure to afford intermediate **I10** which was used as such in the next step.

### Compound 1

### 2-(3,4-Dichlorobenzoyl)-1,2,3,4,8,9-hexahydropyrido[4',3':3,4]pyrazolo[1,5-a]pyrazin-10(7H)-one

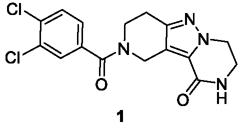

Intermediate **I10** was dissolved in EtOAc (150 mL). The solution was washed NaHCO₃ (sat., aq., 150 mL), dried (MgSO₄), filtered and concentrated under reduced pressure. The residue was diluted in THF (5 mL) and MeCN (1 mL) and TBD (245 mg, 1.76 mmol) was added. The reaction mixture was heated at 100 °C for 105 min in a Biotage microwave. The reaction mixture was cooled to room temperature and a precipitate was formed during the night. The white crystals were filtered off, washed with MeCN and dried at 50 °C under vacuum to afford compound **1** (872 mg, 41% over 2 steps).

**¹H NMR** (400 MHz, DMSO-*d*₆, 101°C) δ ppm 7.78 (br s, 1H), 7.67 (d, *J*=8.1 Hz, 1H), 7.65 (d, *J*=2.0 Hz, 1H), 7.40 (dd, *J*=8.3, 1.9 Hz, 1H), 4.66 (s, 2H), 4.17 - 4.23 (m, 2H), 3.64 - 3.82 (m, 2H), 3.55 - 3.62 (m, 2H), 2.74 (t, *J*=5.9 Hz, 2H); **LCMS** (method B): Rt = 1.53 min, *m*/*z* calcd. for C₁₆H₁₄Cl₂N₄O₂ 364.0, found 365 [M+H]⁺.

### 3.1.2.2. Synthesis of Compound 2

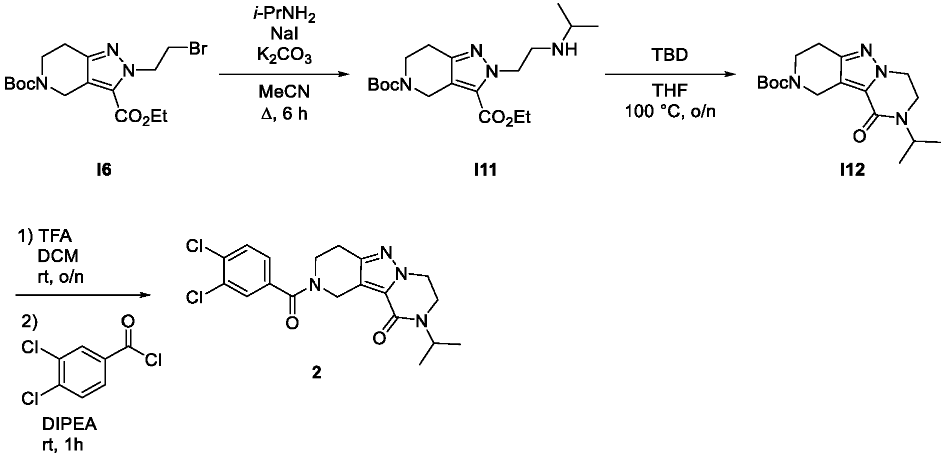

### Intermediate I11

### 5-tert-Butyl 3-ethyl 2-{2-[(propan-2-yl)amino]ethyl}-2,4,6,7-tetrahydro-5H-pyrazolo[4,3-c]pyridine-3,5-dicarboxylate

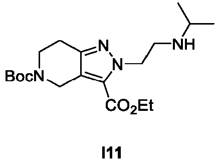

A mixture of intermediate **I6** (0.70 g, 1.74 mmol), isopropylamine (1.03 g, 17.4 mmol), K₂CO₃ (481 mg, 3.48 mmol) and NaI (26.1 mg, 0.17 mmol) in MeCN (115 mL) was stirred under reflux for 6 h. The reaction mixture was filtered over decalite and the filtrate was concentrated under reduced pressure. The residue was purified by column chromatography (silica, heptane/(EtOAc/(EtOH/NH₃ aq. 7M in MeOH), 75/25/2), gradient from 30:70 to 0:100) to afford intermediate **I11** (105 mg, 16%) as a brown oil.

### Intermediate I12

### tert-Butyl 10-oxo-9-(propan-2-yl)-3,4,7,8,9,10-hexahydropyrido[4',3':3,4]pyrazolo[1,5a]-pyrazine-2(1H)-carboxylate

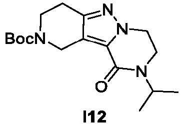

A mixture of intermediate **I11** (105 mg, 0.276 mmol) and TBD (11.5 mg, 82.8 µmol) in THF (2 mL) was stirred at 100 °C overnight. The reaction mixture was concentrated under reduced pressure and the residue was purified by flash column chromatography (silica, heptane/EtOAc, gradient from 80:20 to 0:100) to afford intermediate **I12** (82 mg, 89%).

### Compound 2

### 2-(3,4-Dichlorobenzoyl)-9-(propan-2-yl)-1,2,3,4,8,9-hexahydropyrido[4',3':3,4]pyrazolo[1,5-a]-pyrazin-10(7H)-one

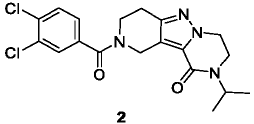

TFA (0.19 mL, 2.45 mmol) was added to a solution of intermediate **I12** (82 mg, 0.24 mmol) in DCM (30 mL) and the reaction mixture was stirred overnight. The mixture was concentrated under reduced pressure. The residue was dissolved in DCM (30mL). DIPEA (0.169 mL, 0.98 mmol) was added followed by 3,4-dichlorobenzoyl chloride (51.4 mg, 0.245 mmol). The reaction mixture was stirred at room temperature for 1 h and the mixture was subjected to a flash column chromatography (silica, heptane/(EtOAc/EtOH, 3:1), gradient from 70:30 to 0:100) to afford compound **2** (82 mg, 82%).

**¹H NMR** (400 MHz, DMSO-*d*₆, 101°C) δ ppm 7.67 (d, *J*=8.1 Hz, 1H), 7.65 (d, *J*=2.0 Hz, 1H), 7.40 (dd, *J*=8.4, 2.0 Hz, 1H), 4.61 - 4.72 (m, 3H), 4.21 - 4.27 (m, 2H), 3.67 - 3.77 (m, 2H), 3.61 - 3.66 (m, 2H), 2.73 (t, *J*=5.9 Hz, 2H), 1.15 (d, *J*=6.8 Hz, 6H); **LCMS** (method D): Rt = 1.85 min, *m*/*z* calcd. for C₁₉H₂₀Cl₂N₄O₂ 406.1, found 407.2 [M+H]⁺; **m.p** 190 °C.

### 3.1.2.3. Synthesis of Compound 3

### 9-Benzyl-2-(3,4-dichlorobenzoyl)-1,2,3,4,8,9-hexahydropyrido[4',3':3,4]pyrazolo[1,5-a]pyrazin-10(7H)-one

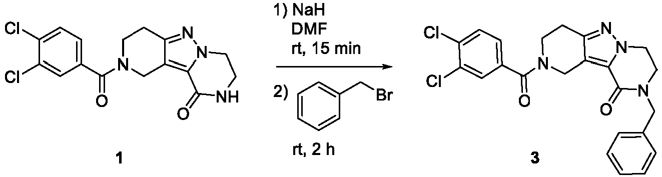

To a solution of compound **1** (125 mg, 0.34 mmol) in anhydrous DMF (4.1 mL) was added NaH (60% dispersion in mineral oil, 20.5 mg, 0.51 mmol) at room temperature. The reaction mixture was stirred for 15 min and benzylbromide (44.8 µL, 0.38 mmol) was added. The reaction mixture was stirred at room temperature for another 2 h. The reaction was quenched with water and diluted with MeOH. The solvents were evaporated under reduced pressure. The crude mixture was purified by flash column chromatography (silica, DCM/MeOH, gradient from 100:0 to 90:10) to afford compound **3** (145 mg, 93%) as a glassy white solid.

**¹H NMR** (400 MHz, DMSO-*d*₆, 81°C) δ ppm 7.67 - 7.71 (m, 2H), 7.43 (dd, *J*=8.1, 2.0 Hz, 1H), 7.24 - 7.37 (m, 5H), 4.71 (br s, 2H), 4.64 (s, 2H), 4.25 - 4.29 (m, 2H), 3.63 - 3.82 (m, 4H), 2.74 (t, *J*=5.8 Hz, 2H) **LCMS** (method C): Rt = 1.05 min, *m*/*z* calcd. for C₂₃H₂₀Cl₂N₄O₂ 454.0, found 455.3 [M+H]⁺.

### 3.1.2.4. Synthesis of Compound 4

### 2-(3,4-Dichlorobenzoyl)-9-[(4-methoxyphenyl)methyl]-1,2,3,4,8,9-hexahydropyrido[4',3':3,4]-pyrazolo[1,5-a]pyrazin-10(7H)-one

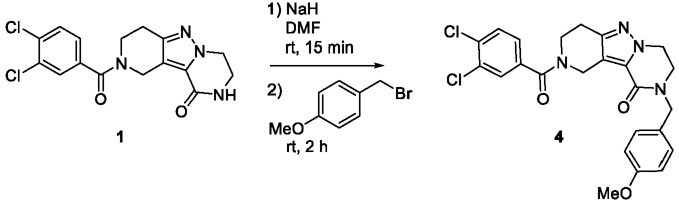

Compound **4** was obtained according to the procedure reported for the synthesis of compound **3.** The crude mixture was purified by flash column chromatography (silica, heptane/(EtOAc/EtOH, 3:1), gradient from 80:20 to 0:100) to afford compound **4** (153 mg, 95%).

**¹H NMR** (400 MHz, DMSO-*d*₆, 101°C) δ ppm 7.68 (d, *J*=8.4 Hz, 1H), 7.66 (d, *J*=2.0 Hz, 1H), 7.41 (dd, *J*=8.1, 2.0 Hz, 1H), 7.20 - 7.27 (m, 2H), 6.85 - 6.93 (m, 2H), 4.70 (s, 2H), 4.57 (s, 2H), 4.21 - 4.27 (m, 2H), 3.74 (s, 3 H), 3.69 - 3.73 (m, 2H), 3.61 - 3.67 (m, 2H), 2.74 (t, *J*=5.8 Hz, 2H); **LCMS** (method B): Rt = 1.98 min, *m*/*z* calcd. for C₂₄H₂₂Cl₂N₄O₃ 484.0, found 485.1 [M+H]⁺.

### 3.1.2.5. Synthesis of Compound 5

### 2-(3,4-Dichlorobenzoyl)-9-[1-(pyridin-4-yl)ethyl]-1,2,3,4,8,9-hexahydropyrido[4',3':3,4-]pyrazolo[1,5-a]pyrazin-10(7H)-one

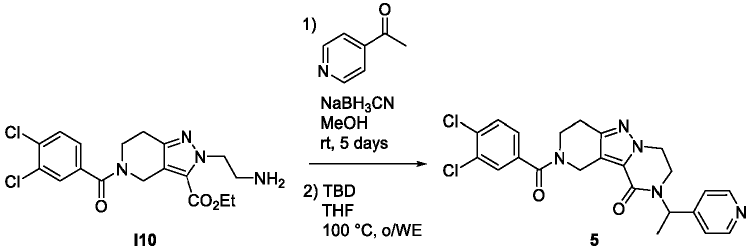

Intermediate **I10** (1.06 g, 1.66 mmol) and 4-acetylpyridine (605 mg, 4.99 mmol) were dissolved in MeOH (20 mL). The mixture was stirred at room temperature and sodium cyanoborohydride (1.10 g, 16.6 mmol) was added portionwise over 5 days twice a day. The reaction mixture was concentrated under reduced pressure. The residue was dissolved in EtOAc and washed with Na₂CO₃ (aq.). The organic layer was dried (MgSO₄), filtered and concentrated under reduced pressure. The crude mixture was purified by flash column chromatography (silica, heptane/(EtOAc/EtOH, 3:1), gradient from 80:20 to 0:100). The residue was dissolved in THF (2 mL) in a microwave tube. TBD (69.5 mg, 0.50 mmol) was added and the reaction mixture was heated at 100 °C over the weekend. The mixture was concentrated under reduced pressure and the crude mixture was purified by flash column chromatography (silica, heptane/(EtOAc/EtOH, 3:1), gradient from 80:20 to 0:100). The residue was dried overnight under vacuum at 50°C to afford compound **5** (18.2 mg, 2.3%) as a white solid.

**¹H NMR** (400 MHz, DMSO-*d*₆, 101°C) δ ppm 8.51 - 8.54 (m, 2H), 7.68 (d, *J*=8.4 Hz, 1H), 7.66 (d, *J*=2.0 Hz, 1H), 7.41 (dd, *J*=8.1, 2.0 Hz, 1H), 7.30 - 7.33 (m, 2H), 5.73 (q, *J*=7.2 Hz, 1H), 4.70 (s, 2H), 4.18 - 4.32 (m, 2H), 3.65 - 3.79 (m, 3H), 3.36 - 3.44 (m, 1H), 2.74 (t, *J*=5.8 Hz, 2H), 1.56 (d, *J*=7.0 Hz, 3H); **LCMS** (method D): Rt = 1.73 min, *m*/*z* calcd. for C₂₃H₂₁Cl₂N₅O₂ 469.1, found 470.2 [M+H]⁺.

### 3.1.2.6. Synthesis of Compound 6

### 2-(3,4-Dichlorobenzoyl)-9-[(2-methoxypyridin-4-yl)methyl]-1,2,3,4,8,9-hexahydropyrido-[4',3':3,4]pyrazolo[1,5-a]pyrazin-10(7H)-one

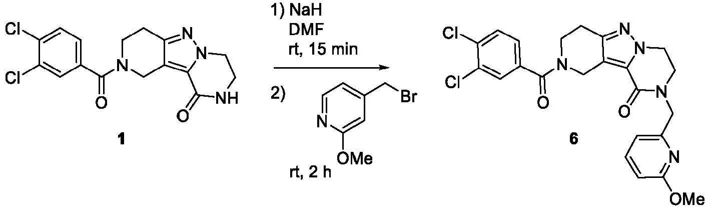

Compound **6** was obtained according to the procedure reported for the synthesis of compound **3.** The crude mixture was purified by flash column chromatography (silica, heptane/EtOAc, gradient from 100:0 to 0:100). A second purification was performed via Prep HPLC (stationary phase: RP XBridge Prep C18 OBD-10µm,30x150mm, mobile phase: NH₄HCO₃ (0.25% in water)/MeCN) to afford compound **6** (59 mg, 37%) as a white solid.

**¹H NMR** (600 MHz, DMSO-*d*₆, 83°C) δ ppm 7.66 - 7.70 (m, 2H), 7.64 (t, *J*=7.7 Hz, 1H), 7.42 (dd, *J*=8.3, 1.8 Hz, 1H), 6.90 (br d, *J*=7.1 Hz, 1H), 6.67 (d, *J*=8.3 Hz, 1H), 4.59 - 4.74 (m, 4H), 4.33 (t, *J*=6.0 Hz, 2H), 3.87 (br t, *J*=6.0 Hz, 2H), 3.80 (s, 3H), 3.57 - 3.83 (m, 2H), 2.75 (br t, *J*=5.5 Hz, 2H); **LCMS** (method C): Rt = 1.01 min, *m*/*z* calcd. for C₂₃H₂₁Cl₂N₅O₃ 485.1, found 486.2 [M+H]⁺.

### 3.1.2.7. Synthesis of Compound 7

### 2-(3,4-Dichlorobenzoyl)-9-[(1,3-thiazol-2-yl)methyl]-1,2,3,4,8,9-hexahydropyrido[4',3':3,4]-pyrazolo[1,5-a]pyrazin-10(7H)-one

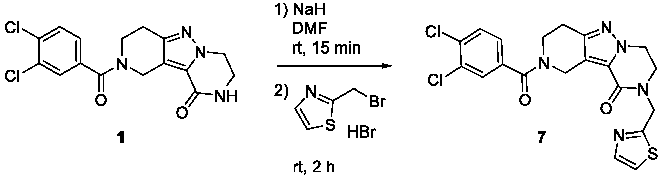

Compound **7** was obtained according to the procedure reported for the synthesis of compound **3.** The crude mixture was purified by flash column chromatography (silica, heptane/EtOAc, gradient from 100:0 to 0:100). A second purification was performed via Prep HPLC (stationary phase: RP XBridge Prep C18 OBD-10µm,30x150mm, mobile phase: NH₄HCO₃ (0.25% in water)/MeCN) to afford compound **7** (99 mg, yield 65%) as a white solid.

**¹H NMR** (400 MHz, DMSO-*d*₆, 81°C) δ ppm 7.73 (d, *J*=3.3 Hz, 1H), 7.66 - 7.71 (m, 2H), 7.63 (d, *J*=3.3 Hz, 1H), 7.42 (dd, *J*=8.1, 2.0 Hz, 1H), 4.95 (s, 2H), 4.69 (br s, 2H), 4.28 - 4.36 (m, 2H), 3.84 - 3.89 (m, 2H), 3.63 - 3.80 (m, 2H), 2.75 (t, *J*=5.8 Hz, 2H); **LCMS** (method C): Rt = 0.88 min, *m*/*z* calcd. for C₂₀H₁₇Cl₂N₅O₂S 461.0, found 462.2 [M+H]⁺.

### 3.1.2.8. Synthesis of Compound 8

### 2-(3,4-Dichlorobenzoyl)-9-[(1H-indol-3-yl)methyl]-1,2,3,4,8,9-hexahydropyrido[4',3':3,4]-pyrazolo[1,5-a]pyrazin-10(7H)-one

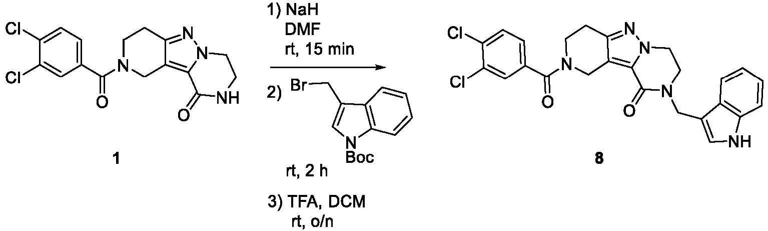

To a mixture of compound **1** (208 mg, 0.57 mmol) in DMF (5 mL) was added NaH (60% dispersion in mineral oil, 34.2 mg, 0.85 mmol) and the reaction mixture was stirred for 15 min at room temperature. *Tert-*Butyl 3-(bromomethyl)-1*H*-indole-1-carboxylate (194 mg, 0.63 mmol) was added and the reaction mixture was stirred for another 2 h. The reaction was quenched with MeOH (1mL) and concentrated under reduced pressure. The crude mixture was purified by flash column chromatography (silica, heptane/(EtOAc/EtOH, 3:1), gradient from 80:20 to 0:100). The product was dissolved in DCM (10 mL) and TFA (0.44 mL, 5.70 mmol) was added. The reaction mixture was stirred at room temperature overnight. The mixture was washed with NaHCO₃ (sat., aq., 250 mL), dried (MgSO₄), filtered and concentrate under reduced pressure. The crude mixture was purified by flash column chromatography (silica, heptane/(EtOAc/EtOH, 3:1), gradient from 80:20 to 0:100) to afford compound **8** (41 mg, 15%) as a light-yellow resin.

**¹H NMR** (400 MHz, DMSO-*d*₆, 101°C) δ ppm 10.75 (s, 1H), 7.69 (d, *J*=8.1 Hz, 1H), 7.67 (d, *J*=2.0 Hz, 1H), 7.56 (d, *J*=7.9 Hz, 1H), 7.42 (dd, *J*=8.1, 2.0 Hz, 1H), 7.36 (d, *J*=8.1 Hz, 1H), 7.34 (d, *J*=2.4 Hz, 1H), 7.04 - 7.10 (m, 1H), 6.93 - 6.98 (m, 1H), 4.79 (s, 2H), 4.74 (s, 2H), 4.13 - 4.18 (m, 2H), 3.66 - 3.80 (m, 2H), 3.59 - 3.65 (m, 2H), 2.73 (t, *J*=5.8 Hz, 2H); **LCMS** (method B): Rt = 1.91 min, *m*/*z* calcd. for C₂₅H₂₁Cl₂N₅O₂ 493.1, found 494.1 [M+H]⁺.

### 3.1.2.9. Synthesis of Compound 9

### 2-(3,4-Dichlorobenzoyl)-9-[(1H-indol-4-yl)methyl]-1,2,3,4,8,9-hexahydropyrido[4',3':3,4]-pyrazolo[1,5-a]pyrazin-10(7H)-one

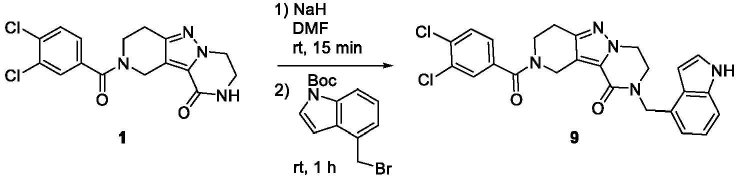

Compound **9** was obtained according to the procedure reported for the synthesis of compound **3.** The crude mixture was purified by flash column chromatography (silica, DMC/MeOH, gradient from 100:0 to 90:10). A second purification was performed via Prep HPLC (stationary phase: RP XBridge Prep C18 OBD-10µm,30x150mm, mobile phase: NH₄HCO₃ (0.25% in water)/MeCN). The residue was triturated in DIPE. The solids were collected by filtration to afford compound **9** (109 mg, 64%) as a greyish solid.

**¹H NMR** (400 MHz, DMSO-*d*₆, 81°C) δ ppm 10.96 (s, 1H), 7.71 (d, *J*=6.4 Hz, 1H), 7.69 (s, 1H), 7.42 - 7.46 (m, 1H), 7.35 (d, *J*=8.1 Hz, 1H), 7.28 (t, *J*=2.8 Hz, 1H), 7.05 (t, *J*=7.6 Hz, 1H), 6.95 (d, *J*=7.0 Hz, 1H), 6.48 (br s, 1H), 4.90 (s, 2H), 4.66 - 4.83 (m, 2H), 4.12 - 4.21 (m, 2H), 3.64 - 3.86 (m, 2H), 3.56 - 3.62 (m, 2H), 2.74 (t, *J*=5.7 Hz, 2H); **LCMS** (method C): Rt = 0.97 min, *m*/*z* calcd. for C₂₅H₂₁Cl₂N₅O₂ 493.1, found 494.3 [M+H]⁺.

### 3.1.2.10. Synthesis of Compound 10

### 2-(3,4-Dichlorobenzoyl)-9-{[4-(difluoromethoxy)phenyl]methyl}-1,2,3,4,8,9-hexahydropyrido-[4',3':3,4]pyrazolo[1,5-a]pyrazin-10(7H)-one

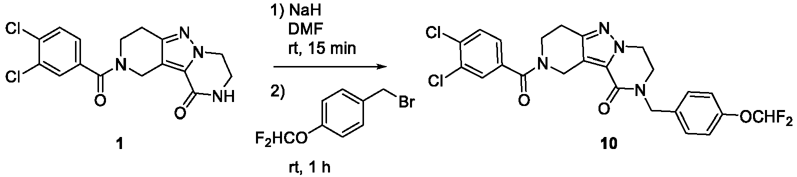

Compound **10** was obtained according to the procedure reported for the synthesis of compound **3.** The crude mixture was purified by flash column chromatography (silica, heptane/EtOAc, gradient from 100:0 to 0:100) to afford compound **10** (147 mg, 85%) as a white foam.

**¹H NMR** (400 MHz, DMSO-*d*₆, 81°C) δ ppm 7.69 (d, *J*=8.5 Hz, 1H), 7.67 (d, *J*=2.0 Hz, 1H), 7.42 (dd, *J*=8.3, 1.8 Hz, 1H), 7.34 - 7.39 (m, 2H), 7.11 - 7.16 (m, 2H), 7.09 (t, *J*=74.2 Hz, 1H), 4.66 - 4.78 (m, 2H), 4.63 (s, 2H), 4.24 - 4.30 (m, 2H), 3.66 - 3.84 (m, 4H), 2.74 (t, *J*=5.7 Hz, 2H).

### 3.1.2.11. Synthesis of Compound 11

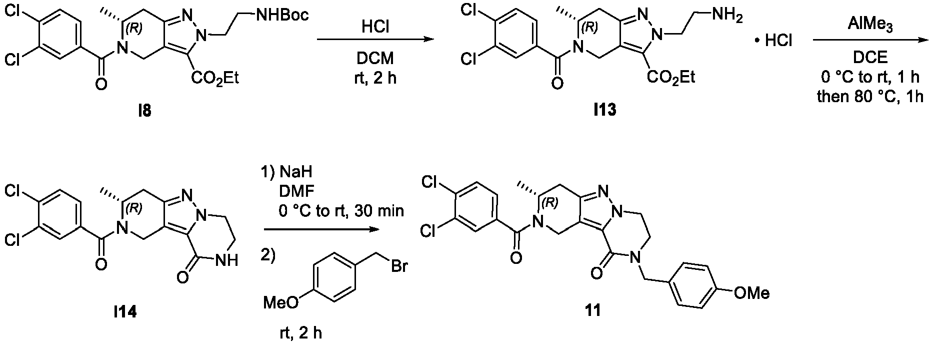

### Intermediate I13

### Ethyl (6R)-2-(2-aminoethyl)-5-(3,4-dichlorobenzoyl)-6-methyl-4,5,6,7-tetrahydro-2H-pyrazolo-[4,3-c]pyridine-3-carboxylate hydrochloride

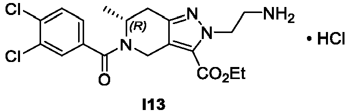

The reaction was performed under anhydrous conditions and under argon atmosphere.

HCl (4N in 1,4-dioxane, 24.8 mL, 99.0 mmol) was added dropwise to a solution of intermediate **I8** (3.47 g, 6.60 mmol) in DCM (60 mL) at room temperature and the reaction mixture was stirred for 2 h. The mixture was concentrated to dryness and co-evaporated with DCM (2 × 40 mL) to afford intermediate **I13** as a white solid which was used as such in the next step.

### Intermediate I14 (C251)

### (3R)-2-(3,4-Dichlorobenzoyl)-3-methyl-1,2,3,4,8,9-hexahydropyrido[4',3':3,4]pyrazolo[1,5-a]-pyrazin-10(7H)-one

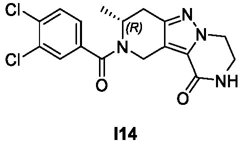

The reaction was performed under anhydrous conditions and under argon atmosphere.

To a solution of intermediate **I13** in DCE (110 mL) was added AlMe₃ (2 M in toluene, 9.91 mL, 19.8 mmol) at 0 °C. The reaction mixture was stirred at room temperature for 1h, and at 80 °C for another hour. The reaction mixture was cooled to room temperature and diluted with DCM (50 mL). Na₂SO₄•10H₂O (~10 g) was added portionwise. The mixture was stirred for 5 minutes and anhydrous Na₂SO₄ was added. The suspension was filtered and washed with DCM (50 mL). The filtrate was washed with NaHCO₃ (sat., aq., 150 mL), HCl (1M, aq., 150 mL) and brine (150 mL). The organic layer was dried (Na₂SO₄), filtered and concentrated under reduced pressure. The crude mixture was purified by flash column chromatography (silica, DCM/MeOH, gradient from 100:0 to 95:5) to afford intermediate **I14** (2.20 g, 85% over 2 steps) as a white solid.

### Compound 11

### (3R)-2-(3,4-Dichlorobenzoyl)-9-[(4-methoxyphenyl)methyl]-3-methyl-1,2,3,4,8,9-hexahydro-pyrido[4',3':3,4]pyrazolo[1,5-a]pyrazin-10(7H)-one

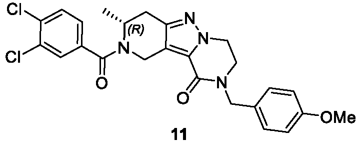

The reaction was performed under anhydrous conditions and under argon atmosphere.

To a solution of intermediate **I14** (350 mg, 0.92 mmol) in DMF (15 mL) at 0 °C was added NaH (60% in mineral oil, 55.4 mg, 1.38 mmol). The reaction mixture was stirred at room temperature for 30 min, then p-methoxybenzyl bromide (204 mg, 1.02 mmol) was added. The reaction mixture was stirred at room temperature for 2 h and diluted with EtOAc (100 mL), washed with H₂O (3 × 100 mL), brine, dried (Na₂SO₄), filtered and concentrated under reduced pressure. The crude mixture was purified by flash column chromatography (silica, DCM/MeOH, gradient from: 100:0 to 97:3). The residue was solubilized in EtOAc (100 mL) and the solution was successively washed with H₂O (3 × 100 mL), and brine (100 mL), dried (Na₂SO₄), filtered and concentrated under reduced pressure to afford compound **11** (391 mg, 84%).

**¹H NMR** (400 MHz, DMSO-*d*₆, 80°C) δ ppm 7.72 (d, *J*=8.2 Hz, 1H), 7.70 (d, *J*=1.7 Hz, 1H), 7.43 (dd, *J*=8.2, 1.7 Hz, 1H), 7.27 (d, *J*=8.5 Hz, 2H), 6.82 (d, *J*=8.5 Hz, 2H), 5.10 (br.s, 1H), 4.82 - 4.54 (m, 3H), 4.39 - 4.22 (m, 3H), 3.76 (s, 3H), 3.68 (t, *J*=5.9 Hz, 2H), 2.95 (dd, *J*=16.2, 5.6 Hz, 1H), 2.58 (d, *J*=16.2 Hz, 1H), 1.18 (d, *J*=6.6 Hz, 3H); **LCMS** (method A): Rt = 10.5 min, *m*/*z* calcd. for C₂₅H₂₄Cl₂N₄O₃ 498, *m*/*z* found 499 [M+H]⁺.

### 3.1.2.12. Synthesis of Compound 12

### (3R)-2-(3,4-Dichlorobenzoyl)-9-[(4-fluorophenyl)methyl]-3-methyl-1,2,3,4,8,9-hexahydro-pyrido[4',3':3,4]pyrazolo[1,5-a]pyrazin-10(7H)-one

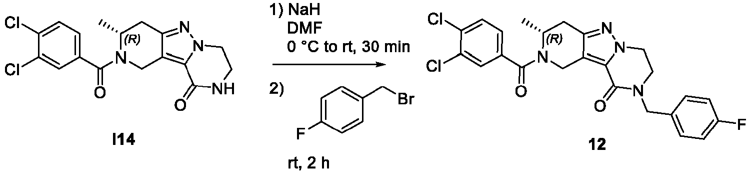

Compound **12** was obtained according to the procedure reported for the synthesis of compound **11.**

The crude mixture was purified by flash column chromatography (silica, DCM/MeOH, gradient from: 100: 0 to 97:3). The residue was solubilized in EtOAc (100 mL) and successively washed with H₂O (3 × 100 mL) and brine (100 mL), dried (Na₂SO₄), filtered and concentrated under reduced pressure to afford compound **12** (360 mg, 86%).

**¹H NMR** (400 MHz, DMSO-*d*₆, 80°C) δ ppm 7.72 (d, *J*=8.2 Hz, 1H), 7.70 (d, *J*=1.7 Hz, 1H), 7.43 (dd, *J*=8.2, 1.7 Hz, 1H), 7.41 - 7.36 (m, 2H), 7.19 - 7.12 (m, 2H), 5.19 - 4.98 (m, 1H), 4.80 - 4.59 (m, 3H), 4.36 - 4.24 (m, 3H), 3.72 (t, *J*=6.1 Hz, 2H), 2.95 (dd, *J*=15.8, 5.9 Hz, 1H), 2.58 (d, *J*=15.8 Hz, 1H), 1.18 (d, *J*=6.7 Hz, 3H); **LCMS** (method A): Rt = 10.7 min, *m*/*z* calcd. for C₂₄H₂₁Cl₂FN₄O2 486, *m*/*z* found 487 [M+H]⁺.

### 3.1.2.13. Synthesis of Compound 13

### (3R)-2-(3,4-Dichlorobenzoyl)-9-{[4-(difluoromethoxy)phenyl]methyl}-3-methyl-1,2,3,4,8,9-hexahydropyrido[4',3':3,4]pyrazolo[1,5-a]pyrazin-10(7H)-one

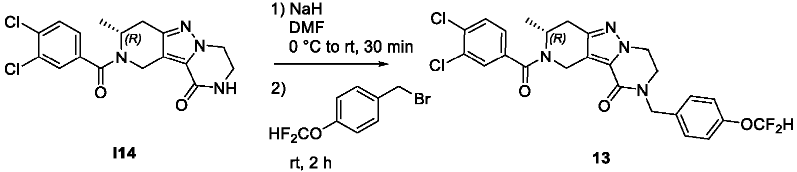

Compound **13** was obtained according to the procedure reported for the synthesis of compound **11.** The crude mixture was purified by flash column chromatography (silica, DCM/MeOH, gradient from 100:0 to 97:3). The residue was solubilized in EtOAc (100 mL) and washed successively with H₂O (3 × 100 mL) and brine (100 mL), dried (Na₂SO₄), filtered and concentrated under reduced pressure to afford compound **13** (375 mg, 80%).

**¹H NMR** (400 MHz, DMSO-*d*₆, 80°C) δ ppm 7.72 (d, *J*=8.2 Hz, 1H), 7.70 (d, *J*=1.7 Hz, 1H), 7.43 (dd, *J*=8.2, 1.7 Hz, 1H), 7.40 (d, *J*=8.3 Hz, 2H), 7.16 (d, *J*=8.3 Hz, 2H), 7.14 (t, *J*=74.0 Hz, 1H), 5.08 (br.s, 1H), 4.80 - 4.52 (m, 3H), 4.38 - 4.23 (m, 3H), 3.73 (t, *J*=6.2 Hz, 2H), 2.96 (dd, *J*=15.6, 5.4 Hz, 1H), 2.58 (d, *J*=15.6 Hz, 1H), 1.18 (d, *J*=6.4 Hz, 3H); **LCMS** (method A): Rt = 10.8 min, *m*/*z* calcd. for C₂₅H₂₂Cl₂F₂N₄O₃ 534, *m*/*z* found 535 [M+H]⁺.

### 3.1.2.14. Synthesis of Compound 14

### (3R)-2-(3,4-Dichlorobenzoyl)-3-methyl-9-{[4-(trifluoromethoxy)phenyl]methyl}-1,2,3,4,8,9-hexahydropyrido[4',3':3,4]pyrazolo[1,5-a]pyrazin-10(7H)-one

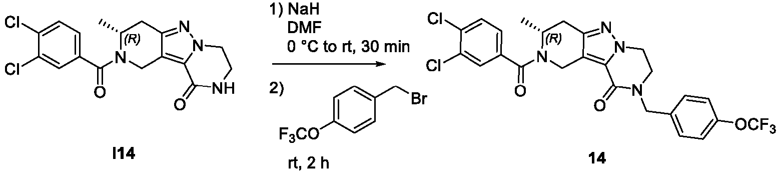

Compound **14** was obtained according to the procedure reported for the synthesis of compound **11.** The crude mixture was purified by flash column chromatography (silica, DCM/MeOH, gradient from 100:0 to 97:3). The residue was solubilized in EtOAc (100 mL) and the mixture was successively washed with H₂O (3 × 100 mL) and brine (100 mL), dried (Na₂SO₄), filtered and concentrated under reduced pressure to afford compound **14** (385 mg, 80%).

**¹H NMR** (400 MHz, DMSO-*d*₆, 80°C) δ ppm 7.72 (d, *J*=8.2 Hz, 1H), 7.70 (d, *J*=1.7 Hz, 1H), 7.48 (d, *J*=8.4 Hz, 2H), 7.43 (dd, *J*=8.2, 1.7 Hz, 1H), 7.32 (d, *J*=8.4 Hz, 2H), 5.09 (br.s, 1H), 4.87 - 4.52 (m, 3H), 4.38 - 4.23 (m, 3H), 3.75 (t, *J*=6.2 Hz, 2H), 2.96 (dd, *J*=16.0, 6.0 Hz, 1H), 2.59 (d, *J*=16.0 Hz, 1H), 1.18 (d, *J*=6.4 Hz, 3H); **LCMS** (method A): Rt = 11.4 min, *m*/*z* calcd. for C₂₅H₂₁Cl₂F₃N₄O₃ 552, *m*/*z* found 553 [M+H]⁺.

### 3.1.2.15. Synthesis of Compound 15

### (3R)-2-(3,4-Dichlorobenzoyl)-3-methyl-9-[(pyridin-2-yl)methyl]-1,2,3,4,8,9-hexahydropyrido-[4',3':3,4]pyrazolo[1,5-a]pyrazin-10(7H)-one

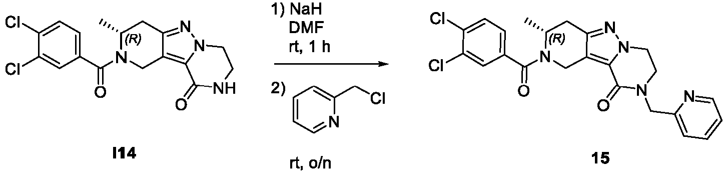

The reaction was performed under anhydrous conditions and under argon atmosphere.

NaH (60% in mineral oil, 66.4 mg, 1.66 mmol) was added to a solution of intermediate **I14** (130 mg, 0.33 mmol) in DMF (4 mL) and the reaction mixture was stirred at room temperature for 1 h. 2-Picolyl chloride (109 mg, 0.66 mmol) was added and the reaction mixture was stirred at room temperature overnight. The mixture was combined with another fraction (0.05 mmol) and diluted with H₂O (40 mL). The mixture was extracted with EtOAc (2 × 100 mL). The combined organic layers were washed with NaHCO₃ (sat., aq., 50 mL) and brine (3 × 50 mL), dried (Na₂SO₄), filtered and concentrated under reduced pressure. The crude mixture was purified by flash column chromatography (silica, DCM/MeOH, gradient from 100:0 to 90:10). A second purification was performed by reverse phase flash column chromatography (C-18, water/MeCN, gradient from 75:25 to 25:75) to afford compound **15** (90 mg, 50%) as a white solid.

**¹H NMR** (400 MHz, DMSO-*d*₆, 80°C) δ ppm 8.52 (ddd, *J*=4.8, 1.6, 0.8 Hz, 1H), 7.76 (td, *J*=7.6, 2.0 Hz, 1H), 7.71 (d, *J*=8.4 Hz, 1H), 7.69 (d, *J*=2.0 Hz, 1H), 7.42 (dd, *J*=8.0, 2.0 Hz, 1H), 7.36 (d, *J*=7.6 Hz, 1H), 7.28 (ddd, *J*=8.0, 5.2, 0.8 Hz, 1H), 5.05 (br.s, 1H), 4.80 - 4.72 (m, 2H), 4.63 (br.s, 1H), 4.39 - 4.26 (m, 3H), 3.90 - 3.80 (m, 2H), 2.95 (dd, *J*=16.0, 6.0 Hz, 1H), 2.58 (d, *J*=16.0 Hz, 1H), 1.17 (d, J=6.4 Hz, 3H); **LCMS** (method A): Rt = 8.6 min, *m*/*z* calcd. for C₂₃H₂₁Cl₂N₅O₂ 469, *m*/*z* found 470 [M+H]⁺.

### 3.1.2.16. Synthesis of Compound 16

### (3R)-2-(3,4-Dichlorobenzoyl)-3-methyl-9-[(pyridin-3-yl)methyl]-1,2,3,4,8,9-hexahydropyrido-[4',3':3,4]pyrazolo[1,5-a]pyrazin-10(7H)-one

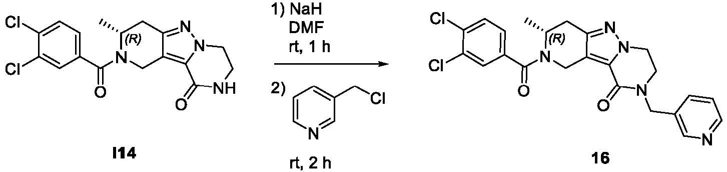

Compound **16** was obtained according to the procedure reported for the synthesis of compound **15.**

The crude mixture was purified by flash column chromatography (silica, DCM/MeOH, gradient from 100:0 to 90:10). The residue was co-evaporated with EtOAc (2 × 5 mL) to afford compound **16** (163 mg, 85%) as a white solid.

**¹H NMR** (400 MHz, DMSO-*d*₆, 80°C) δ ppm 8.57 (d, *J*=2.0 Hz, 1H), 8.50 (dd, *J*=4.8, 1.6 Hz, 1H), 8.50 (dt, *J*=7.6, 1.6 Hz, 1H), 7.71 (d, *J*=8.0 Hz, 1H), 7.69 (d, *J*=1.6 Hz, 1H), 7.42 (dd, *J*=8.0, 2.0 Hz, 1H), 7.42 (ddd, *J*=7.6, 4.8, 0.8 Hz, 1H), 5.08 (br.s, 1H), 4.73 - 4.64 (m, 3H), 4.36 - 4.26 (m, 3H), 3.81 - 3.71 (m, 2H), 2.95 (dd, *J*=15.6, 5.6 Hz, 1H), 2.57 (d, *J*=15.6 Hz, 1H), 1.17 (d, *J*=6.8 Hz, 3H); **LCMS** (method A): Rt = 7.9 min, *m*/*z* calcd. for C₂₃H₂₁Cl₂N₅O₂ 469, *m*/*z* found 470 [M+H]⁺.

### 3.2.1.17. Synthesis of Compound 17

### (3R)-2-(3,4-Dichlorobenzoyl)-3-methyl-9-[(pyridin-4-yl)methyl]-1,2,3,4,8,9-hexahydropyrido-[4',3':3,4]pyrazolo[1,5-a]pyrazin-10(7H)-one

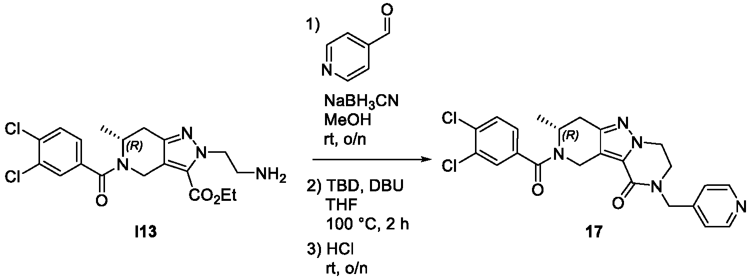

Sodium cyanoborohydride (134 mg, 2.02 mmol) was added to a mixture of intermediate **I13** (440 mg, 0.67 mmol) and 4-pyridinecarboxaldehyde (108 mg, 1.01 mmol) in MeOH (5 mL). The reaction mixture was stirred at room temperature overnight. Additional amount of 4-pyridinecarboxaldehyde (108 mg, 1.01 mmol) and sodium cyanoborohydride (134 mg, 2.02 mmol) was added and the reaction mixture was stirred overnight. The mixture was concentrated under reduced pressure. The residue was dissolved in THF (3 mL). DBU (0.50 mL, 3.37 mmol) and TBD (28.1 mg, 0.20 mmol) were added. The reaction mixture was stirred at 100 °C for 2 h in a sealed pressure tube and the mixture was concentrated under reduced pressure. The crude mixture was purified by flash column chromatography (silica, heptane/(EtOAc/EtOH, 3:1), gradient from 80:20 to 0:100). The residue was dissolved in MeOH (3 mL). HCl (1M aq., 1 mL) was added and the mixture was stirred at room temperature overnight. The solution was concentrated in a sample concentrator. The residue was dissolved in DCM (2 mL) and the solution was washed with Na₂CO₃ (aq.) and dried over an HM-N isolute cartridge. The filtrate was concentrated under reduced pressure. The crude mixture was purified by flash column chromatography (silica, heptane/(EtOAc/EtOH, 3:1), gradient from 80:20 to 0:100) to afford compound **17** (48 mg, 15%). **¹H NMR** (400 MHz, DMSO-*d*₆, 101°C) δ ppm 8.48 - 8.54 (m, 2H), 7.68 (d, *J*=8.1 Hz, 1H), 7.65 (d, *J*=2.0 Hz, 1H), 7.39 (dd, *J*=8.1, 2.0 Hz, 1H), 7.27 - 7.32 (m, 2H), 4.94 - 5.17 (m, 1H), 4.67 (s, 2H), 4.23 - 4.36 (m, 3H), 3.73 - 3.78 (m, 2H), 2.89 - 2.98 (m, 2H), 2.57 (d, *J*=15.8 Hz, 1H), 1.17 (d, *J*=7.0 Hz, 3H); **LCMS** (method B): Rt = 1.72 min, *m*/*z* calcd. for C₂₃H₂₁Cl₂N₅O₂ 469.1, found 470.1 [M+H]⁺.

### 3.2.1.18. Synthesis of Compound 18

### (3R)-2-(3,4-Dichlorobenzoyl)-3-methyl-9-[(1-methyl-1H-pyrazol-5-yl)methyl]-1,2,3,4,8,9-hexahydropyrido[4',3':3,4]pyrazolo[1,5-a]pyrazin-10(7H)-one

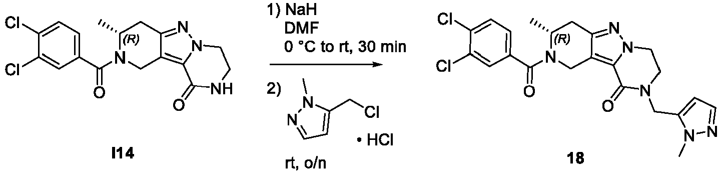

The reaction was performed under anhydrous conditions and under argon atmosphere.

To a solution of intermediate **I14** (130 mg, 285 µmol, 83% purity) in DMF (5 mL) was added NaH (60% in mineral oil, 12.5 mg, 0.31 mmol). The reaction mixture was stirred at room temperature for 30 min. To a solution of 5-(chloromethyl)-1-methyl-1*H*-pyrazole hydrochloride (52.3 mg, 0.31 mmol) in DMF (5 mL) was added NaH (60% in mineral oil, 12.5 mg, 0.31 mmol). The mixture was stirred at room temperature for 10 min and added to the mixture of intermediate **I14** and NaH in DMF. The reaction mixture was stirred at room temperature overnight, diluted with H₂O (150 mL) and extracted with EtOAc (3 × 150 mL). The combined organic extracts were washed with NaHCO₃ (sat., aq., 3 × 150 mL), brine (150 mL), dried (Na₂SO₄), filtered and concentrated to dryness. The crude mixture was purified by flash column chromatography (silica, cyclohexane/EtOAc, gradient from 80:20 to 50:50). A second purification was performed by reverse phase flash column chromatography (C-18, MeCN/H₂O, gradient from 1:9 to 1:1). The residue was further purified by flash column chromatography (silica, DCM/MeOH, gradient from 100:0 to 95:5). The product was co-evaporated with EtOH and dried at 50 °C under vacuum overnight to obtain compound **18** (83.2 mg, 62%).

**¹H NMR** (400 MHz, DMSO-*d*₆, 80°C) δ ppm 7.71 (d, *J*=8.0 Hz, 1H), 7.69 (d, *J*= 2.1 Hz, 1H), 7.42 (dd, *J*=8.0, 2.1Hz, 1H), 7.34 (d, *J*=1.8 Hz, 1H), 6.27 (d, *J*=1.8 Hz, 1H), 5.08 (br.s, 1H), 4.83 - 4.52 (m, 3H), 4.38 - 4.21 (m, 3H), 3.80 (s, 3H), 3.74 - 3.69 (m, 2H), 2.95 (dd, *J*=16.3, 6.6 Hz, 1H), 2.57 (d, *J*=16.3 Hz, 1H), 1.17 (d, *J*=6.8 Hz, 3H); **LCMS** (method A): Rt = 9.1 min, *m*/*z* calcd. for C₂₂H₂₂Cl₂N₆O₂ 472, *m*/*z* found 473 [M+H]⁺.

### 3.2.1.19. Synthesis of Compound 19

### (3R)-2-(3,4-Dichlorobenzoyl)-3-methyl-9-[(1-methyl-1H-pyrazol-3-yl)methyl]-1,2,3,4,8,9-hexahydropyrido[4',3':3,4]pyrazolo[1,5-a]pyrazin-10(7H)-one

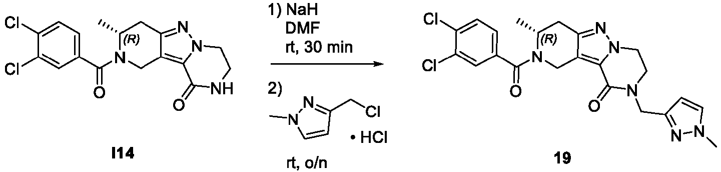

Compound **19** was obtained according to the procedure reported for the synthesis of compound **18.**

The crude mixture was purified by flash column chromatography (silica, EtOAc/MeOH, gradient from 99:1 to 92:8). The residue was triturated in Et₂O (3 mL) and successively co-evaporated with EtOAc and a mixture of EtOAc and EtOH (1:1, 4 × 5 mL) to afford compound **19** (92 mg, 43%). **¹H NMR** (400 MHz, DMSO-*d₆*, 80°C) δ ppm 7.71 (d, *J*=8.0 Hz, 1H), 7.69 (d, *J*=2.0 Hz, 1H), 7.58 (d, *J*=2.4 Hz, 1H), 7.42 (dd, *J*=8.0, 2.0 Hz, 1H), 6.15 (d, *J*=2.0 Hz, 1H), 5.06 (br.s, 1H), 4.63 (br.s, 1H), 4.63 - 4.54 (m, 2H), 4.32 - 4.22 (m, 3H), 3.80 (s, 3H), 3.75 - 3.71 (m, 2H), 2.94 (dd, *J*=16.0, 5.2 Hz, 1H), 2.57 (d, *J*=16.0 Hz, 1H), 1.17 (d, *J*=6.8 Hz, 3H); **LCMS** (method A): Rt = 9.1 min, *m*/*z* calcd. for C₂₂H₂₂Cl₂N₆O₂ 472, *m*/*z* found 473 [M+H]⁺.

### 3.2.1.20. Synthesis of Compound 20

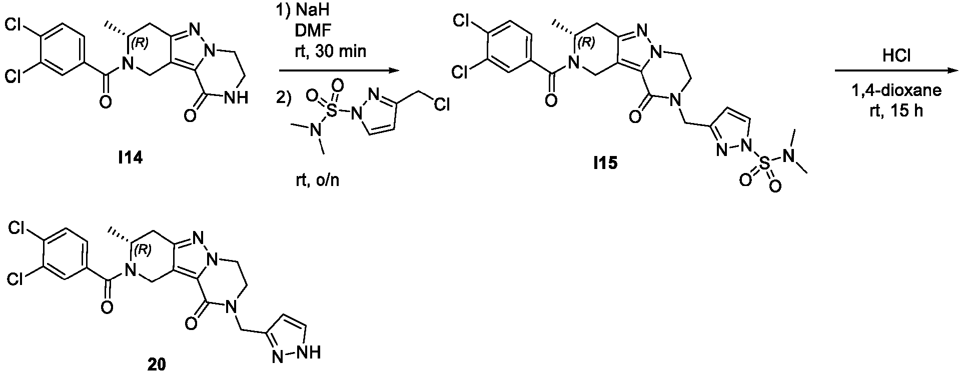

### Intermediate I15

### 3-{[(3R)-2-(3,4-Dichlorobenzoyl)-3-methyl-10-oxo-1,3,4,7,8,10-hexahydropyrido[4',3':3,4]-pyrazolo[1,5-a]pyrazin-9(2H)-yl]methyl}-N,N-dimethyl-1H-pyrazole-1-sulfonamide

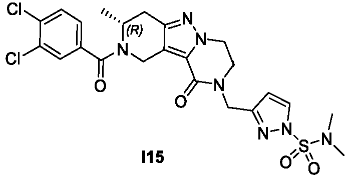

The reaction was performed under anhydrous conditions and under argon atmosphere.

To a solution of intermediate **I14** (185 mg, 0.49 mmol) in DMF (9 mL) was added NaH (60% in mineral oil, 21.4 mg, 0.54 mmol). The reaction mixture was stirred at room temperature for 30 min. A solution of 3-(chloromethyl)-*N,N*-dimethyl-1*H*-pyrazole-1-sulfonamide (120 mg, 0.54 mmol) in DMF (9 mL) was added and the reaction mixture was stirred at room temperature overnight. Additional quantity of 3-(chloromethyl)-*N,N*-dimethyl-1*H*-pyrazole-1-sulfonamide (21.4 mg, 0.54 mmol) was added and the reaction mixture was stirred for another 3 h. The reaction mixture was diluted with H₂O (45 mL) and extracted with EtOAc (3 × 45 mL). The combined organic layers were washed with NaHCO₃ (sat., aq., 3 × 45 mL) and brine (45 mL), dried (Na₂SO₄), filtered and concentrated to dryness. The crude mixture was combined with another fraction (0.11 mmol) and purified by flash column chromatography (silica, EtOAc/MeOH, gradient from 99:1 to 92:8) to afford intermediate **I15** (208 mg, 70%).

### Compound 20

### (3R)-2-(3,4-Dichlorobenzoyl)-3-methyl-9-[(1H-pyrazol-3-yl)methyl]-1,2,3,4,8,9-hexahydro-pyrido[4',3':3,4]pyrazolo[1,5-a]pyrazin-10(7H)-one

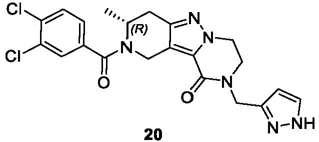

The reaction was performed under anhydrous conditions and under argon atmosphere.

Intermediate **I15** (188 mg, 0.33 mmol) was solubilized in HCl (4N in 1,4-dioxane, 9.13 mL, 36.5 mmol). The reaction mixture was stirred at room temperature for 15 h and quenched with NaHCO₃ (sat., aq., 9 mL). The aqueous phase was extracted with EtOAc (3 × 25 mL). The combined organic layers were washed with water (25 mL) and brine (25 mL), dried (Na₂SO₄), filtered and concentrated under reduced pressure. The crude mixture was purified by flash column chromatography (silica, DCM/MeOH, 96:4). The residue was triturated in Et₂O (3 mL), co-evaporated with EtOAc (2 × 5 mL) and EtOH (2 × 5 mL) and dried under vacuum overnight at 55 °C to afford compound **20** (60 mg, 39%) as a white solid.

**¹H NMR** (400 MHz, DMSO-*d*₆, 80°C) δ ppm 12.52 (br.s, 1H), 7.71 (d, *J*=8.0 Hz, 1H), 7.69 (d, *J*=2.0 Hz, 1H), 7.62 (br.s, 1H), 7.42 (dd, *J*=8.0, 2.0 Hz, 1H), 6.19 (d, *J*=1.6 Hz, 1H), 5.07 (br.s, 1H), 4.79 - 4.53 (m, 3H), 4.37 - 4.21 (m, 3H), 3.78 - 3.68 (m, 2H), 2.95 (dd, *J*=16.0, 6.0 Hz, 1H), 2.57 (d, *J*=16.0 Hz, 1H), 1.17 (d, *J*=6.8 Hz, 3H); **LCMS** (method A): Rt = 8.8 min, *m*/*z* calcd. for C₂₁H₂₀Cl₂N₆O₇ 458, *m*/*z* found 459 [M+H]⁺.

### 3.2.1.21. Synthesis of Compound 21

### (3R)-2-(3,4-Dichlorobenzoyl)-3-methyl-9-[(1,2-oxazol-3-yl)methyl]-1,2,3,4,8,9-hexahydro-pyrido[4',3':3,4]pyrazolo[1,5-a]pyrazin-10(7H)-one

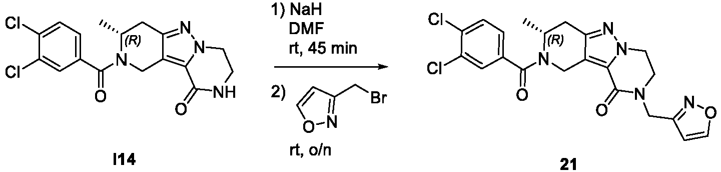

Compound **21** was obtained according to the procedure reported for the synthesis of compound **15.**

The crude mixture was purified by flash column chromatography (silica, DCM/MeOH, gradient from 100:0 to 95:5). A second purification was performed by preparative HPLC (XBridge column 5µm OBD, 30x150mm, 40 mL/min, H₂O +0.1% HCOOH/MeOH: 54/46). The residue was taken up in a mixture of EtOAc (30 mL) and NaHCO₃ (sat., aq., 30 mL). The layers were separated and the aqueous phase was extracted with EtOAc (2 × 25 mL). The combined organic extracts were dried (Na₂SO₄), filtered and concentrated under reduced pressure to afford compound **21** (52 mg, 28%) as a white solid.

**¹H NMR** (400 MHz, DMSO-*d*₆, 80°C) δ ppm 8.82 (d, *J*=1.6 Hz, 1H), 7.71 (d, *J*=8.4 Hz, 1H), 7.69 (d, *J*=2.0 Hz, 1H), 7.42 (dd, *J*=8.4, 2.0 Hz, 1H), 6.54 (d, *J*=1.6 Hz, 1H), 5.06 (br.s, 1H), 4.80 - 4.72 (m, 2H), 4.60 (br.s, 1H), 4.38 - 4.26 (m, 3H), 3.87 - 3.77 (m, 2H), 2.95 (dd, *J*=16.0, 5.6 Hz, 1H), 2.58 (d, *J*=16.0 Hz, 1H), 1.17 (d, *J*=6.8 Hz, 3H); **LCMS** (method A): Rt = 9.4 min, *m*/*z* calcd. for C₂₁H₁₉Cl₂N₅O₃ 459, *m*/*z* found 460 [M+H]⁺.

### 3.2.1.22. Synthesis of Compound 22

### (3R)-2-(3,4-Dichlorobenzoyl)-3-methyl-9-[(1,3-thiazol-4-yl)methyl]-1,2,3,4,8,9-hexahydro-pyrido[4',3':3,4]pyrazolo[1,5-a]pyrazin-10(7H)-one

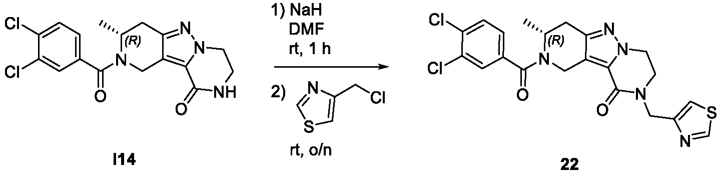

Compound **22** was obtained according to the procedure reported for the synthesis of compound **15.** The crude mixture was purified by flash column chromatography (silica, DCM/MeOH, gradient from 100:0 to 95:5) to afford 120 mg of compound **22** containing impurities. The fraction was combined with another batch (0.38 mmol) and purified by flash column chromatography (silica, DCM/MeOH, gradient from 100:0 to 90:10). A second purification was performed by flash column chromatography (C-18, H₂O/MeCN, gradient from 60:40 to 0:100). The residue was purified again by flash column chromatography (silica, EtOAc) to afford compound **22** (50 mg, 15%) as a white solid.

**¹H NMR** (400 MHz, DMSO-*d*₆, 80°C) δ ppm 9.04 (d, *J*=1.6 Hz, 1H), 7.71 (d, *J*=8.4 Hz, 1H), 7.69 (d, *J*=2.0 Hz, 1H), 7.59 (d, *J*=1.6 Hz, 1H), 7.42 (dd, *J*=8.0, 1.6 Hz, 1H), 5.07 (br.s, 1H), 4.84 - 4.75 (m, 2H), 4.60 (br.s, 1H), 4.37 - 4.26 (m, 3H), 3.88 - 3.78 (m, 2H), 2.95 (dd, *J*=16.0, 6.0 Hz, 1H), 2.58 (d, *J*=16.0 Hz, 1H), 1.17 (d, *J*=6.8 Hz, 3H); **LCMS** (method A): Rt = 9.3 min, *m*/*z* calcd. for C₂₁H₁₉CL₂N₅O₂S 475, *m*/*z* found 476 [M+H]⁺.

### 3.2.1.23. Synthesis of Compound 23

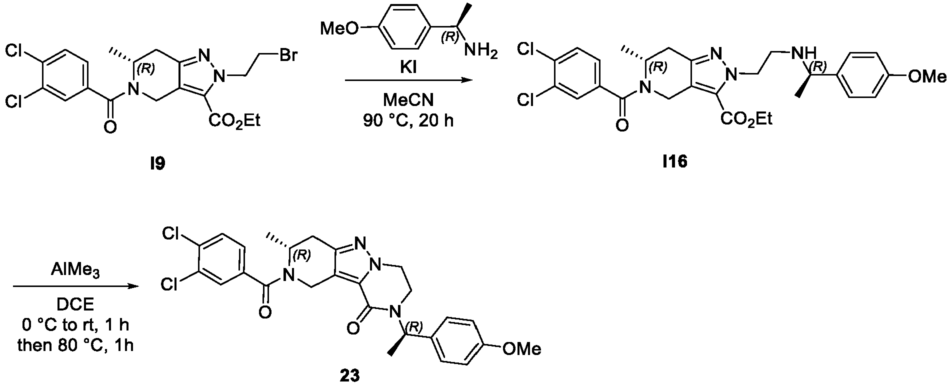

### Intermediate I16

### Ethyl (6R)-5-(3,4-dichlorobenzoyl)-2-(2-{[(1R)- 1-(4-methoxyphenyl)ethyl]amino }ethyl)-6-methyl-4,5,6,7-tetrahydro-2H-pyrazolo[4,3-c]pyridine-3-carboxylate

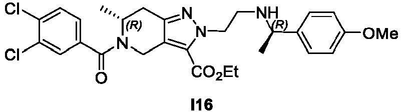

The reaction was performed under anhydrous conditions and under argon atmosphere.

To a solution of intermediate **I9** (700 mg, 1.39 mmol) in MeCN (25 mL) were added KI (92.2 mg, 0.56 mmol) and (*R*)-(-)-1-(4-methoxy-phenyl)-ethylamine (1.0 mL, 6.95 mmol). The reaction mixture was stirred at 90 °C for 20 h and diluted with H₂O (150 mL). The aqueous phase was extracted with EtOAc (3 × 150 mL). The combined organic extracts were dried (Na₂SO₄), filtered and concentrated to dryness. The crude mixture was purified by flash column chromatography (silica, DCM/MeOH, gradient from 100:0 to 95:5) to afford intermediate **I16** (523 mg, 67%).

### Compound 23

### (3R)-2-(3,4-Dichlorobenzoyl)-9-[(1R)-1-(4-methoxyphenyl)ethyl]-3-methyl-1,2,3,4,8,9-hexahydropyrido[4',3':3,4]pyrazolo[1,5-a]pyrazin-10(7H)-one

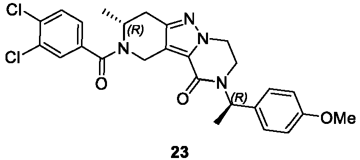

The reaction was performed under anhydrous conditions and under argon atmosphere.

To a solution of intermediate **I16** (524 mg, 0.94 mmol) in DCE (6 mL) at 0 °C was added AlMe₃ (2M in toluene, 1.40 mL, 2.80 mmol). The reaction mixture was stirred at room temperature for 1 h and at 80 °C for 1 h. An additional quantity of AlMe₃ (2M in toluene, 1.40 mL, 2.80 mmol) was added and the reaction mixture was stirred at 80 °C for another hour. The reaction mixture was diluted with DCM (150 mL) and Na₂SO₄•10H₂O (1.5 g) was added. The mixture was stirred for 30 min, dried (Na₂SO₄) and filtered. The organic layer was washed with HCl (1N, aq., 3 × 150 mL), dried (Na₂SO₄), filtered and evaporated under reduced pressure. The crude mixture was purified by flash column chromatography (silica, DCM/MeOH, gradient from 100:0 to 95:5) to afford compound **23** (340 mg, 71%).

**¹H NMR** (400 MHz, DMSO-*d*₆, 80°C) δ ppm 7.71 (d, *J*=8.3 Hz, 1H), 7.69 (d, *J*= 2.1 Hz, 1H), 7.42 (dd, *J*=8.3, 2.1 Hz, 1H), 7.30 (d, *J*=8.6 Hz, 2H), 6.93 (d, *J*=8.6 Hz, 2H), 5.78 (q, *J*=7.0 Hz, 1H), 5.07 (br.s, 1H), 4.63 (br.s, 1H), 4.38 - 4.21 (m, 2H), 4.19 - 4.11 (m, 1H), 3.77 (s, 3H), 3.71 - 3.62 (m, 1H), 3.33 - 3.26 (m, 1H), 2.95 (dd, *J*=16.3, 6.6 Hz, 1H), 2.57 (d, *J*=16.3 Hz, 1H), 1.54 (d, *J*=7.0 Hz, 3H), 1.18 (d, *J*=6.8 Hz, 3H); **LCMS** (method A): Rt = 10.9 min, *m*/*z* calcd. for C₂₆H₂₆Cl₂N₄O₃ 512, *m*/*z* found 513 [M+H]⁺.

### 3.2.1.24. Synthesis of Compound 24

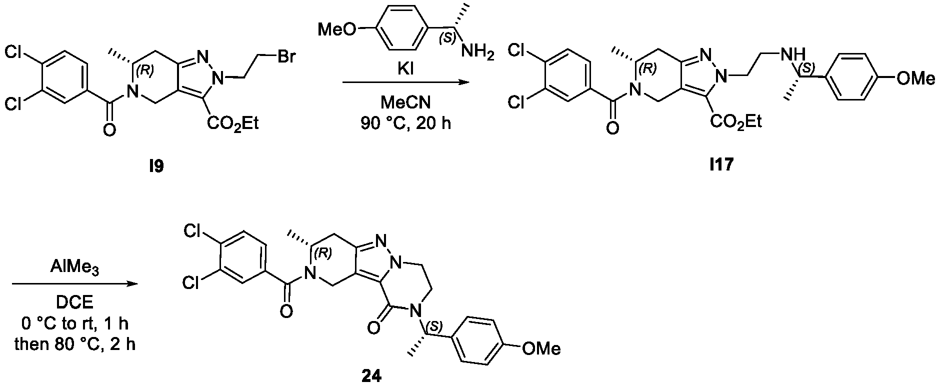

### Intermediate I17

### Ethyl (6R)-5-(3,4-dichlorobenzoyl)-2-(2-{[(1S)-1-(4-methoxyphenyl)ethyl]amino }ethyl)-6-methyl-4,5,6,7-tetrahydro-2H-pyrazolo[4,3-c]pyridine-3-carboxylate

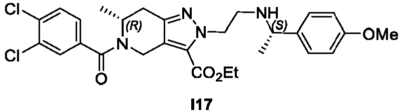

Intermediate **I17** was obtained according to the procedure reported for the synthesis of intermediate **I16.**

The crude mixture was purified by flash column chromatography (silica, cyclohexane/EtOAc, gradient from 90:10 to 0:100). A second purification was performed by flash column chromatography (silica, DCM/MeOH, gradient from 100:0 to 95:5) to afford intermediate **I17** (631 mg, 81%).

### Compound 24

### (3R)-2-(3,4-dichlorobenzoyl)-9-[(1S)-1-(4-methoxyphenyl)ethyl]-3-methyl-1,2,3,4,8,9-hexahydropyrido[4',3':3,4]pyrazolo[1,5-a]pyrazin-10(7H)-one

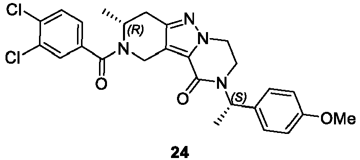

Compound **24** was obtained according to the procedure reported for the synthesis of compound **23.** The crude mixture was purified by flash column chromatography (silica, DCM/MeOH, gradient from 100;0 to 95:5) to afford compound **24** (447 mg, 77%, 95% purity).

**¹H NMR** (400 MHz, DMSO-*d*₆, 80°C) δ ppm 7.71 (d, *J*=8.3 Hz, 1H), 7.69 (d, *J*=2.1 Hz, 1H), 7.42 (dd, *J*=8.3, 2.1Hz, 1H), 7.28 (d, *J*=8.6 Hz, 2H), 6.92 (d, *J*=8.6 Hz, 2H), 5.78 (q, *J*=7.0 Hz, 1H) 5.11 (br.s, 1H), 4.62 (br.s, 1H), 4.37 - 4.21 (m, 2H), 4.16 - 4.05 (m, 1H), 3.76 (s, 3H), 3.70 - 3.63 (m, 1H), 3.30 - 3.22 (m, 1H), 2.94 (dd, *J*=16.3, 6.6 Hz, 1H), 2.56 (d, *J*=16.3 Hz, 1H), 1.54 (d, *J*=7.0 Hz, 3H), 1.17 (d, *J*=6.8 Hz, 3H); **LCMS** (method A): Rt = 10.8 min, *m*/*z* calcd. for C₂₆H₂₆Cl₂N₄O₃ 512, *m*/*z* found 513 [M+H]⁺.

### 3.2.1.25. Synthesis of Compound 25

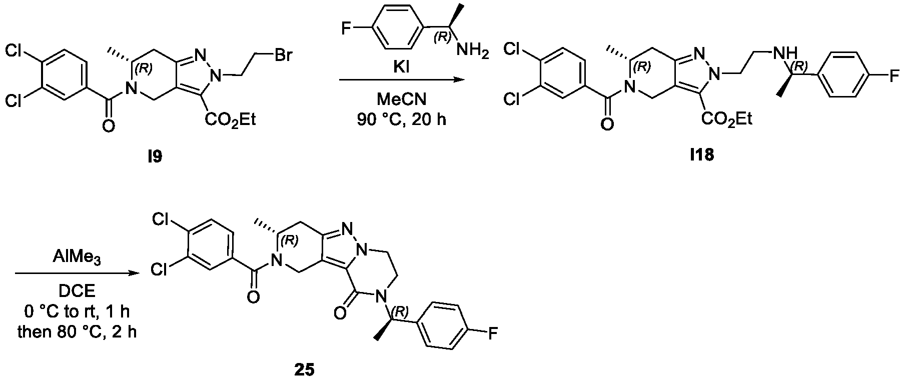

### Intermediate I18

### Ethyl (6R)-5-(3,4-dichlorobenzoyl)-2-(2-{[(1R)-1-(4-fluorophenyl)ethyl]amino }ethyl)-6-methyl-4,5,6,7-tetrahydro-2H-pyrazolo[4,3-c]pyridine-3-carboxylate

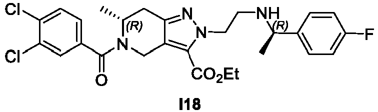

Intermediate **I18** was obtained according to the procedure reported for the synthesis of intermediate **I16.**

The crude mixture was purified by flash column chromatography (silica, cyclohexane/EtOAc, gradient from 90:10 to 0:100) to afford intermediate **I18** (397 mg, 79%).

### Compound 25

### (3R)-2-(3,4-Dichlorobenzoyl)-9-[(1R)-1-(4-fluorophenyl)ethyl]-3-methyl-1,2,3,4,8,9-hexahydropyrido[4',3':3,4]pyrazolo[1,5-a]pyrazin-10(7H)-one

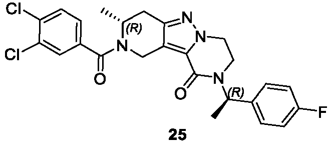

Compound **25** was obtained according to the procedure reported for the synthesis of compound **23.**

The crude mixture was purified by flash column chromatography (silica, DCM/MeOH, gradient from 100:0 to 95:5). A purification was performed via Prep SFC (stationary phase: Chiralpak Daicel ID 20 × 250 mm, mobile phase: CO₂, *i*-PrOH + 0.4% i-PrNH₂) to afford compound **25** (195 mg, 56%).

**¹H NMR** (400 MHz, DMSO-*d*₆) δ ppm 1.16 (d, *J*=6.8 Hz, 3H), 1.54 (d, *J*=7.0 Hz, 3H), 2.55 (d, *J*=16.1 Hz, 1H), 2.87 - 2.95 (m, 1H), 3.21 - 3.41 (m, 1H), 3.56 - 3.74 (m, 1H), 4.05 - 4.36 (m, 3H), 4.53 (br s, 1H), 5.05 (br s, 1H), 5.78 (q, *J*=7.0 Hz, 1H), 7.09 - 7.16 (m, 2H), 7.36 - 7.43 (m, 3H), 7.62 - 7.71 (m, 2H); **LCMS** (method D): Rt = 2.14 min, *m*/*z* calcd. for C₂₅H₂₃Cl₂FN₄O₂, 500.1; *m*/*z* found 501.0 [M+H]⁺.

### 3.2.1.26. Synthesis of Compound 26

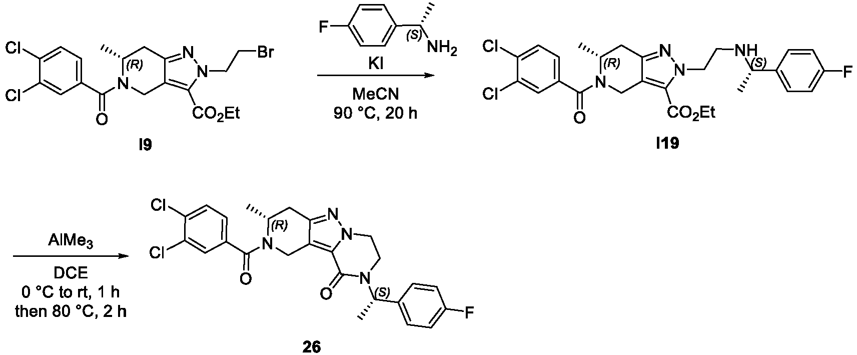

### Intermediate I19

### Ethyl (6R)-5-(3,4-dichlorobenzoyl)-2-(2-{[(1S)-1-(4-fluorophenyl)ethyl]amino }ethyl)-6-methyl-4,5,6,7-tetrahydro-2H-pyrazolo[4,3-c]pyridine-3-carboxylate

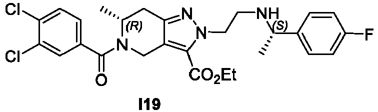

Intermediate **I19** was obtained according to the procedure reported for the synthesis of intermediate **I16.**

The crude mixture was purified by flash column chromatography (silica, cyclohexane/EtOAc, gradient from 90:10 to 0:100) to afford intermediate **I19** (709 mg, 72%).

### Compound 26

### (3R)-2-(3,4-Dichlorobenzoyl)-9-[(1S)-1-(4-fluorophenyl)ethyl]-3-methyl-1,2,3,4,8,9-hexahydro-pyrido[4',3':3,4]pyrazolo[1,5-a]pyrazin-10(7H)-one

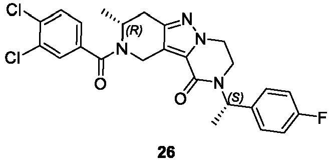

Compound **26** was obtained according to the procedure reported for the synthesis of compound **23.**

The crude mixture was purified by flash column chromatography (silica, DCM/EtOAc, gradient from 50:50 to 20:80, then DCM/MeOH, gradient from 100:0 to 95: 5). A second purification was performed by flash column chromatography (silica, DCM/THF, gradient from 100:0 to 70:30). The residue was finally purified via Prep SFC (stationary phase: Chiralpak Diacel AD 20 × 250 mm, mobile phase: CO₂, EtOH + 0.4% i-PrNH₂) to afford compound **26** (80 mg, 26%).

**¹H NMR** (400 MHz, DMSO-*d*₆) δ ppm 1.16 (d, *J*=6.8 Hz, 3H), 1.55 (d, *J*=7.3 Hz, 3H), 2.56 (d, *J*=16.1 Hz, 1H), 2.92 (dd, *J*=16.0, 5.8 Hz, 1H), 3.30 (ddd, *J*=13.2, 7.0, 4.7 Hz, 1H), 3.69 (ddd, *J*=13.0, 8.1, 4.6 Hz, 1H), 4.08 - 4.17 (m, 1H), 4.21 - 4.32 (m, 2H), 4.55 - 4.74 (m, 1H), 4.94 - 5.22 (m, 1H), 5.79 (q, *J*=7.2 Hz, 1H), 7.09 - 7.16 (m, 2H), 7.35 - 7.42 (m, 2H), 7.64 - 7.71 (m, 2H); **LCMS** (method D): Rt = 2.14 min, *m*/*z* calcd. for C₂₅H₂₃Cl₂FN₄O₂, 500.1; *m*/*z* found 501.2 [M+H]⁺.

### 3.2.1.27. Synthesis of Compound 27

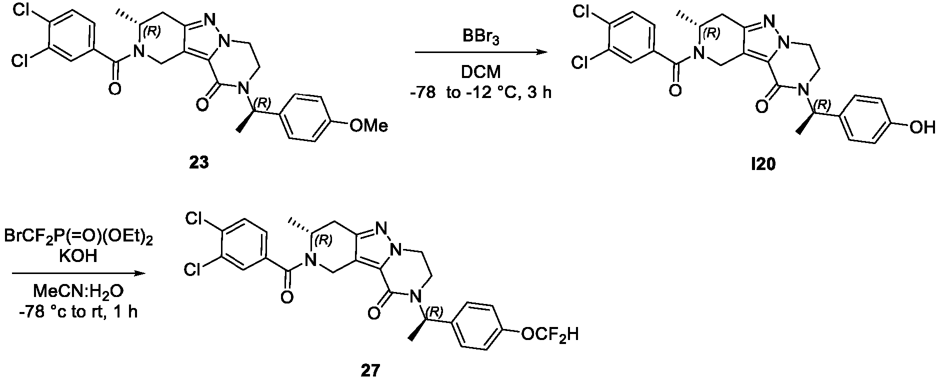

### Intermediate I20

### (3R)-2-(3,4-Dichlorobenzoyl)-9-[(1R)-1-(4-hydroxyphenyl)ethyl]-3-methyl-1,2,3,4,8,9-hexa-hydropyrido[4',3':3,4]pyrazolo[1,5-a]pyrazin-10(7H)-one

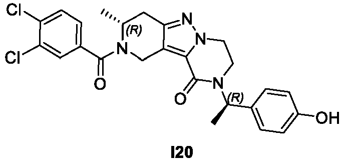

The reaction was performed under anhydrous conditions and under argon atmosphere.

To a solution of compound **23** (200 mg, 0.39 mmol) in DCM (4 mL) at -78 °C was added BBr₃ (1M in DCM, 2.34 mL, 2.34 mmol). The reaction mixture was stirred at -78°C for 1 h, warmed to -35 °C and stirred for 1 h, warmed to -12 °C and stirred for another hour. The reaction was quenched with NaHCO₃ (sat., aq., 15 mL) and the mixture was stirred for 15 min and concentrated under reduced pressure. MeOH (10 mL) was added and the reaction mixture was stirred at room temperature overnight. The aqueous layer was extracted with EtOAc (3 × 100 mL). The combined organic layers were dried (Na₂SO₄), filtered and concentrated to dryness. The crude mixture was purified by flash column chromatography (silica, DCM/EtOAc, gradient from 60:40 to 0:10) to afford intermediate **I20** (135 mg, 65%, 93% purity).

### Compound 27

### (3R)-2-(3,4-Dichlorobenzoyl)-9-{(1R)-1-[4-(difluoromethoxy)phenyl]ethyl}-3-methyl-1,2,3,4,8,9-hexahydropyrido[4',3':3,4]pyrazolo[1,5-a]pyrazin-10(7H)-one

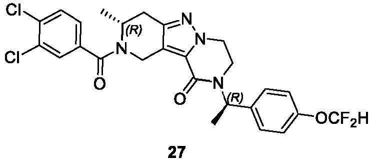

The reaction was performed under argon atmosphere.

To a suspension of intermediate **I20** (100 mg, 0.20 mmol) and KOH (225 mg, 4.00 mmol) in H₂O (350 µL) and MeCN (350 µL) at -78 °C was added diethyl[(bromodifluoro)methyl]phosphonate (71 µL, 0.40 mmol). The reaction mixture was stirred at room temperature for 1 h and diluted with EtOAc (50 mL). The mixture was washed with NaHCO₃ (sat., aq.), brine, dried (Na₂SO₄), filtered and concentrated under reduced pressure. The crude mixture was purified by flash column chromatography (silica, DCM/MeOH, gradient from 100:0 to 97:3) to afford compound **27** (48 mg, 43%) as a light yellow solid.

**¹H NMR** (400 MHz, DMSO-*d₆*, 80°C) δ ppm 7.73 (d, *J*=8.2 Hz, 1H), 7.70 (d, *J*=1.6 Hz, 1H), 7.47 - 7.41 (m, 3H), 7.18 (d, *J*=8.4 Hz, 2H), 7.15 (t, *J*=74.2 Hz, 1H), 5.81 (q, *J*=7.2 Hz, 1H), 5.05 (br.s, 1H), 4.65 (br.s, 1H), 4.37 - 4.15 (m, 3H), 3.75 - 3.67 (m, 1H), 3.39 - 3.31 (m, 1H), 2.95 (dd, *J*=16.0, 6.0 Hz, 1H), 2.58 (d, *J*=16.0 Hz, 1H), 1.56 (d, *J*=7.2 Hz, 3H), 1.18 (d, *J*=6.7 Hz, 3H); **LCMS** (method A): Rt = 11.1 min, *m*/*z* calcd. for C₂₆H₂₄Cl₂F₂N₄O₃ 548, *m*/*z* found 549 [M+H]⁺.

### 3.2.1.28. Synthesis of Compound 28

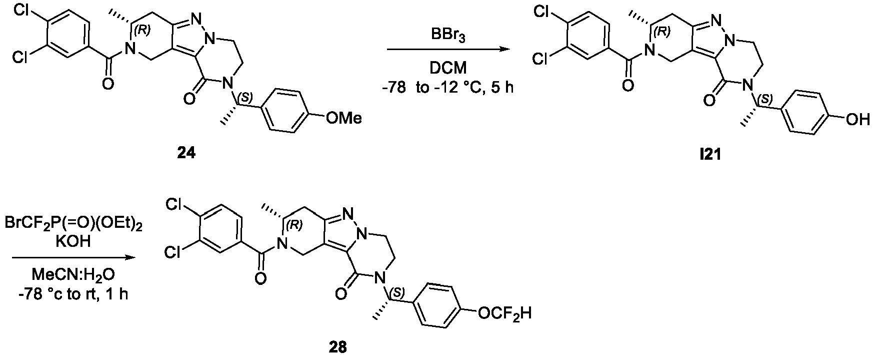

### Intermediate I21

### (3R)-2-(3,4-Dichlorobenzoyl)-9-[(SR)-1-(4-hydroxyphenyl)ethyl]-3-methyl-1,2,3,4,8,9-hexahydropyrido[4',3':3,4]pyrazolo[1,5-a]pyrazin-10(7H)-one

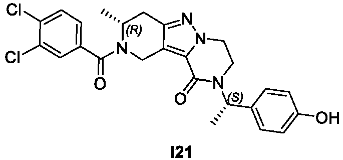

Intermediate **I21** was obtained according to the procedure reported for the synthesis of intermediate **I20**.

The crude mixture was purified by flash column chromatography (silica, DCM/EtOAc, gradient from 60:40 to 0:100) to afford intermediate **I21** (173 mg, 65%, 91% purity).

### Compound 28

### (3R)-2-(3,4-Dichlorobenzoyl)-9-{(1S)-1-[4-(difluoromethoxy)phenyl]ethyl}-3-methyl-1,2,3,4,8,9-hexahydropyrido[4',3':3,4]pyrazolo[1,5-a]pyrazin-10(7H)-one

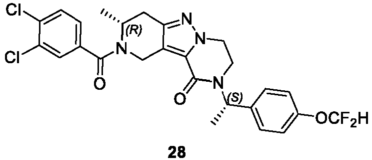

Compound **28** was obtained according to the procedure reported for the synthesis of compound **27.**

The crude mixture was purified several times (3 times) by flash column chromatography (silica, DCM/MeOH, gradient from 100:0 to 97:3) to afford compound **28** (67 mg, 47%) as a white solid. **¹H NMR** (400 MHz, DMSO-*d*₆, 80°C) δ ppm 7.72 (d, *J*=8.2 Hz, 1H), 7.70 (d, *J*= 1.9 Hz, 1H), 7.46 - 7.41 (m, 3H), 7.17 (d, *J*=8.4 Hz, 2H), 7.14 (t, *J*=74.1 Hz, 1H), 5.81 (q, *J*=7.2 Hz, 1H), 5.10 (br.s, 1H), 4.62 (br.s, 1H), 4.37 - 4.26 (m, 2H), 4.21 - 4.13 (m, 1H), 3.77 - 3.68 (m, 1H), 3.37 - 3.29 (m, 1H), 2.95 (dd, *J*=16.0, 5.6 Hz, 1H), 2.58 (d, *J*=16.0 Hz, 1H), 1.57 (d, *J*=6.9 Hz, 3H), 1.18 (d, J=6.9 Hz, 3H); **LCMS** (method A): Rt = 11.1 min, *m*/*z* calcd. for C₂₆H₂₄Cl₂F₂N₄O₃ 548, *m*/*z* found 549 [M+H]⁺.

### 3.2.1.29. Synthesis of Compound 29

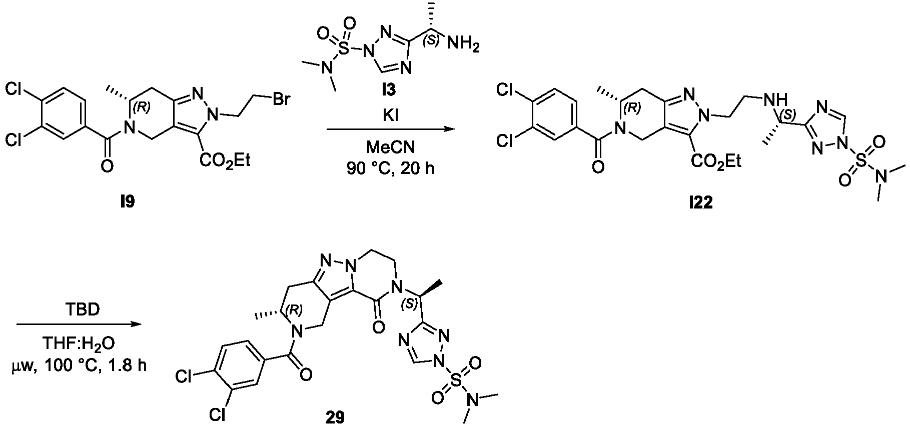

### Intermediate I22

### Ethyl (6R)-5-(3,4-dichlorobenzoyl)-2-[2-({(1S)-1-[1-(dimethylsulfamoyl)-1H-1,2,4-triazol-3-yl]ethyl}amino)ethyl]-6-methyl-4,5,6,7-tetrahydro-2H-pyrazolo[4,3-c]pyridine-3-carboxylate

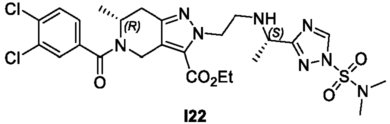

The reaction was performed under anhydrous conditions and under argon atmosphere.

Intermediate **I9** (362 mg, 0.74 mmol) was dissolved in MeCN (12 mL). KI (49.2 mg, 0.30 mmol) and intermediate **I3** (820 mg, 3.70 mmol) were added. The reaction mixture was stirred at 90 °C for 20 h Additional quantity of KI (49.2 mg, 0.30 mmol) was added and the reaction mixture was stirred for another 1.5 h. The reaction mixture was diluted with H₂O (10 mL) and extracted with EtOAc (3 × 20 mL). The combined organic layers were dried (Na₂SO₄), filtered and concentrated under reduced pressure. The crude mixture was purified by flash column chromatography (silica, DCM/MeOH, gradient from 100:0 to 95:5). A second purification was performed by flash column chromatography (silica, DCM/MeOH, 95:5) to give a pure fraction of intermediate I22 (292 mg, 61%) and another fraction containing impurities (49 mg, 9%, 84% purity).

### Compound 29

### 3-{(1S)-1-[(3R)-2-(3,4-Dichlorobenzoyl)-3-methyl-10-oxo-1,3,4,7,8,10-hexahydropyrido-[4',3':3,4]pyrazolo[1,5-a]pyrazin-9(2H)-yl]ethyl}-N,N-dimethyl-1H-1,2,4-triazole-1-sulfonamide

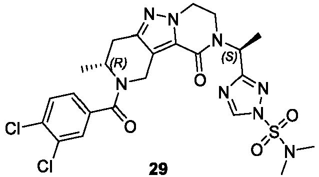

The reaction was performed under argon atmosphere.

Intermediate **I22** (292 mg, 0.45 mmol) was dissolved in MeCN (1.1 mL) and H₂O (5.9 mL). TBD (37.9 mg, 0.27 mmol) was added and the reaction mixture was stirred at 100 °C under microwave irradiation for 1.8 h. The reaction mixture was concentrated to dryness. The crude mixture was purified by flash column chromatography (silica, DCM/MeOH, gradient from 98:2 to 95:5) to give compound **29** (215 mg, 71%, 87% purity).

28 mg of compound **29** was co-evaporated with EtOAc (3 × 2 mL) and dried under vacuum at 50 °C for 20 h to give pure compound **29** (24 mg) as a white solid.

**¹H NMR** (400 MHz, DMSO-*d₆*, 80°C) δ ppm 9.00 (s, 1H), 7.72 (d, *J*=8.3 Hz, 1H), 7.69 (d, *J*= 2.1 Hz, 1H), 7.42 (dd, *J*=8.3, 2.1Hz, 1H), 5.86 (q, *J*=6.9 Hz, 1H), 5.06 (br.s, 1H), 4.61 (br.s, 1H), 4.39 - 4.19 (m, 3H), 3.86 - 3.78 (m, 1H), 3.65 - 3.58 (m, 1H), 2.95 (dd, *J*=16.3, 6.6 Hz, 1H), 2.91 (s, 6H), 2.58 (d, *J*=16.3 Hz, 1H), 1.61 (d, *J*=6.9 Hz, 3H), 1.17 (d, *J*=6.8 Hz, 3H); **LCMS** (method A): Rt = 10.1 min, *m*/*z* calcd. for C₂₃H₂₆Cl₂N₈O₄S 580, *m*/*z* found 581 [M+H]⁺.

### 3.2.1.30. Synthesis of Compound 30

### (3R)-2-(3,4-Dichlorobenzoyl)-3-methyl-9-[(1S)-1-(1H-1,2,4-triazol-3-yl)ethyl]-1,2,3,4,8,9-hexa-hydropyrido[4',3':3,4]pyrazolo[1,5-a]pyrazin-10(7H)-one

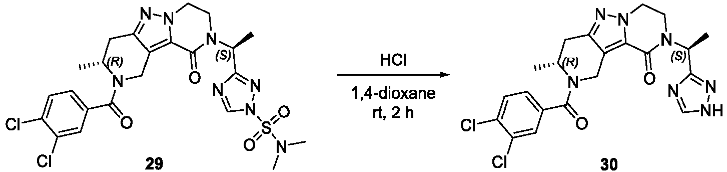

The reaction was performed under anhydrous conditions and under argon atmosphere.

Compound **29** (178 mg, 0.27 mmol, 87% purity) was solubilized in 1,4-dioxane (1 mL) and HCl (4N in 1,4-dioxane, 5 mL, 20 mmol) was added. The reaction mixture was stirred at room temperature for 2 h and concentrated to dryness. The residue was diluted in DCM (5 mL) and washed with NaHCO₃ (sat., aq., 3 × 5 mL). The combined organic layers were dried (Na₂SO₄), filtered and concentrated under reduced pressure. The crude mixture was purified by flash column chromatography (silica, DCM/MeOH, gradient from 100:0 to 95:5). The residue was co-evaporated with EtOH (3 × 3 mL) and dried under vacuum at 50 °C for 48 h to give compound **30** (102 mg, 80%) as a white solid.

**¹H NMR** (400 MHz, DMSO-*d₆*, 80°C) δ ppm 13.74 (br.s, 1H), 8.24 (s, 1H), 7.71 (d, *J*=8.3 Hz, 1H), 7.69 (d, *J*=2.1 Hz, 1H), 7.42 (dd, *J*=8.3, 2.1Hz, 1H), 5.86 (q, *J*=7.2 Hz, 1H), 5.08 (br.s, 1H), 4.62 (br.s, 1H), 4.35 - 4.15 (m, 3H), 3.81 - 3.72 (m, 1H), 3.62 - 3.53 (m, 1H), 2.95 (dd, *J*=16.3, 6.6 Hz, 1H), 2.57 (d, *J*=16.3 Hz, 1H), 1.57 (d, *J*=7.2 Hz, 3H), 1.17 (d, *J*=6.8 Hz, 3H); **LCMS** (method A): Rt = 8.5 min, *m*/*z* calcd. for C₂₁H₂₁Cl₂N₇O₂ 473, *m*/*z* found 474 [M+H]⁺.

### 3.2.1.31. Synthesis of Compounds 31 and 32

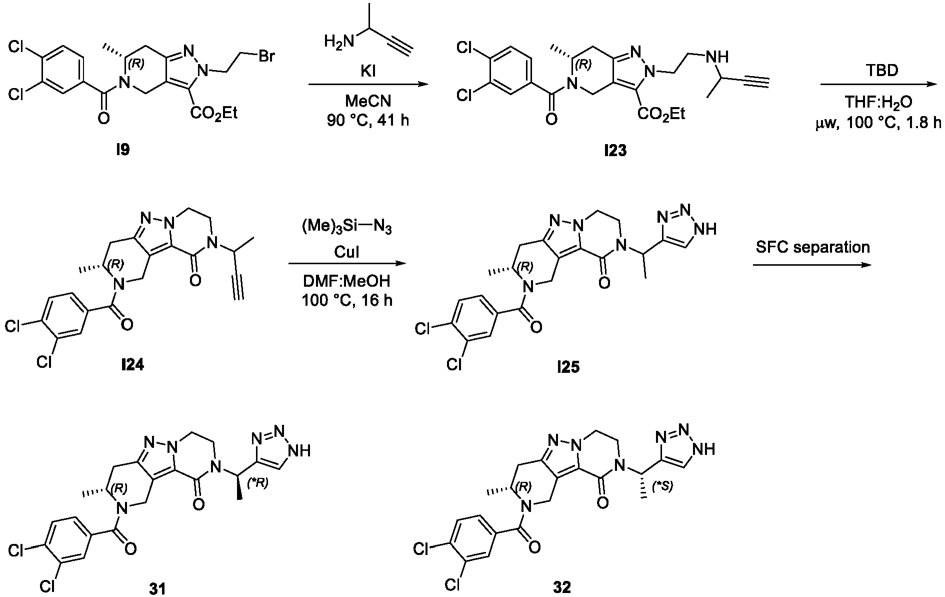

### Intermediate I23

### Ethyl (6R)-2-{2-[(but-3-yn-2-yl)amino]ethyl}-5-(3,4-dichlorobenzoyl)-6-methyl-4,5,6,7-tetrahydro-2H-pyrazolo[4,3-c]pyridine-3-carboxylate

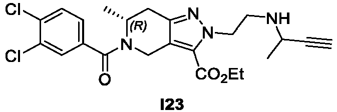

The reaction was performed under anhydrous conditions and under argon atmosphere.

Intermediate **I9** (214 mg, 0.42 mmol) was dissolved in MeCN (7.60 mL). KI (28.2 mg, 0.17 mmol) and 1-methyl-prop-2-ynylamine (179 µL, 2.12 mmol) were added. The reaction mixture was stirred at 90 °C for 24 h. An additional quantity of KI (28.2 mg, 0.17 mmol) was added and the reaction mixture was stirred at 90 °C for 17 h. 1-Methyl-prop-2-ynylamine (71.5 µL, 0.85 mmol) was added and the reaction mixture was stirred at 90 °C for another 2 h. The reaction mixture was transferred into a sealed tube, 1-methyl-prop-2-ynylamine (179 µL, 2.12 mmol) was added and the reaction mixture was stirred at 90 °C for 16 h. The reaction mixture was diluted in water (30 mL) and extracted with EtOAc (3 × 50 mL). The combined organic layers were dried (Na₂SO₄), filtered and concentrated under reduced pressure. The crude mixture was purified by flash column chromatography (silica, DCM/MeOH, gradient from 100:0 to 95:5) to afford intermediate **I23** (177 mg, 78%, 89% purity) as a yellowish gum.

### Intermediate I24

### (3R)-9-(But-3-yn-2-yl)-2-(3,4-dichlorobenzoyl)-3-methyl-1,2,3,4,8,9-hexahydropyrido[4',3':3,4]-pyrazolo[1,5-a]pyrazin-10(7H)-one

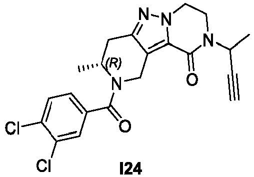

The reaction was performed under anhydrous conditions and under argon atmosphere.

Intermediate **I23** (177 mg, 0.33 mmol, 89% purity) was dissolved in MeCN (0.6 mL) and THF (2.92 mL). TBD (13.8 mg, 0.10 mmol) was added and the reaction mixture was stirred at 100 °C under microwave irradiations for 1.8 h. Additional quantity of TBD (13.8 mg, 0.10 mmol) was added and the reaction mixture was stirred at 100 °C under microwave irradiations for another 1.8 h. The reaction mixture was concentrated under reduced pressure. The crude mixture was purified by flash column chromatography (silica, DCM/MeOH, gradient from 100:0 to 95:5) to afford intermediate **I24** (156 mg, 84%, 77% purity).

### Compounds 31 and 32

### (3R)-2-(3,4-Dichlorobenzoyl)-3-methyl-9-[(1*R)-1-(1H-1,2,3-triazol-4-yl)ethyl]-1,2,3,4,8,9-hexahydropyrido[4',3':3,4]pyrazolo[1,5-a]pyrazin-10(7H)-one

### (3R)-2-(3,4-Dichlorobenzoyl)-3-methyl-9-[(1*S)-1-(1H-1,2,3-triazol-4-yl)ethyl]-1,2,3,4,8,9-hexahydropyrido[4',3':3,4]pyrazolo[1,5-a]pyrazin-10(7H)-one

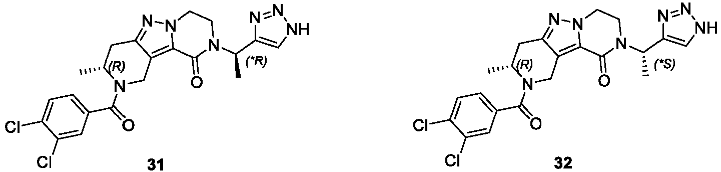

The reaction was performed under anhydrous conditions and under argon atmosphere.

Intermediate **I24** (126 mg, 0.22 mmol, 77% purity) was dissolved in DMF (1.35 mL) and MeOH (150 µL). CuI (2.14 mg, 0.01 mmol) and trimethylsilylazide (44.3 µL, 0.34 mmol) were added. The reaction mixture was stirred at 100 °C for 16 h. The mixture was combined with another fraction (0.05 mmol) and concentrated to dryness. The residue was diluted with EtOAc (5 mL) and washed with water (3 × 5 mL). The organic layer was dried (Na₂SO₄), filtered and concentrated under reduced pressure. The crude mixture was purified by flash column chromatography (silica, DCM/MeOH, gradient from 100:0 to 95:5). The residue was co-evaporated with EtOAc (3 × 2 mL) and dried under vacuum at 50°C to afford compound **I25** (77 mg, 72%) as a white solid.

The diastereoisomers were separated via Prep SFC (stationary phase: Chiralpak Daicel ID 20 x 250 mm, mobile phase: CO₂, EtOH + 0.4% i-PrNH₂) to afford compounds **31** and **32.**

### Compound 31

**¹H NMR** (400 MHz, DMSO-*d*₆, 81°C) δ ppm 14.10 - 14.97 (m, 1H), 7.80 (s, 1H), 7.66 - 7.72 (m, 2H), 7.41 (dd, *J*=8.1, 2.0 Hz, 1H), 5.89 (q, *J*=7.0 Hz, 1H), 4.87 - 5.25 (m, 1H), 4.44 - 4.83 (m, 1H), 4.15 - 4.35 (m, 3H), 3.66 - 3.75 (m, 1H), 3.40 - 3.46 (m, 1H), 2.93 (dd, *J*=16.0, 5.8 Hz, 1H), 2.56 (d, *J*=15.8 Hz, 1H), 1.55 (d, *J*=7.0 Hz, 3H), 1.16 (d, *J*=6.8 Hz, 3H); **LCMS** (method D): Rt = 1.65 min, *m*/*z* calcd. for C₂₁H₂₁Cl₂N₇O₂ 473.1, *m*/*z* found 474.2 [M+H]⁺.

### Compound 32

**¹H NMR** (400 MHz, DMSO-*d*₆, 81°C) δ ppm 14.45 - 14.80 (m, 1H), 7.78 (s, 1H), 7.66 - 7.72 (m, 2H), 7.41 (dd, *J*=8.1, 2.0 Hz, 1H), 5.89 (q, *J*=7.0 Hz, 1H), 4.86 - 5.30 (m, 1H), 4.42 - 4.83 (m, 1H), 4.23 - 4.32 (m, 2H), 4.13 - 4.21 (m, 1H), 3.67 - 3.76 (m, 1H), 3.37 - 3.49 (m, 1H), 2.93 (dd, *J*=15.8, 5.7 Hz, 1H), 2.56 (d, *J*=15.8 Hz, 1H), 1.56 (d, *J*=7.0 Hz, 3H), 1.16 (d, *J*=6.8 Hz, 3H); **LCMS** (method D): Rt = 1.65 min, *m*/*z* calcd. for C₂₁H₂₁Cl₂N₇O₂ 473.1, *m*/*z* found 474.2 [M+H]⁺.

### 3.2. SYNTHESIS OF THE 7-MEMBERED RINGS

### 3.2.1. Synthesis of Intermediate 126

### Ethyl 2-{3-[(tert-butoxycarbonyl)amino]propyl}-5-(3,4-dichlorobenzoyl)-4,5,6,7-tetrahydro-2H-pyrazolo[4,3-c]pyridine-3-carboxylate

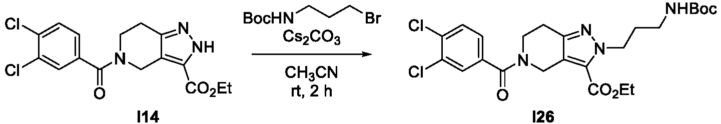

A mixture of intermediate **I14** (1.00 g, 2.70 mmol), Cs₂CO₃ (1.32 g, 4.05 mmol) and 3-(boc-amino)propyl bromide (676 mg, 2.84 mmol) in MeCN (30 mL) was stirred at room temperature for 2 h. The mixture was filtered and the filtrate was concentrated under reduced pressure. The crude mixture was purified by flash column chromatography (silica, heptane/(EtOAc/EtOH,3:1), gradient from 90:10 to 0:100) to afford intermediate **I26** (850 mg, 60%).

### 3.2.2. Synthesis of Compounds

### 3.2.2.1. Synthesis of Compound 33

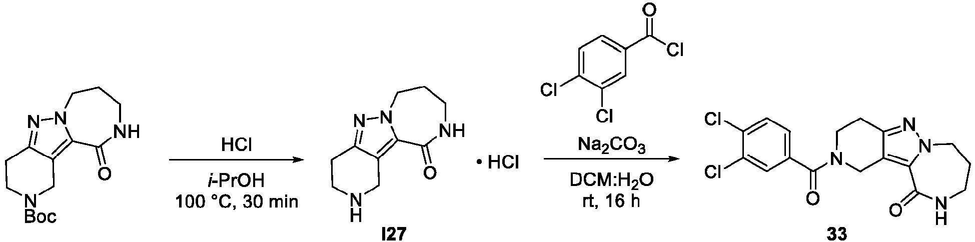

### Intermediate I27

### 1,2,3,4,7,8,9,10-Octahydro-11H-pyrido[4',3':3,4]pyrazolo[1,5-a][1,4]diazepin-11-one hydrochloride

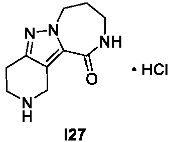

A pressure tube was charged with tert-butyl 11-oxo-3,4,7,8,9,10-hexahydro-1*H*-pyrido[2,3]-pyrazolo[2,4-*b*][1,4]diazepine-2-carboxylate (250 mg, 0.78 mmol) in *i*-PrOH (5 mL). HCl (6M in *i*-PrOH, 1.3 mL, 7.80 mmol) was added and the reaction mixture was stirred at 100 °C for 30 min. The mixture was concentrated under reduced pressure. The residue was triturated in DIPE. The solids were collected by filtration and dried to afford intermediate **I27** (200 mg, quant.) as a white solid.

### Compound 33

### 2-(3,4-Dichlorobenzoyl)-1,2,3,4,7,8,9,10-octahydro-11H-pyrido[4',3':3,4]pyrazolo[1,5-a][1,4]-diazepin-11-one

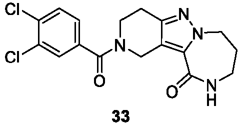

A pressure tube was charged with intermediate **I27** (198 mg, 0.82 mmol), 3,4-dichlorobenzoyl chloride (185 mg, 0.86 mmol), DCM (2.6 mL), water (2.61 mL) and Na₂CO₃ (432 mg, 4.08 mmol). The reaction mixture was stirred at room temperature for 16 h. The precipitate was collected by filtration and washed with water and DIPE. The product was dried under vacuum to afford compound **33** (220 mg, 71%) as a white solid.

**¹H NMR** (400 MHz, DMSO-*d*₆, 100°C) δ ppm 7.72 (br s, 1 H), 7.66 (d, *J*=8.1 Hz, 1H), 7.63 (d, *J*=2.0 Hz, 1H), 7.39 (dd, *J*=8.1, 2.0 Hz, 1H), 4.63 (s, 2H), 4.34 (t, *J*=6.4 Hz, 2H), 3.62 - 3.78 (m, 2H), 3.19 - 3.27 (br s, 2H), 2.70 (t, *J*=5.8 Hz, 2H), 2.09 - 2.16 (m, 2H); **LCMS** (method C): Rt = 0.79 min, *m*/*z* calcd. for C₁₇H₁₆Cl₂N₄O₂ 378.0, found 379.1 [M+H]⁺; **m.p** 237 °C.

### 3.2.2.2. Synthesis of Compound 34

### 2-(3,4-Dichlorobenzoyl)-10-methyl-1,2,3,4,7,8,9,10-octahydro-11H-pyrido[4',3':3,4]pyrazolo-[1,5-a][1,4]diazepin-11-one

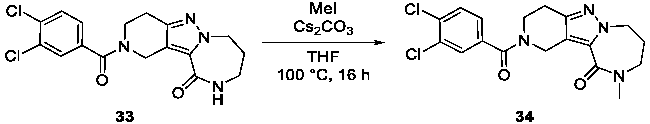

Compound **33** (60.0 mg, 158 µmol), MeI (19.7 µL, 0.32 mmol) and Cs₂CO₃ (103 mg, 0.32 mmol) were dispensed into a microwave vial. THF (2 mL) was added and the reaction mixture was stirred at 100 °C for 16 h. The mixture was concentrated under reduced pressure. The residue was partitioned between water and 2-MeTHF. The layers were separated and the organic phase was dried (MgSO₄), filtered and concentrated under reduced pressure. The crude mixture was purified by flash column chromatography (silica, heptane/(EtOAc/EtOH, 3:1), gradient from 100:0 to 0:100). The product was triturated in DIPE and MeCN. The solids were collected by filtration and dried under vacuum to afford compound **34** (29 mg, 47%) as a pale yellow solid.

**¹H NMR** (400 MHz, DMSO-*d*₆, 100°C) δ ppm 7.67 (d, *J*=8.1 Hz, 1H), 7.64 (d, *J*=2.0 Hz, 1H), 7.40 (dd, *J*=8.4, 2.0 Hz, 1H), 4.60 (s, 2H), 4.28 (t, *J*=6.8 Hz, 2H), 3.63 - 3.77 (m, 2H), 3.36 - 3.41 (m, 2H), 3.00 (s, 3H), 2.70 (t, *J*=5.9 Hz, 2H), 2.16 - 2.23 (m, 2H); **LCMS** (method C): Rt = 0.83 min, *m*/*z* calcd. for C₁₈H₁₈Cl₂N₄O₂ 392.1, found 393.1 [M+H]⁺.

### 3.2.2.4. Synthesis of Compound 36

### 2-(3,4-Dichlorobenzoyl)-10-[(4-methoxyphenyl)methyl]-1,2,3,4,7,8,9,10-octahydro-11H-pyrido[4',3':3,4]pyrazolo[1,5-a][1,4]diazepin-11-one

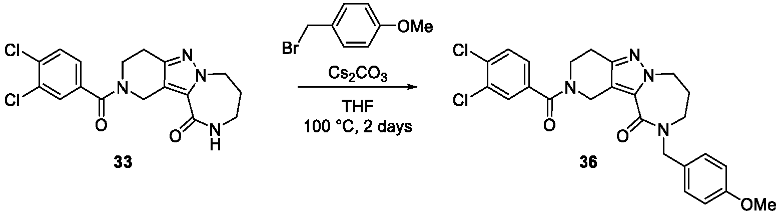

Compound **36** was obtained according to the procedure reported for the synthesis of compound **34.**

The crude mixture was purified by flash column chromatography (silica, heptane/(EtOAc/EtOH, 3:1), gradient from 100:0 to 0:100). The product was triturated in DIPE. The solids were collected by filtration and dried under vacuum to afford compound **36** (31 mg, 39%) as a white solid.

**¹H NMR** (400 MHz, DMSO-*d*₆, 100°C) δ ppm 7.67 (d, *J*=8.1 Hz, 1H), 7.64 (d, *J*=2.0 Hz, 1H), 7.40 (dd, *J*=8.3, 1.9 Hz, 1H), 7.22 - 7.28 (m, 2H), 6.86 - 6.92 (m, 2H), 4.64 (s, 2H), 4.57 (s, 2H), 4.24 (t, *J*=6.8 Hz, 2H), 3.75 (s, 3H), 3.67 - 3.74 (m, 2H), 3.31 - 3.37 (m, 2H), 2.71 (t, *J*=5.8 Hz, 2H), 1.97 - 2.05 (m, 2H); **LCMS** (method C): Rt = 1.04 min, *m*/*z* calcd. for C₂₅H₂₄Cl₂N₄O₃ 498.1, found 499.1 [M+H]⁺; **m.p** 236 °C.

### 3.2.2.5. Synthesis of Compound 37

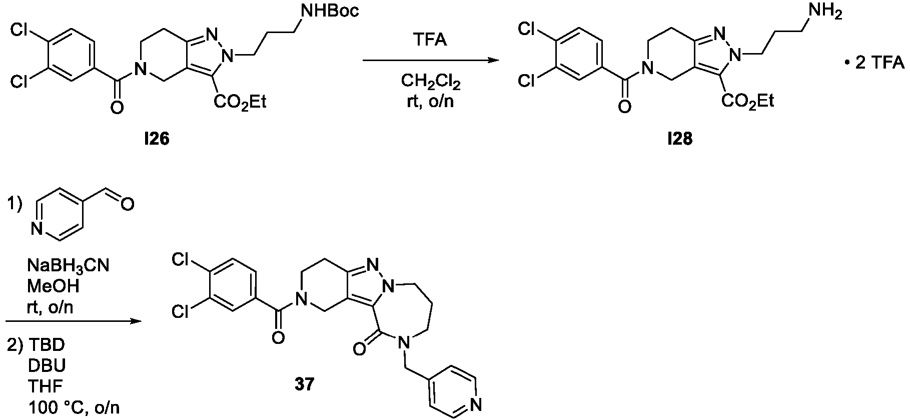

### Intermediate I28

### Ethyl 2-(3-aminopropyl)-5-(3,4-dichlorobenzoyl)-4,5,6,7-tetrahydro-2H-pyrazolo[4,3-c]-pyridine-3-carboxylate • 2TFA

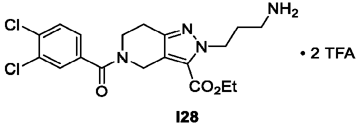

TFA (1.24 mL, 16.2 mmol) was added to a solution of intermediate **I26** (850 mg, 1.62 mmol) in DCM (100 mL). The reaction mixture was stirred overnight at room temperature and concentrated under reduced pressure to afford intermediate **I28** which was used as such in the next step.

### Compound 37

### 2-(3,4-Dichlorobenzoyl)-10-[(pyridin-4-yl)methyl]-1,2,3,4,7,8,9,10-octahydro-11H-pyrido-[4',3':3,4]pyrazolo[1,5-a][1,4]diazepin-11-one

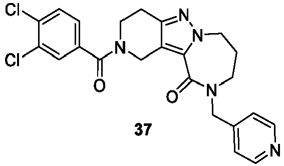

Sodium cyanoborohydride (56.6 mg, 0.86 mmol) was added to a mixture of intermediate **I28** (373 mg, 0.57 mmol) and 4-pyridinecarboxaldehyde (61.2 mg, 0.57 mmol) in MeOH (10 mL). The reaction mixture was stirred at room temperature for 4 h. Additional amounts of 4-pyridinecarboxaldehyde (61.2 mg, 0.57 mmol) and sodium cyanoborohydride (56.6 mg, 0.86 mmol) were added. The reaction mixture was stirred overnight and concentrated under reduced pressure.

The residue was dissolved in THF (3mL). TBD (23.8 mg, 0.17 mmol) and DBU (0.43 mL, 2.85 mmol) were added and the reaction mixture was stirred at 100 °C overnight in a pressure tube. The mixture was concentrated under reduced pressure and the crude mixture was purified by flash column chromatography (silica, heptane/(EtOAc/EtOH, 3:1), gradient from 80:20 to 0:100) to afford compound **37** (99 mg, 37%).

**¹H NMR** (400 MHz, DMSO-*d*₆, 101°C) δ ppm 8.47 - 8.54 (m, 2H), 7.67 (d, *J*=8.1 Hz, 1H), 7.64 (d, *J*=1.8 Hz, 1H), 7.40 (dd, *J*=8.3, 1.9 Hz, 1H), 7.27 (d, *J*=5.7 Hz, 2H), 4.68 (s, 2H), 4.64 (s, 2H), 4.32 (t, *J*=6.7 Hz, 2H), 3.64 - 3.78 (m, 2H), 3.43 (dd, *J*=6.8, 5.5 Hz, 2H), 2.73 (t, *J*=5.9 Hz, 2H), 2.09 - 2.17 (m, 2H); **LCMS** (method B): Rt = 1.66 min, *m*/*z* calcd. for C₂₃H₂₁Cl₂N₅O₂ 469.0, found 470.2 [M+H]⁺.

### 3.2.2.6. Synthesis of Compound 38

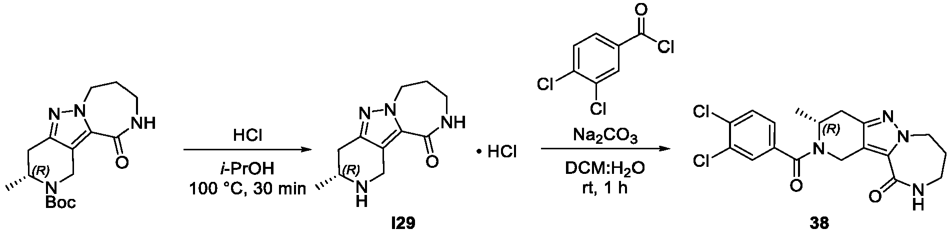

### Intermediate I29

### (3R)-3-Methyl-1,2,3,4,7,8,9,10-octahydro-11H-pyrido[4',3':3,4]pyrazolo[1,5-a][1,4]diazepin-11-one hydrochloride

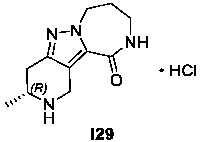

A pressure tube was charged with *tert*-butyl (3R)-3-methyl-11-oxo-3,4,7,8,9,10-hexahydro-1*H-*pyrido[2,3]pyrazolo[2,4-*b*][1,4]diazepine-2-carboxylate (250 mg, 0.78 mmol) in *i*-PrOH (5 mL). HCl (6M in *i*-PrOH, 1.3 mL, 7.80 mmol) was added and the reaction mixture was stirred at 100 °C for 30 min. The mixture was concentrated under reduced pressure. The residue was triturated in DIPE. The solids were collected by filtration and dried to afford intermediate **I29** (200 mg, quant.) as a white solid.

### Compound 38

### (3R)-2-(3,4-Dichlorobenzoyl)-3-methyl-1,2,3,4,7,8,9,10-octahydro-11H-pyrido[4',3':3,4]-pyrazolo[1,5-a][1,4]diazepin-11-one

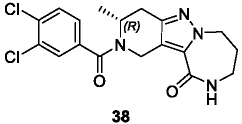

A pressure tube was charged with intermediate **I29** (200 mg, 0.78 mmol), 3,4-dichlorobenzoyl chloride (177 mg, 0.82 mmol), DCM (2.5 mL, 39 mmol), and water (2.5 mL). Na₂CO₃ (413 mg, 3.90 mmol) was added portionwise and the reaction mixture was stirred at room temperature for 1h. The layers were separated and the organic phase was dried (MgSO₄), filtered and concentrated under reduced pressure. The crude mixture was purified by flash column chromatography (silica, heptane/(EtOAc/EtOH, 3:1), gradient from 100:0 to 0:100). The product was triturated in DIPE, collected by filtration and dried under vacuum to afford compound **38** (172 mg, 56%) as a white fluffy solid.

**¹H NMR** (400 MHz, DMSO-*d*₆, 100°C) δ ppm 7.74 (br s, 1H), 7.66 (d, *J*=8.4 Hz, 1H), 7.62 (d, *J*=1.8 Hz, 1H), 7.37 (dd, *J*=8.3, 1.9 Hz, 1H), 4.87 - 5.12 (m, 1H), 4.47 - 4.74 (m, 1H), 4.29 - 4.41 (m, 2H), 4.20 (br d, *J*=17.4 Hz, 1H), 3.21 - 3.28 (m, 2H), 2.94 (s, 1H), 2.90 (dd, *J*=15.8, 5.9 Hz, 1H), 2.52 (d, *J*=15.8 Hz, 1H), 2.09 - 2.18 (m, 2H), 1.14 (d, *J*=6.8 Hz, 3H); **LCMS** (method C): Rt = 0.83 min, *m*/*z* calcd. for C₁₈H₁₈Cl₂N₄O₂ 392.1, found 393.2 [M+H]⁺.

### 3.2.2.7. Synthesis of Compound 39

### (3R)-2-(3,4-Dichlorobenzoyl)-3-methyl-10-(2-methylpropyl)-1,2,3,4,7,8,9,10-octahydro-11H-pyrido[4',3':3,4]pyrazolo[1,5-a][1,4]diazepin-11-one

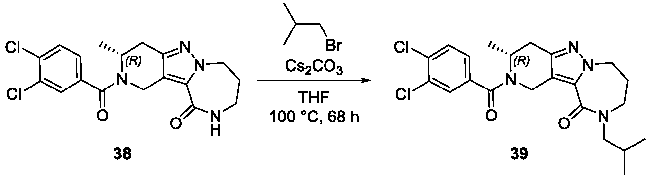

Compound **39** was obtained according to the procedure reported for the synthesis of compound **34.**

The crude mixture was purified by flash column chromatography (silica, heptane/EtOAc, gradient from 100:0 to 0:100) to afford compound **39** (45 mg, 66%).

**¹H NMR** (400 MHz, CDCl₃, 57°C) δ ppm 7.43 - 7.54 (m, 2H), 7.23 (dd, *J*=8.1, 1.6 Hz, 1H), 5.28 (s, 1H), 4.56 - 5.55 (m, 1H), 4.28 - 4.48 (m, 3H), 3.32 - 3.53 (m, 3H), 3.18 - 3.30 (m, 1H), 2.91 - 3.07 (m, 1H), 2.62 (br d, *J*=15.9 Hz, 1H), 2.21 - 2.32 (m, 2H), 1.90 - 2.05 (m, 1H), 1.25 (br d, *J*=6.9 Hz, 3H), 0.92 - 1.01 (m, 6H); **LCMS** (method D): Rt = 2.02 min, *m*/*z* calcd. for C₂₂H₂₆Cl₂N₄O₂ 448.1, found 449.3 [M+H]⁺.

### 3.2.2.8. Synthesis of Compound 40

### (3R)-2-(3,4-Dichlorobenzoyl)-10-[(4-methoxyphenyl)methyl]-3-methyl-1,2,3,4,7,8,9,10-octahydro-11H-pyrido[4',3':3,4]pyrazolo[1,5-a][1,4]diazepin-11-one

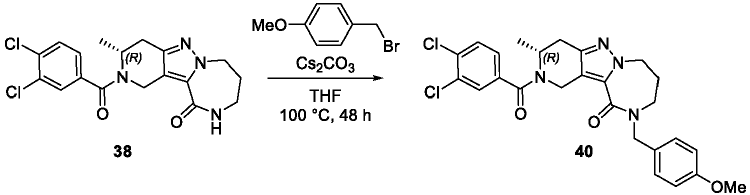

Compound **40** was obtained according to the procedure reported for the synthesis of compound **34.**

The crude mixture was purified by flash column chromatography (silica, heptane/(EtOAc/EtOH, 3:1), gradient from 100:0 to 0:100). Another purification was performed via Prep SFC (stationary phase: Chiralcel Diacel OJ 20 × 250 mm, mobile phase: CO₂, EtOH + 0.4% i-PrNH₂) to afford compound **40** (38 mg, 29%).

**¹H NMR** (400 MHz, DMSO-*d*₆, 100°C) δ ppm 7.67 (d, *J*=8.1 Hz, 1H), 7.64 (d, *J*=2.0 Hz, 1H), 7.38 (dd, *J*=8.3, 1.9 Hz, 1H), 7.24 - 7.29 (m, 2H), 6.86 - 6.92 (m, 2H), 4.87 - 5.13 (m, 1H), 4.52 - 4.71 (m, 3H), 4.18 - 4.29 (m, 3H), 3.75 (s, 3H), 3.31 - 3.39 (m, 2H), 2.91 (dd, *J*=16.2, 6.1 Hz, 1H), 2.52 (d, *J*=16.1 Hz, 1H), 1.97 - 2.05 (m, 2H), 1.16 (d, *J*=6.8 Hz, 3H); **LCMS** (method D): Rt = 2.04 min, *m*/*z* calcd. for C₂₆H₂₆Cl₂N₄O₃ 512.1, found 513.3 [M+H]⁺.

### 3.2.2.9. Synthesis of Compound 41

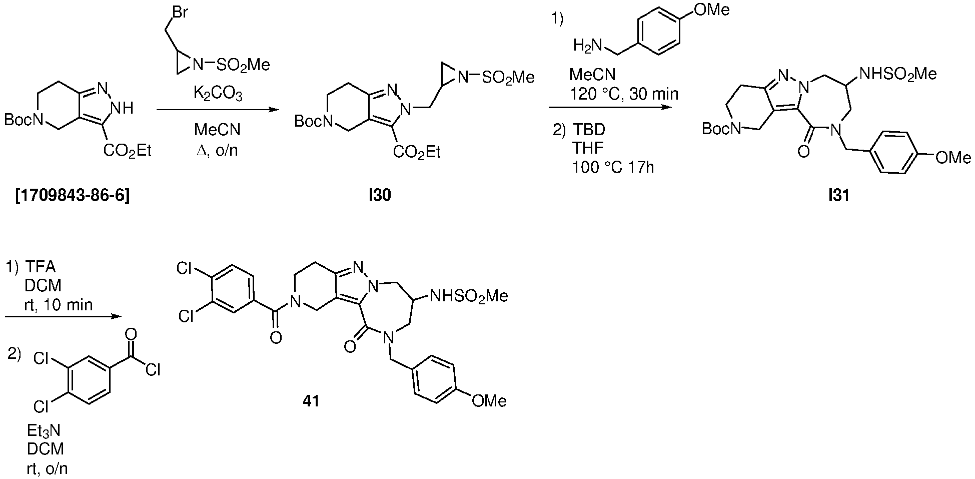

### Intermediate I30

### 5-tert-Butyl 3-ethyl 2-{ [1-(methanesulfonyl)aziridin-2-yl]methyl }-2,4,6,7 -tetrahydro-5H-pyrazolo[4,3-c]pyridine-3,5-dicarboxylate

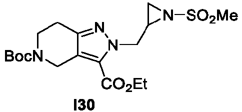

A mixture of 5-*tert* butyl 3-ethyl-3,7-dihydro-2*H*-pyrazolo[4,3-*c*]pyridine-3,5(4*H*)-dicarboxylate (2.55 g, 8.63 mmol), epibromohydrin (5.00 g, 23.4 mmol) and K₂CO₃ (3.58 g, 25.9 mmol) in MeCN (75 mL) was stirred under reflux overnight. The reaction mixture was filtered over decalite while still hot. The filtrate was concentrated under reduced pressure and the residue was purified by flash column chromatography (silica, heptane/EtOAc, gradient from 80:20 to 0:100) to afford intermediate **I30** (850 mg, 23%).

### Intermediate 31

### tert-Butyl 8-[(methanesulfonyl)amino]-10-[(4-methoxyphenyl)methyl]-11-oxo-1,3,4,7,8,9,10,11-octahydro-2H-pyrido[4',3':3,4]pyrazolo[1,5-a][1,4]diazepine-2-carboxylate

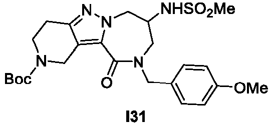

A mixture of intermediate **I30** (600 mg, 1.40 mmol), 4-methoxybenzyl bromide (192 mg, 1.40 mmol) in MeCN (5 mL) was stirred at 120 °C for 30 min. The reaction mixture was concentrated under reduced pressure and the crude mixture was purified by flash column chromatography (silica, heptane/(EtOAc/EtOH, 3:1), gradient from 80:20 to 0:100). The residue (275 mg) was dissolved in THF (2 mL) and TBD (58.5 mg, 0.42 mmol) was added. The reaction mixture was stirred at 100 °C in a sealed tube for 17 h. The mixture was concentrated under reduced pressure and the crude mixture was purified by flash column chromatography (silica, heptane/(EtOAc/EtOH, 3:1), gradient from 80:20 to 0:100) to afford intermediate **I31** (86 mg, 12%) as a clear oil.

### Compound 41

### N-{2-(3,4-Dichlorobenzoyl)-10-[(4-methoxyphenyl)methyl]-11-oxo-1,3,4,7,8,9,10,11-octahydro-2H-pyrido[4',3':3,4]pyrazolo[1,5-a][1,4]diazepin-8-yl}methanesulfonamide

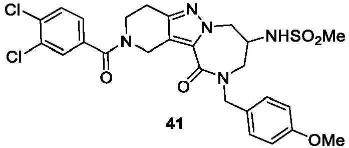

TFA (0.13 mL, 1.66 mmol) was added to a solution of intermediate **I31** (86.0 mg, 0.17 mmol) in DCM (10 mL) and the reaction mixture was stirred at room temperature for 10 min. The mixture was concentrated under reduced pressure. The residue was dissolved in DCM (20 mL). Et₃N (92 µL, 0.66 mmol) was added followed by 3,4-dichlorobenzoyl chloride (36.4 mg, 0.17 mmol) and the reaction mixture was stirred at room temperature overnight. The mixture was washed with water, dried (MgSO₄), filtered and concentrated under reduced pressure. The crude mixture was purified by flash column chromatography (silica, heptane/(EtOAC/EtOH, 3:1), gradient from 80:20 to 0:100). The residue was dissolved in MeOH (5 mL), and water was added dropwise until the solution stayed turbid. The mixture was stirred for 2 h. The precipitate was collected by filtration and dried under vacuum at 50 °C to afford compound **41** (61 mg, 62%).

**¹H NMR** (400 MHz, DMSO-*d*₆, 150°C) δ ppm 7.63 (d, *J*=8.1 Hz, 1H), 7.59 (d, *J*=1.8 Hz, 1H), 7.36 (dd, *J*=8.1, 2.0 Hz, 1H), 7.21 - 7.27 (m, 2H), 6.86 - 6.91 (m, 2H), 6.81 (br s, 1H), 4.81 (d, *J*=14.5 Hz, 1H), 4.58 - 4.69 (m, 2H), 4.39 - 4.49 (m, 2H), 4.24 - 4.31 (m, 1H), 3.91 - 4.01 (m, 1H), 3.76 (s, 3H), 3.70 - 3.75 (m, 2H), 3.40 - 3.48 (m, 1H), 3.26 - 3.34 (m, 1H), 2.90 (s, 3H), 2.74 (t, *J*=5.8 Hz, 2H); **LCMS** (method B): Rt = 1.84 min, *m*/*z* calcd. for C₂₆H₂₇Cl₂N₅O₅S 591.1, found 592.1 [M+H]⁺.

### 3.2.2.10. Synthesis of Compound 42

### a) Synthesis of Intermediate I34

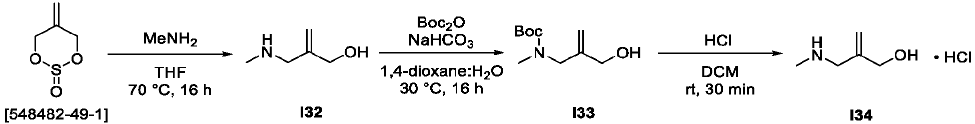

### Intermediate I32

### 2-((Methylamino)methyl)prop-2-en-1-ol

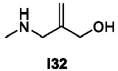

A mixture of 5-methylene-1,3,2-dioxathiane 2-oxide (20.0 g, 149 mmol) in methanamine (2M in THF, 223 mL) in a sealed tube was stirred at 70 °C for 16 h. The mixture was filtered and the filtrate was concentrated under reduced pressure to give intermediate **I32** as yellow liquid which was used as such in the next step.

### Intermediate I33

### tert-Butyl (2-(hydroxymethyl)allyl)(methyl)carbamate

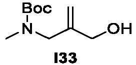

To a solution of intermediate **I32** in 1,4-dioxane (60 mL) and H₂O (60 mL) were added Boc₂O (22.7 g, 104 mmol) and NaHCO₃ (12.5 g, 148 mmol). The reaction mixture was stirred at 30 °C for 16 h. The mixture was diluted with EtOAc (400 mL) and washed with brine (3 × 80 mL). The organic layer was dried (Na₂SO₄), filtered and concentrated under reduced pressure. The crude mixture was purified by flash column chromatography (silica, petroleum ether/EtOAc, gradient from 20:1 to 1:1) to give intermediate **I33** (12.5 g, 42% over 2 steps) as light yellow liquid.

### Intermediate I34

### 2-((Methylamino)methyl)prop-2-en-1-ol hydrochloride

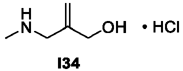

To a solution of intermediate **I33** (9.50 g, 47.2 mmol) in DCM (20 mL) was added HCl (4M in 1,4-dioxane, 30 mL) at 0 °C. The reaction mixture was stirred at 20 °C for 30 min and concentrated under reduced pressure to afford intermediate **I34** as yellow oil which was used in the next step without further purification.

### b) Synthesis of Compound 42

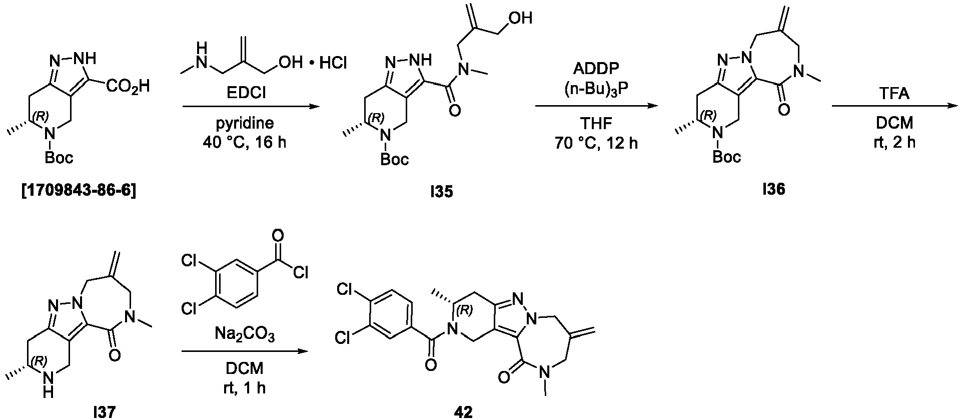

### Intermediate I35

### (R)-tert-Butyl 3-((2-(hydroxymethyl)allyl)(methyl)carbamoyl)-6-methyl-6,7-dihydro-2H-pyrazolo[4,3-c]pyridine-5(4H)-carboxylate

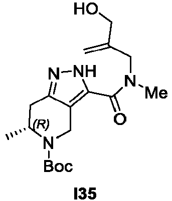

To a mixture of (*R*)-5-(tert-butoxycarbonyl)-6-methyl-4,5,6,7-tetrahydro-2*H*-pyrazolo[4,3-*c*]-pyridine-3-carboxylic acid (4.09 g, 14.5 mmol) and intermediate **I34** (2.0 g) in pyridine (15 mL) was added EDCI (4.18 g, 21.8 mmol). The reaction mixture was stirred at 40 °C for 16 h and diluted with EtOAc (400 mL). The resulting solution was washed with HCl (1N, aq.) (4 × 30 mL), dried (Na₂SO₄), filtered and concentrated under reduced pressure. The crude mixture was purified by flash column chromatography (silica, petroleum ether/EtOAc, gradient from 1:1 to 0:1). The residue was triturated with EtOAc and petroleum ether (1:1, 10 mL) to give intermediate **I35** (2.2 g, 35%, 85% purity) as light yellow solid.

### Intermediate I36

### (R)-tert-Butyl 3,10-dimethyl-8-methylene-11-oxo-3,4,8,9,10,11-hexahydro-1H-pyrido[4',3':3,4]-pyrazolo[1,5-a][1,4]diazepine-2(7H)-carboxylate

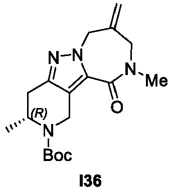

To a mixture of intermediate **I35** (1.60 g, 3.73 mmol) in THF (2 mL) were added ADDP (1.88 g, 7.46 mmol) and tributylphosphine (1.51 g, 7.46 mmol) under N₂. The reaction mixture was stirred at 70 °C for 12 h and diluted with water (100 mL). The layers were separated and the aqueous phase was extracted with EtOAc (2 × 150 mL). The combined organic extracts were washed with brine (40 mL), dried (Na₂SO₄), filtered and concentrated under reduced pressure. The crude mixture was purified by flash column chromatography (silica, petroleum ether/EtOAc, gradient from 5:1 to 0:1) to give intermediate **I36** (1.7 g, 61%, 94% purity) as white solid.

### Intermediate I37

### (3R)-3,10-Dimethyl-8-methylidene-1,2,3,4,7,8,9,10-octahydro-11H-pyrido[4',3':3,4]pyrazolo-[1,5-a][1,4]diazepin-11-one • TFA

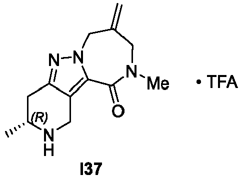

A sealed tube was charged with intermediate **I36** (108 mg, 0.31 mmol) and DCM (1 mL). TFA (0.24 mL, 3.13 mmol) was added and the reaction mixture was stirred at room temperature for 2 h. The mixture was concentrated under reduced pressure to afford intermediate **I37** which was used as such in the next step.

### Compound 42

### (3R)-2-(3,4-Dichlorobenzoyl)-3,10-dimethyl-8-methylidene-1,2,3,4,7,8,9,10-octahydro-11H-pyrido[4',3':3,4]pyrazolo[1,5-a][1,4]diazepin-11-one

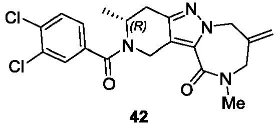

Intermediate **I37** was dissolved in DCM (1 mL) and water (1 mL). 3,4-Dichlorobenzoyl chloride (70.9 mg, 0.33 mmol) was added followed by Na₂CO₃ (166 mg, 1.56 mmol) portionwise over 10 min. The reaction mixture was stirred at room temperature for 1 h. The layers were separated and the organic phase was dried (MgSO₄), filtered and concentrated under reduced pressure. The crude mixture was purified by flash column chromatography (silica, heptane/EtOAc) A second purification was performed via Prep HPLC (stationary phase: RP XBridge Prep C18 OBD-10µm, 50 × 150mm, mobile phase: NH₄HCO₃ (0.25% in H₂O)/MeOH). The residue was co-evaporated with MeOH and dried under vacuum to give compound **42** (72 mg, 53% over 2 steps) as a fluffy white solid.

**¹H NMR** (400 MHz, DMSO-*d*₆, 100°C) δ ppm 7.67 (d, *J*=8.4 Hz, 1H), 7.63 (d, *J*=2.0 Hz, 1H), 7.38 (dd, *J*=8.1, 2.0 Hz, 1H), 5.12 - 5.20 (m, 2H), 4.97 (s, 2H), 4.55 - 4.70 (m, 1H), 4.21 (br d, *J*=17.2 Hz, 1H), 3.95 (s, 2H), 3.01 (s, 3H), 2.87 - 2.93 (m, 1H), 2.53 (d, *J*=16.1 Hz, 1H), 1.15 (d, *J*=6.8 Hz, 3H); **LCMS** (method C): Rt = 0.95 min, *m*/*z* calcd. for C₂₀H₂₀Cl₂N₄O₂ 418.2, found 419.2 [M+H]⁺.

### 3.2.2.11. Synthesis of Compound 43

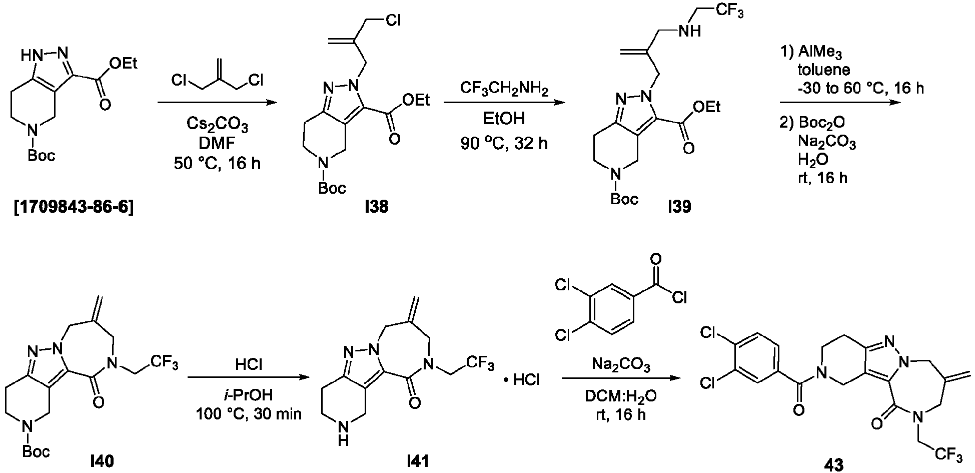

### Intermediate I38

### 5-tert-Butyl 3-ethyl 2-(2-(chloromethyl)allyl)-6,7-dihydro-2H-pyrazolo[4,3-c]pyridine-3,5(4H)-dicarboxylate

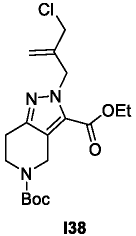

A mixture of 5-tert-butyl 3-ethyl 6,7-dihydro-1H-pyrazolo[4,3-c]pyridine-3,5(4H)-dicarboxylate (60.0 g, 203 mmol), 3-chloro-2-(chloromethyl)prop-1-ene (50.8 g, 406 mmol) and Cs₂CO₃ (99.3 g, 305 mmol) in DMF (600 mL) was degassed and purged with N₂ (3 times). The reaction mixture was stirred at 50 °C for 16 h under N₂ atmosphere. The mixture was poured into water (3 L) and stirred for 5 min. The aqueous phase was extracted with EtOAc (3 × 1 L). The combined organic extracts were washed with brine (3 L), dried (Na₂SO₄), filtered and concentrated under reduced pressure. The crude mixture was purified by flash column chromatography (silica, petroleum ether/EtOAc, gradient from 100:1 to 10:1) to give intermediate **I38** (30.00 g, 38%) as a yellow oil.

### Intermediate I39

### 5-tert-Butyl 3-ethyl 2-(2-(((2,2,2-trifluoroethyl)amino)methyl)allyl)-6,7-dihydro-2H-pyrazolo[4,3-c]pyridine-3,5(4H)-dicarboxylate

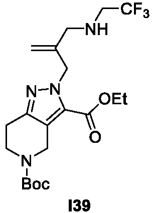

A solution of intermediate **I38** (500 mg, 1.30 mmol) and 2,2,2-trifluoroethanamine (3.86 g, 39.0 mmol) in EtOH (50.00 mL) was stirred at 90 °C for 32 h. The mixture was diluted with EtOAc (120 mL) and washed with NaHCO₃ (sat., aq., 120 mL). The organic phase was dried (Na₂SO₄), filtered and concentrated under reduced pressure. The crude mixture was purified by flash column chromatography (silica) to afford intermediate **I39** (460 mg, 79%) as yellow oil.

### Intermediate I40

### tert-Butyl 8-methylene-11-oxo-10-(2,2,2-trifluoroethyl)-3,4,8,9,10,11-hexahydro-1H-pyrido-[4',3':3,4]pyrazolo[1,5-a][1,4]diazepine-2(7H)-carboxylate

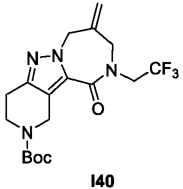

To a solution of intermediate **I39** (460.0 mg, 1.03 mmol) in toluene (10.00 mL) was added Al(Me)₃ (2M in toluene, 2.06 mL) at -30 °C. The reaction mixture was stirred at 60 °C for 16 h. The reaction was quenched with H₂O (10.00 mL). Na₂CO₃ (109.2 mg, 1.03 mmol) and Boc₂O (270 mg, 1.24 mmol) were added and the reaction mixture was stirred at 20 °C for 16 h. The mixture was diluted with EtOAc (50 mL) and washed with HCl (1M, aq., 50 mL). The organic phase was dried (Na₂SO₄), filtered and concentrated under reduced pressure. The crude mixture was purified by flash column chromatography (silica) to afford intermediate **I40** (390 mg, 95%) as yellow solid.

### Intermediate I41

### 8-Methylidene-10-(2,2,2-trifluoroethyl)-1,2,3,4,7,8,9,10-octahydro-11H-pyrido[4',3':3,4]-pyrazolo[1,5-a][1,4]diazepin-11-one hydrochloride

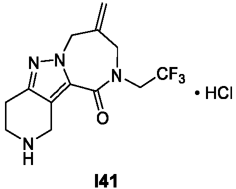

A sealed tube was charged with intermediate **I40** (214 mg, 0.51 mmol) and *i*-PrOH (3.26 mL). HCl (6M in *i*-PrOH, 0.85 mL, 5.10 mmol) was added and the reaction mixture was stirred at 100 °C for 30 min. The mixture was concentrated under reduced pressure. The residue was triturated in DIPE, filtered and dried to afford intermediate **I41** (169 mg, 95%) as a white solid.

### Compound 43

### 2-(3,4-Dichlorobenzoyl)-8-methylidene-10-(2,2,2-trifluoroethyl)-1,2,3,4,7,8,9,10-octahydro-11H-pyrido[4',3':3,4]pyrazolo[1,5-a][1,4]diazepin-11-one

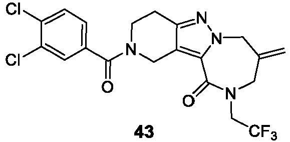

A sealed tube was charged with intermediate **I41** (169 mg, 0.48 mmol), 3,4-dichlorobenzoyl chloride (109 mg, 0.51 mmol), DCM (1.5 mL) and water (1.5 mL). Na₂CO₃ (255 mg, 2.41 mmol) was added portionwise and the reaction mixture was stirred at room temperature for 16 h. The layers were separated and the organic phase was dried (MgSO₄), filtered and concentrated under reduced pressure. The crude mixture was purified by flash column chromatography (silica, heptane/EtOAc, gradient from 100:0 to 0:100) to afford compound **43** (183 mg, 78%).

**¹H NMR** (400 MHz, DMSO-*d*₆, 100°C) δ ppm 8.66 (dd, *J*=4.8, 1.5 Hz, 1H), 8.44 (d, *J*=2.4 Hz, 1H), 7.69 - 7.73 (m, 1H), 7.65 - 7.69 (m, 2H), 7.56 (dd, *J*=8.0, 4.7 Hz, 1H), 7.41 (dd, *J*=8.1, 2.0 Hz, 1H), 7.12 - 7.18 (m, 2H), 6.78 - 6.83 (m, 2H), 6.42 (br s, 1H), 4.34 (br d, *J*=2.4 Hz, 2H), 4.29 (s, 2H), 3.72 (s, 3H), 3.58 - 3.66 (m, 2H), 2.41 (t, *J*=5.8 Hz, 2H); **LCMS** (method C): Rt = 1.04 min, *m*/*z* calcd. for C₂₇H₂₃Cl₂N₅O₃ 535.1, found 536.1 [M+H]⁺.

### 3.2.2.12. Synthesis of Compound 44

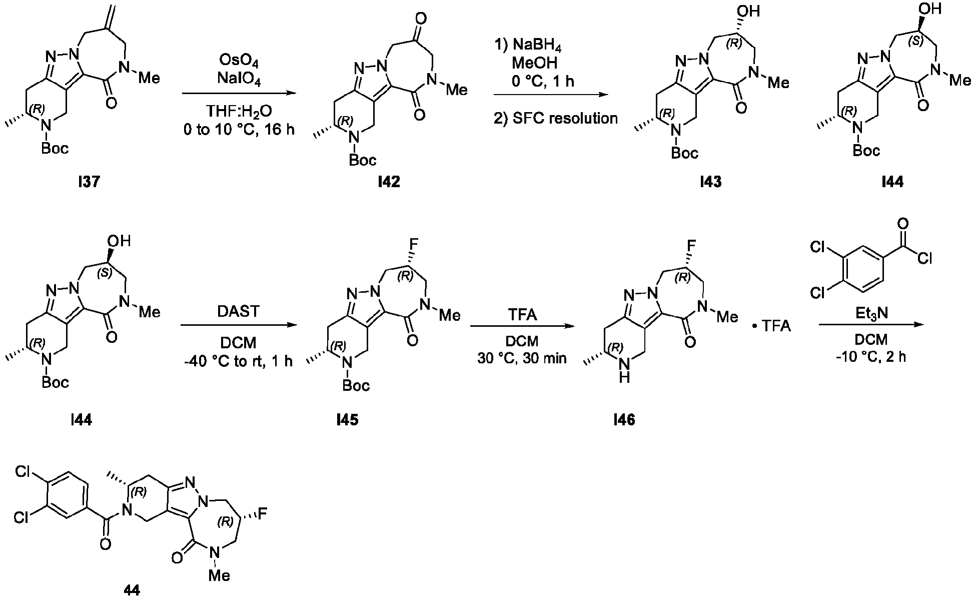

### Intermediate I42

### tert-Butyl (3R)-3,10-dimethyl-8,11-dioxo-1,3,4,7,8,9,10,11-octahydro-2H-pyrido[4',3':3,4]-pyrazolo[1,5-a][1,4]diazepine-2-carboxylate

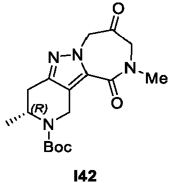

To a mixture of intermediate **I37** (1.30 g, 3.49 mmol) in THF (60 mL) and H₂O (20 mL) were added OsO₄ (177 mg, 698 µmol) and NaIO₄ (2.24 g, 10.5 mmol) at 0 °C. The reaction mixture was stirred at 10 °C for 16 h. The mixture combined with another fraction (2 g scale) and diluted with EtOAc (100 mL) and Na₂SO₃ (sat., aq, 100 mL). The aqueous phase was extracted with EtOAc (3 × 100 mL). The combined organic extracts were washed with brine (20 mL), dried (Na₂SO₄), filtered and concentrated under reduced pressure. The residue was purified by flash column chromatography (silica, petroleum ether/EtOAc, gradient from 1:1 to 0:1) to afford intermediate **I42** (2.9 g, 70% purity) as a yellow solid.

### Intermediates 143 and 144

### tert-Butyl (3R,8R)-8-hydroxy-3,10-dimethyl-11-oxo-1,3,4,7,8,9,10,11-octahydro-2H-pyrido-[4',3':3,4]pyrazolo[1,5-a][1,4]diazepine-2-carboxylate

### tert-Butyl (3R,8S)-8-hydroxy-3,10-dimethyl-11-oxo-1,3,4,7,8,9,10,11-octahydro-2H-pyrido-[4',3':3,4]pyrazolo[1,5-a][1,4]diazepine-2-carboxylate

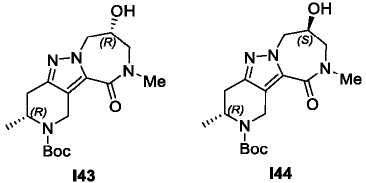

To a mixture of intermediate **I42** (2.9 g, 70% purity) in MeOH (30 mL) was added NaBH₄ (551 mg, 14.6 mmol) at 0 °C. The reaction mixture was stirred at 0 °C for 1 h and the reaction was quenched with H₂O (80 mL) at 0 °C. The mixture was extracted with EtOAc (3 × 80 mL). The combined organic layers were washed with brine (40 mL), dried (Na₂SO₄), filtered and concentrated under reduced pressure. The residue was combined with another batch (200 mg scale) and purified by flash column chromatography (silica, petroleum ether/EtOAc, gradient from 1:1 to 0:1) to give a mixture of intermediates **I43** and **I44** (1.95 g, 89% purity) as a yellow oil.

The diastereoisomers were separated by SFC (column: AD (250 mm × 30mm, 5 µm); mobile phase: CO₂/*i*-PrOH (with 1% NH₃ in H₂O), isocratic elution:80:20) to afford intermediate **I43** and intermediate **I44.**

SFC analysis: Column: Chiralpak AD-3 100×4.6mm I.D., 3µm; mobile phase: i-PrOH (0.05% DEA) in CO₂ from 5% to 40%; flow rate: 3mL/min; wavelength: 254nm
**I44:** Rt = 1.918 min

### Intermediate I45

### tert-Butyl (3R,8R)-8-fluoro-3,10-dimethyl-11-oxo-1,3,4,7,8,9,10,11-octahydro-2H-pyrido-[4',3':3,4]pyrazolo[1,5-a][1,4]diazepine-2-carboxylate

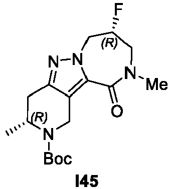

To a mixture of intermediate **I44** (700 mg, 1.70 mmol) in DCM (15 mL) was added DAST (1.09 g, 6.79 mmol) at -40 °C. The reaction mixture was stirred at room temperature for 1 h. The reaction was quenched with H₂O (20 mL) and NaHCO₃ (sat., aq, 20 mL). The layers were separated and the aqueous phase was extracted with EtOAc (3 × 80 mL). The combined organic extracts were washed with brine (40 mL), dried (Na₂SO₄), filtered and concentrated under reduced pressure. The crude mixture was combined with another batch (700 mg scale) and purified by flash column chromatography (silica, petroleum ether/EtOAc, gradient from 3:1 to 0:1) to afford intermediate **I45** (470 mg, 93% purity) as a light yellow solid.

### Intermediate I46

### (3R,8R)-8-Fluoro-3,10-dimethyl-1,2,3,4,7,8,9,10-octahydro-11H-pyrido[4',3':3,4]pyrazolo-[1,5-a][1,4]diazepin-11-one • TFA

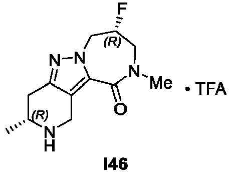

Intermediate **I46** was obtained according to the procedure reported for the synthesis of intermediate **I49.**

### Compound 44

### (3R,8R)-8-fluoro-3,10-dimethyl-1,2,3,4,7,8,9,10-octahydro-11H-pyrido[4',3':3,4]pyrazolo-[1,5-a][1,4]diazepin-11-one

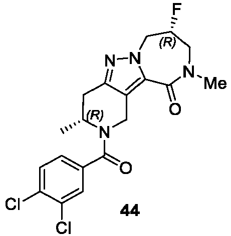

Compound **44** was obtained according to the procedure reported for the synthesis of compound **46.**

**MS** (ESI): *m*/*z* calcd. for C₁₉H₁₉Cl₂FN₄O₂ 424; *m*/*z* found, 425 [M+H]⁺; **¹H NMR** (400 MHz, DMSO-*d*₆) δ ppm 7.75 - 7.66 (m, 2H), 7.43 (dd, *J*=1.9, 8.2 Hz, 1H), 4.64 - 4.17 (m, 8H), 3.07 (br s, 3H), 2.95 (br dd, *J*=5.9, 15.9 Hz, 1H), 2.58 (br d, *J*=15.8 Hz, 1H), 1.17 (d, *J*=6.8 Hz, 3H).

### 3.2.2.15. Synthesis of Compound 48

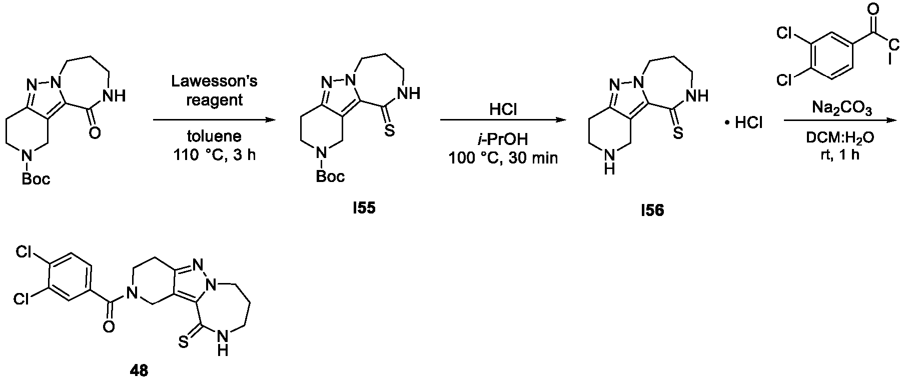

### Intermediate I55

### tert-Butyl 11-thioxo-3,4,8,9,10,11-hexahydro-1H-pyrido[4',3':3,4]pyrazolo[1,5-a][1,4]diazepine-2(7H)-carboxylate

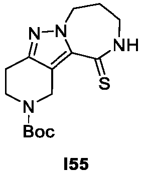

To a solution of *tert*-butyl 11-oxo-1,3,4,7,8,9,10,11-octahydro-2*H*-pyrido[4',3':3,4]pyrazolo-[1,5-*a*][1,4]diazepine-2-carboxylate (250 mg, 816 µmol) in toluene (5 mL) was added Lawesson's reagent (165 mg, 408 µmol). The reaction mixture was stirred at 110 °C for 3 h. The mixture was concentrated under reduced pressure and the residue was purified by prep-TLC (EtOAc) to afford intermediate **I55** (258 mg, 98%) as yellow solid.

### Intermediate I56

### 1,2,3,4,7,8,9,10-octahydro-11H-pyrido[4',3':3,4]pyrazolo[1,5-a] [1,4]diazepine-11-thione hydrochloride

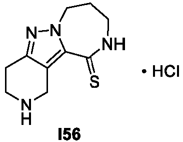

A sealed tube was charged with intermediate I55 (54.0 mg, 0.17 mmol) in *i*-PrOH (1.07 mL). HCl (6M in *i*-PrOH, 0.28 mL, 1.68 mmol) was added and the reaction mixture was stirred at 100 °C for 30 min. The mixture was concentrated under reduced pressure. The residue was triturated in DIPE, filtered and dried to afford intermediate **I56** (35 mg, 81%) as a yellow solid.

### Compound 48

### (3,4-dichlorophenyl)(11-sulfanylidene-1,3,4,7,8,9,10,11-octahydro-2H-pyrido[4',3':3,4]pyrazolo-[1,5-a][1,4]diazepin-2-yl)methanone

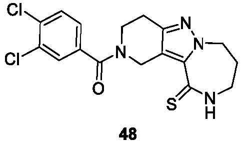

A sealed tube was charged intermediate **I56** (35 mg, 135 µmol), 3,4-dichlorobenzoyl chloride (30.7 mg, 0.14 mmol), DCM (0.5 mL) and water (0.5 mL). Na₂CO₃ (71.7 mg, 0.68 mmol) was added portionwise and the reaction mixture was stirred at room temperature for 1 h. The layers were separated and the organic phase was dried (MgSO₄), filtered and concentrated under reduced pressure. The crude mixture was purified by flash column chromatography (silica, heptane/(EtOAc/EtOH, 3:1), gradient from 100:0 to 0:100). The residue was triturated in DIPE, filtered and dried under vacuum to give compound **48** (31 mg, 58%) as a pale yellow solid.

**¹H NMR** (400 MHz, DMSO-*d*₆, 100°C) δ ppm 10.01 - 10.29 (m, 1H), 7.66 (d, *J*=8.1 Hz, 1H), 7.63 (d, *J*=2.0 Hz, 1H), 7.40 (dd, *J*=8.1, 2.0 Hz, 1H), 4.70 (s, 2H), 4.26 (t, *J*=6.9 Hz, 2H), 3.66 - 3.76 (m, 2H), 3.25 (q, *J*=6.2 Hz, 2H), 2.71 (t, *J*=5.9 Hz, 2H), 2.20 (quin, *J*=6.8 Hz, 2H); **LCMS** (method C): Rt = 1.01 min, *m*/*z* calcd. for C₁₇H₁₆Cl₂N₄OS 394.0, *m*/*z* found 395.1 [M+H]⁺.

### 3.2.2.15. Synthesis of compound C51 and analogues

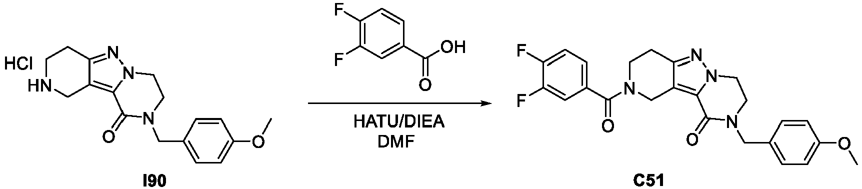

To a solution of 3,4-difluorobenzoic acid (30 mg, 0.19 mmol) in N,N-dimethylformamide (3 mL) was added 2-(7-azabenzotriazol-1-yl)-N,N,N',N'-tetramethyluronium hexafluorophosphate (105 mg, 0.276 mmol). Then Intermediate **I90** (50 mg, 0.14 mmol, 95% purity) and N,N-diisopropylethylamine (90 mg, 0.70 mmol) were added. The mixture was stirred at room temperature overnight. The mixture was poured into water (10 mL) and extracted with ethyl acetate (10 mL) twice. The combined organic phases were washed with brine (5 mL), dried over anhydrous sodium sulfate and filtered. The filtrate was concentrated *in vacuo* to give a residue, which was purified by Prep. HPLC (Column: Waters Xbrige C18 (5 µm 19 * 150 mm), Mobile phase A: water (0.1 % ammonium bicarbonate), Mobile phase B: acetonitrile, UV: 214 nm, Flow rate: 15 mL/min, Gradient: 20 - 75 % (%B)) to give **compound C51** (35 mg, 56% yield, 98.7% purity from LCMS) as a white solid.

The compounds listed in the table below were made in a similar way :

| Co. No. | Structure | Reagents | | yield |
|---|---|---|---|---|
| **C53** | 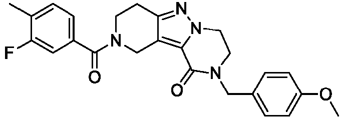 | 1. | Intermediate **I90** | 32% |
| | | 2. | 3-fluoro-4-methylbenzoic acid | |
| | | 3. | HATU | |
| | | 4. | N,N-diisopropylethylamine | |
| | | 5. | DMF | |
| **C49** | 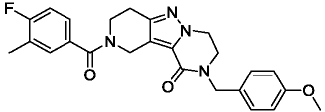 | 1. | Intermediate **I90** | 31% |
| | | 2. | 4-fluoro-3-methylbenzoic acid | |
| | | 3. | HATU | |
| | | 4. | N,N-diisopropylethylamine | |
| | | 5. | DMF | |
| **C52** | 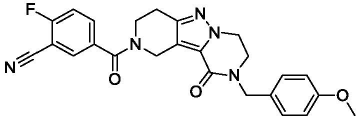 | 1. | Intermediate **I90** | 90% |
| | | 2. | 3-cyano-4-fluorobenzoic acid | |
| | | 3. | HATU | |
| | | 4. | N,N-diisopropylethylamine | |
| | | 5. | DMF | |
| **C54** | 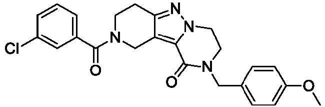 | 1. | Intermediate **I90** | 26% |
| | | 2. | 3-chlorobenzoic acid | |
| | | 3. | HATU | |
| | | 4. | N,N-diisopropylethylamine | |
| | | 5. | DMF | |
| **C50** | 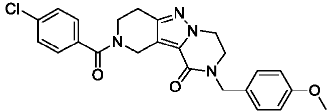 | 1. | Intermediate **I90** | 39% |
| | | 2. | 4-chlorobenzoic acid | |
| | | 3. | HATU | |
| | | 4. | N,N-diisopropylethylamine | |
| | | 5. | DMF | |
| **C62** | 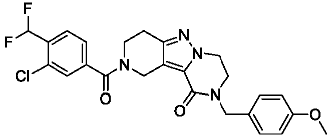 | 1. | Intermediate **I90** | 43% |
| | | 2. | 3-chloro-4-(difluoromethyl)benzoic acid **I60** | |
| | | 3. | HATU | |
| | | 4. | N,N-diisopropylethylamine | |
| | | 5. | DMF | |
| **C57** | 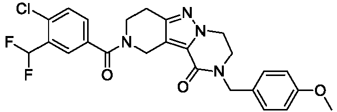 | 1. | Intermediate **I90** | 29% |
| | | 2. | 4-chloro-3-(difluoromethyl)benzoic acid **I64** | |
| | | 3. | HATU | |
| | | 4. | N,N-diisopropylethylamine | |
| | | 5. | DMF | |
| **C56** | 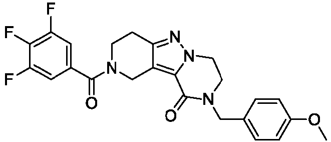 | 1. | Intermediate **I90** | 62% |
| | | 2. | 3,4,5-trifluorobenzoic acid | |
| | | 3. | HATU | |
| | | 4. | N,N-diisopropylethylamine | |
| | | 5. | DMF | |
| **C64** | 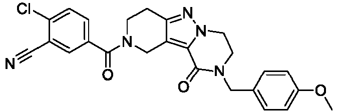 | 1. | Intermediate **I90** | 59% |
| | | 2. | 4-chloro-3-cyanobenzoic acid | |
| | | 3. | HATU | |
| | | 4. | N,N-diisopropylethylamine | |
| | | 5. | DMF | |
| **C70** | 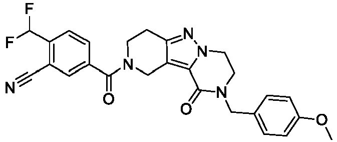 | 1. | Intermediate **I90** | 35% |
| | | 2. | 3-cyano-4-(difluoromethyl)benzoic acid **I69** | |
| | | 3. | HATU | |
| | | 4. | N,N-diisopropylethylamine | |
| | | 5. | DMF | |
| **C68** | 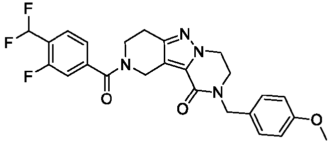 | 1. | Intermediate **I90** | 33% |
| | | 2. | 4-(difluoromethyl)-3-fluorobenzoic acid **I71** | |
| | | 3. | HATU | |
| | | 4. | N,N-diisopropylethylamine | |
| | | 5. | DMF | |

### 3.2.2.16. Synthesis of compound C59 and analogues

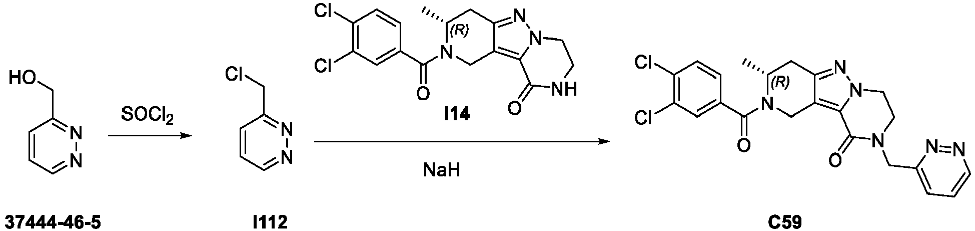

To a solution of pyridazin-3-ylmethanol (220.0 mg, 2.0 mmol) in DCM (10 mL) was added a solution of sulfurous dichloride (595.0 mg, 5.0 mmol) in DCM (2 mL) dropwise at 0 °C. The mixture was stirred at room temperature for 2 hours. The mixture was concentrated to afford the crude intermediate **I112** (200.0 mg, 78.1% yield, 90% purity from ¹H NMR) as yellow oil. **¹H NMR** (400 MHz, CD₃OD) δ 9.71 - 9.69 (m, 1H), 8.77 (s, 1H), 8.64 - 8.62 (m, 1H), 5.13 - 5.12 (m, 2H).

To a solution of intermediate **I112** (100 mg, 0.26 mmol, the preparation has been reported in EP19176933) in DMF (2 mL) was added 60% wt. NaH in mineral oil (16.0 mg, 0.4 mmol) at 0 °C under nitrogen. The mixture was stirred at 0 °C for 30 minutes. Then intermediate **I14** (40.0 mg, 0.31 mmol) was added to this mixture. The mixture was stirred at 25 °C for 3 hours. The mixture was treated with water (10 mL) at 0 °C and stirred for 10 minutes. Then EtOAc (10 mL) was added to extract the desired compound for three times. The combined organic phases were washed with brine (30 mL), dried over Na₂SO₄, filtered and concentrated. The residue was purified by pre-HPLC to afford compound **C59** (41.0 mg, 33.6% yield, 98.2 % purity from LCMS) as a pale yellow solid.

The compounds listed in the table below were made in a similar way :

| Co. No. | Structure | Reagents | | Yield |
|---|---|---|---|---|
| **C60** | 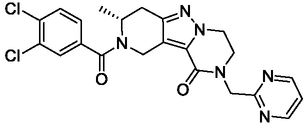 | 1. | Intermediate **I14** | 36.0% |
| | | 2. | 2-(chloromethyl)pyrimidine | |
| | | 3. | NaH | |
| | | 4. | DMF | |
| **C65** | 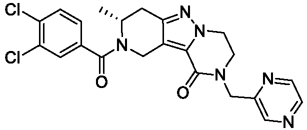 | 1. | Intermediate **I14** | 23.6% |
| | | 2. | 2-(bromomethyl)pyrazine | |
| | | 3. | NaH | |
| | | 4. | DMF | |
| **C58** | 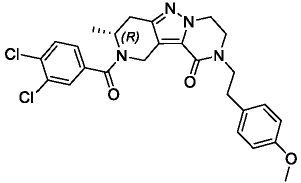 | 1. | Intermediate **I14** | 19.5% |
| | | 2. | 1-(2-bromoethyl)-4-methoxybenzene | |
| | | 3. | NaH | |
| | | 4. | DMF | |
| **C69** | 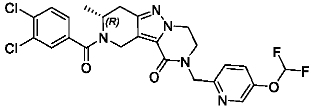 | 1. | Intermediate **I14** | 18% |
| | | 2. | 2-(bromomethyl)-5-(difluoromethoxy)pyridine | |
| | | 3. | NaH | |
| | | 4. | N,N-dimethylformamide | |
| **C66** | 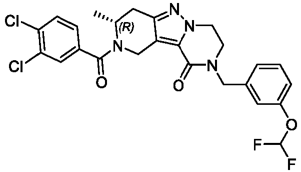 | 1. | Intermediate **I14** | 64% |
| | | 2. | 1-(bromomethyl)-3(difluoromethoxy)benzene | |
| | | 3. | NaH | |
| | | 4. | N,N-dimethylformamide | |
| **C67** | 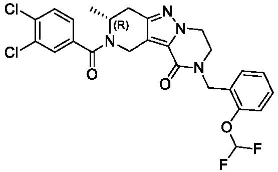 | 1. | Intermediate **I14** | 64% |
| | | 2. | 1-(bromomethyl)-2(difluoromethoxy)benzene | |
| | | 3. | NaH | |
| | | 4. | N,N-dimethylformamide | |

### 3.2.2.17. Synthesis of compound C81 and analogues

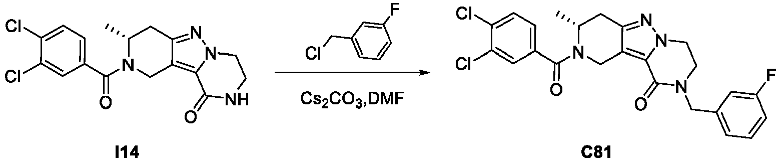

A mixture of 1-(chloromethyl)-3-fluorobenzene (70 mg, 0.18 mmol), intermediate **I14** (31 mg, 0.21 mmol) and cesium carbonate (176 mg, 0.56 mmol) in N,N-dimethylformamide (2 mL) was stirred at room temperature for 3 hours. The mixture was diluted with water (10 mL) and extracted with ethyl acetate (10 mL). The organic layer was washed with brine (10 mL), dried over Na₂SO₄ and concentrated. The residue was purified by chromatography on silica gel (dichloromethane : methanol = 50 : 1) and further purified by prep-TLC (dichloromethane : methanol = 20 : 1) to afford **compound C81** (37 mg, 42% yield, 97.35% purity by LCMS) as a white solid.

The compounds listed in the table below were made in a similar way :

| Co. No. | Structure | Reagents | | Yield |
|---|---|---|---|---|
| **C82** | 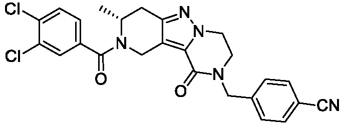 | 1. | Intermediate **I14** | 37% |
| | | 2. | 4-(chloromethyl)benzonitrile | |
| | | 3. | cesium carbonate | |
| | | 4. | N,N-dimethylformamide | |
| **C80** | 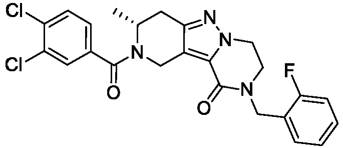 | 1. | Intermediate **I14** | 39% |
| | | 2. | 1-(chloromethyl)-2-fluorobenzene | |
| | | 3. | cesium carbonate | |
| | | 4. | N,N-dimethylformamide | |

### 3.2.2.18. Synthesis of compound C83

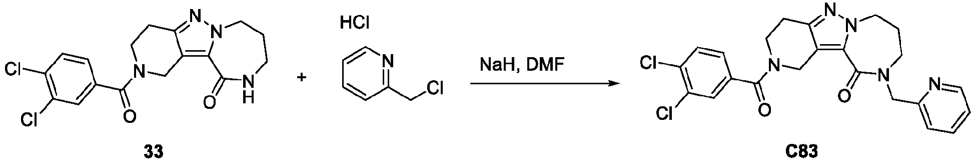

A tube was charged with 2-(3,4-dichlorobenzoyl)-1,2,3,4,7,8,9,10-octahydro-11H-pyrido[4',3':3,4]pyrazolo[1,5-a][1,4]diazepin-11-one **33** (73 mg, 0.19 mmol) in dry DMF (1.2 mL) under N₂ atm. NaH 60 wt% in mineral oil (38.5 mg, 0.96 mmol) was added and the mixture was stirred for 1h at RT. 2-(chloromethyl)pyridine hydrochloride (66 mg, 0.39 mmol) was added and the mixture was stirred at rt for 16h. The reaction mixture was quenched with water and extracted with Me-THF. The organic layer was separated, dried over MgSO₄, filtered and concentrated in vacuo. The residue was purified via Prep HPLC (Stationary phase: RP XBridge Prep C18 OBD-10µm,30x150mm, Mobile phase: 0.25% NH₄HCO₃ solution in water, CH₃CN). The residue was dissolved in MeOH and concentrated in vacuo to afford 2-(3,4-dichlorobenzoyl)-10-(pyridin-2-ylmethyl)-1,2,3,4,7,8,9,10-octahydro-11H-pyrido[4',3':3,4]pyrazolo[1,5-a][1,4]diazepin-11-one **C83** (18.8 mg, yield 21%) as a white foam.

### 3.2.2.19. Synthesis of compound C63

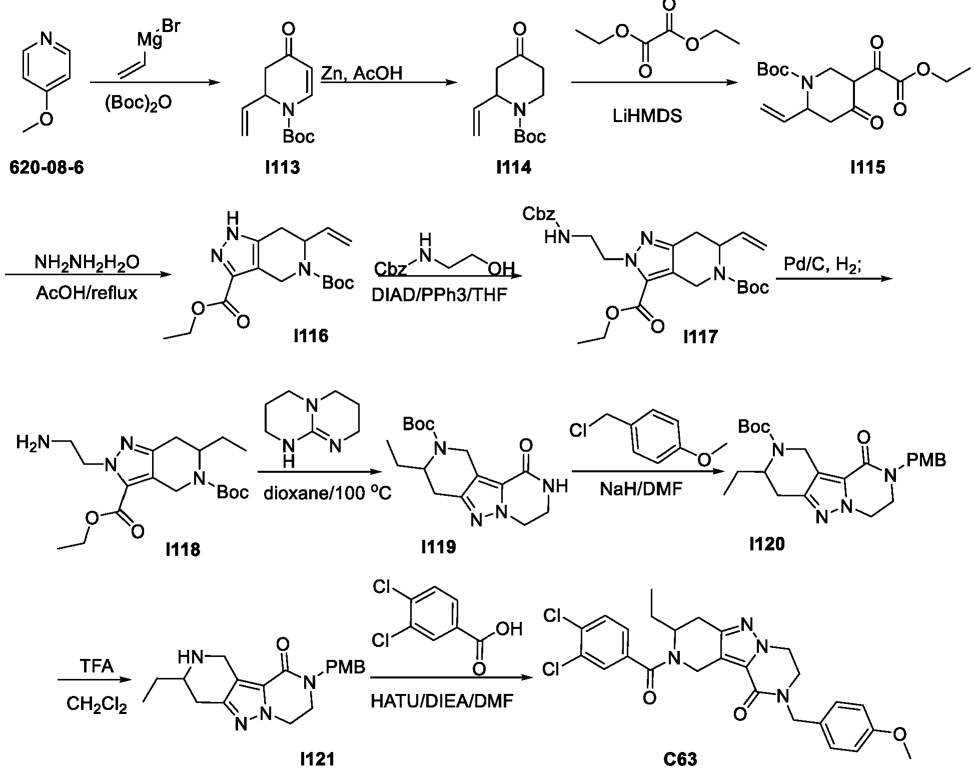

To a solution of 4-methoxypyridine (10.5 g, 96.12 mmol) in tetrahydrofuran (100 mL) was added di-tert-butyl dicarbonate (21.0 g, 96.22 mmol) portionwise at 0 °C under nitrogen atmosphere. Then the mixture was added 1 M vinylmagnesium bromide in tetrahydrofuran (100 mL, 100 mmol) dropwise over 30 min at 0 °C. The mixture was stirred at room temperature for 3 hours. The mixture was treated with hydrogen chloride (1 M, 200 mL) at 5 °C and stirred for 10 minutes. Then the mixture was extracted with ethyl acetate (100 mL) for three times. The combined organic layers were washed with 10% aqueous sodium bicarbonate solution (100 mL), then brine (100 mL), dried over Na₂SO₄ and filtered. The filter was evaporated to give intermediate **I113** (8.5 g, 35 % yield, 90 % purity from ¹H NMR). **¹H NMR** (400 MHz, CDCl₃) δ 7.79 (s, 1H), 5.84 - 5.76 (m, 1H), 5.30 - 5.11 (m, 3H), 5.09 - 5.02 (m, 1H), 2.93 - 2.87 (m, 1H), 2.54 - 2.51 (m, 1H), 1.47 (s, 9H).

To a slurry of intermediate **I113** (9.00 g, 36.28 mmol, 90% purity) in acetic acid (50 mL) was added activated zinc dust (12.1 g, 185.0 mmol) at 25 °C. After addition, the resulting mixture was stirred at 70 °C for 14 hours. The mixture was filtered and the filter cake was washed with ethanol (200 mL). The filtrate was concentrated *in vacuo* to dryness. The residue was purified by column C18 (Mobile Phase A: water (+ 0.1% ammonium bicarbonate), Mobile Phase B: acetonitrile, Gradient: 05 - 55% (%B)) to give intermediate **I114.** (2.31 g, 25% yield, 90% purity from ¹H NMR) as brown oil. **¹H NMR** (300 MHz, CDCl₃) δ 5.85 - 5.74 (m, 1H), 5.30 - 5.15 (m, 3H), 4.23 - 4.15 (m, 1H), 3.40 - 3.30 (m, 1H), 2.77 - 2.33 (m, 4H), 1.53 (s, 9H).

To a solution of intermediate **I114** (10.5 g, 41.9 mmol, 90% purity) in tetrahydrofuran (50 mL) was added lithium bis(trimethylsilyl)amide (63 mL, 63 mmol) dropwise at -65 °C. The mixture was stirred at -65 °C for 30 min. Then diethyl oxalate (7.8 g, 53.8 mmol) was added and the mixture was stirred at -65 °C to room temperature overnight. The reaction mixture was poured into water (100 mL) and extracted with dichloromethane (50 mL) for three times. The combined organic layers were washed with brine (50 mL), dried over anhydrous sodium sulfate and filtered. The filtrate was concentrated to give a crude . The crude was purified by chromatography on silica gel (petroleum ether: ethyl acetate = 3 : 1) to give intermediate **I115** (7.5 g) as a yellow oil. **LCMS** (ESI): Rₜ = 2.210 min, mass calcd. for C₁₆H₂₃NO₆ 325.15, m/z found 270.1 [M+H-56]⁺.

A solution of intermediate **I115** (7.5 g, 16.13 mmol, 70% purity) and hyrazine hydrate (1.5 g, 39.8 mmol, 85% purity) in acetic acid glacial (20 mL) was stirred at 100 °C for 4 hour. The reaction mixture was poured into water (100 mL) and extracted with dichloromethane (200 mL) for three times. The combined organic layers were washed with brine (100 mL), dried over anhydrous sodium sulfate and filtered. The filtrate was concentrated to give a residue, which was purified by chromatography on silica gel (petroleum ether : ethyl acetate = 1 : 1) to give intermediate **I116** (1.2 g, 27% yield, 90% purity by LCMS) as a yellow solid. **¹H NMR** (400 MHz, CDCl₃) δ 5.75 - 5.66 (m, 1H), 5.29 - 5.49 (m, 4H), 4.43 - 4.33 (m, 2H), 4.19 - 4.13 (m, 1H), 3.05 - 2.92 (m, 2H), 1.47 (s, 9H), 1.3 (t, J = 7.2 Hz, 3H).

To a mixture of intermediate **I116** (570 mg, 1.60 mmol, 90% purity) in tetrahydrofuran (30 mL) at 0 °C was added benzyl (2-hydroxyethyl)carbamate (360 mg, 1.84 mmol), triphenylphosphine (670 mg, 2.55 mmol) and diisopropyl azodicarboxylate (610 mg, 3.02 mmol) successively. After addition, the mixture was stirred at 0°C to room temperature overnight. The mixture was concentrated *in vacuo* to give a residue, which was purified by column C18 (Mobile Phase A: water (+ 0.1% ammonium bicarbonate), Mobile Phase B: acetonitrile, Gradient: 05 - 65% (%B)) to give intermediate **I117** (437 mg, 55% yield, 100% purity from LCMS) as yellow oil. **LCMS** (ESI): Rₜ = 1.78 min, mass calcd. for C C₂₆H₃₄N₄O₆ 498.25, m/z found 499.1 [M+H]⁺.

To a mixture of intermediate **I117** (230 mg, 0.46 mmol, 100% purity) in ethanol (15 mL) was added 10% palladium on activated carbon (ca 50% moisture, 23 mg) at room temperature. The mixture was stirred at room temperature under hydrogen atmosphere (1 atm) for 16 hours. The mixture was filtered. The filtrate was concentrated *in vacuo* to give intermediate **I118** (200 mg, 81% yield, 68% purity from LCMS) as grey oil. **LCMS** (ESI): Rₜ = 1.62 min, mass calcd. for C₁₈H₃₀N₄O₄ 366.23, m/z found 367.1 [M+H]⁺.

To a mixture of intermediate **I118** (200 mg, 0.37 mmol, 68% purity) in 1,4-dioxane (8 mL) was added 2,3,4,6,7,8-hexahydro-1H-pyrimido[1,2-a]pyrimidine (20 mg, 0.14 mmol) at room temperature. The mixture was stirred at 100 °C for 2 hours. The mixture was concentrated *in vacuo* to give a residue, which was purified by column C18 (Mobile Phase A: water (+ 0.1% ammonium bicarbonate), Mobile Phase B: acetonitrile, Gradient: 05 - 45% (%B)) to give intermediate **I119** (95 mg, 80% yield, 100% purity from LCMS) as a white solid. **LCMS** (ESI): Rₜ = 1.45 min, mass calcd. for C₁₆H₂₄N₄O₃ 320.18, m/z found 321.1 [M+H]⁺.

To an ice-cooled mixture of 60% wt. sodium hydride in mineral oil (24 mg, 0.60 mmol) in N,N-dimethylformamide (4 mL) was added intermediate **I119** (125 mg, 0.39 mmol, 100% purity) below 10 °C. After the mixture was stirred at 10 °C for 30 mins, a solution of 4-methoxybenzylchloride (73 mg, 0.47 mmol) in N,N-dimethylformamide (1 mL) was added dropwise. The mixture was stirred at 10 °C for 120 mins. Then the mixture was quenched with water (50 mL) and extracted with ethyl ether (30 mL) twice. The combined organic layers were washed with brine (80 mL), dried over Na₂SO₄ and filtered. The filtrate was concentrated *in vacuo* to give intermediate **I120** (198 mg, 100% yield, 87% purity from LCMS) as grey oil. **LCMS** (ESI): Rₜ = 1.71 min, mass calcd. for C₂₄H₃₂N₄O₄ 440.24, m/z found 441.1 [M+H]⁺.

To a solution of intermediate **I120** (198 mg, 0.39 mmol, 87% purity) in dichloromethane (5 mL) was added trifluoroacetic acid (1 mL) at room temperature. The resulting mixture was stirred at room temperature overnight. The mixture was concentrated *in vacuo* to dryness. Then the residue was diluted with dichloromethane (50 mL) and neutralized with saturated sodium bicarbonate aqueous solution (50 mL). The mixture was extracted with dichloromethane (50 mL) twice. The combined organic layers were washed with brine (50 mL) and dried over sodium sulfate. The mixture was concentrated *in vacuo* to give intermediate **I121** (146 mg, 99% yield, 90% purity from LCMS) as a grey solid. **LCMS** (ESI): Rₜ = 1.36 min, mass calcd. for C₁₉H₂₄N₄O₂ 340.19, m/z found 341.1 [M+H]⁺.

To a mixture of intermediate **I121** (145 mg, 0.38 mmol, 100% purity) and 3,4-dichlorobenzoic acid (100 mg, 0.52 mmol) in dichloromethane (15 mL) was added N,N-diisopropylethylamine (250 mg, 1.93 mmol) and O-(7-azabenzotriazol-1-yl)-N,N,N',N'-tetramethyluronium hexafluorophosphate (290 mg, 0.76 mmol) at room temperature. The resulting mixture was stirred at 25 °C for 16 hours. The mixture was concentrated *in vacuo* to give a residue, which was purified by column C18 (Mobile Phase A: water (+ 0.02 % ammonium acetate), Mobile Phase B: acetonitrile, Gradient: 05 - 60 % (%B)) to give compound C63 (79 mg, 40% yield, 99% purity from LCMS) as a white solid.

### 3.2.2.20. Synthesis of compound C61*R

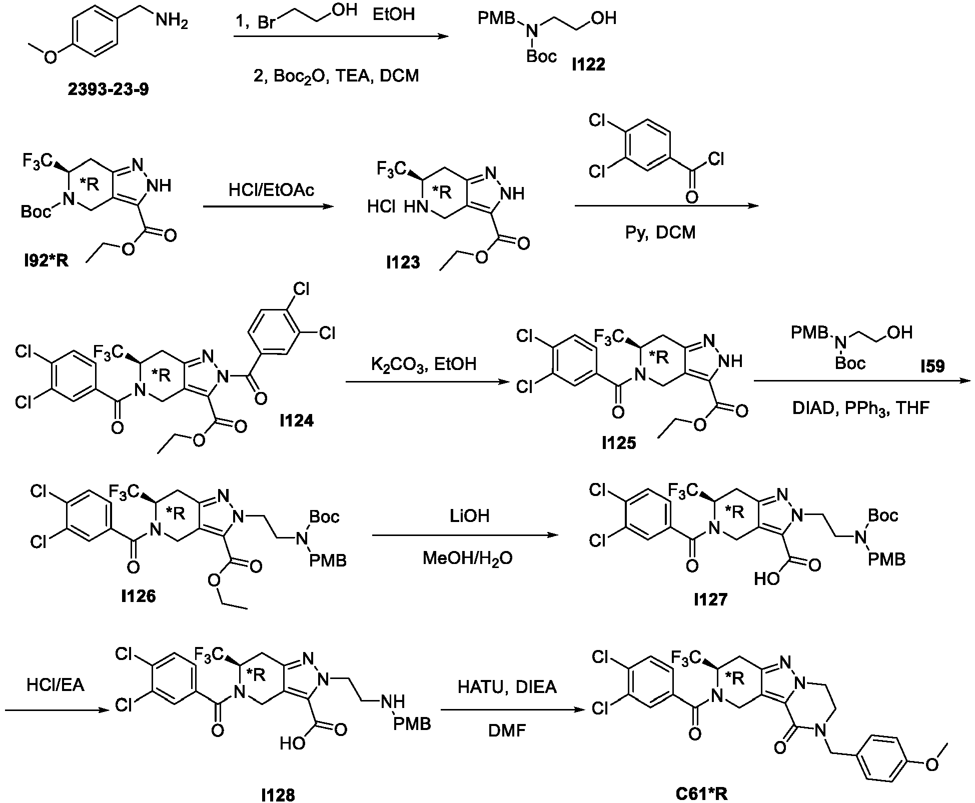

To a solution of (4-methoxyphenyl)methanamine (16.3 g, 98 % purity, 116 mmol) in ethanol (60 mL) was added 2-bromoethanol (5.00 g, 96 % purity, 38.4 mmol). After stirring at 85 °C overnight, the mixture was concentrated *in vacuo* to dryness. The residue was dissolved in dichloromethane (60 mL) and triethylamine (7.80 g, 77.083 mmol), di-tert-butyl dicarbonate (33.6 g, 154 mmol) were added. The mixture was stirred at room temperature for 2 hours. The mixture was treated with saturated ammonium chloride aqueous solution (100 mL), brine (50 mL) and extracted with dichloromethane (50 mL) for three times. The combined organic phase were dried over Na₂SO₄(s), filtered and concentrated to give the crude product, which was purified by silica gel column chromatography (petroleum ether : ethyl acetate = 10 : 1 to 2 : 1) to give intermediate **I122** (6.88 g, 90 % purity from ¹H NMR, 57 % yield) as yellow oil. **LCMS** (ESI): Rₜ = 1.36 min, mass calcd. for C₁₅H₂₃NO₄ 281.16, m/z found 226.1 [M-56+H]⁺. **¹H NMR** (400 MHz, CDCl₃) 7.16 (d, *J* = 6.8 Hz, 2H), 6.86 (d, *J* = 8.4 Hz, 2H), 4.41 (s, 2H), 3.80 (s, 3H), 3.67 (s, 2H), 3.37 (s, 2H), 3.12 (br s, 1H), 1.48 (s, 9H).

To a solution of intermediate **I92*R** (904 mg, 2.49 mmol) in ethyl acetate (4 mL) was added 3 M HCl/EA (20 mL, 60 mmol) at room temperature. After stirring at room temperature for 4 hours, the mixture was concentrated *in vacuo* to give intermediate **I123** (715 mg, 90 % purity from ¹H NMR, 86 % yield) as a yellow solid, which was used in the next step without further purification. **¹H NMR** (400 MHz, DMSO-*d6*) 10.95 (br s, 3H), 4.77 - 4.70 (m, 1H), 4.42 - 4.28 (m, 4H), 3.31 - 3.26 (m, 1H), 3.11 - 3.04 (m, 1H), 1.31 (t, *J* = 7.2 Hz, 3H). **LCMS** (ESI): Rₜ = 1.50 min, mass calcd. for C₁₀H₁₃ClF₃N₃O₂ 299.1, m/z found 264.2 [M-HCl+H]⁺.

To a solution of intermediate **I123** (120 mg, 90 % purity, 0.360 mmol) in pyridine (5 mL) was added 3,4-dichlorobenzoyl chloride (400 mg, 1.91 mmol) in dichloromethane (1 mL) at 0 °C. The mixture was stirred at room temperature overnight. The mixture was concentrated *in vacuo* to give a residue, which was purified by C18 column (acetonitrile : water containing 0.1 % ammonium bicarbonate = 10 % to 80 %) to give intermediate **I124** (340 mg, 55 % purity, 83 % yield) as a yellow solid. **LCMS** (ESI): Rₜ = 2.056 min, mass calcd. for C₂₄H₁₆Cl₄F₃N₃O₄ 609.21, m/z found 610.0 [M+H]⁺.

A mixture of intermediate **I124** (340 mg, 55 % purity, 0.307 mmol) and potassium carbonate (120 mg, 0.868 mmol) in ethanol (10 mL) was stirred at 40 °C for 2 hours. The mixture was concentrated *in vacuo* to give a residue, which was purified by C18 column (acetonitrile : water (0.1 % ammonium bicarbonate = 10 % to 70 %) to give intermediate **I125** (120 mg, 90 % purity from NMR, 81 % yield) as a white solid. **LCMS** (ESI): Rₜ = 1.67 min, mass calcd. for C₁₇H₁₄Cl₂F₃N₃O₃ 435.04, m/z found 435.9 [M+H]⁺. **¹H NMR** (400 MHz, CDCl₃) 7.65 - 7.51 (m, 2H), 7.40 - 7.30 (m, 1H), 5.95 (br s, 0.7H), 5.58 (br s, 0.3H), 4.91 - 4.22 (m, 5H), 3.33 - 3.15 (m, 2H), 1.26 (t, *J* = 7.2 Hz, 3H).

To a mixture of intermediate **I125** (120 mg, 90 % purity, 0.248 mmol), intermediate **I122** (150 mg, 90 % purity, 0.480 mmol) and triphenylphosphine (130 mg, 0.496 mmol) in tetrahydrofuran (5 mL) was added diisopropyl azodicarboxylate (100 mg, 0.495 mmol) at 0 °C. The mixture was stirred at room temperature overnight. The mixture was concentrated *in vacuo* to give a residue, which was purified by C18 column (acetonitrile : water containing 0.1 % ammonium bicarbonate = 10 % to 80 %) to give intermediate **I126** (30 mg, 100 % purity, 17 % yield) as a yellow solid. **LCMS** (ESI): Rₜ = 2.047 min, mass calcd. for C₃₂H₃₅Cl₂F₃N₄O₆ 698.19, m/z found 699.2 [M+H]⁺.

A mixture of intermediate **I126** (35 mg, 0.05 mmol) and lithium hydroxide hydrate (11 mg, 0.25 mmol) in tetrahydrofuran/water (0.5 mL/0.2 mL) was stirred at 25 °C for 5 hours. The mixture was concentrated to give crude intermediate **I127** (50 mg, crude, 60% purity from LCMS) as a white solid, which was used directly in the next step without further purification. **LCMS** (ESI): Rₜ = 1.49 min, mass calcd. for C₃₀H₃₁Cl₂F₃N₄O₆ 670.16, m/z found 671.5 [M+H]⁺.

A solution of intermediate **I127** (50 mg, 0.0744 mmol) in 4 M HCl/EtOAc (2 mL) was stirred at 25 °C for 3 hours. The mixture was concentrated *in vacuo* to give the residue, which was purified by C18 column (acetonitrile : water containing 0.1 % ammonium bicarbonate = 10 % to 80 %) to give intermediate **I128** (29 mg, 89 % yield, 40 % purity from LCMS) as a white solid. **LCMS** (ESI): Rₜ = 1.42 min, mass calcd. for C₂₅H₂₃Cl₂F₃N₄O₄ 570.1, m/z found 571.4 [M+H]⁺.

A mixture of intermediate **I128** (25 mg, 0.044 mmol), 2-(7-azabenzotriazol-1-yl)-N,N,N',N'-tetramethyluronium hexafluorophosphate (25 mg, 0.066 mmol) and N,N-diisopropylethylamine (17 mg, 0.132 mmol) in N,N-dimethylformamide (1 mL) was stirred at room temperature for 1 hour. The mixture was purified by C18 column (acetonitrile : water containing 0.1 % ammonium bicarbonate = 10 % to 80 %) to give compound **C61*R** (9 mg, 38 % yield, 99.67 % purity from LCMS) as a white solid.

### 3.2.2.21. Synthesis of compound C61*S

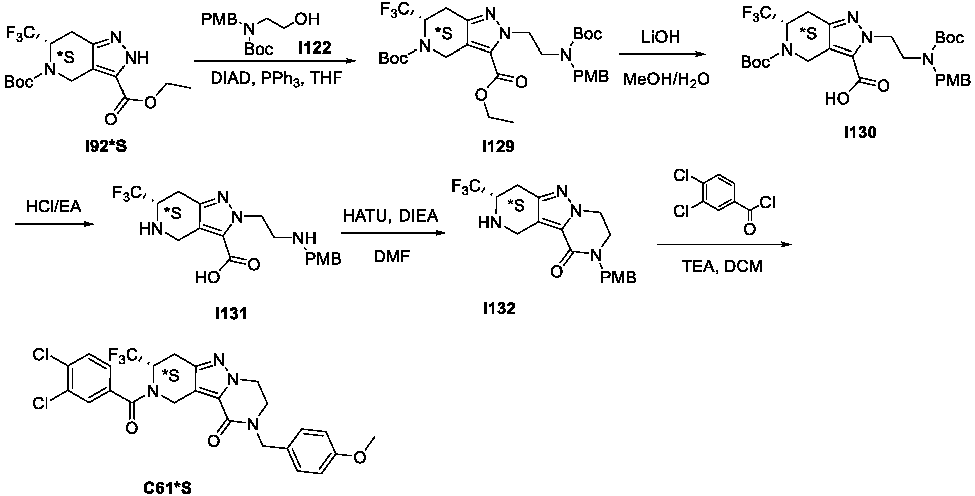

To a mixture of intermediate **I92*S** (0.8 g, 2.20 mmol), intermediate **I122** (1.0 g, 90 % purity, 3.20 mmol) and triphenylphosphine (1.1 g, 4.19 mmol) in tetrahydrofuran (15 mL) was added diisopropyl azodicarboxylate (0.85 g, 4.20 mmol) at 0 °C. The mixture was stirred at room temperature overnight. The mixture was concentrated *in vacuo* to give a residue, which was purified by C18 column (acetonitrile : water containing 0.1 % ammonium bicarbonate = 10 % to 80 %) to give intermediate **I129** (1.0 g, 90 % purity from NMR, 65 % yield) as a yellow solid. **¹H NMR** (400 MHz, CDCl₃) 7.12 - 7.04 (m, 2H), 6.82 (d, *J =* 8.4 Hz, 2H), 5.42 - 5.31 (m, 0.5H), 5.16 - 5.03 (m, 1.5H), 4.79 - 4.58 (m, 2H), 4.42 - 4.05 (m, 5H), 3.78 (s, 3H), 3.65 - 3.42 (m, 2H), 3.14 - 3.02 (m, 2H), 1.52 (s, 9H), 1.43 - 1.38 (m, 12H).

To a solution of intermediate **I129** (550 mg, 90 % purity, 0.790 mmol) in methanol (30 mL) was added lithium hydroxide monohydrate (300 mg, 7.15 mmol) in 20 mL of water. Then the reaction mixture was stirred at 25 °C for 3 hours. The mixture was concentrated *in vacuo* to remove organic solvent. The aqueous layer was diluted with water (100 mL) and acidified with 1 M hydrochloric acid solution to pH ~ 5. The mixture was extracted with ethyl acetate (100 mL) twice. The combined organic layers were dried over Na₂SO₄ (s), filtered and concentrated to give intermediate **I130** (450 mg, 95 % purity from NMR, 90 % yield) as a yellow solid. **¹H NMR** (400 MHz, CDCl₃) 7.15 - 7.02 (m, 2H), 6.85 - 6.78 (m, 2H), 5.39 (br s, 0.5H), 5.19 - 5.05 (m, 1.5H), 4.78 - 4.61 (m, 2H), 4.31 - 4.12 (m, 3H), 3.77 (s, 3H), 3.68 - 3.46 (m, 2H), 3.18 - 3.03 (m, 2H), 1.54 - 1.50 (m, 9H), 1.41 (s, 9H).

A solution of intermediate **I130** (450 mg, 95 % purity, 0.714 mmol) in HCl/ ethyl acetate (30 mL) was stirred at 25 °C for 3 hours. The mixture was concentrated *in vacuo* to give intermediate **I131** (240 mg, 100 % purity, 72 % yield) as a yellow solid. **LCMS** (ESI): Rₜ = 1.142 min, mass calcd. for C18H21F3N4O3 398.16, m/z found 399.2 [M+H]⁺.

A mixture of intermediate **I131** (140 mg, 100 % purity, 0.301 mmol), 2-(7-Azabenzotriazol-1-yl)-N,N,N',N'-tetramethyluronium hexafluorophosphate (160 mg, 0.421 mmol) and N,N-diisopropylethylamine (200 mg, 1.55 mmol) in N,N-dimethylformamide (15 mL) was stirred at room temperature 2 hours. The reaction was treated with ice water (50 mL) and extracted with ethyl acetate (50 mL) twice. The combined organic phases were washed with brine (50 mL) twice. The organic layer was dried over Na₂SO₄ (s), filtered and concentrated. The residue was purified by C18 column (acetonitrile : water containing 0.1 % ammonium bicarbonate = 5 % to 40 %) to give intermediate **I132** (100 mg, 97 % purity, 85 % yield) as a yellow solid. **¹H NMR** (400 MHz, MeOD) 7.26 (d, *J =* 8.4 Hz, 2H), 6.90 (d, *J =* 8.0 Hz, 2H), 4.65 (s, 2H), 4.23 - 4.28 (m, 3H), 3.98 (d, *J* = 16.8 Hz, 1H), 3.77 (s, 3H), 3.69 (t, *J* = 6.4 Hz, 2H), 3.65 - 3.55 (m, 1H), 2.97 - 2.92 (m, 1H), 2.77 - 2.70 (m, 1H).

To a solution of intermediate **I132** (100 mg, 97 %, purity, 0.255 mmol) in pyridine (5 mL) was added 3,4-dichlorobenzoyl chloride (100 mg, 0.477 mmol) in dichloromethane (1 mL) at 0 °C. The mixture was stirred at room temperature overnight. The solvent was removed under reduced pressure. The residue was purified by C18 column (acetonitrile : water containing 0.1 % ammonium bicarbonate = 10 % to 70 %) to give compound **C61*S** (60 mg, 99.5 % purity, 42 % yield) as a white solid.

### 3.2.2.22. Synthesis of Compound C55*R and C55*S

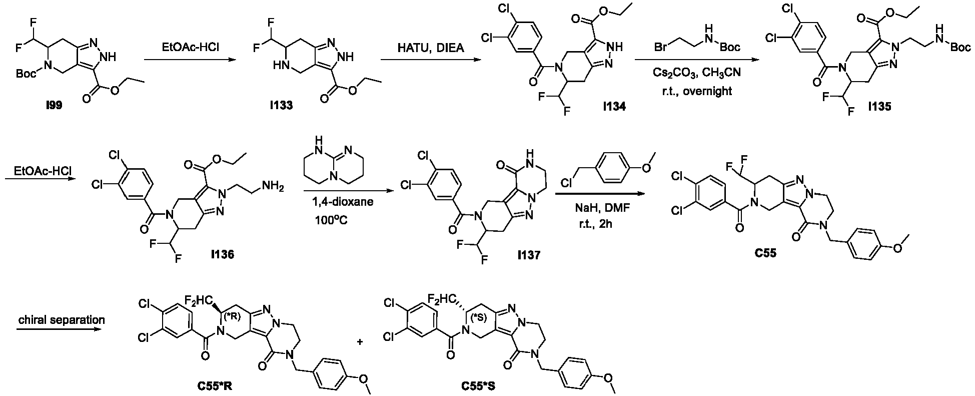

A mixture of intermediate **I99** (6 g, 17.4mmol) in 4 M HCl/EtOAc (50 mL) was stirred at room temperature for 1 hour. Then the mixture was concentrated *in vacuo* to dryness. The residue was neutralized with saturated aqueous sodium hydrogencarbonate (50 mL) and extracted with EtOAc (50 mL) for three times. The combined organic phases were washed with brine (50 mL), dried over Na₂SO₄ and filtered. The filtrate was concentrated under reduced pressure to give intermediate **I133** (4.5 g, 90 % purity from ¹H NMR, 95 % yield) as a yellow solid. **¹H NMR** (400 MHz, CDCl₃) δ 6.01 - 5.69 (m, 1H), 4.33 - 4.20 (m, 3H), 3.96 - 3.75 (m, 1H), 3.23 - 3.13 (m, 1H), 2.88 - 2.83 (m, 1H), 2.69 - 2.62 (m, 1H), 2.47 - 2.32 (m, 3H), 2.07 - 2.04 (m, 1H), 1.31 (t, J = 7.2 Hz, 3H).

To a solution of 3,4-dichlorobenzoic acid (3.5 g, 18 mmol), 2-(7-azabenzotriazol-1-yl)-N,N,N',N'-tetramethyluronium hexafluorophosphate (6.8 g, 18 mmol) and N,N-diisopropylethylamine (4.6 g, 3.6 mmol) in N,N-dimethylformamide (25 mL) was added intermediate **I133** (3.1 g, 95 % purity, 12 mmol). Then the mixture was stirred at room temperature overnight. The mixture was diluted with ethyl acetate (30 mL). and washed with brine (20 mL), dried over Na₂SO₄(s) and filtered. The filtrate was concentrated under reduced pressure to give a residue, which was purified by silica gel column chromatography (petroleum ether : ethyl acetate = 10 : 1 to 4 : 1) to give intermediate **I134** (3 g, 90% purity, 56 % yield) as a yellow solid. ¹**H NMR** (400 MHz, CDCl₃) δ 7.55 - 7.56 (m, 2H), 7.32 (s, 1H), 6.03 - 5.41 (m, 2H), 4.82 - 4.07 (m, 4H), 3.15 (br s, 2H), 1.26 (t, *J =* 7.2 Hz, 3H).

To a solution of intermediate **I134** (3 g, 90 % purity, 6.48 mmol) and tert-butyl (2-bromoethyl)carbamate (2.88 g, 18 mmol) in acetonitrile (50 mL) was added cesium carbonate (6.3 g, 19.4 mmol) at room temperature. Then the mixture was stirred at room temperature overnight. The mixture was concentrated under reduced pressure to dryness, The residue was diluted with ethyl acetate (50 mL) and washed with water (50 mL) twice and brine (50 mL). The organic phase was dried over Na₂SO₄(s), filtered and concentrated to give a residue, which was purified by silica gel column chromatography (petroleum ether: ethyl acetate = 2 : 1 to 1 : 1) to give intermediate **I135** (1.9 g, 90% purity, 43 % yield) as yellow oil. **¹H NMR** (400MHz, CDCl₃) δ 7.55 - 7.45 (m, 2H), 3.48 (s, 2H), 3.09 - 3.03 (m, 2H), 1.40 (s, 12H).

A mixture of intermediate **I135** (2.3 g, 3.7 mmol) in 4 M HCl/EtOAc (30 mL) was stirred at room temperature for 1 hour. Then the mixture was concentrated under reduced pressure. The residue was neutralized with saturated aqueous sodium hydrogencarbonate (30 mL) and extracted with ethyl acetate (30 mL) for three times. The combined organic phases were washed with brine (20 mL), dried over Na₂SO₄ and filtered. The filtrate was concentrated *in vacuo* to give intermediate **I136** (1.5 g, 90 % purity, 91 % yield) as a white solid. **¹H NMR** (400 MHz, DMSO-*d6*) δ 7.79 - 7.73 (m, 2H), 7.47 - 7.46 (m, 1H), 6.41 - 6.01 (m, 1H), 5.43 - 5.32 (m, 1H), 4.83 - 4.11 (m, 8H), 3.01 (s, 3H), 1.36 - 1.06 (m, 3H).

To a solution of intermediate **I136** (600 mg, 90 % purity, 1.2 mmol) in 1,4-dioxane (15 mL) was added 2,3,4,6,7,8-hexahydro-1H-pyrimido[1,2-a]pyrimidine (51 mg, 0.36 mmol) at room temperature. The reaction mixture was stirred at 100 °C under nitrogen atmosphere for 2 hours. The mixture was concentrated under reduced pressure to dryness. The residue was dissolved in ethyl acetate (25 mL) and the mixture was washed with water (15 mL) twice and brine (15 mL). the organic phase was dried over Na₂SO₄(s), filtered and concentrated to give intermediate **I137** (500 mg, 90% purity, 93 % yield) as a white solid. **¹H NMR** (400MHz, DMSO-d6) δ 8.25 - 8.19 (m, 1H), 7.75 (s, 2H), 7.47 - 7.46 (m, 1H), 6.28 - 5.99 (m, 1H), 5.40 - 5.26 (m, 1H), 4.60 - 4.02 (m, 4H), 3.60 - 3.57 (m, 2H), 3.27 - 3.18 (m, 4H).

To a solution of intermediate **I137** (400 mg, 90 % purity, 0.88 mmol) in N,N-dimethylformamide (8 mL) was added 1-(chloromethyl)-4-methoxybenzene (320 mg, 1 mmol) and sodium hydride (80 mg, 60%.wt, 1.76 mmol) at room temperature. Then the mixture was stirred at room temperature for 2 hours. The mixture was quenched with aq.NH₄Cl (25 mL) and extracted with EtOAc (25 mL) for three times, the combined organic phases were washed with water (15 mL) twice and brine (15 mL), dried over Na₂SO₄(s) and filtered. The filtrate was concentrated *in vacuo* to give compound **C55** (300 mg, 90% purity, 60 % yield) as a white solid. **¹H NMR** (400MHz, CDCl3) δ 7.56 - 7.53 (m, 2H), 7.32 - 7.30 (m, 1H), 7.26 - 7.21 (m, 2H), 6.91 - 6.83 (m, 2H), 6.03 - 5.48 (m, 2H), 4.70 - 4.62 (m, 2H), 4.26 (s, 2H), 3.86 - 3.78 (m, 3H), 3.62 (s, 2H), 3.11 - 3.89 (m, 2H), 1.58 (s, 2H), 0.88 - 0.83 (m, 1H).

A racemic mixture of compound **C55** (300 mg, 90 % purity, 0.56 mmol) was separated by chiral Prep. HPLC (separation condition: Column: Chiralpak IB 4.6 µm 20 * 250 mm; Mobile Phase: Hex : EtOH : DEA = 95 : 5 : 0.2 at 15 mL/min; Temp: 30 °C; Wavelength: 230 nm) to give compounds **C55*R** (56.3 mg, 99 % purity, 38 % yield, 99.9 % ee) and **C55*S** (59 mg, 99 % purity, 38 % yield, 99.9 % ee) as yellow solids.

**Compound C55*R** : Chiral HPLC (Chiralpak IB 5 um 4.6 * 250 mm; Mobile Phase: IE-Hex-EtOH-40-60.1cm at 1 mL/min; Temp: 30 °C; Wavelength: 254 nm R_{T} = 11.173 min).

**Compound C55*S** : Chiral HPLC (Chiralpak IB 5 um 4.6 * 250 mm; Mobile Phase: IE-Hex-EtOH-40-60.1cm at 1 mL/min; Temp: 30 °C; Wavelength: 254 nm R_{T} = 12.795 min).

### 3.2.2.23. Synthesis of compound C79:

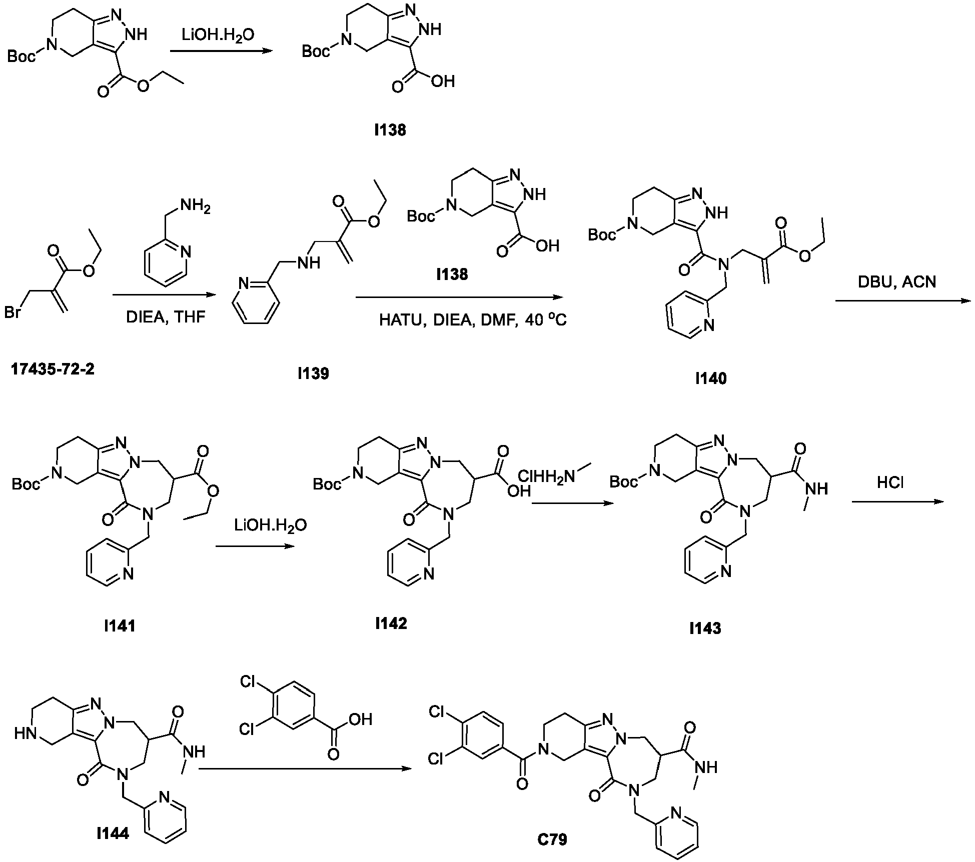

A mixture of intermediate 5-(tert-butyl) 3-ethyl 2,4,6,7-tetrahydro-5H-pyrazolo[4,3-c]pyridine-3,5-dicarboxylate (1 g, 3.39 mmol) and lithium hydroxide hydrate (427 mg, 0.48 mmol) in tetrahydrofuran/water (10 mL/3 mL) was stirred at room temperature overnight. The mixture was diluted with water (10 mL) and acidified with 1 N hydrochloric acid to pH = 6. Then the mixture was extracted with ethyl acetate (10 mL) for three times. The combined organic layers were washed with brine (20 mL), dried over Na₂SO₄ and concentrated *in vacuo* to afford intermediate **I138** (200 mg, 22% yield, 100% purity from LCMS) as yellow solids. **LCMS** (ESI): Rₜ = 0.94 min, mass calcd. for C₁₂H₁₇N₃O₄ 267.1, m/z found 266.2 [M-H]⁻.

**¹H NMR** (400 MHz, DMSO-d6) δ 4.48 (s, 2H), 3.60 - 3.57 (m, 2H), 2.67 - 2.64 (m, 2H), 1.41 (s, 9H).

To a mixture of pyridin-2-ylmethanamine (1.12 g, 10.4 mmol), N-diisopropyl-ethylamine (4.0 g, 31.08 mmol) in tetrahydrofuran (20 mL) was added ethyl 2-(bromomethyl)acrylate (2.00 g, 10.4 mmol). Then the mixture was stirred at room temperature overnight. The reaction mixture was poured into water (100 mL) and extracted with ethyl acetate (100 mL). The organic layer was concentrated *in vacuo* to give a residue, which was purified by column chromatography on silica gel (dichloromethane : methanol = 50 : 1) to give intermediate **I139** (1.0 g, 43% yield, 80% purity from LCMS) as yellow oil. **LCMS** (ESI): Rₜ = 1.24 min, mass calcd. for C₁₂H₁₆N₂O₂ 220.1, m/z found 221.1 [M+H]⁺.

A mixture of intermediate **I138** (1.21 g, 4.54 mmol), intermediate **I139** (1.00 g, 4.54 mmol), 2-(7-azabenzotriazol-1-yl)-N,N,N',N'-tetramethyluronium hexafluorophosphate (2.59 g, 6.81 mmol) and N-diisopropyl-ethylamine (1.76 g, 13.6 mmol) in N,N-dimethylformamide (20 mL) was stirred at room temperature overnight. The reaction mixture was poured into water (50 mL) and extracted with ethyl acetate (30 mL) for three times. The combined organic layers were washed with brine (30 mL), and concentrated. The residue was purified by column chromatography on silica gel (dichloromethane : methanol = 50 :1) to give intermediate **I140** (300 mg, 14% yield, 80% purity from LCMS) as yellow oil. **LCMS** (ESI): Rₜ = 1.50 min, mass calcd. for C₂₄H₃₁N₅O₅ 469.2, m/z found 470.5 [M+H]⁺.

A mixture of intermediate **I140** (300 mg, 0.64 mmol) and 1,8-diazabicyclo[5.4.0]-7-undecene (48 mg, 0.32 mmol) in acetonitrile (10 mL) was stirred at 50 °C for 3 hours under nitrogen. Then the mixture was concentrated *in vacuo* to give the residue, which was purified by column chromatography on silica gel (dichloromethane : methanol = 50 : 1) to give intermediate **I141** (120 mg, 40% yield, 80% purity from LCMS) as yellow oil. **LCMS** (ESI): Rₜ = 1.51 min, mass calcd. for C₂₄H₃₁N₅O₅ 469.2, m/z found 470.5 [M+H]⁺.

A mixture of intermediate **I141** (120 mg, 0.26 mmol) and lithium hydroxide hydrate (32 mg, 0.767 mmol) in tetrahydrofuran/water (2 : 1, 3 mL) was stirred at room temperature for 5 hours. Then the mixture was acidified with 1 N hydrochloric acid to pH = 6. The mixture was concentrated *in vacuo* to give crude intermediate **I142** (200 mg, crude) as yellow oil, which was used for the next step directly.

A mixture of crude intermediate **I142** (200 mg crude, 0.45 mmol) , methanamine hydrochloride (93 mg, 1.36 mmol), 2-(7-azabenzotriazol-1-yl)-N,N,N',N'-tetramethyluronium hexafluorophosphate (258 mg, 0.68 mmol) and N-diisopropyl-ethylamine (293 mg, 2.27 mmol) in N,N-dimethylformamide (5 mL) was stirred at room temperature for 2 hours. Then the mixture was poured into water (10 mL) and extracted with ethyl acetate (10 mL). The organic layer was washed with brine (10 mL), dried over Na₂SO₄ and concentrated. The residue was purified by column chromatography on silica gel (dichloromethane : methanol = 30 :1) to give intermediate **I143** (25 mg, 13% yield, 90% purity from LCMS) as yellow oil. **LCMS** (ESI): Rₜ = 1.37 min, mass calcd. for C₂₃H₃₀N₆O₄ 454.2, m/z found 455.4 [M+H]⁺.

A mixture of intermediate **I143** (25 mg, 0.055 mmol) in hydrogen chloride/ether (1 mL) was stirred at room temperature for 3 hours. The mixture was concentrated *in vacuo* to give intermediate **I144** (25 mg, 100% yield, 90% purity from LCMS) as yellow oil. **LCMS** (ESI): Rₜ = 0.64 min, mass calcd. for C₁₈H₂₂N₆O₂ 354.2, m/z found 355.4 [M+H]⁺.

A mixture of intermediate **I144** (25 mg, 0.07 mmol), 3,4-dichlorobenzoic acid (14 mg, 0.07 mmol), 2-(7-azabenzotriazol-1-yl)-N,N,N',N'-tetramethyluronium hexafluorophosphate (53 mg, 0.14 mmol), N-diisopropyl-ethylamine (45 mg, 0.35 mmol) in N,N-dimethylformamide (1 mL) was stirred at room temperature for 3 hours. The reaction mixture was treated with water (10 mL) and extracted with ethyl acetate (10 mL). The organic layer was dried over Na₂SO₄ and filtered. The filtrate was concentrated *in vacuo* to give a residue, which was purified by Pre-HPLC (ACN/H2O = 50 %) to get **compound C79** (10 mg, 27% yield, 99.7% purity by LCMS) as a white solid.

### 3.2.2.24 Synthesis of compounds C85*R and C85*S

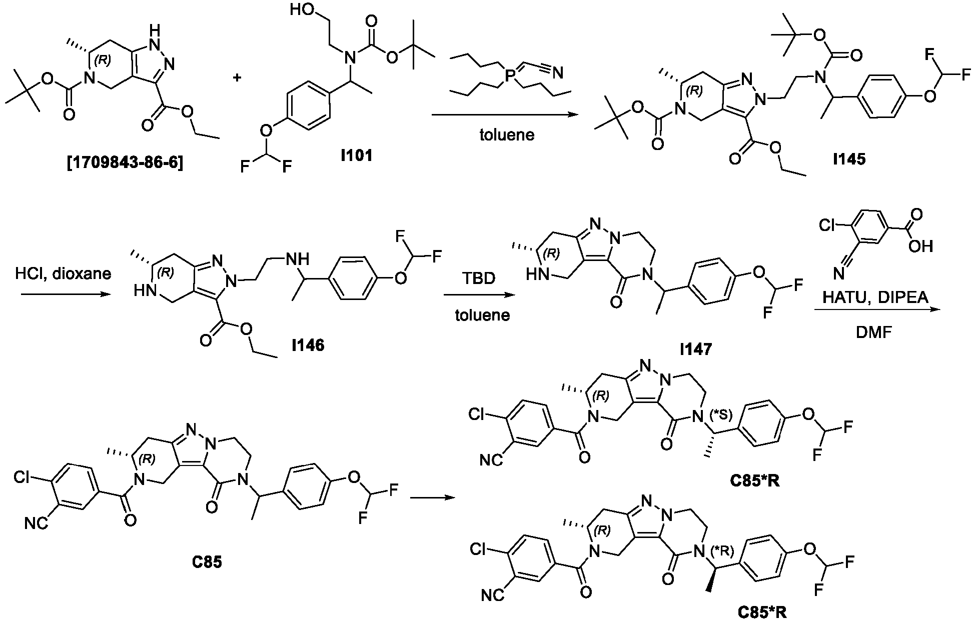

A tube was charged with tert-butyl (1-(4-(difluoromethoxy)phenyl)ethyl)(2-hydroxyethyl)carbamate (500 mg, 1.62 mmol), **I101** (642 mg, 1.94 mmol) in dry toluene (2 mL). The mixture was purged with nitrogen for 2 min. Cyanomethylenetributylphosphorane (2.1 mL, 1 M, 2.10 mmol) was added and the vial was capped. The reaction mixture was heated at 100°C for 16 h. The mixture was concentrated in vacuo and the residue was purified by column chromatography (heptane/EtOAc from 100/5 to 50/50) to afford 5-(tert-butyl) 3-ethyl (6R)-2-(2-((tert-butoxycarbonyl)(1-(4-(difluoromethoxy)phenyl)ethyl)amino)ethyl)-6-methyl-2,4,6,7-tetrahydro-5H-pyrazolo[4,3-c]pyridine-3,5-dicarboxylate **I145** (899 mg, yield 89 %) as a pale brown oil.

A tube was charged with 5-(tert-butyl) 3-ethyl (6R)-2-(2-((tert-butoxycarbonyl)(1-(4-(difluoromethoxy)phenyl)ethyl)amino)ethyl)-6-methyl-2,4,6,7-tetrahydro-5H-pyrazolo[4,3-c]pyridine-3,5-dicarboxylate **I145** (899 mg, 1.44 mmol) in dry 1,4-dioxane (11.3 mL). HCl (3.6 mL, 4M in dioxane, 14.44 mmol) was added and the mixture was stirred at rt for 16 h. The mixture was concentrated in vacuo. The resulting residue was triturated in DIPE, filtered off and dried under vacuum to afford ethyl (6R)-2-(2-((1-(4-(difluoromethoxy)phenyl)ethyl)amino)ethyl)-6-methyl-4,5,6,7-tetrahydro-2H-pyrazolo[4,3-c]pyridine-3-carboxylate **I146** (715 mg, quantitative yield).

A tube was charged with ethyl (6R)-2-(2-((1-(4-(difluoromethoxy)phenyl)ethyl)amino)ethyl)-6-methyl-4,5,6,7-tetrahydro-2H-pyrazolo[4,3-c]pyridine-3-carboxylate **I146** (715 mg, 1.44 mmol) in toluene (18 mL). 1,5,7-Triazabicyclo[4.4.0]ec-5-ene (TBD) (602 mg, 4.33 mmol) was added and the vial capped. The mixture was stirred at 100°C for 2h (full conversion). The mixture was cooled and washed with water. The organic layer was separated, dried over MgSO₄, filtered and concentrated in vacuo. The residue was purified by column chromatography on a 25 g SNAP cartridge in a biotage system using a gradient from (heptane/ [EtOAc-EtOH(3:1)+2%NH₄OH]) from 0/100 to 100/0) to afford (3R)-9-(1-(4-(difluoromethoxy)phenyl)ethyl)-3-methyl-1,2,3,4,8,9-hexahydropyrido[4',3':3,4]pyrazolo[1,5-a]pyrazin-10(7H)-one **I147** (430 mg, yield 79%) as an oil.

To a solution of (3R)-9-(1-(4-(difluoromethoxy)phenyl)ethyl)-3-methyl-1,2,3,4,8,9-hexahydropyrido[4',3':3,4]pyrazolo[1,5-a]pyrazin-10(7H)-one **I147** (338 mg, 0.81 mmol) and 4-chloro-3-cyanobenzoic acid (185 mg, 0.97 mmol) in dry DMF (5.4 mL), were added HATU (460 mg, 1.21 mmol) and DIPEA (0.56 mL, 3.23 mmol) at RT. The reaction mixture was stirred at rt for 16 h. The reaction mixture was poured into water (10 mL) and a pink precipitate was formed. The solid was filtered off and the product was purified via Prep HPLC (Stationary phase: RP XBridge Prep C18 OBD-10µm,50x150mm, Mobile phase: 0.25% NH₄HCO₃ solution in water, CH₃CN) to get a yellow foam. The obtained product **C85** was further purified via SFC to separate the enantiomers: A purification was performed via Prep SFC (Stationary phase: Chiralcel Diacel OJ 20 x 250 mm, Mobile phase: CO₂, EtOH + 0.4 iPrNH₂) yielding **C85*R** (87 mg, yield 20%) and **C85*S** (79 mg, yield 18%), both as yellowish solids.

### 3.2.2.25. Synthesis of C119*R and C119*S

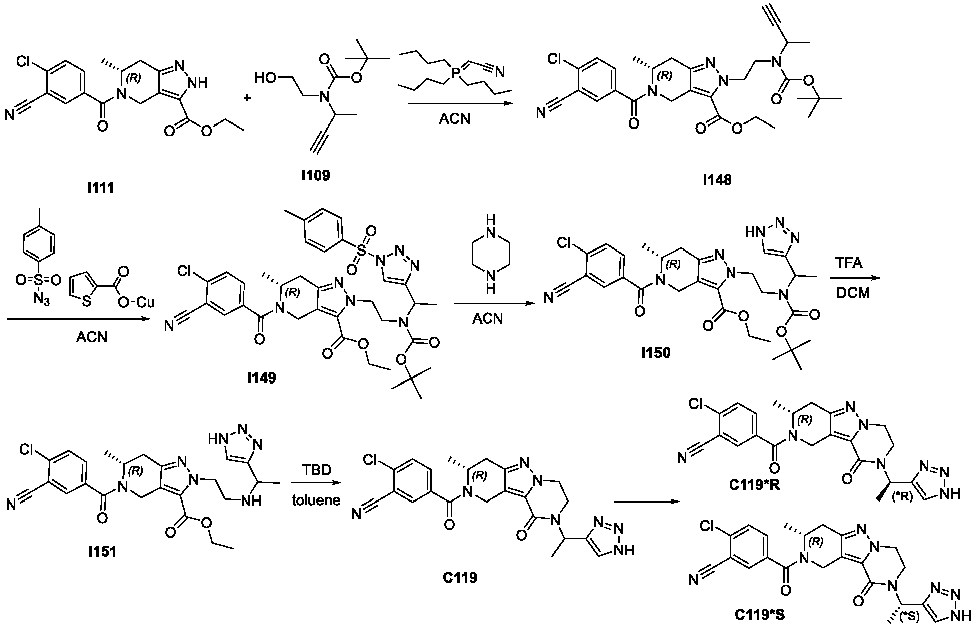

A tube was charged with ethyl (R)-5-(4-chloro-3-cyanobenzoyl)-6-methyl-4,5,6,7-tetrahydro-2H-pyrazolo[4,3-c]pyridine-3-carboxylate **I111** (836 mg, 2.24 mmol), tert-butyl but-3-yn-2-yl(2-hydroxyethyl)carbamate **I109** (573 mg, 2.69 mmol) in dry toluene (14 mL). The mixture was purged with nitrogen for 2 minutes. Cyanomethylenetributylphosphorane (0.77 mL, 2.92 mmol) was added and the vial was capped. The mixture was heated at 100°C for 16h. The mixture was concentrated in vacuo and the residue was purified by column chromatography (heptane/EtOAc from 100/0 to 50/50) to afford ethyl (6R)-2-(2-(but-3-yn-2-yl(tert-butoxycarbonyl)amino)ethyl)-5-(4-chloro-3-cyanobenzoyl)-6-methyl-4,5,6,7-tetrahydro-2H-pyrazolo[4,3-c]pyridine-3-carboxylate **I148** (979 mg, yield 64%) as a red oil.

**I148** (979 mg, 1.72 mmol) was dissolved in DCM (21 mL) and cooled in an icebath. p-toluenesulfonyl azide (1.39 mL, 2.07 mmol) diluted in DCM was added followed by copper(i) thiophene-2-carboxylate (32.9 mg, 0.17 mmol) and stirred for 1 hour. The reaction mixture was filtered, partially concentrated and purified by column chromatography (heptane/EtOAc from 100/0 to 0/100) to afford ethyl (6R)-2-(2-((tert-butoxycarbonyl)(1-(1-tosyl-1H-1,2,3-triazol-4-yl)ethyl)amino)ethyl)-5-(4-chloro-3-cyanobenzoyl)-6-methyl-4,5,6,7-tetrahydro-2H-pyrazolo[4,3-c]pyridine-3-carboxylate **I149** (1.32 g, yield quantitative) as a white foam.

A tube was charged with ethyl (6R)-2-(2-((tert-butoxycarbonyl)(1-(1-tosyl-1H-1,2,3-triazol-4-yl)ethyl)amino)ethyl)-5-(4-chloro-3-cyanobenzoyl)-6-methyl-4,5,6,7-tetrahydro-2H-pyrazolo[4,3-c]pyridine-3-carboxylate **I149** (1.32 g, 1.72 mmol) in dry acetonitrile (ACN) (15 mL). Piperazine (620 mg, 7.21 mmol) was added and the mixture was stirred at 100°C for 1h. The mixture was cooled and concentrated in vacuo. The residue was purified by column chromatography (heptane/EtOAc from 100/0 to 0/100) to afford ethyl (6R)-2-(2-((1-(1H-1,2,3-triazol-4-yl)ethyl)(tert-butoxycarbonyl)amino)ethyl)-5-(4-chloro-3-cyanobenzoyl)-6-methyl-4,5,6,7-tetrahydro-2H-pyrazolo[4,3-c]pyridine-3-carboxylate **I150** (544 mg, yield 52%) as a white foam.

A tube was charged with ethyl (6R)-2-(2-((1-(1H-1,2,3-triazol-4-yl)ethyl)(tert-butoxycarbonyl)amino)ethyl)-5-(4-chloro-3-cyanobenzoyl)-6-methyl-4,5,6,7-tetrahydro-2H-pyrazolo[4,3-c]pyridine-3-carboxylate **I150** (544 mg, 0.89 mmol) in dry DCM (4 mL). TFA (0.82 mL, 10.7 mmol) was added and the mixture was stirred at rt for 2h. The mixture was concentrated in vacuo. The residue was portioned between water and Me-THF and neutralized with Na₂CO₃. The organic layer was separated, dried over MgSO₄, filtered and concentrated in vacuo. The product ethyl (6R)-2-(2-((1-(1H-1,2,3-triazol-4-yl)ethyl)amino)ethyl)-5-(4-chloro-3-cyanobenzoyl)-6-methyl-4,5,6,7-tetrahydro-2H-pyrazolo[4,3-c]pyridine-3-carboxylate **I151** was used as such in the next step.

A tube was charged with **I151** (455 mg, 0.62 mmol) in dry toluene (5 mL). 1,5,7-triazabicyclo[4.4.0]dec-5-ene (600 mg, 4.31 mmol) was added and the vial capped. The reaction mixture was stirred at 100°C for 2 h. The mixture was cooled and washed with water. The organic layer was separated, dried over MgSO₄, filtered and concentrated in vacuo. The product was purified by column chromatography (heptane/EtOAc from 100/0 to 0/100). The resulting product was triturated in DIPE, filtered off and dried under vacuum to afford 5-((3R)-9-(1-(1H-1,2,3-triazol-4-yl)ethyl)-3-methyl-10-oxo-1,2,3,4,7,8,9,10-octahydropyrido[4',3':3,4]pyrazolo[1,5-a]pyrazine-2-carbonyl)-2-chlorobenzonitrile **C119** (198 mg, yield 69%). The racemic mixture was purified by Prep SFC (Stationary phase: Chiralpak Diacel AD 20 x 250 mm, Mobile phase: CO₂, iPrOH + 0.4 iPrNH₂). The two fractions obtained were triturated in DIPE, filtered off and dried under vacuum to afford **C119*R** (50 mg, yield 17%) and **C119*S** (50 mg, yield 17%).

### 3.2.2.26. Synthesis of compound C75 and analogues

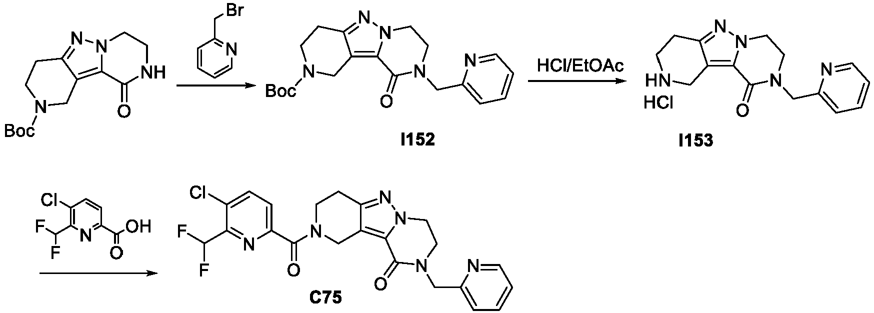

To a solution of 60 % wt. sodium hydride in mineral oil (1.2 g, 20 mmol) in N,N-dimethylformamide (30 mL) was added tert-butyl 10-oxo-3,4,7,8,9,10-hexahydro-pyrido[4',3':3,4]pyrazolo[1,5-a]pyrazine-2(1H)-carboxylate (3 g, 10 mmol) at 0 °C . After stirring at 0°C for 30 min, the mixture was add 2-(bromomethyl) pyridine (2.64 g, 15 mmol) at 0 °C. Then the mixture was stirred at 0 °C under nitrogen atmosphere for 2 hours. The mixture was quenched with saturated ammonium chloride (30 mL) and extracted with ethyl acetate (100 mL) twice. The combined organic layers were washed with brine (50 mL), dried over Na₂SO₄(s) and filtered. The filtrate was concentrated *in vacuo* to give a residue which was purified by C18 column (acetonitrile : water = 50 - 70 %) to afford intermediate **I152** (3 g, 90 % purity from ¹H NMR, 77 % yield) as yellow oil. **LCMS** (ESI): Rₜ = 1.46 min, mass calcd. for C₂₀H₂₅N₅O₃ 383.20, m/z found 384.5 [M+H]⁺. **¹H NMR** (400 MHz, CDCl₃) δ 8.55 (d, *J =* 4.4 Hz, 1H), 7.70 - 7.66 (m, 1H), 7.37 (d, *J =* 8.0 Hz, 1H), 7.24 - 7.20 (t, 1H), 4.84 (s, 2H), 4.71 (s, 2H), 4.33 - 4.30 (t, *J =* 5.6 Hz, 2H), 3.88 - 3.86 (m, 2H), 3.71 (s, 2H), 2.77 (s, 2H), 1.48 (s, 9H).

To a solution of intermediate **I152** (1.00 g, 2.6 mmol) in 4 M HCl/EtOAc (20 mL) was stirred at room temperature for 0.5 hours. The reaction mixture was concentrated under reduce pressure to give intermediate **I53** hydrochloride salt (900 mg, 80 % purity from ¹H NMR, crude) as a yellow solid. **LCMS** (ESI): Rₜ = 0.34 min, mass calcd. for C₁₅H₁₇N₅O 283.14, m/z found 284.3 [M+H]⁺. **¹H NMR** (400 MHz, CDCl₃) δ 9.73 (s, 2H), 8.80 - 8.79 (d, *J =* 5.6 Hz, 1H), 8.41 (s, 1H), 7.95 (d, *J* = 4.0 Hz, 1H), 7.86 (s, 1H), 5.02 (s, 2H), 4.49 (t, *J* = 6.0 Hz, 2H), 4.22 (s, 2H), 4.01 - 3.98(m, 2H), 3.38 (s, 2H), 2.95 - 2.93 (m, 2H).

A solution of intermediate **I153** (100 mg, 0.28 mmol), intermediate **I76** (62 mg, 0.28 mmol), 2-(7-azabenzotriazol-1-yl)-N,N,N',N'-tetramethyluronium hexafluorophosphate (175 mg, 0.42 mmol) and N,N-diisopropylethylamine (115 mg, 0.84 mmol) in N,N-dimethylformamide (5 mL) was stirred at room temperature for 2 hours. The mixture was poured into water (20 mL) and extracted with ethyl acetate (20 mL) twice. The combined organic layers were washed with brine (50 mL), dried over Na₂SO₄(s) and filtered. The filtrate was concentrated *in vacuo* to give a residue, which was purified by C18 column (acetonitrile : water = 35 % to 65 %) to give **compound C75** (80 mg, 99.8 % purity, 60 % yield) as a white solid.

The compounds listed in the table below were made in a similar way :

| Co. No. | Structure | Reagents | Yield |
|---|---|---|---|
| **C71** | 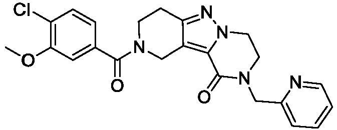 | 1. Intermediate **I153** | 27% |
| | | 2. 4-chloro-3-methoxybenzoic acid | |
| | | 3. HATU | |
| | | 4. N,N-diisopropylethylamine | |
| | | 5. DMF | |
| **C78** | 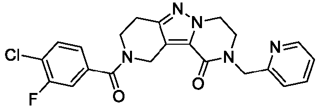 | 1. Intermediate **I153** | 24% |
| | | 2. 4-chloro-3-fluorobenzoic acid | |
| | | 3. HATU | |
| | | 4. N,N-diisopropylethylamine | |
| | | 5. DMF | |
| **C72** | 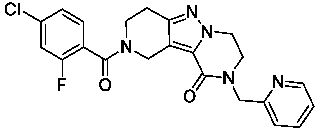 | 1. Intermediate **I153** | 23% |
| | | 2. 4-chloro-2-fluorobenzoic acid | |
| | | 3. HATU | |
| | | 4. N,N-diisopropylethylamine | |
| | | 5. DMF | |
| **C73** | 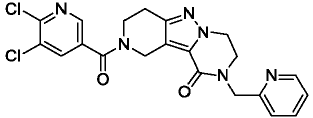 | 1. Intermediate **I153** | 39% |
| | | 2. 5,6-dichloronicotinic acid | |
| | | 3. HATU | |
| | | 4. N,N-diisopropylethylamine | |
| | | 5. DMF | |
| **C74** | 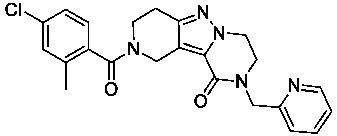 | 1. Intermediate **I153** | 35% |
| | | 2. 4-chloro-2-methylbenzoic acid | |
| | | 3. HATU | |
| | | 4. N,N-diisopropylethylamine | |
| | | 5. DMF | |
| **C76** | 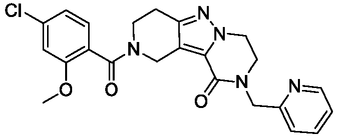 | 1. Intermediate **I153** | 47% |
| | | 2. 4-chloro-2-methoxybenzoic acid | |
| | | 3. HATU | |
| | | 4. N,N-diisopropylethylamine | |
| | | 5. DMF | |

### 3.2.2.27. Synthesis of compound C77

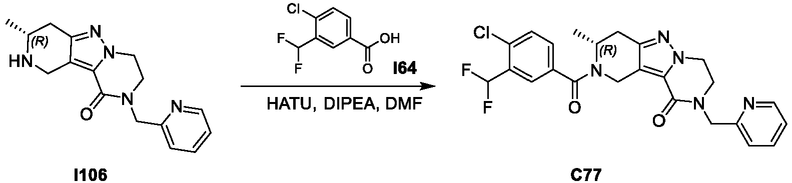

To a solution of intermediate **I106** (100 mg, 95 % purity, 0.34 mmol) and 4-chloro-3-(difluoromethyl)benzoic acid (76 mg, 0.37 mmol) in N,N-dimethylformamide (5 mL) was added O-(7-Azabenzotriazol-1-yl)-N,N,N',N'-tetramethyluronium hexafluorophosphate (192 mg, 0.51 mmol) and N,N-diisopropylethylamine (130 mg, 1.01 mmol) at 0 °C. After stirring at room temperature under nitrogen atmosphere for 2 hours, the mixture was poured into water (30 mL) and extracted with ethyl acetate (30 mL) for three times. The combined organic layers were washed with water (30 mL), dried over Na₂SO₄(s) and filtered. The filtrate was concentrated *in vacuo* to give the residue, which was purified by C18 column (acetonitrile : water = 35 % to 65 %) to give compound **C77** (17 mg, 10 % yield, 99.1% purity) as a white solid.

### 3.2.2.28. Synthesis of compound C94 and 96, 97, 100-102, 104, 107-117, 124 and 125

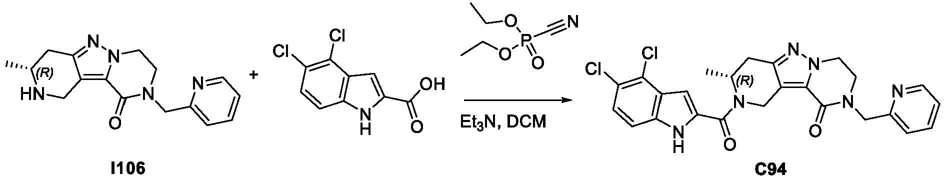

A tube was charged with **I106** (300 mg, 1.00 mmol), 4,5-dichloro-1h-indole-2-carboxylic acid (279 mg, 1.21 mmol) and (0.56 mL, 4.04 mmol) in dry DCM (6 mL) and the vial was sealed in at N₂-atm. Diethyl cyanophosphonate (0.22 mL, 1.31 mmol) was added dropwise and the mixture was stirred at rt for 16 h. The reaction mixture was directly purified by column chromatography (DCM/MeOH from 100/0 to 90/10). The obtained residue was recrystalized in MeOH/ACN. The grey crystals were collected by filtration to afford (R)-2-(4,5-dichloro-1H-indole-2-carbonyl)-3-methyl-9-(pyridin-2-ylmethyl)-1,2,3,4,8,9-hexahydropyrido[4',3':3,4]pyrazolo[1,5-a]pyrazin-10(7H)-one **C94** (302 mg, yield 59%).

The compounds listed in the table below were obtained in an analogous manner.

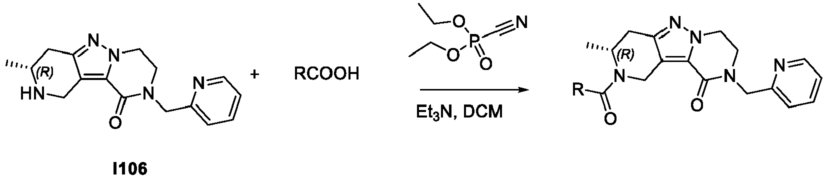

| Co. No. | Structure | RCCOH | Yield % |
|---|---|---|---|
| **C94** | 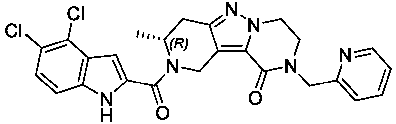 | 231295-84-4 | 59 |
| | | 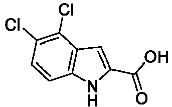 | |
| **C96*** | 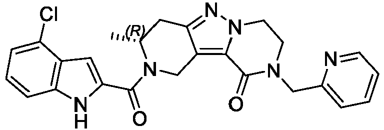 | 24621-73-6 | 38 |
| | | 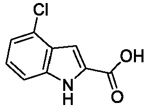 | |
| **C97** | 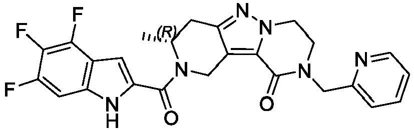 | 884494-64-8 | 71 |
| | | 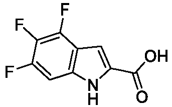 | |
| **C100** | 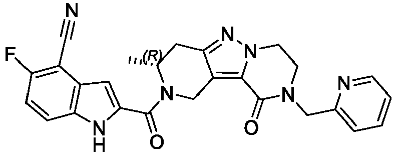 | 2091608-10-3 | 62 |
| | | 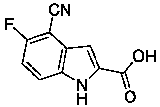 | |
| **C101** | 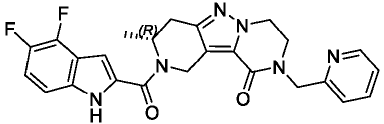 | 884494-61-5 | 61 |
| | | 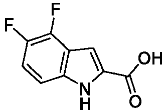 | |
| **C102** | 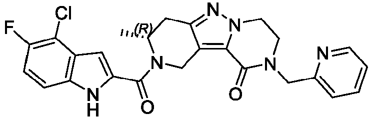 | 169674-08-2 | 64 |
| | | 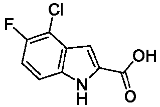 | |
| **C104** | 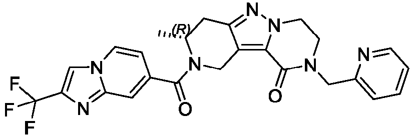 | 1260777-52-3 | 54 |
| | | 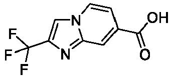 | |
| **C107** | 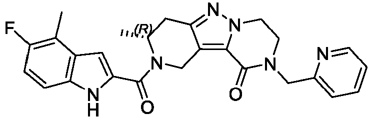 | 50536-62-4 | 57 |
| | | 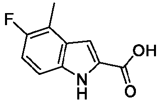 | |
| **C108** | 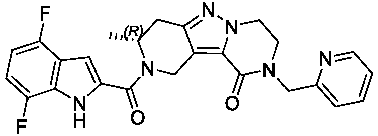 | 247564-67-6 | 61 |
| | | 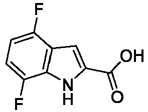 | |
| **C109** | 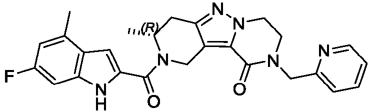 | 1249870-64-1 | 60 |
| | | 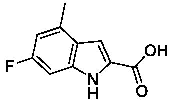 | |
| **C110** | 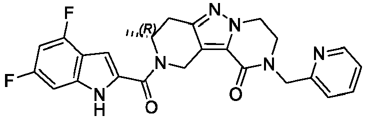 | 247564-66-5 | 65 |
| | | 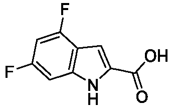 | |
| **C111** | 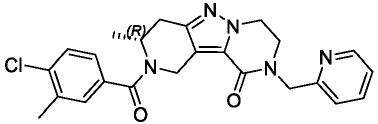 | 7697-29-2 | 43 |
| | | 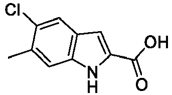 | |
| **C112** | 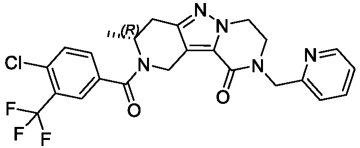 | 1737-36-6 | 41 |
| | | 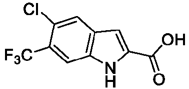 | |
| **C113** | 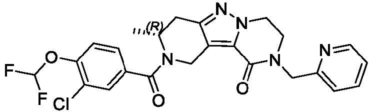 | 1192829-74-5 | 18 |
| | | 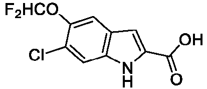 | |
| **C114** | 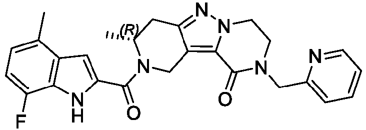 | 383132-49-8 | 54 |
| | | 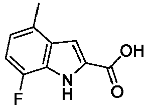 | |
| **C115** | 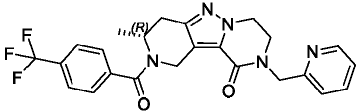 | 455-24-3 | 47 |
| | | 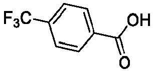 | |
| **C116** | 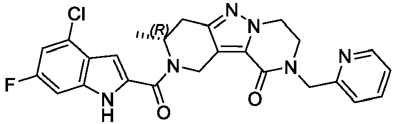 | 383133-62-8 | 72 |
| | | 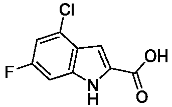 | |
| **C117** | 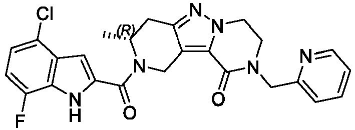 | 383133-62-8 | 64 |
| | | 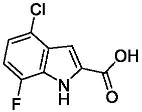 | |
| **C124** | 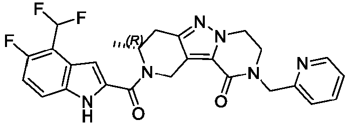 | **I85** | 35 |
| | | 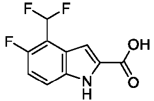 | |
| **C125** | 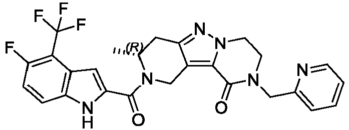 | **I79** | 11 |
| | | 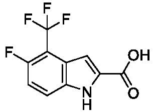 | |

| | | | |
|---|---|---|---|
| * 1-propanephosphonic anhydride (1.5 eq.) was used (instead of DECP), Et₃N (4 eq.) was replaced by DIPEA and DCM was substituted by DMF. | | | |

### 3.2.2.29. Synthesis of compounds C126 and 123, 127-131

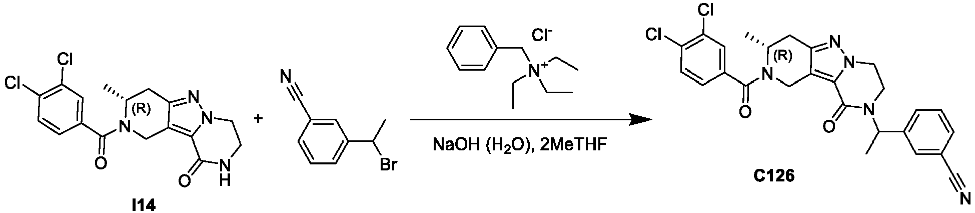

A solution of **I14** (50 mg, 0.132 mmol) in 2-MeTHF (5 mL) was added to a Radley tube containing 3-(1-bromoethyl)benzonitrile [1096159-01-1] (33 mg, 0.158 mmol). Then, a solution of benzyltriethylammonium chloride [56-37-1] (3 mg, 0.013 mmol) in distilled water (10 µL) and a solution of NaOH (50% in H₂O) [1310-73-2] (348 µL, 1.515 g/mL, 6.59 mmol) were added. The bi-phasic mixture was stirred vigorously for 1 hour at 50°C, and then overnight at room temperature. H₂O (500 µL) was added, the biphasic mixture was filtered over HM-N drying cartridges and rinsed with 2-MeTHF (2 x 6 mL). The filtrate was concentrated, and the residue was purified by Prep HPLC (Stationary phase: RP XBridge Prep C18 OBD-10µm,30x150mm, Mobile phase: 0.25% NH₄HCO₃ solution in water, CH₃CN) to yield **C126** as a solid.

The compounds listed in the table below were obtained in an analogous manner.

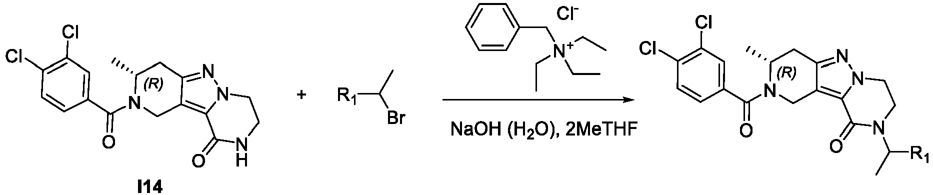

| Co. No. | Reagent | Product | Yield (%) |
|---|---|---|---|
| **C131** | [88563-83-1] | 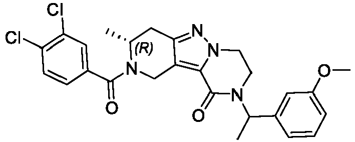 | 31 |
| | 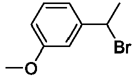 | | |
| **C130** | [1824272-95-8] | 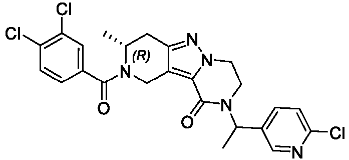 | 35 |
| | 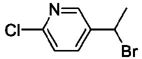 | | |
| **C129** | [40603-95-0] | 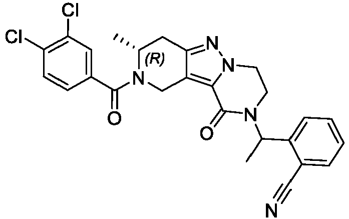 | 67 |
| | 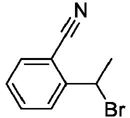 | | |
| **C128** | [1253571-23-1] | 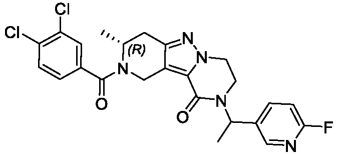 | 60 |
| | 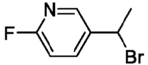 | | |
| **C127** | [68120-57-0] | 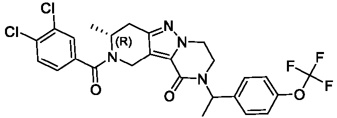 | 49 |
| | 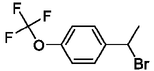 | | |
| **C123** | [194152-29-9] | 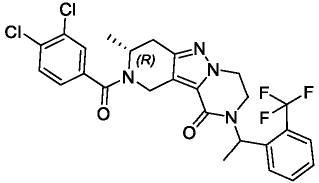 | RS not isolate d |
| | 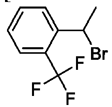 | | |

### 3.2.2.30. Synthesis of compound C122 and 91, 95, 106 and 120

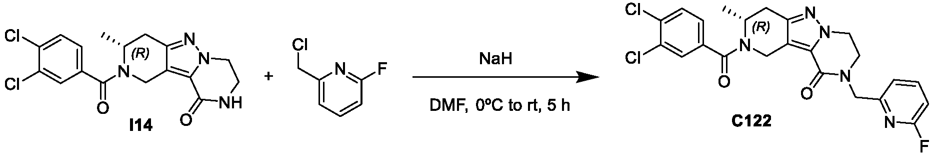

NaH [7646-69-7] (14 mg, 0.360 mmol, 60%) was added to a solution of **I14** (100 mg, 0.24 mmol) in DMF (2 mL) at 0°C. The mixture was warmed to room temperature and stirred for 30 minutes before 2-(chloromethyl)-6-fluoropyridine [315180-16-6] (0.054 mL, 0.48 mmol) was added. The reaction mixture was stirred at room temperature for 5 hours, and then, diluted with EtOAc and water. The organic layer was separated, washed with an aqueous saturated solution of NaHCO₃, brine (2 x 15 mL), dried over Na₂SO₄ and concentrated in vacuo. The crude was purified by column chromatography (DCM/MeOH from 100 to 95:5). The product was co-evaporated with EtOH and dried under vacuum. Then, it was triturated in Et₂O (6 mL) and washed with EtOH (10 mL) to afford compound **C122** (86 mg, 48% yield) as a beige powder.

The compounds listed in the table below were obtained in an analogous manner.

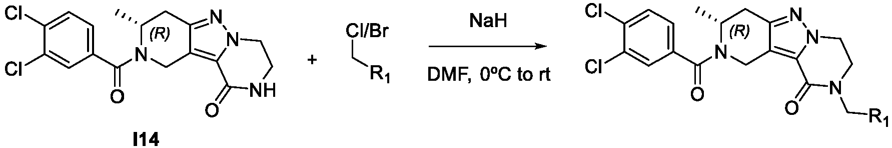

| Co. No. | Reagent | Structure | Yield (%) |
|---|---|---|---|
| **C120** | [946773-13-3] | 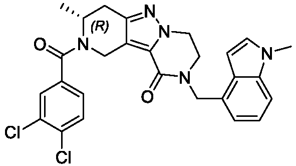 | 20 |
| | 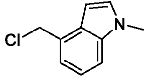 | | |
| **C106** | [101990-70-9] | 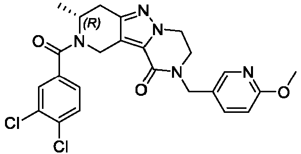 | 67 |
| | 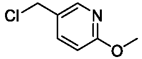 | | |
| **C95** | [1261686-95-6] | 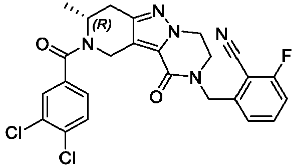 | 56 |
| | 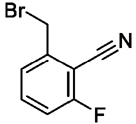 | | |
| **C91** | [70258-18-3] | 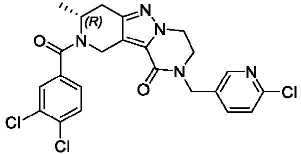 | 79 |
| | 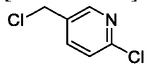 | | |

### 3.2.2.30. Synthesis of compound C121

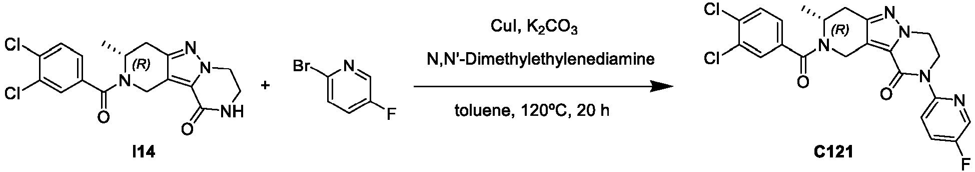

To a solution of **I14** (150 mg, 0.36 mmol) in toluene (1.2 mL), 2-bromo-5-fluoropyridine [41404-58-4] (53 mg, 0.24 mmol), CuI [7681-65-4 ] (14 mg, 0.072 mmol), N,N'-dimethylethylenediamine [110-70-3] (0.023 mL, 0.216 mmol, 0.819 g/mL) and K₂CO₃ [584-08-7] (99 mg, 0.52 mmol) were successively added under argon atmosphere. The reaction mixture was heated at 120°C for 20 hours. H₂O (20 mL) and EtOAc (20 mL) were added to the reaction mixture, the aqueous layer was separated and extracted with EtOAc (2 x 10 mL). The combined organic layers were washed with brine (10 mL), dried over Na₂SO₄, filtered and concentrated in vacuo. The residue was purified by column chromatography (DCM/MeOH from 100:0 to 95:5). The obtained solid was co-evaporated with EtOH, dried under vacuum and recrystallized with EtOAc/EtOH. The solid was collected by filtration to yield **C121** (130 mg, 72% yield) as a white powder.

### 3.2.2.31 Synthesis of compound C118 and 86-88, 90, 92, 93, 98, 99, and 103.

### Intermediate I154 and analogues

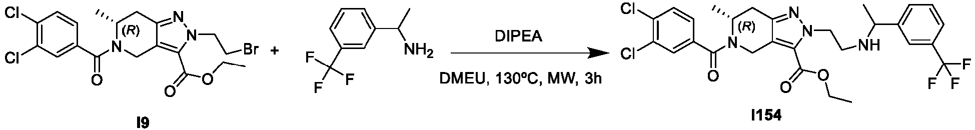

DIPEA [7087-68-5] (0.1 mL, 0.589 mmol, 0.75 g/mL) was added to a solution of intermediate **I9** (300 mg, 0.491 mmol) and 1-(3-trifluoromethylphenyl)ethylamine [59382-36-4] (139 mg, 0.736 mmol) in DMEU (3 mL). The reaction was stirred at under the microwave at 125°C for 1 hour. The reaction mixture was used in the next step without any purification.

The compounds listed in the table below were obtained in an analogous manner.

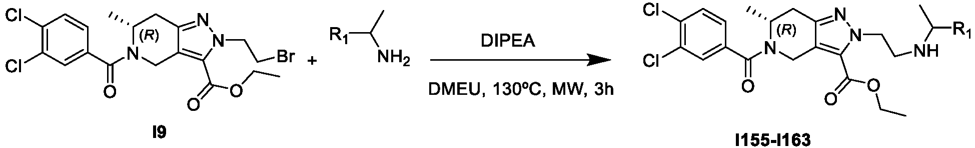

| Reagent | Intermediate obtained |
|---|---|
| [2179323-61-6] | 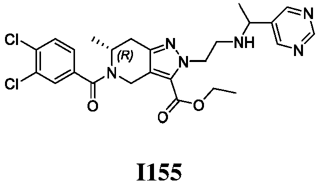 |
| 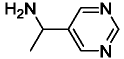 | |
| [42088-91-5] | 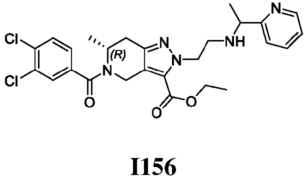 |
| 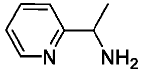 | |
| [24358-62-1] | 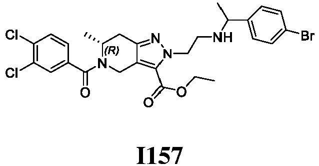 |
| 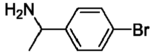 | |
| [618-36-0] | 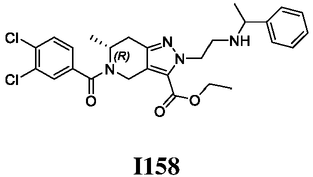 |
| 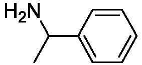 | |
| [50919-08-9] | 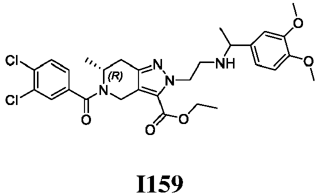 |
| 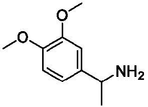 | |
| [306761-19-3] | 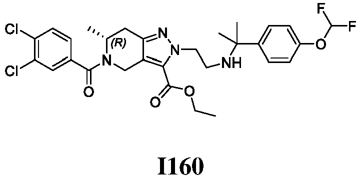 |
| 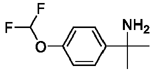 | |
| [86225-78-7] | 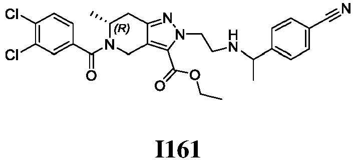 |
| 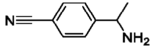 | |
| [1179072-43-5] | 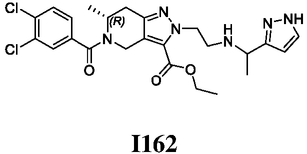 |
| 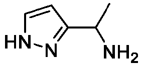 | |
| [105321-49-1] | 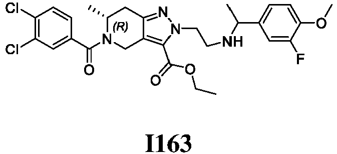 |
| 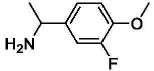 | |

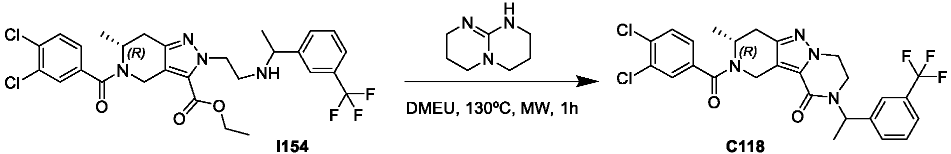

1,5,7-Triazabicyclo[4.4.0]dec-5-ene [5807-14-7] (150 mg, 1.08 mmol) was added to a solution of **I154** (293 mg, 0.49 mmol) in 1,3-dimethyl-2-imidazolidinone [80-73-9] (3 mL) in a microwave vial. The reaction mixture was heated 130°C for 1 hour in the microwave. Then, it was diluted with CH₃CN, and purified by RP-HPLC (Stationary phase: RP Xbridge Prep C18 OBD-10µm,50x150mm, Mobile phase: 0.25% NH₄HCO₃ solution in water, CH₃CN). The solid was recrystallized with MeOH (2 mL). The product was filtered, washed with H₂O and dried at 50°C under vacuum to yield compound **C118** (85 mg, 31% yield).

The compounds listed in the table below were obtained in an analogous manner.

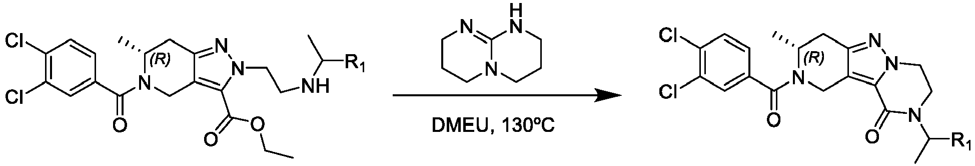

| Reagent | Compound structure | Co. No. | Yield (%) |
|---|---|---|---|
| **I155** | 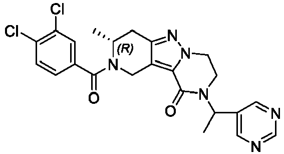 | **C103** | 35 |
| 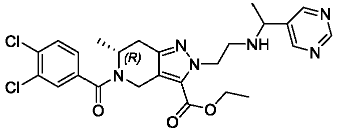 | | | |
| **I156** | 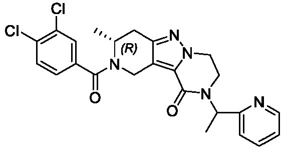 | **C99** | 62 |
| 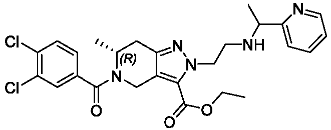 | | | |
| **I157** | 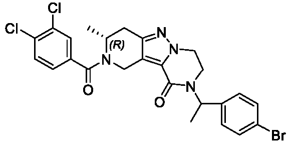 | **C98** | 59 |
| 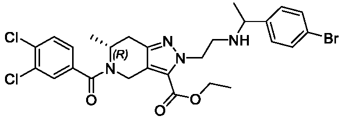 | | | |
| **I158** | 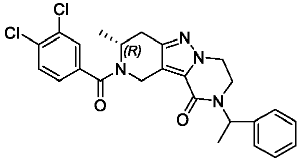 | **C93** | 57 |
| 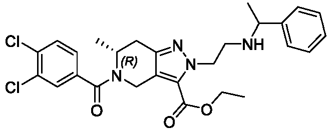 | | | |
| **I159** | 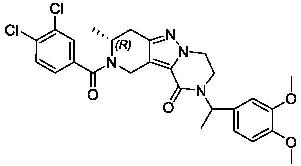 | **C92** | 58 |
| 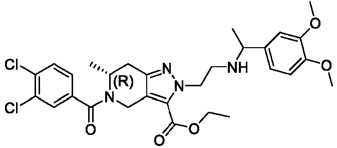 | | | |
| **I160** | 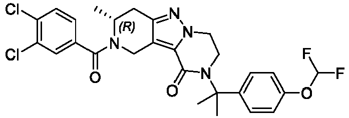 | **C90** | 9 |
| 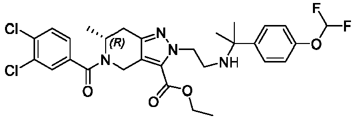 | | | |
| **I161** | 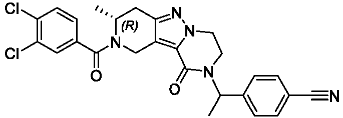 | **C88** | 48 |
| 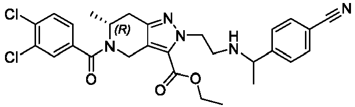 | | | |
| **I162** | 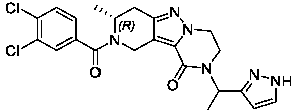 | **C87** | 58 |
| 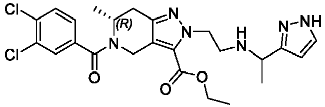 | | | |
| **I163** | 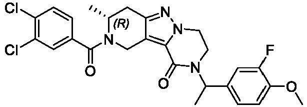 | **C86** | 23 |
| 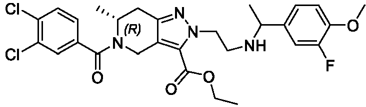 | | | |

### 3.2.2.32 Synthesis of compound C84 and 89 and 105.

### Synthesis of intermediate I164

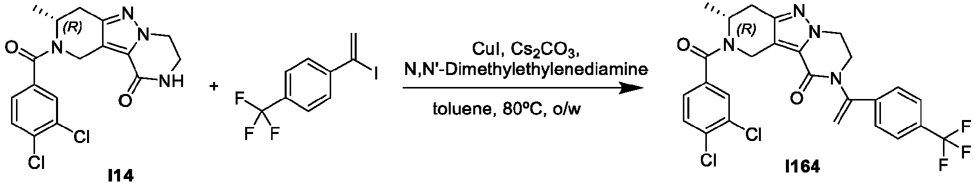

In a vial under a N2 atm, **I14** (250 mg, 0.659 mmol), 1-(1-Iodoethenyl)-4-(trifluoromethyl)benzene [359887-55-1] (393 mg, 1.318 mmol), CuI [7681-65-4] (75 mg, 0.396 mmol), Cs₂CO₃ [534-17-8] (430 mg, 1.318 mmol) were dissolved in toluene [108-88-3] (3 mL). Then, N,N'-dimethylethylenediamine [110-70-3] (85 µL, 0.791 mmol, 0.819 g/mL) was added and the reaction mixture was stirred at 80°C over the weekend. The reaction mixture was quenched with NH₄Cl saturated aqueous solution and extracted with EtOAc (3 times). The combined organic layers were dried over MgSO₄, filtered and concentrated under reduced pressure. The crude residue was purified by column chromatography (heptane/EtOAc from 50/50 to 20/80) to give **I164** (186 mg, 51% yield, 0.339 mmol) as a white solid.

The intermediate listed in the table below were obtained in an analogous manner.

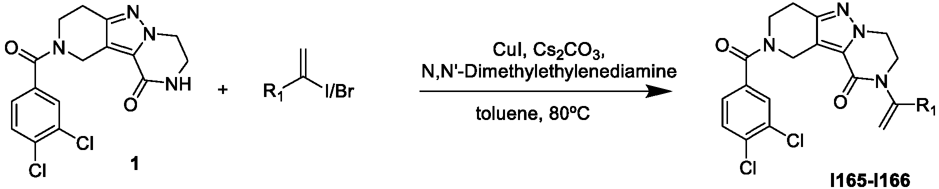

| Reagent | Intermediate | Yield (%) |
|---|---|---|
| [98-81-7] | 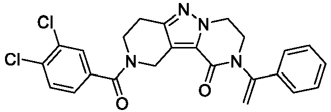 **I165** | 81 |
| 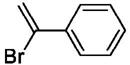 | | |
| [359887-55-1] | 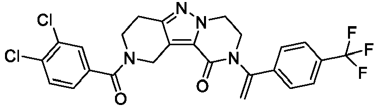 **I166** | 74 |
| 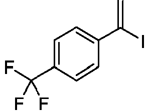 | | |

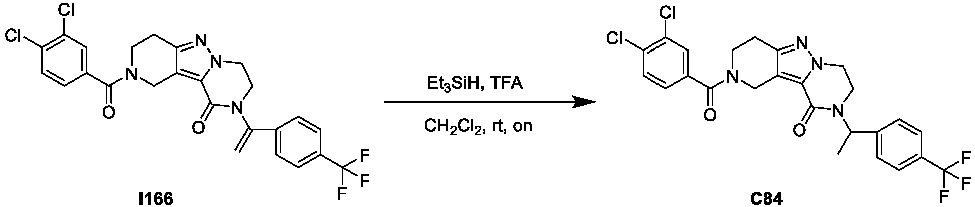

To a vigorously solution of **I166** (68 mg, 0.146 mmol) (68 mg, 0.146 mmol) in CH₂Cl₂ (1.5 mL) under a N₂ atmosphere, Et₃SiH [617-86-7] (116 µL, 0.728 mmol, 0.728 g/mL) was added. TFA [1493-13-6] (32 µL, 0.364 mmol, 1.696 g/mL,) was added dropwise at 0°C. The reaction mixture was stirred at room temperature overnight. The crude mixture was poured into an aqueous NaOH solution (1M) and extracted with DCM (4 times). Combined organic layers were dried over MgSO4, filtered and concentrated. The residue was purified by column chromatography (100% EtOAc) to afford **C84** (34 mg, 28% yield) as a white solid.

The compounds listed in the table below were obtained in an analogous manner.

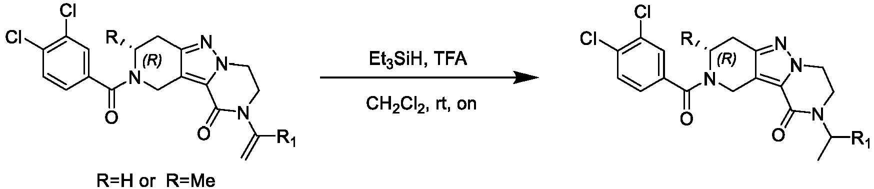

| Reagent | Compound structure | Cmpd n° | Yield (%) |
|---|---|---|---|
| **I165** | 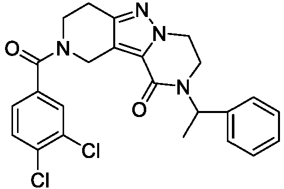 | **C89** | 29 |
| 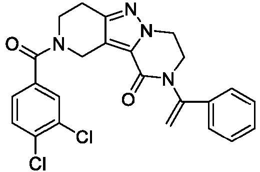 | | | |
| **I164** | 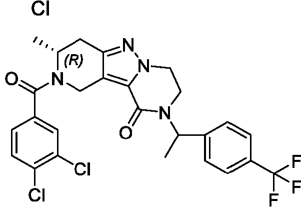 | **C105** | 74 |
| 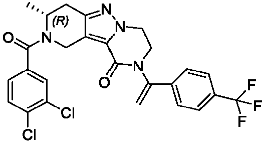 | | | |

### 3.2.2.33 Separation of diastereoisomers

The diastereoisomers listed in the table below were obtained by SFC separation of the corresponding mixture.

| Co. No. | Structure | Method |
|---|---|---|
| **C88*S** | 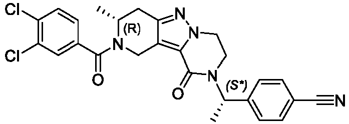 | Prep SFC (Stationary phase: Chiralpak Diacel AD 20 x 250 mm, Mobile phase: CO₂, EtOH + 0.4 iPrNH₂) |
| **C88*R** | 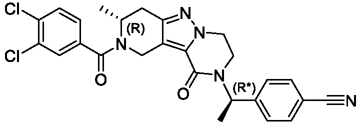 | Prep SFC (Stationary phase: Chiralpak Diacel AD 20 x 250 mm, Mobile phase: CO₂, EtOH + 0.4 iPrNH₂) |
| **C123*R** | 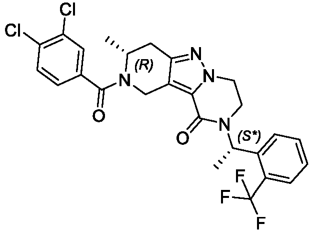 | Prep SFC (Stationary phase: Chiralpak Diacel AD 20 x 250 mm, Mobile phase: CO₂, EtOH-iPrOH (50-50) + 0.4% iPrNH₂) |
| **C123*S** | 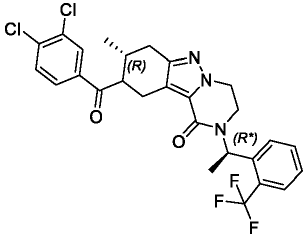 | Prep SFC (Stationary phase: Chiralpak Diacel AD 20 x 250 mm, Mobile phase: CO₂, EtOH-iPrOH (50-50) + 0.4% iPrNH₂) |
| **C105*R** | 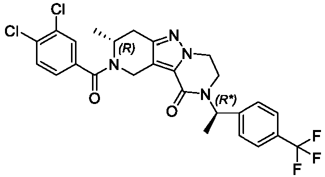 | Prep SFC (Stationary phase: Chiralpak Daicel IC 20 x 250 mm, Mobile phase: CO₂, EtOH + 0.4 iPrNH₂) |
| **C105*S** | 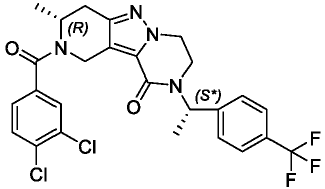 | Prep SFC (Stationary phase: Chiralpak Daicel IC 20 x 250 mm, Mobile phase: CO₂, EtOH + 0.4 iPrNH₂) |

### 3.2.2.34 Synthesis of compound C132 and 133-250.

### General Procedure X1

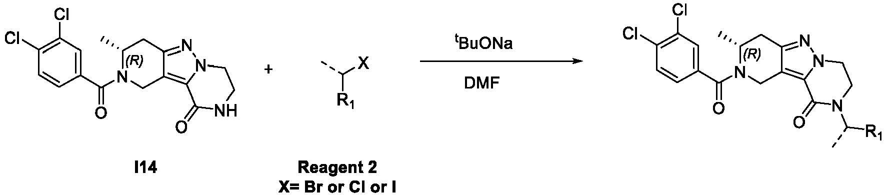

**I14** (1 mmol) and **Reagent** 2 (1.2 mmol) were placed in a vial. DMF (1 mL) and a solution of ^{t}BuONa (3 mmol, 3.0 eq.) [865-48-5] in THF were added. The reaction mixture was stirred for 16 hours at 60 °C. After cooling to the ambient temperature, the mixture was quenched with excess of acetic acid and evaporated. The residue was dissolved in DMSO, filtered, and the solution was subjected to HPLC purification*.

### General Procedure X2

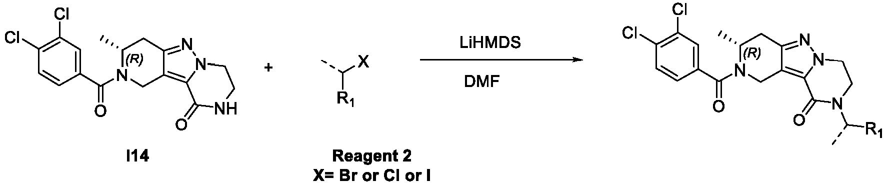

**I14** (1 mmol) and **Reagent 2** (1.3 mmol) were placed in a vial. Then dry DMF (1 mL) and a solution of LiHMDS (2 mmol, 2.0 eq.) in THF were added. The reaction mixture was stirred for 16 hours at 60 °C. After cooling to the ambient temperature, the mixture was quenched with excess of acetic acid and evaporated. The residue was dissolved in DMSO, filtered, and the solution was subjected to HPLC purification*.

*The purification was performed using Agilent 1260 Infinity systems equipped with DAD and mass-detector. Waters Sunfire C18 OBD Prep Column, 100 A, 5 µm, 19 mm x 100 mm with SunFire C18 Prep Guard Cartridge, 100 A, 10 µm, 19 mm x 10 mm was used. Deionized Water (phase A) and HPLC-grade Methanol (phase B) were used as an eluent. In some cases, ammonia or TFA was used as an additive to improve the separation of the products. In these cases, free bases and TFA salts of the products were formed respectively.

The compounds listed in the table below were synthetized by using method X1 or method X2

| **Co. No.** | Structure | Method | HPLC method | Yield (%) |
|---|---|---|---|---|
| **C132** | 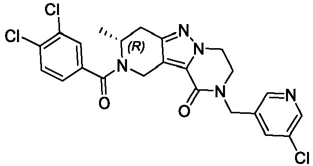 | X1 | RP, MeOH+ammonia, gradient 50 - 100% | 21 |
| **C133** | 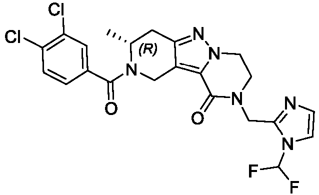 | X1 | RP, MeOH+ammonia, gradient 40 - 90% | 19 |
| **C134** | 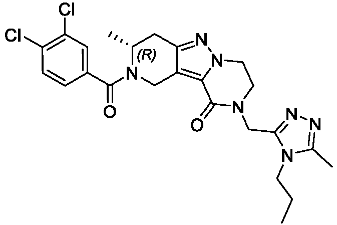 | X1 | RP, MeOH+ammonia, gradient 30 - 80% | 32 |
| **C135** | 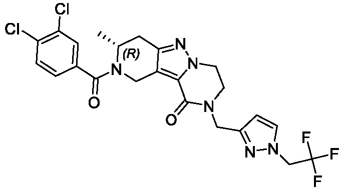 | X1 | RP, MeOH+ammonia, gradient 40 - 90% | 39 |
| **C136** | 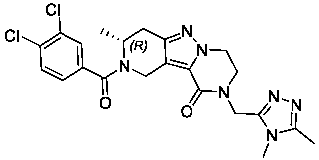 | X1 | RP, MeOH+ammonia, gradient 30 - 80% | 11 |
| **C137** | 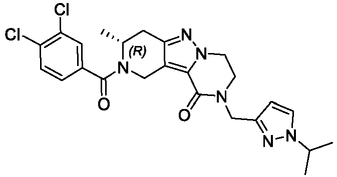 | X2 | RP, MeOH+ammonia, gradient 50 - 100% | 45 |
| **C138** | 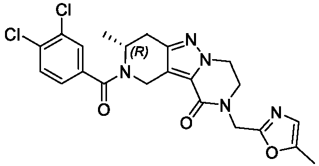 | X1 | RP, MeOH+ammonia, gradient 30 - 80% | 29 |
| **C139** | 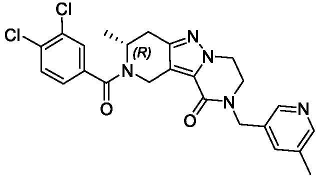 | X1 | RP, MeOH+ammonia, gradient 50 - 100% | 40 |
| **C140** | 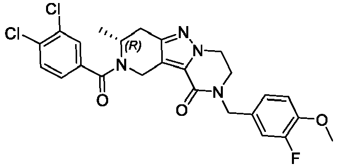 | X1 | RP, MeOH+ammonia, gradient 50 - 100% | 43 |
| **C141** | 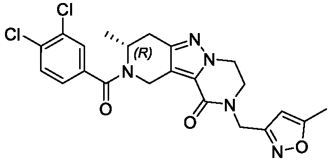 | X2 | RP, MeOH+ammonia, gradient 40 - 90% | 41 |
| **C142** | 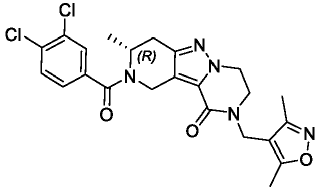 | X1 | RP, MeOH+ammonia, gradient 40 - 90% | 37 |
| **C143** | 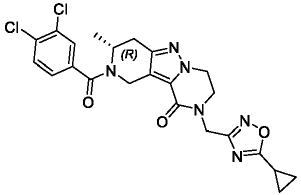 | X1 | RP, MeOH+ammonia, gradient 30 - 80% | 25 |
| **C144** | 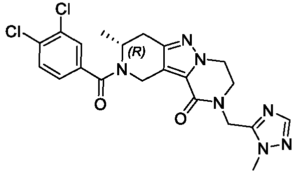 | X1 | RP, MeOH+ammonia, gradient 30 - 80% | 27 |
| **C145** | 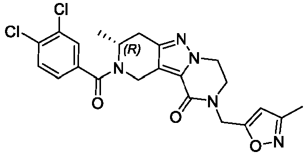 | X1 | RP, MeOH+ammonia, gradient 40 - 90% | 29 |
| **C146** | 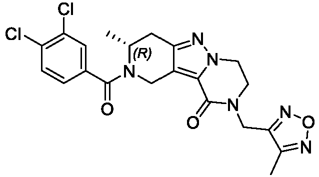 | X1 | RP, MeOH+ammonia, gradient 40 - 90% | 23 |
| **C147** | 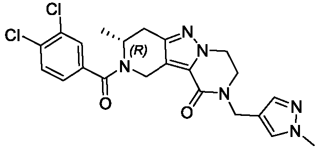 | X1 | RP, MeOH+ammonia, gradient 40 - 90% | 20 |
| **C148** | 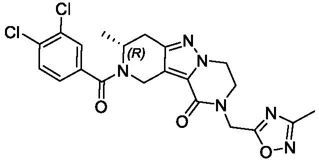 | X1 | RP, MeOH+ammonia, gradient 30 - 80% | 14 |
| **C149** | 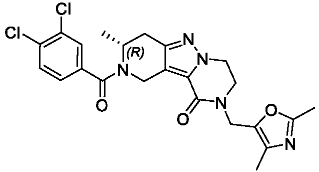 | X1 | RP, MeOH+ammonia, gradient 40 - 90% | 4 |
| **C150** | 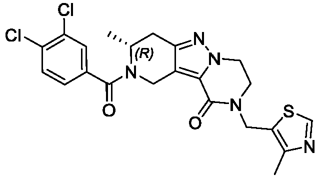 | X1 | RP, MeOH+ammonia, gradient 50 - 100% | 22 |
| **C151** | 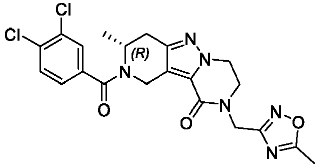 | X1 | RP, MeOH+ammonia, gradient 30 - 80% | 16 |
| **C152** | 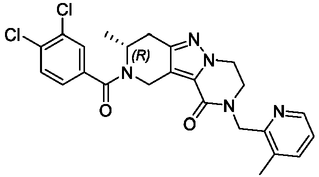 | X1 | RP, MeOH+ammonia, gradient 50 - 100% | 15 |
| **C153** | 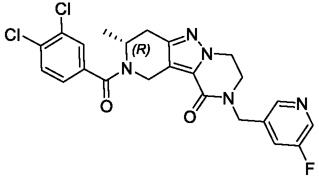 | X1 | RP, MeOH+ammonia, gradient 50 - 100% | 21 |
| **C154** | 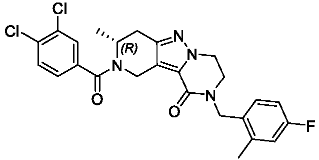 | X1 | RP, MeOH+ammonia, gradient 60 - 100% | 34 |
| **C155** | 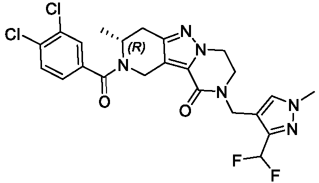 | X1 | RP, MeOH+ammonia, gradient 40 - 90% | 35 |
| **C156** | 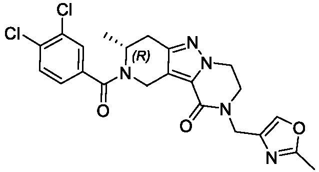 | X1 | RP, MeOH+ammonia, gradient 30 - 80% | 33 |
| **C157** | 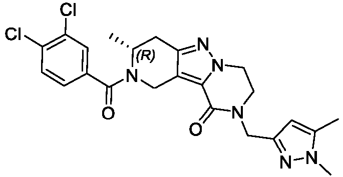 | X1 | RP, MeOH+ammonia, gradient 40 - 90% | 14 |
| **C158** | 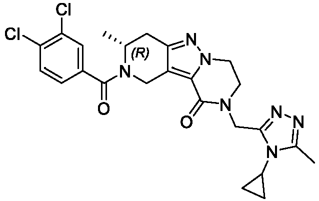 | X1 | RP, MeOH+ammonia, gradient 30 - 80% | 13 |
| **C159** | 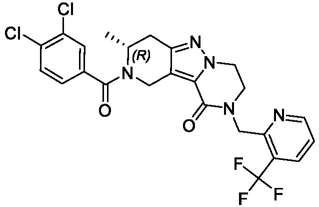 | X1 | RP, MeOH+ammonia, gradient 50 - 100% | 12 |
| **C160** | 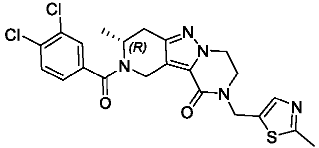 | X1 | RP, MeOH+ammonia, gradient 50 - 100% | 16 |
| **C161** | 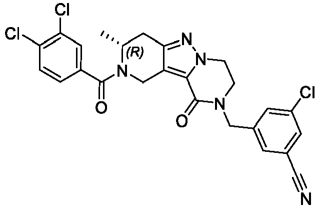 | X1 | RP, MeOH+ammonia, gradient 50 - 100% | 19 |
| **C162** | 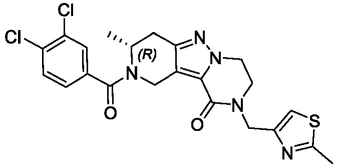 | X1 | RP, MeOH+ammonia, gradient 50 - 100% | 30 |
| **C163** | 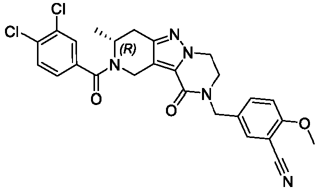 | X1 | RP, MeOH+ammonia, gradient 50 - 100% | 41 |
| **C164** | 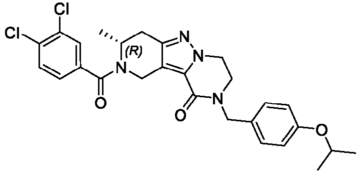 | X1 | RP, MeOH+ammonia, gradient 60 - 100% | 26 |
| **C165** | 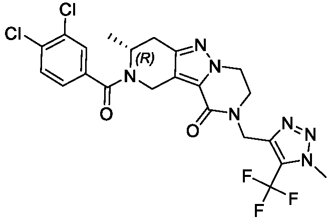 | X1 | RP, MeOH+ammonia, gradient 40 - 90% | 44 |
| **C166** | 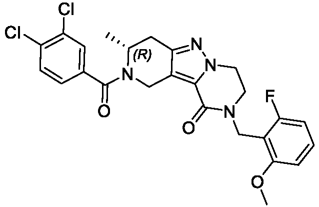 | X1 | RP, MeOH+ammonia, gradient 50 - 100% | 42 |
| **C167** | 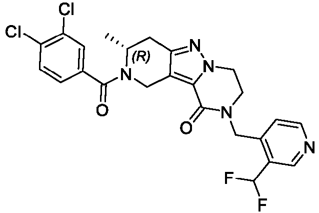 | X1 | RP, MeOH+ammonia, gradient 40 - 90% | 19 |
| **C168** | 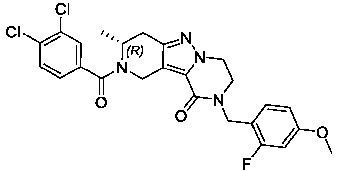 | X1 | RP, MeOH+ammonia, gradient 50 - 100% | 25 |
| **C169** | 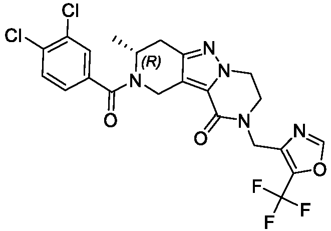 | X1 | RP, MeCN+ammonia, gradient 40 - 90% | 14 |
| **C170** | 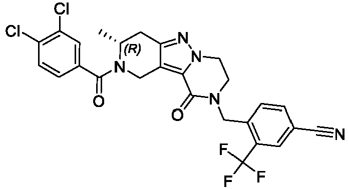 | X1 | RP, MeOH+ammonia, gradient 60 - 100% | 15 |
| **C171** | 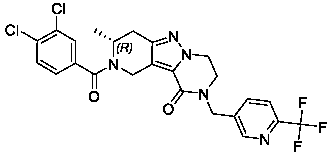 | X1 | RP, MeOH+ammonia, gradient 50 - 100% | 34 |
| **C172** | 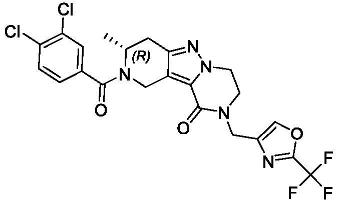 | X1 | RP, MeOH+ammonia, gradient 40 - 90% | 29 |
| **C173** | 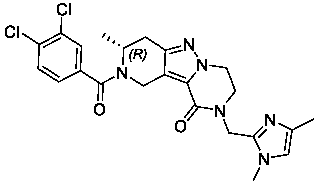 | X1 | RP, MeOH+ammonia, gradient 40 - 90% | 19 |
| **C174** | 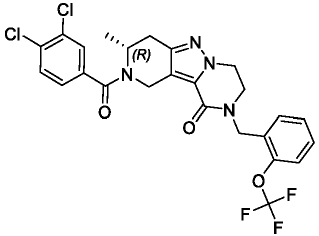 | X1 | RP, MeOH+ammonia, gradient 60 - 100% | 31 |
| **C175** | 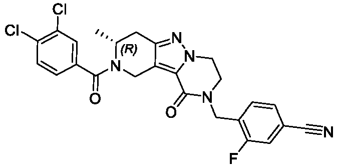 | X1 | RP, MeOH+ammonia, gradient 50 - 100% | 16 |
| **C176** | 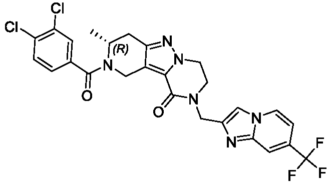 | X1 | RP, MeOH+ammonia, gradient 50 - 100% | 20 |
| **C177** | 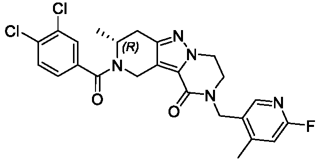 | X1 | RP, MeOH+ammonia, gradient 50 - 100% | 40 |
| **C178** | 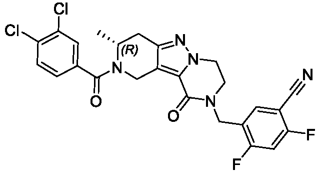 | X1 | RP, MeOH+ammonia, gradient 50 - 100% | 26 |
| **C179** | 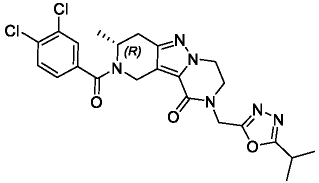 | X1 | RP, MeOH+ammonia, gradient 30 - 80% | 31 |
| **C180** | 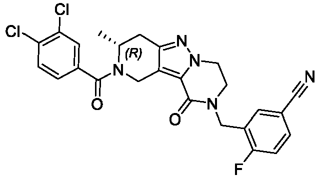 | X1 | RP, MeOH+ammonia, gradient 50 - 100% | 20 |
| **C181** | 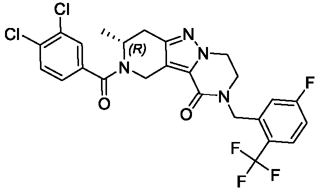 | X1 | RP, MeOH+ammonia, gradient 60 - 100% | 17 |
| **C182** | 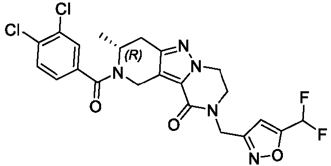 | X1 | RP, MeOH+ammonia, gradient 40 - 90% | 53 |
| **C183** | 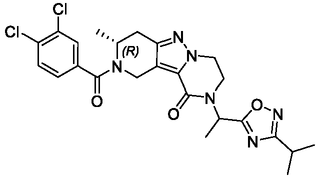 | X1 | RP, MeOH+ammonia, gradient 50 - 100% | 23 |
| **C184** | 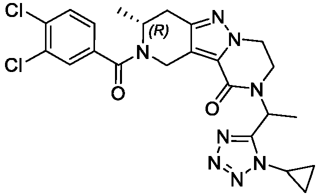 | X1 | RP, MeCN+ammonia, gradient 40 - 90% | 33 |
| **C185** | 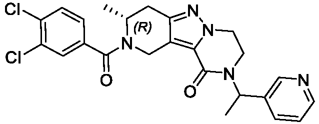 | X1 | RP, MeOH+ammonia, gradient 50 - 100% | 13 |
| **C186** | 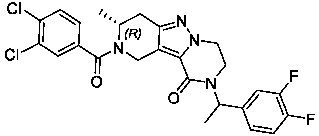 | X1 | RP, MeOH+ammonia, gradient 60 - 100% | 28 |
| **C187** | 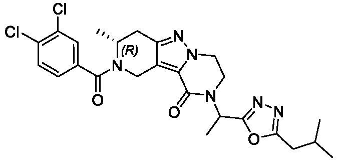 | X2 | RP, MeOH+ammonia, gradient 40 - 90% | 14 |
| **C188** | 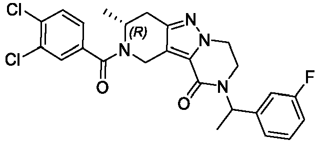 | X2 | RP, MeOH+ammonia, gradient 60 - 100% | 28 |
| **C189** | 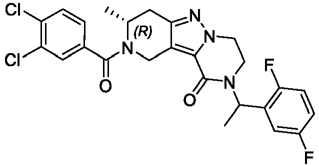 | X2 | RP, MeOH+ammonia, gradient 60 - 100% | 24 |
| **C190** | 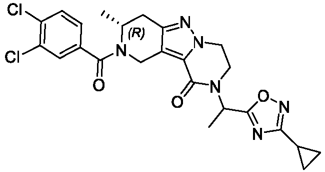 | X1 | RP, MeOH+ammonia, gradient 40 - 90% | 19 |
| **C191** | 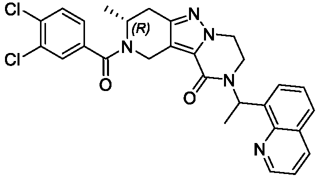 | X1 | RP, MeOH+ammonia, gradient 50 - 100% | 21 |
| **C192** | 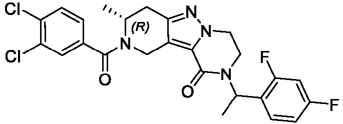 | X1 | RP, MeOH+ammonia, gradient 60 - 100% | 32 |
| **C193** | 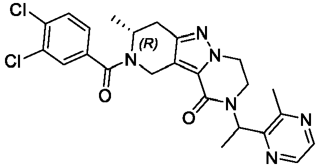 | X1 | RP, MeOH+ammonia, gradient 40 - 90% | 41 |
| **C194** | 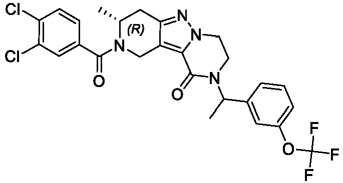 | X1 | RP, MeOH+ammonia, gradient 60 - 100% | 30 |
| **C195** | 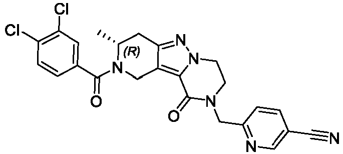 | X1 | RP, MeOH+ammonia, gradient 40 - 90% | 9 |
| **C196** | 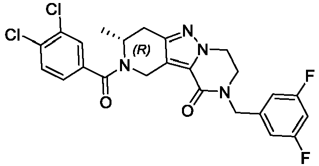 | X1 | RP, MeOH+ammonia, gradient 60 - 100% | 13 |
| **C197** | 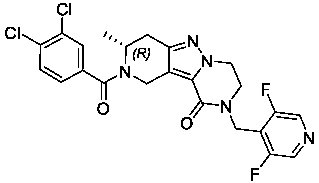 | X1 | RP, MeOH+ammonia, gradient 50 - 100% | 28 |
| **C198** | 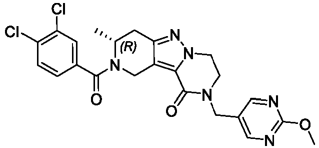 | X1 | RP, MeOH+ammonia, gradient 40 - 90% | 12 |
| **C199** | 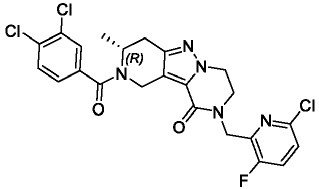 | X1 | RP, MeCN+ammonia, gradient 50 - 100% | 18 |
| **C200** | 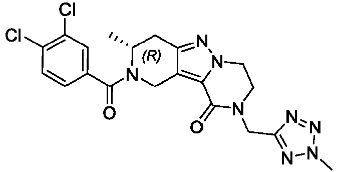 | X1 | RP, MeOH+ammonia, gradient 30 - 80% | 20 |
| **C201** | 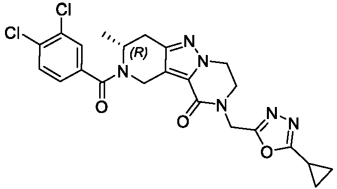 | X1 | RP, MeOH+ammonia, gradient 30 - 80% | 17 |
| **C202** | 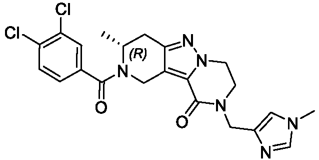 | X1 | RP, MeOH+ammonia, gradient 30 - 80% | 38 |
| **C203** | 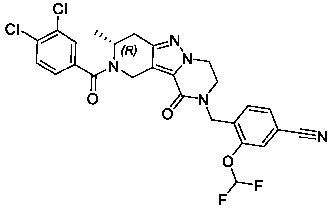 | X1 | RP, MeOH+ammonia, gradient 50 - 100% | 30 |
| **C204** | 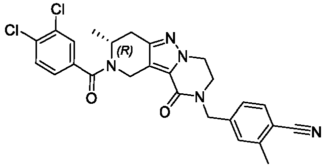 | X1 | RP, MeOH+ammonia, gradient 50 - 100% | 15 |
| **C205** | 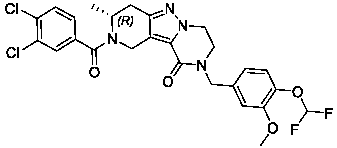 | X1 | RP, MeOH+ammonia, gradient 50 - 100% | 24 |
| **C206** | 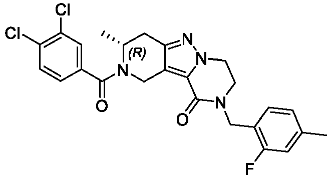 | X1 | RP, MeOH+ammonia, gradient 65 - 85% | 14 |
| **C207** | 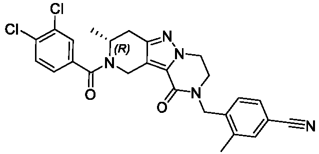 | X1 | RP, MeCN+water, gradient 45 - 70% | 10 |
| **C208** | 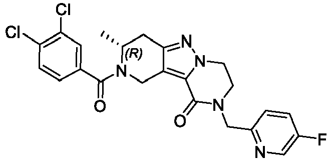 | X1 | RP, MeCN+water, gradient 25 - 50% | 14 |
| **C209** | 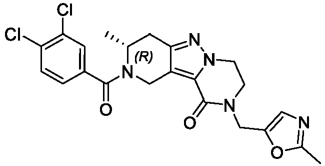 | X1 | RP, MeCN+HCOOH, gradient 20 - 40% | 15 |
| **C210** | 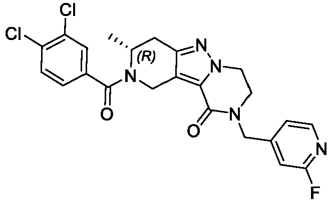 | X1 | RP, MeOH+ammonia, gradient 50 - 100% | 12 |
| **C211** | 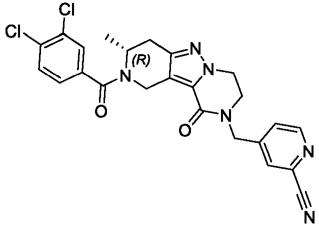 | X1 | RP, MeCN+HCOOH, gradient 25 - 55% | 10 |
| **C212** | 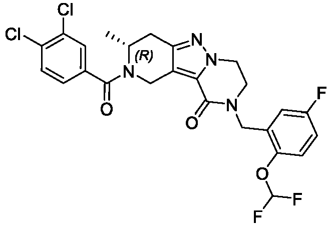 | X1 | RP, MeOH+ammonia, gradient 55 - 70% | 21 |
| **C213** | 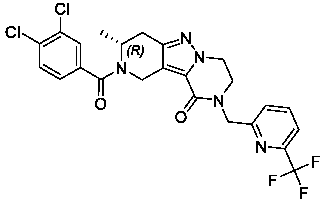 | X1 | RP, MeOH+ammonia, gradient 50 - 100% | 9 |
| **C214** | 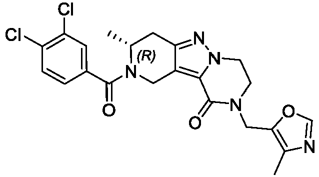 | X1 | RP, MeOH+ammonia, gradient 30 - 80% | 15 |
| **C215** | 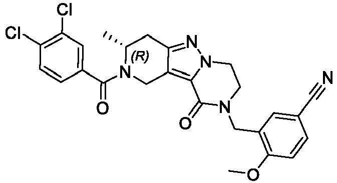 | X1 | RP, MeCN+HCOOH, gradient 35 - 70% | 19 |
| **C216** | 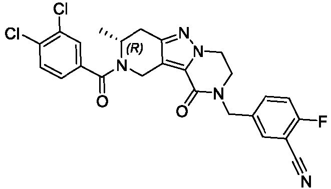 | X1 | RP, MeCN+HCOOH, gradient 40 - 70% | 16 |
| **C217** | 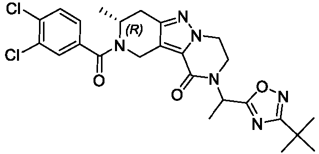 | X2 | RP, MeOH+ammonia, gradient 50 - 100% | 19 |
| **C218** | 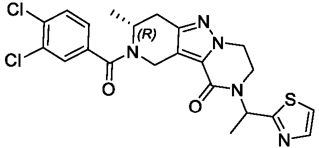 | X1 | RP, MeCN+ammonia, gradient 50 - 100% | 14 |
| **C219** | 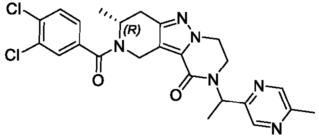 | X1 | RP, MeOH+ammonia, gradient 40 - 90% | 27 |
| **C220** | 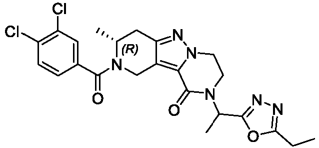 | X1 | RP, MeOH+ammonia, gradient 30 - 80% | 17 |
| **C221** | 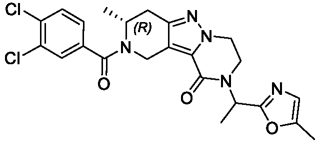 | X1 | RP, MeCN+ammonia, gradient 40 - 90% | 19 |
| **C222** | 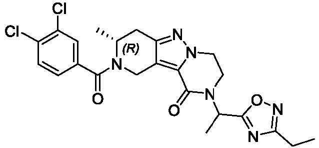 | X1 | RP, MeCN+HCOOH, gradient 35 - 60% | 10 |
| **C223** | 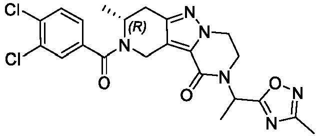 | X1 | RP, MeOH+ammonia, gradient 30 - 80% | 15 |
| **C224** | 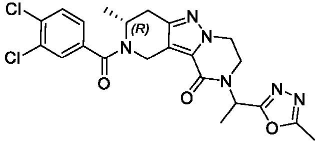 | X1 | RP, MeOH+ammonia, gradient 30 - 80% | 16 |
| **C225** | 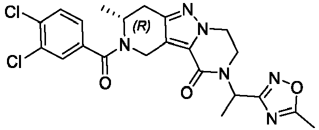 | X1 | RP, MeOH+ammonia, gradient 30 - 80% | 16 |
| **C226** | 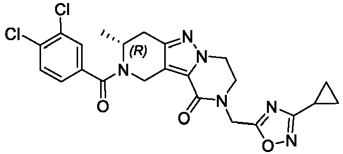 | X1 | RP, MeCN+HCOOH, gradient 30 - 55% | 10 |
| **C227** | 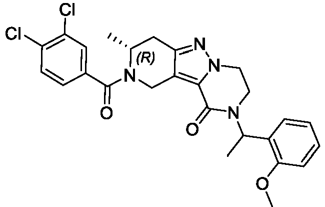 | X1 | RP, MeOH+ammonia, gradient 50 - 100% | 13 |
| **C228** | 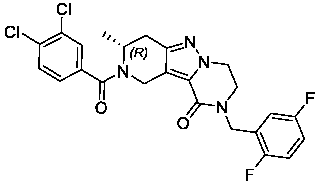 | X2 | RP, MeOH+ammonia, gradient 60 - 100% | 17 |
| **C229** | 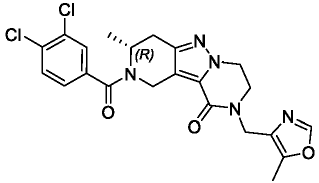 | X1 | RP, MeCN+ammonia, gradient 30 - 80% | 9 |
| **C230** | 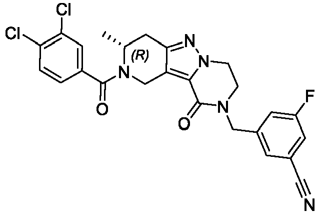 | X1 | RP, MeOH+ammonia, gradient 50 - 100% | 14 |
| **C231** | 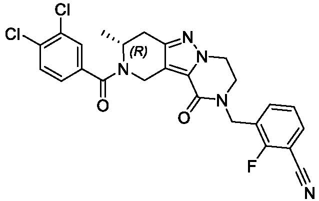 | X1 | RP, MeOH+ammonia, gradient 50 - 100% | 9 |
| **C232** | 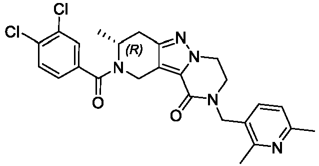 | X1 | RP, MeCN+HCOOH, gradient 15 - 40% | 13 |
| **C233** | 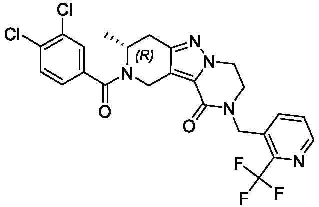 | X1 | RP, MeCN+HCOOH, gradient 40 - 70% | 14 |
| **C234** | 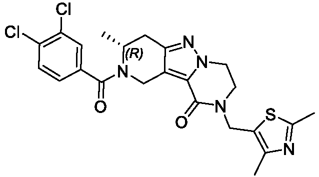 | X1 | RP, MeCN+HCOOH, gradient 30 - 60% | 12 |
| **C235** | 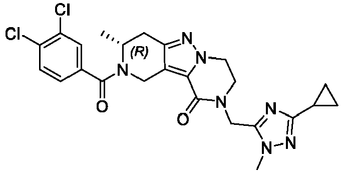 | X1 | RP, MeCN+HCOOH, gradient 20 - 45% | 9 |
| **C236** | 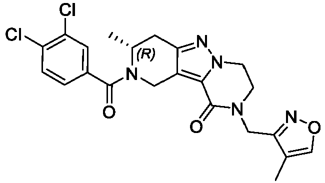 | X1 | RP, MeCN+ammonia, gradient 40 - 90% | 5 |
| **C237** | 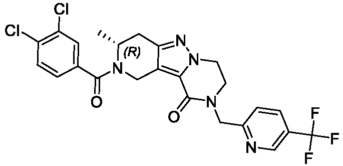 | X1 | RP, MeCN+ammonia, gradient 50 - 100% | 7 |
| **C238** | 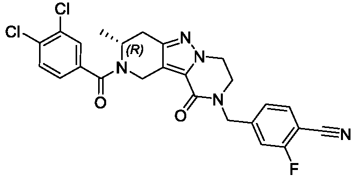 | X1 | RP, MeCN+water, gradient 35 - 65% | 7 |
| **C239** | 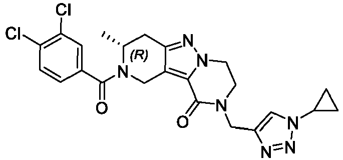 | X1 | RP, MeCN+ammonia, gradient 35 - 60% | 11 |
| **C240** | 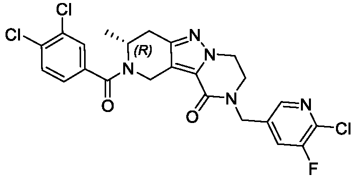 | X1 | RP, MeOH+ammonia, gradient 50 - 100% | 9 |
| **C241** | 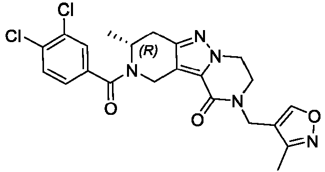 | X1 | RP, MeOH+ammonia, gradient 40 - 90% | 9 |
| **C242** | 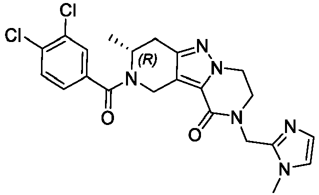 | X2 | RP, MeCN+HCOOH, gradient 10 - 40% | 21 |
| **C243** | 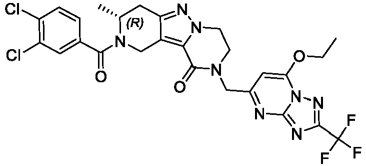 | X1 | RP, MeCN+water, gradient 35 - 60% | 3 |
| **C244** | 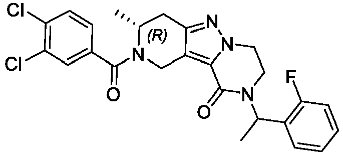 | X1 | RP, MeCN+HCOOH, gradient 55 - 75% | 19 |
| **C245** | 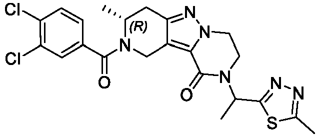 | X1 | RP, MeOH+ammonia, gradient 40 - 90% | 28 |
| **C246** | 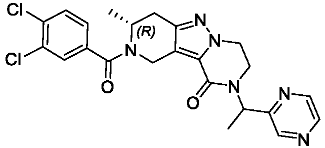 | X1 | RP, MeOH+ammonia, gradient 30 - 80% | 13 |
| **C247** | 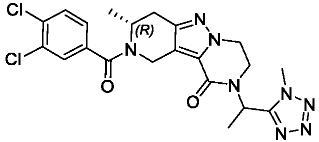 | X1 | RP, MeCN+ammonia, gradient 30 - 80% | 13 |
| **C248** | 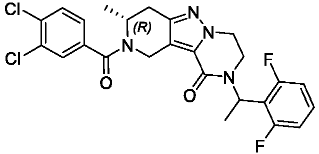 | X1 | RP, MeCN+HCOOH, gradient 55 - 80% | 7 |
| **C249** | 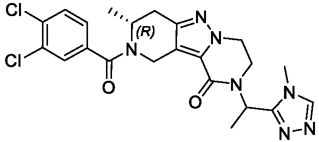 | X1 | RP, MeCN+ammonia, gradient 20 - 45% | 8 |
| **C250** | 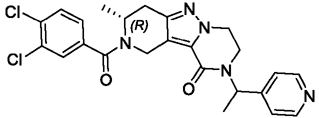 | X1 | RP, MeOH+ammonia, gradient 50 - 100% | 4 |

### 3.2.3. LCMS data for compounds C49-C250

| Cmpd n° | Rt (min) | [M+H]⁺ | [M-H]⁻ | LCMS method |
|---|---|---|---|---|
| **C49** | 3.74 | 449 | | G |
| **C50** | 4.00 | 451 | | G |
| **C51** | 3.76 | 452 | | G |
| **C52** | 3.66 | 460 | | G |
| **C53** | 3.11 | 449 | | G |
| **C54** | 4.17 | 451 | | G |
| **C55*R** | 3.41 | 535 | | H |
| **C55*S** | 3.41 | 535 | | H |
| **C56** | 3.41 | 471 | | G |
| **C57** | 2.39 | 501 | | G |
| **C58** | 4.05 | 513 | | H |
| **C59** | 3.51 | 470 | | G |
| **C60** | 3.37 | 471 | | H |
| **C61*S** | 4.02 | 552 | | H |
| **C62** | 2.50 | 501 | | G |
| **C63** | 4.16 | 513 | | H |
| **C64** | 3.08 | 476 | | G |
| **C65** | 2.62 | 471 | | H |
| **C61*R** | 3.58 | 553 | | H |
| **C66** | 4.14 | 535 | | H |
| **C67** | 3.44 | 535 | | H |
| **C68** | 3.55 | 485 | | H |
| **C69** | 3.85 | 536 | | H |
| **C70** | 3.38 | 492 | | H |
| **C71** | 3.00 | 452 | | H |
| **C72** | 2.86 | 440 | | H |
| **C73** | 2.90 | 457 | | H |
| **C74** | 2.93 | 436 | | H |
| **C75** | 2.79 | 472 | | H |
| **C76** | 2.31 | 452 | | G |
| **C77** | 3.46 | 486 | | H |
| **C78** | 3.05 | 440 | | H |
| **C79** | 3.00 | 527 | | H |
| **C80** | 4.14 | 487 | | G |
| **C81** | 4.27 | 486 | | G |
| **C82** | 3.84 | 494 | | H |
| **C83** | 1.76 | 470 | | B |
| **C84** | 1.19 | 537 | | C |
| **C85*R** | 1.96 | 540 | 598 [M+CH₃COO]⁻ | E |
| **C85*S** | 1.95 | 540 | 598 [M+CH₃COO]⁻ | E |
| **C86** | 1.14 | 531 | | C |
| **C87** | 0.92 | 473 | 531 [M+CH₃COO]⁻ | C |
| **C88** | 1.09 | 508 | 566 [M+CH₃COO]⁻ | C |
| **C89** | 1.12 | 469 | | C |
| **C90** | 1.19 | 563 | 621 [M+CH₃COO]⁻ | C |
| **C91** | 10.10 | 504 | | A |
| **C92** | 1.09 | 543 | | C |
| **C93** | 2.15 | 483 | | I |
| **C94** | 1.05 | 509 | 507 | C |
| **C95** | 10.50 | 512 | | A |
| **C96** | 0.98 | 475 | 473 | C |
| **C97** | 0.97 | 495 | 493 | C |
| **C98** | 2.29 | 561 | | I |
| **C99** | 1.94 | 484 | | I |
| **C100** | 0.88 | 484 | 482 | C |
| **C101** | 0.93 | 477 | 475 | C |
| **C102** | 0.97 | 493 | 491 | C |
| **C103** | 1.80 | 485 | | I |
| **C104** | 1.56 | 510 | 508 | I |
| **C105** | 1.23 | 551 | | C |
| **C105*R** | 2.23 | 551 | 609 [M+CH₃COO]⁻ | D |
| **C105*S** | 2.24 | 551 | 609 [M+CH₃COO]⁻ | D |
| **C106** | 9.90 | 500 | | A |
| **C107** | 0.94 | 473 | 471 | C |
| **C108** | 0.92 | 477 | 475 | C |
| **C109** | 0.95 | 473 | 471 | C |
| **C110** | 0.94 | 477 | 475 | C |
| **C111** | 0.94 | 450 | | C |
| **C112** | 0.98 | 504 | | C |
| **C113** | 0.92 | 502 | | C |
| **C114** | 0.95 | 473 | 471 | C |
| **C115** | 0.93 | 470 | | C |
| **C116** | 0.99 | 493 | 491 | C |
| **C117** | 0.98 | 493 | 491 | C |
| **C118** | 1.26 | 551 | | C |
| **C119*R** | 1.47 | 465 | | J |
| **C119*S** | 1.48 | 465 | | J |
| **C120** | 10.90 | 522 | | A |
| **C121** | 10.60 | 474 | | A |
| **C122** | 10.00 | 488 | | A |
| **C123*R** | 2.17 | 551 | | E |
| **C123*S** | 2.19 | 551 | | E |
| **C124** | 0.97 | 509 | 507 | C |
| **C125** | 1.05 | 527 | 525 | C |
| **C88*R** | 2.03 | 508 | | D |
| **C88*S** | 2.03 | 508 | | D |
| **C126** | 2.05 | 508 | | I |
| **C127** | 2.31 | 567 | 625 [M+CH₃COO]⁻ | D |
| **C128** | 1.95 | 502 | 560 [M+CH₃COO]⁻ | D |
| **C129** | 2.00 | 508 | 566 [M+CH₃COO]⁻ | D |
| **C130** | 2.02 | 518 | 516 | I |
| **C131** | 2.07 | 513 | 571 [M+CH₃COO]⁻ | J |
| **C132** | 1.43 | 504 | | F |
| **C133** | 1.34 | 509 | | F |
| **C134** | 1.13 | 516 | | F |
| **C135** | 1.26 | 541 | | F |
| **C136** | 1.04 | 488 | | F |
| **C137** | 1.39 | 501 | | F |
| **C138** | 1.33 | 474 | | F |
| **C139** | 0.99 | 484 | | F |
| **C140** | 1.34 | 517 | | F |
| **C141** | 1.23 | 474 | | F |
| **C142** | 1.35 | 488 | | F |
| **C143** | 1.38 | 501 | | F |
| **C144** | 1.22 | 474 | | F |
| **C145** | 1.33 | 474 | | F |
| **C146** | 1.38 | 475 | | F |
| **C147** | 1.26 | 473 | | F |
| **C148** | 1.30 | 475 | | F |
| **C149** | 1.33 | 488 | | F |
| **C150** | 1.32 | 490 | | F |
| **C151** | 1.36 | 475 | | F |
| **C152** | 1.23 | 484 | | F |
| **C153** | 1.33 | 488 | | F |
| **C154** | 1.52 | 501 | | F |
| **C155** | 1.22 | 523 | | F |
| **C156** | 1.31 | 474 | | F |
| **C157** | 1.32 | 487 | | F |
| **C158** | 1.26 | 514 | | F |
| **C159** | 1.51 | 538 | | F |
| **C160** | 1.32 | 490 | | F |
| **C161** | 1.49 | 530 | | F |
| **C162** | 1.35 | 490 | | F |
| **C163** | 1.24 | 524 | | F |
| **C164** | 1.57 | 527 | | F |
| **C165** | 1.25 | 542 | | F |
| **C166** | 1.45 | 517 | | F |
| **C167** | 1.19 | 520 | | F |
| **C168** | 1.49 | 517 | | F |
| **C169** | 1.40 | 528 | | F |
| **C170** | 1.48 | 562 | | F |
| **C171** | 1.44 | 538 | | F |
| **C172** | 1.33 | 528 | | F |
| **C173** | 1.09 | 487 | | F |
| **C174** | 1.45 | 553 | | F |
| **C175** | 1.47 | 512 | | F |
| **C176** | 1.26 | 577 | | F |
| **C177** | 1.26 | 502 | | F |
| **C178** | 1.33 | 530 | | F |
| **C179** | 1.34 | 503 | | F |
| **C180** | 1.37 | 512 | | F |
| **C181** | 1.44 | 555 | | F |
| **C182** | 1.40 | 510 | | F |
| **C183** | 1.52 | 517 | | F |
| **C184** | 1.36 | 515 | | F |
| **C185** | 1.16 | 484 | | F |
| **C186** | 1.53 | 519 | | F |
| **C187** | 1.25 | 531 | | F |
| **C188** | 1.39 | 501 | | F |
| **C189** | 1.48 | 519 | | F |
| **C190** | 1.45 | 515 | | F |
| **C191** | 1.44 | 534 | | F |
| **C192** | 1.49 | 519 | | F |
| **C193** | 1.24 | 499 | | F |
| **C194** | 1.44 | 567 | | F |
| **C195** | 1.21 | 495 | | F |
| **C196** | 1.51 | 505 | | F |
| **C197** | 1.21 | 506 | | F |
| **C198** | 1.30 | 501 | | F |
| **C199** | 1.39 | 522 | | F |
| **C200** | 1.26 | 475 | | F |
| **C201** | 1.30 | 501 | | F |
| **C202** | 0.89 | 473 | | F |
| **C203** | 1.47 | 560 | | F |
| **C204** | 1.27 | 508 | | F |
| **C205** | 1.32 | 565 | | F |
| **C206** | 1.56 | 501 | | F |
| **C207** | 1.49 | 508 | | F |
| **C208** | 1.37 | 488 | | F |
| **C209** | 1.37 | 474 | | F |
| **C210** | 1.33 | 488 | | F |
| **C211** | 1.34 | 495 | | F |
| **C212** | 1.38 | 553 | | F |
| **C213** | 1.48 | 538 | | F |
| **C214** | 1.29 | 474 | | F |
| **C215** | 1.42 | 524 | | F |
| **C216** | 1.46 | 512 | | F |
| **C217** | 1.52 | 531 | | F |
| **C218** | 1.43 | 490 | | F |
| **C219** | 1.23 | 499 | | F |
| **C220** | 1.34 | 503 | | F |
| **C221** | 1.38 | 488 | | F |
| **C222** | 1.36 | 503 | | F |
| **C223** | 1.36 | 489 | | F |
| **C224** | 1.29 | 489 | | F |
| **C225** | 1.33 | 489 | | F |
| **C226** | 1.33 | 501 | | F |
| **C227** | 1.51 | 513 | | F |
| **C228** | 1.47 | 505 | | F |
| **C229** | 1.18 | 474 | | F |
| **C230** | 1.48 | 512 | | F |
| **C231** | 1.43 | 512 | | F |
| **C232** | 1.08 | 498 | | F |
| **C233** | 1.42 | 538 | | F |
| **C234** | 1.35 | 504 | | F |
| **C235** | 1.18 | 514 | | F |
| **C236** | 1.23 | 474 | | F |
| **C237** | 1.33 | 538 | | F |
| **C238** | 1.38 | 512 | | F |
| **C239** | 1.30 | 500 | | F |
| **C240** | 1.49 | 522 | | F |
| **C241** | 1.18 | 474 | | F |
| **C242** | 0.95 | 473 | | F |
| **C243** | 1.37 | 623 | | F |
| **C244** | 1.51 | 501 | | F |
| **C245** | 1.18 | 505 | | F |
| **C246** | 1.20 | 485 | | F |
| **C247** | 1.29 | 489 | | F |
| **C248** | 1.51 | 519 | | F |
| **C249** | 1.06 | 488 | | F |
| **C250** | 0.99 | 484 | | F |

### 3.2.4. SFC data

| Cmpd n° | Rt (min) | [M+H]⁺ | [M-H]⁻ | SFC method |
|---|---|---|---|---|
| **C85*R** | 3.04 | 599 [M+iPrNH₂] | 538 | SFC_C |
| **C85*S** | 3.4 | 599 [M+iPrNH₂] | 538 | SFC_C |
| **C105*R** | 4.9 | 610 [M+iPrNH₂] | 549 | SFC_A |
| **C105*S** | 5.66 | 610 [M+iPrNH₂] | 549 | SFC_A |
| **C119*R** | 5.19 | 465 | 465 | SFC_D |
| **C119*S** | 5.85 | 465 | 465 | SFC_D |
| **C123*R** | 4.88 | 511 | | SFC_B |
| **C123*S** | 5.29 | 511 | | SFC_B |
| **C88*R** | 7.51 | 508 | 506 | SFC_E |
| **C88*S** | 8.45 | 508 | 506 | SFC_E |

### 3.2.5. ¹H NMR data

| Co. No. | NMR Description |
|---|---|
| **C49** | 1H NMR (400 MHz, DMSO-*d6*) δ 7.42 - 7.40 (m, 1H), 7.35 - 7.32 (m, 1H), 7.25 - 7.20 (m, 3H), 6.91 - 6.90 (m, 2H), 4.78 (s, 1H), 4.61 - 4.53 (m, 3H), 4.26 (s, 2H), 3.91 - 3.88 (m, 1H), 3.73 (s, 3H), 3.64 - 3.49 (m, 3H), 2.74 (s, 2H), 2.28 (s, 3H). |
| **C50** | 1H NMR (400 MHz, DMSO-*d6*) δ 7.56 - 7.48 (m, 4H), 7.27 - 7.22 (m, 2H), 6.99 - 6.80 (m, 2H), 4.80 (s, 1H), 4.61 - 4.51 (m, 3H), 4.26 (s, 2H), 3.91 (s, 1H), 3.74 (s, 3H), 3.65 - 3.56 (m, 3H), 2.74 (s, 2H). |
| **C51** | 1H NMR (400 MHz, DMSO-*d6*) δ 7.65 - 7.51 (m, 2H), 7.40 - 7.32 (m, 1H), 7.30 - 7.19 (m, 2H), 6.97 - 6.86 (m, 2H), 4.79 (s, 1H), 4.61 - 4.51 (m, 3H), 4.26 (s, 2H), 3.97 - 3.81 (m, 1H), 3.73 (s, 3H), 3.66 - 3.55 (m, 3H), 2.75 (s, 2H). |
| **C52** | 1H NMR (400 MHz, DMSO-*d6*) δ 8.11 (s, 1H), 7.90 (s, 1H), 7.66 - 7.62 (m, 2H), 7.26 - 7.21 (m, 1H), 6.91 (s, 2H), 4.80 (s, 1H), 4.61 - 4.51 (m, 3H), 4.27 (s, 2H), 3.91 (s, 1H), 3.73 (s, 3H), 3.66 - 3.55 (m, 3H), 2.75 (s, 2H). |
| **C53** | 1H NMR (400 MHz, DMSO-*d6*) δ 7.39 - 7.36 (m, 1H), 7.26 - 7.19 (m, 4H), 6.90 (s, 2H), 4.79 (s, 1H), 4.61 - 4.51 (m, 3H), 4.25 (s, 2H), 3.89 (s, 1H), 3.73 (s, 3H), 3.64 - 3.57 (m, 3H), 2.73 (s, 2H), 2.28 (s, 3H). |
| **C54** | 1H NMR (400 MHz, DMSO-*d6*) δ 7.57 - 7.49 (m, 3H), 7.43 - 7.41 (m, 1H), 7.28 - 7.21 (m, 2H), 6.97 - 6.85 (m, 2H), 4.80 (s, 1H), 4.61 - 4.50 (m, 3H), 4.25 (s, 2H), 3.90 (s, 1H), 3.73 (s, 3H), 3.66 - 3.55 (m, 3H), 2.74 (s, 2H). |
| **C55*R** | 1H NMR (400 MHz, CD3OD) δ 7.71 - 7.69 (m, 2H), 7.44 (d, J = 7.6 Hz, 1H), 7.33 - 7.20 (m, 2H), 6.92 (s, 2H), 6.26 - 5.80 (m, 1H), 5.60 - 5.42 (m, 1H), 4.69 - 4.37 (m, 4H), 4.31 (s, 2H), 3.79 (s, 3H), 3.73 (s, 2H), 3.26 - 2.91 (m, 2H). |
| **C55*S** | 1H NMR (400 MHz, CD₃OD) δ 7.68 - 7.66 (m, 2H), 7.41 (d, J = 8.0 Hz, 1H), 7.31 - 7.17 (m, 2H), 6.95 - 6.83 (m, 2H), 6.25 - 5.77 (m, 1H), 5.59 - 5.39 (m, 1H), 4.77 - 4.38 (m, 4H), 4.28 (s, 2H), 3.76 (s, 3H), 3.69 (s, 2H), 3.21 - 2.89 (m, 2H). |
| **C56** | 1H NMR (400 MHz, DMSO-*d6*) δ 7.61 - 7.44 (m, 2H), 7.28 - 7.20 (m, 2H), 6.97 - 6.81 (m, 2H), 4.78 (s, 1H), 4.61 - 4.52 (m, 3H), 4.26 (s, 2H), 3.89 (s, 1H), 3.73 (s, 3H), 3.65 - 3.55 (m, 3H), 2.74 (s, 2H). |
| **C57** | 1H NMR (400 MHz, DMSO-*d6*) δ 7.75 - 7.67 (m, 3H), 7.39 - 7.12 (m, 3H), 6.97 - 6.85 (m, 2H), 4.81 (s, 1H), 4.61 - 4.51 (m, 3H), 4.26 (s, 2H), 3.91 (s, 1H), 3.73 (s, 3H), 3.66 - 3.52 (m, 3H), 2.75 (s, 2H). |
| **C58** | 1H NMR (400 MHz, CDCl₃) δ 7.55 - 7.49 (m, 2H), 7.24 (s, 1H), 7.15 - 7.10 (m, 2H), 6.86 - 6.82 (m, 2H), 5.43 - 5.38 (m, 1H), 4.79 - 4.12 (m, 4H), 3.76 - 3.44 (m, 7H), 3.12 - 2.60 (m, 4H), 1.25 (s, 3H). |
| **C59** | 1H NMR (400 MHz, CDCl₃) δ 9.16 (dd, J = 4.8, 1.2 Hz, 1H), 7.63 (br s, 1H), 7.52 (s, 2H), 7.51 - 7.49 (m, 1H), 7.24 (s, 1H), 5.73 - 5.37 (m, 1H), 5.01 - 4.69 (m, 3H), 4.38 - 4.35 (m, 3H), 4.02 - 3.98 (m, 2H), 3.16 - 2.91 (m, 1H), 2.67 (br s, 1H), 1.25 (s, 3H). |
| **C60** | 1H NMR (400 MHz, CDCl₃) δ 8.71 - 8.69 (m, 2H), 7.52 (s, 1 H), 7.47 (br s, 1H), 7.25 - 7.21 (m, 2H), 5.71 - 5.37 (m, 1H), 5.13 - 4.87 (m, 2H), 4.71 - 4.29 (m, 4H), 4.03 - 3.88 (m, 2H), 3.16 - 2.95 (m, 1H), 2.76 - 2.61 (m, 1H), 1.25 (s, 3H). |
| **C61*S** | 1H NMR (400 MHz, DMSO-*d6*) 7.85 - 7.72 (m, 2H), 7.56 - 7.44 (m, 1H), 7.33 - 7.15 (m, 2H), 6.96 - 6.82 (m, 2H), 6.00 - 5.85 (m, 0.6H), 5.44 - 5.29 (m, 0.4H), 4.92 (br s, 0.4H), 4.71 - 4.45 (m, 3.2H), 4.38 - 4.17 (m, 2.4H), 3.73 (s, 3H), 3.69 - 3.57 (m, 2H), 3.41 - 3.35 (m, 0.3H), 3.27 - 3.17 (m, 0.7H), 3.12 - 3.02 (m, 0.6H), 2.98 - 2.88 (m, 0.4H). |
| **C62** | 1H NMR (400 MHz, DMSO-*d6*) δ 7.79 (d, J = 7.6 Hz, 1H), 7.72 (s, 1H), 7.58 (d, J = 7.2 Hz, 1H), 7.40 - 7.13 (m, 3H), 6.93 - 6.87 (m, 2H), 4.82 (s, 1H), 4.62 - 4.50 (m, 3H), 4.28 - 4.24 (m, 2H), 3.92 (s, 1H), 3.74 (s, 3H), 3.66 - 3.61 (m, 2H), 3.54 (s, 1H), 2.77 - 2.73 (m, 2H). |
| **C63** | 1H NMR (300 MHz, DMSO-*d6*, 60 oC) δ 7.78 - 7.73 (m, 2H), 7.46 (dd, J = 8.4, 1.8 Hz, 1H), 7.29 (d, J = 7.8 Hz, 2H), 6.98 - 6.92 (m, 2H), 5.86 - 5.43 (m, 1H), 4.68 - 4.58 (m, 2H), 4.31 - 4.27 (m, 2H), 3.78 (s, 3H), 3.71 - 3.68 (m, 2H), 3.19 - 3.16 (m, 1H), 3.04 - 2.95 (m, 1H), 2.77 - 2.62 (m, 1H), 1.83 - 1.27 (m, 3H), 0.99 - 0.79 (m, 3H). |
| **C64** | 1H NMR (400 MHz, DMSO-*d6*) δ 8.17 (s, 1H), 7.91 - 7.79 (m, 2H), 7.28 - 7.20 (m, 2H), 6.97 - 6.85 (m, 2H), 4.81 (s, 1H), 4.61 - 4.52 (m, 3H), 4.33 - 4.19 (m, 2H), 3.91 (s, 1H), 3.73 (s, 3H), 3.66 - 3.61 (m, 2H), 3.54 (s, 1H), 2.75 (s, 2H). |
| **C65** | 1H NMR (400 MHz, CDCl₃) δ 8.67 (s, 1H), 8.53 - 8.52 (m, 2H), 7.52 (s, 1H), 7.49 (d, J = 8.0 Hz, 1H), 7.23 (s, 1H), 5.79 - 5.30 (m, 1H), 4.92 - 4.89 (m, 1H), 4.77 (br s, 1H), 4.56 - 4.20 (m, 4H), 3.93 - 3.90 (m, 2H), 3.05 (br s, 1H), 2.66 (d, J = 15.6Hz, 1H), 1.25 (s, 3H). |
| **C61*R** | 1H NMR (400 MHz, CD₃OD) 7.72-7.69 (m, 2H), 7.46 - 7.44 (m, 1H), 7.30-7.23 (m, 2H), 6.91-6.89 (m, 2H), 5.99-5.56 (m, 1H), 4.95-4.91(m, 1H), 4.67-4.60 (m, 3H), 4.37-4.30 (m, 2H), 3.78-3.71 (m, 5H), 3.26-3.15 (m, 2H). |
| **C66** | 1H NMR (400 MHz, DMSO-*d6*) δ 7.75 - 7.73 (m, 2H), 7.46 - 7.05 (m, 6H), 5.46 - 5.23 (m, 1H), 4.69 - 4.51 (m, 3H), 4.31 (s, 2H), 4.16 - 4.10 (m, 1H), 3.72 (s, 2H), 2.94 (d, J = 10.0 Hz, 1H), 2.63 - 2.56 (m, 1H), 1.23 - 1.13 (m, 3H). |
| **C67** | 1H NMR (400 MHz, CDCl₃) δ 7.53 (d, J = 1.6 Hz, 1H), 7.50 (d, J = 8.4 Hz, 1H), 7.45 - 7.41 (m, 1H), 7.35 - 7.31 (m, 1H), 7.24 - 7.20 (m, 2H), 7.13 (d, J = 8.0 Hz, 1H), 6.58 (t, J = 73.6 Hz, 1H), 5.80 - 5.63 (m, 0.4H), 5.48 - 5.30 (m, 0.6H), 4.81 - 4.67 (m, 2.6H), 4.56 - 4.29 (m, 3.4H), 3.76 - 3.71 (m, 2H), 3.14 - 2.96 (m, 1H), 2.65 (d, J = 16.8 Hz, 1H), 1.25 (s, 3H). |
| **C68** | 1H NMR (400 MHz, DMSO-*d6*) δ 7.76 - 7.72 (m, 1H), 7.52 (d, J = 10.8 Hz, 1H), 7.42 (t, J = 7.6 Hz, 1H), 7.28 - 7.13 (m, 3H), 6.93 - 6.87 (m, 2H), 4.83 - 4.79 (m, 1H), 4.61 - 4.50 (m, 3H), 4.28 - 4.23 (m, 2H), 3.91-3.90 (m, 1H), 3.74 (s, 3H), 3.66 - 3.69 (m, 2H), 3.54 - 3.52 (m, 1H), 2.79 - 2.73 (m, 2H). |
| **C69** | 1HNMR (400 MHz, DMSO-*d6*) δ 8.43 (s, 1H), 7.75 - 7.73 (m, 2H), 7.65 (br s, 1H), 7.48 - 7.43 (m, 2H), 7.30 (t, J = 73.2 Hz, 1H), 5.42 - 5.22 (m, 1H), 4.79 - 4.35 (m, 2H), 4.48 - 4.08 (m, 4H), 3.86 (br s, 2H), 2.97 - 2.91 (m, 1H), 1.18 - 1.12 (m, 3H). |
| **C70** | 1H NMR (400 MHz, DMSO-*d6*) δ 8.18 (s, 1H), 7.95 - 7.93 (m, 2H), 7.46 - 7.19 (m, 3H), 6.93 - 6.87 (m, 2H), 4.85 - 4.83 (m, 1H), 4.62 - 4.50 (m, 3H), 4.28 - 4.24 (m, 2H), 3.96 - 3.89 (m, 1H), 3.74 - 3.73 (m, 3H), 3.67 - 3.60 (m, 2H), 3.53 - 3.49 (m, 1H), 2.78 - 2.74 (m, 2H). |
| **C71** | 1H NMR (400 MHz, DMSO-*d6*) δ 8.51 - 8.49 (m, 1H), 7.76 - 7.75 (m, 1H), 7.50 (d, J = 8.0 Hz, 1H), 7.37 - 7.29 (m, 2H), 7.18 (s, 1H), 7.02 (d, J = 8.0 Hz, 1H), 4.78 - 4.58 (m, 4H), 4.33 (s, 2H), 3.88 - 3.86 (m, 6H), 3.57 (s, 1H), 2.75 (s, 2H) |
| **C72** | 1H NMR (400 MHz, DMSO-*d6*) δ 8.53 - 8.49 (m, 1H), 7.79 - 7.75 (m, 1H), 7.62 - 7.58 (m, 1H), 7.53 - 7.47 (m, 1H), 7.39 - 7.26 (m, 3H), 4.80 (d, J = 11.2 Hz, 2H), 4.68 (s, 1H), 4.45 (s, 1H), 4.36 - 4.30 (m, 2H), 3.94 (s, 1H), 3.87 - 3.79 (m, 2H), 3.52 - 3.51 (m, 1H), 2.77 (s, 1H), 2.67 (s, 1H). |
| **C73** | 1H NMR (400 MHz, DMSO-*d6*) δ 8.52 (s, 2H), 8.27 (s, 1H), 7.79 - 7.72 (m, 1H), 7.38 - 7.21 (m, 2H), 4.79 (s, 2H), 4.70 (s, 1H), 4.59 (s, 1H), 4.36 - 4.34 (m, 2H), 3.92 - 3.73 (m, 3H), 3.64 (s, 1H), 2.76 (s, 2H). |
| **C74** | 1H NMR (400 MHz, CDCl₃) δ 8.56 - 8.53 (m, 1H), 7.71 - 7.65 (m, 1H), 7.41 - 7.28 (m, 1H), 7.23 - 7.09 (m, 4H), 5.21 - 4.77 (m, 4H), 4.34 - 4.31 (m, 2H), 4.22 - 3.52 (m, 4H), 2.96 - 2.69 (m, 2H), 2.31 - 2.23 (m, 3H). |
| **C75** | 1H NMR (400 MHz, DMSO-*d6*) δ 8.54 - 8.49 (m, 1H), 8.29 - 8.26 (m, 1H), 7.90 - 7.72 (m, 2H), 7.39 - 7.08 (m, 3H), 4.83 (s, 1.4H), 4.80 (s, 1.4H), 4.69 (s, 0.6H), 4.60 (s, 0.6H), 4.37 - 4.30 (m, 2H), 3.98 - 3.95 (m, 0.6H), 3.88 - 3.85 (m, 1.4H), 3.82 - 3.79 (m, 0.6H), 3.65 - 3.62 (m, 1.4H), 2.83 - 2.81 (m, 2H). |
| **C76** | 1H NMR (400 MHz, CDCl₃) δ 8.55 - 8.53 (m, 1H), 7.71 - 7.64 (m, 1 H), 7.40 - 7.28 (m, 1H), 7.26 - 7.16 (m, 2H), 7.01 - 6.90 (m, 2H), 5.14 - 4.98 (m, 1H), 4.86 - 4.78 (m, 2H), 4.56 - 4.30 (m, 4H), 3.90 - 3.84 (m, 2H), 3.82 - 3.77 (m, 3H), 3.56 - 3.48 (m, 1H), 2.91 - 2.66 (m, 2H). |
| **C77** | 1H NMR (400 MHz, CD₃OD) δ 8.52 (m, 1H), 7.84 (m, 1H), 7.76 (m, 1H), 7.68 - 7.61 (m, 2H), 7.46 - 7.35 (m, 2H), 7.11 (t, J = 54.4 Hz, 1H), 5.61 - 5.42 (m, 1H), 5.00 - 4.86 (m, 2H), 4.76 - 4.58 (m, 1H), 4.40 (m, 3H), 3.91 (m, 2H), 3.10 - 3.06 (m, 1H), 2.69 (m, 1H), 1.27 (m, 3H). |
| **C78** | 1H NMR (400 MHz, DMSO-*d6*) δ 8.52 (s, 1H), 7.76 - 7.67 (m, 2H), 7.564 (d, J = 9.2 Hz, 1H), 7.34 - 7.29 (m, 3H), 4.77 - 4.56 (m, 4H), 4.34 (s, 2H), 3.90 - 3.85 (m, 3H), 3.55 (s, 1H), 2.75 (s, 2H). |
| **C79** | 1H NMR (400 MHz, CD₃OD) δ 8.52 - 8.47 (m, 1H), 7.82 - 7.80 (m, 1H), 7.76 - 7.66 (m, 2H), 7.45 - 7.33 (m, 3H), 5.12 - 5.00 (m, 1H), 4.75 - 4.57 (m, 4H), 4.03 - 4.02 (m, 1H), 3.73 - 3.57 (m, 3H), 3.25 - 3.13 (m, 2H), 2.86 - 2.82 (m, 2H), 2.72 (s, 3H). |
| **C80** | 1H NMR (400 MHz, CDCl₃) δ 7.53 - 7.49 (m, 2H), 7.41 - 7.38 (m, 1H), 7.33 - 7.29 (m, 1H), 7.26 - 7.24 (m, 1H), 7.16 - 7.06 (m, 2H), 5.68 - 5.42 (m, 1H), 4.82 - 4.29 (m, 6H), 3.76 - 3.73 (m, 2H), 3.08 - 2.98 (m, 1H), 2.67 - 2.63 (m, 1H), 1.29 - 1.21 (m, 3H). |
| **C81** | 1H NMR (400 MHz, CDCl₃) δ 7.53 - 7.51 (m, 2H), 7.33 - 7.31 (m, 1H), 7.27 - 7.26 (m, 0.5H), 7.25 - 7.24 (m, 0.5H), 7.09 - 6.98 (m, 3H), 5.64 - 5.37 (m, 1H), 4.78 - 4.28 (m, 6H), 3.66 - 3.63 (m, 2H), 3.11 - 2.99 (m, 1H), 2.71 - 2.60 (m, 1H), 1.25 (s, 3H). |
| **C82** | 1H NMR (400 MHz, CDCl₃) δ 7.67 - 7.64 (m, 2H), 7.53 - 7.49 (m, 2H), 7.43 - 7.41 (m, 2H), 7.26 - 7.24 (m, 1H), 5.69 - 5.33 (m, 1H), 4.82 - 4.31 (m, 6H), 3.68 - 3.64 (m, 2H), 3.09 - 2.99 (m, 1H), 2.71 - 2.63 (m, 1H), 1.25 (s, 3H). |
| **C83** | 1H NMR (400 MHz, DMSO-*d6*, 100°C) δ ppm 2.11 (quin, J=6.5 Hz, 2 H) 2.72 (t, J=5.9 Hz, 2 H) 3.46 - 3.51 (m, 2 H) 3.71 (br s, 2 H) 4.32 (t, J=6.8 Hz, 2 H) 4.61 (s, 2 H) 4.75 (s, 2 H) 7.26 (dd, J=7.5, 4.8 Hz, 1 H) 7.32 (d, J=7.9 Hz, 1 H) 7.39 (dd, J=8.1, 2.0 Hz, 1 H) 7.64 (d, J=1.8 Hz, 1 H) 7.67 (d, J=8.1 Hz, 1 H) 7.73 (td, J=7.7, 1.8 Hz, 1 H) 8.50 (d, J=4.9 Hz, 1 H) |
| **C84** | 1H NMR (400 MHz, DMSO-*d6*, 80°C) δ ppm 1.61 (d, J=7.0 Hz, 3 H) 2.77 (t, J=5.8 Hz, 2 H) 3.39 (ddd, J=13.1, 6.7, 4.6 Hz, 1 H) 3.64 - 3.86 (m, 3 H) 4.14 - 4.36 (m, 2 H) 4.73 (br s, 2 H) 5.80 - 5.91 (m, 1 H) 7.45 (dd, J=8.4, 2.0 Hz, 1 H) 7.56 - 7.64 (m, 2 H) 7.67 - 7.75 (m, 4 H) |
| **C85*R** | 1H NMR (400 MHz, DMSO-*d6*,, 100°C) δ ppm 1.17 (d, J=6.9 Hz, 3 H) 1.54 (d, J=7.1 Hz, 3 H) 2.55 (d, J=16.0 Hz, 1 H) 2.87 - 2.97 (m, 1 H) 3.35 (ddd, J=13.2, 6.7, 4.7 Hz, 1 H) 3.69 (ddd, J=13.2, 8.2, 4.8 Hz, 1 H) 4.11 - 4.39 (m, 3 H) 4.44 - 4.81 (m, 1 H) 4.90 - 5.27 (m, 1 H) 5.78 (q, J=7.1 Hz, 1 H) 7.09 (t, J=74.2 Hz, 1 H) 7.12 - 7.19 (m, 2 H) 7.41 (d, J=8.5 Hz, 2 H) 7.72 - 7.77 (m, 1 H) 7.77 - 7.82 (m, 1 H) 8.00 (d, J=1.9 Hz, 1 H) |
| **C85*S** | 1H NMR (400 MHz, DMSO-*d6*, 100°C) δ ppm 1.17 (br d, J=6.9 Hz, 3 H) 1.56 (br d, J=6.6 Hz, 3 H) 2.55 (br d, J=15.9 Hz, 1 H) 2.89 - 2.95 (m, 1 H) 3.26 - 3.37 (m, 1 H) 3.64 - 3.78 (m, 1 H) 4.06 - 4.39 (m, 3 H) 4.42 - 4.80 (m, 1 H) 4.90 - 5.29 (m, 1 H) 5.73 - 5.85 (m, 1 H) 7.08 (br t, J=74.2 Hz, 1 H) 7.14 (br d, J=8.2 Hz, 2 H) 7.40 (br d, J=7.5 Hz, 2 H) 7.71 - 7.83 (m, 2 H) 7.97 - 8.02 (m, 1 H) |
| **C86** | 1H NMR (400 MHz, DMSO-*d6*, 150°C) δ ppm 1.18 (br d, J=6.6 Hz, 3 H) 1.54 (br dd, J=6.9, 3.4 Hz, 3 H) 2.54 - 2.61 (m, 1 H) 2.94 (br dd, J=15.5, 5.8 Hz, 1 H) 3.32 - 3.43 (m, 1 H) 3.64 - 3.73 (m, 1 H) 3.85 (s, 3 H) 4.11 - 4.19 (m, 1 H) 4.20 - 4.26 (m, 1 H) 4.31 (br dd, J=17.6, 4.6 Hz, 1 H) 4.67 (br s, 1 H) 5.08 (br dd, J=17.2, 10.3 Hz, 1 H) 5.73 (br d, J=7.3 Hz, 1 H) 7.07 - 7.19 (m, 3 H) 7.38 (br d, J=8.1 Hz, 1 H) 7.61 (s, 1 H) 7.66 (br d, J=7.9 Hz, 1 H) |
| **C87** | 1H NMR (400 MHz, DMSO-*d6*, 100°C) δ ppm 1.18 (d, J=6.8 Hz, 3 H) 1.53 (dd, J=6.9, 3.9 Hz, 3 H) 2.58 (br d, J=16.1 Hz, 1 H) 2.93 (br d, J=5.7 Hz, 1 H) 3.45 (br s, 1 H) 3.64 - 3.73 (m, 1 H) 4.12 - 4.20 (m, 1 H) 4.22 - 4.28 (m, 1 H) 4.28 - 4.34 (m, 1 H) 4.67 (br s, 1 H) 5.08 (br s, 1 H) 5.83 (q, J=7.1 Hz, 1 H) 6.22 (br s, 1 H) 7.42 (dd, J=8.1, 1.1 Hz, 1 H) 7.62 (br s, 1 H) 7.67 (d, J=1.8 Hz, 1 H) 7.69 - 7.72 (m, 1 H) 12.47 (br s, 1 H) |
| **C88** | 1H NMR (400 MHz, DMSO-*d6*, 100°C) δ ppm 1.18 (d, J=7.0 Hz, 3 H) 1.60 (dd, J=7.0, 4.2 Hz, 3 H) 2.58 (d, J=15.8 Hz, 1 H) 2.93 (br d, J=5.7 Hz, 1 H) 3.35 - 3.46 (m, 1 H) 3.75 (qd, J=8.5, 4.6 Hz, 1 H) 4.17 - 4.25 (m, 1 H) 4.26 - 4.29 (m, 1 H) 4.31 (br d, J=6.2 Hz, 1 H) 4.65 (br s, 1 H) 5.09 (br s, 1 H) 5.78 - 5.85 (m, 1 H) 7.41 (dd, J=8.3, 1.9 Hz, 1 H) 7.57 (dd, J=8.1, 4.2 Hz, 2 H) 7.67 (dd, J=1.8, 1.1 Hz, 1 H) 7.70 (d, J=8.1 Hz, 1 H) 7.78 (dd, J=8.5, 2.8 Hz, 2 H) |
| **C89** | 1H NMR (400 MHz, DMSO-*d6*, 100°C) δ ppm 1.57 (d, J=7.3 Hz, 3 H) 2.76 (t, J=5.9 Hz, 2 H) 3.32 (ddd, J=13.3, 6.9, 4.7 Hz, 1 H) 3.63 - 3.81 (m, 3 H) 4.10 - 4.19 (m, 1 H) 4.22 - 4.30 (m, 1 H) 4.73 (s, 2 H) 5.82 (q, J=7.0 Hz, 1 H) 7.26 - 7.33 (m, 1 H) 7.34 - 7.40 (m, 4 H) 7.44 (dd, J=8.1, 2.0 Hz, 1 H) 7.66 - 7.73 (m, 2 H) |
| **C90** | 1H NMR (400 MHz, DMSO-*d6*, 100°C) δ ppm 1.13 (d, J=6.8 Hz, 3 H) 1.72 (s, 6 H) 2.54 (br d, J=15.8 Hz, 1 H) 2.86 - 2.93 (m, 1 H) 3.79 - 3.85 (m, 2 H) 4.14 (br d, J=16.9 Hz, 1 H) 4.28 - 4.33 (m, 2 H) 4.50 - 4.68 (m, 1 H) 4.85 - 5.02 (m, 1 H) 7.07 (t, J=74.4 Hz, 1 H) 7.05 - 7.08 (m, 2 H) 7.36 (dd, J=8.3, 1.9 Hz, 1 H) 7.39 - 7.42 (m, 2 H) 7.61 (d, J=1.8 Hz, 1 H) 7.65 (d, J=8.1 Hz, 1 H) |
| **C91** | 1H NMR (400 MHz, DMSO-*d6*, 80°C) δ ppm 1.18 (d, J=6.8 Hz, 3 H) 2.59 (d, J=16.0 Hz, 1 H) 2.96 (dd, J=15.9, 5.9 Hz, 1 H) 3.76 - 3.82 (m, 2 H) 4.26 - 4.36 (m, 3 H) 4.64 - 4.74 (m, 3 H) 4.96 - 5.20 (m, 1 H) 7.43 (dd, J=8.2, 1.9 Hz, 1 H) 7.47 (d, J=8.3 Hz, 1 H) 7.69 (d, J=1.9 Hz, 1 H) 7.72 (d, J=8.3 Hz, 1 H) 7.82 (dd, J=8.3, 2.4 Hz, 1 H) 8.42 (d, J=2.3 Hz, 1 H) |
| **C92** | 1H NMR (400 MHz, DMSO-*d6*, 100°C) δ ppm 1.20 (d, J=7.0 Hz, 3 H) 1.51 - 1.58 (m, 3 H) 2.59 (d, J=15.8 Hz, 1 H) 2.94 (br d, J=5.9 Hz, 1 H) 3.26 - 3.41 (m, 1 H) 3.63 - 3.74 (m, 1 H) 3.76 - 3.81 (m, 6 H) 4.09 - 4.21 (m, 1 H) 4.22 - 4.40 (m, 2 H) 4.49 - 4.80 (m, 1 H) 4.92 - 5.23 (m, 1 H) 5.72 - 5.83 (m, 1 H) 6.90 - 6.98 (m, 3 H) 7.40 - 7.46 (m, 1 H) 7.66 - 7.74 (m, 2 H) |
| **C93** | 1H NMR (400 MHz, DMSO-*d6*, 100°C) δ ppm 1.19 (d, J=6.8 Hz, 3 H) 1.55 - 1.61 (m, 3 H) 2.54 - 2.64 (m, 1 H) 2.91 - 2.96 (m, 1 H) 3.26 - 3.40 (m, 1 H) 3.66 - 3.78 (m, 1 H) 4.10 - 4.22 (m, 1 H) 4.23 - 4.38 (m, 2 H) 4.53 - 4.78 (m, 1 H) 4.97 - 5.23 (m, 1 H) 5.79 - 5.88 (m, 1 H) 7.27 - 7.34 (m, 1 H) 7.34 - 7.40 (m, 4 H) 7.41 - 7.45 (m, 1 H) 7.65 - 7.74 (m, 2 H) |
| **C94** | 1H NMR (400 MHz, DMSO-*d6*, 80°C) δ ppm 1.28 (d, J=6.9 Hz, 3 H) 2.71 (d, J=15.9 Hz, 1 H) 3.02 - 3.14 (m, 1 H) 3.81 - 3.95 (m, 2 H) 4.30 - 4.44 (m, 2 H) 4.51 (br d, J=17.4 Hz, 1 H) 4.72 - 4.87 (m, 2 H) 5.16 (quin, J=6.6 Hz, 1 H) 5.38 (d, J=17.3 Hz, 1 H) 6.86 (d, J=0.8 Hz, 1 H) 7.31 (ddd, J=7.5, 4.8, 1.2 Hz, 1 H) 7.35 - 7.42 (m, 2 H) 7.44 - 7.51 (m, 1 H) 7.79 (td, J=7.7, 1.8 Hz, 1 H) 8.55 (ddd, J=4.8, 1.8, 0.9 Hz, 1 H) 11.99 (br s, 1 H) |
| **C95** | 1H NMR (400 MHz, DMSO-*d6*) δ ppm 1.09 - 1.24 (m, 3 H) 2.56 - 2.66 (m, 1 H) 2.89 - 3.01 (m, 1 H) 3.79 - 3.91 (m, 2 H) 4.05 - 4.21 (m, 1 H) 4.32 - 4.57 (m, 3 H) 4.74 - 4.96 (m, 2 H) 5.24 (br s, 1 H) 7.35 - 7.51 (m, 3 H) 7.70 - 7.80 (m, 3 H) |
| **C96** | 1H NMR (400 MHz, DMSO-*d6*, 80°C) δ ppm 1.25 (d, J=6.9 Hz, 3 H) 2.69 (d, J=15.9 Hz, 1 H) 2.99 - 3.07 (m, 1 H) 3.79 - 3.91 (m, 2 H) 4.28 - 4.42 (m, 2 H) 4.49 (br d, J=17.3 Hz, 1 H) 4.77 (d, J=3.2 Hz, 2 H) 5.16 (quin, J=6.5 Hz, 1 H) 5.38 (d, J=17.3 Hz, 1 H) 6.78 - 6.85 (m, 1 H) 7.09 - 7.16 (m, 1 H) 7.16 - 7.23 (m, 1 H) 7.28 (ddd, J=7.5, 4.9, 1.2 Hz, 1 H) 7.34 - 7.40 (m, 1 H) 7.41 - 7.49 (m, 1 H) 7.76 (td, J=7.7, 1.8 Hz, 1 H) 8.52 (ddd, J=4.8, 1.8, 0.9 Hz, 1 H) 11.79 (br s, 1 H) |
| **C97** | 1H NMR (400 MHz, DMSO-*d6*, 80°C) δ ppm 1.24 (d, J=6.8 Hz, 3 H) 2.68 (br d, J=16.0 Hz, 1 H) 3.01 - 3.10 (m, 1 H) 3.80 - 3.92 (m, 2 H) 4.28 - 4.41 (m, 2 H) 4.47 (br d, J=17.4 Hz, 1 H) 4.78 (s, 2 H) 5.14 (quin, J=6.6 Hz, 1 H) 5.34 (d, J=17.3 Hz, 1 H) 6.95 (d, J=0.8 Hz, 1 H) 7.20 - 7.32 (m, 2 H) 7.37 (d, J=7.7 Hz, 1 H) 7.77 (td, J=7.7, 1.8 Hz, 1 H) 8.53 (ddd, J=4.8, 1.8, 0.9 Hz, 1 H) 11.92 (br s, 1 H) |
| **C98** | 1H NMR (400 MHz, DMSO-*d6*, 150°C) δ ppm 1.20 (d, J=6.8 Hz, 3 H) 1.58 (dd, J=7.0, 3.7 Hz, 3 H) 2.60 (d, J=16.1 Hz, 1 H) 2.96 (dd, J=15.8, 5.9 Hz, 1 H) 3.40 (dddd, J=13.2, 8.4, 6.8, 4.8 Hz, 1 H) 3.72 (dddd, J=13.1, 8.1, 4.9, 3.0 Hz, 1 H) 4.14 - 4.38 (m, 3 H) 4.63 - 4.75 (m, 1 H) 5.10 (dd, J=17.2, 10.6 Hz, 1 H) 5.73 - 5.82 (m, 1 H) 7.34 (dd, J=8.1, 3.3 Hz, 2 H) 7.40 (dd, J=8.1, 2.0 Hz, 1 H) 7.53 (dd, J=8.6, 2.0 Hz, 2 H) 7.63 (d, J=1.8 Hz, 1 H) 7.68 (d, J=8.1 Hz, 1 H) |
| **C99** | 1H NMR (400 MHz, DMSO-*d6*, 150°C) δ ppm 1.20 (dd, J=6.8, 2.6 Hz, 3 H) 1.62 (dd, J=7.0, 3.3 Hz, 3 H) 2.60 (d, J=15.8 Hz, 1 H) 2.97 (br dd, J=16.0, 5.8 Hz, 1 H) 3.58 - 3.70 (m, 1 H) 3.81 (dddd, J=13.1, 8.1, 4.7, 3.6 Hz, 1 H) 4.16 - 4.37 (m, 3 H) 4.62 - 4.77 (m, 1 H) 5.08 (dd, J=17.3, 6.1 Hz, 1 H) 5.83 (qd, J=7.1, 2.1 Hz, 1 H) 7.23 - 7.31 (m, 1 H) 7.35 - 7.44 (m, 2 H) 7.63 (t, J=2.0 Hz, 1 H) 7.68 (dd, J=8.1, 3.1 Hz, 1 H) 7.72 - 7.80 (m, 1 H) 8.51 - 8.59 (m, 1 H) |
| **C100** | 1H NMR (400 MHz, DMSO-*d6*, 80°C) δ ppm 1.25 (d, J=6.9 Hz, 3 H) 2.69 (d, J=16.0 Hz, 1 H) 3.01 - 3.09 (m, 1 H) 3.80 - 3.94 (m, 2 H) 4.29 - 4.42 (m, 2 H) 4.49 (br d, J=17.5 Hz, 1 H) 4.78 (d, J=2.2 Hz, 2 H) 5.04 - 5.17 (m, 1 H) 5.33 (br d, J=17.4 Hz, 1 H) 6.92 (d, J=0.9 Hz, 1 H) 7.23 - 7.32 (m, 2 H) 7.37 (d, J=7.8 Hz, 1 H) 7.77 (td, J=7.7, 1.8 Hz, 1 H) 7.82 (ddd, J=9.0, 4.6, 0.8 Hz, 1 H) 8.53 (ddd, J=4.8, 1.8, 0.9 Hz, 1 H) 12.19 (br s, 1 H) |
| **C101** | 1H NMR (400 MHz, DMSO-*d6*, 80°C) δ ppm 1.24 (d, J=6.9 Hz, 3 H) 2.68 (d, J=16.0 Hz, 1 H) 3.01 - 3.08 (m, 1 H) 3.87 (ddd, J=7.0, 5.3, 2.2 Hz, 2 H) 4.30 - 4.42 (m, 2 H) 4.47 (br d, J=17.5 Hz, 1 H) 4.78 (d, J=2.3 Hz, 2 H) 5.15 (quin, J=6.6 Hz, 1 H) 5.36 (d, J=17.3 Hz, 1 H) 6.92 (s, 1 H) 7.15 - 7.32 (m, 3 H) 7.37 (d, J=7.8 Hz, 1 H) 7.77 (td, J=7.7, 1.8 Hz, 1 H) 8.53 (ddd, J=4.8, 1.8, 0.9 Hz, 1 H) 11.84 (s, 1 H) |
| **C102** | 1H NMR (400 MHz, DMSO-*d6*, 80°C) δ ppm 1.25 (d, J=6.9 Hz, 3 H) 2.69 (d, J=16.0 Hz, 1 H) 2.99 - 3.08 (m, 1 H) 3.86 (ddd, J=7.0, 5.3, 2.2 Hz, 2 H) 4.30 - 4.41 (m, 2 H) 4.49 (br d, J=17.4 Hz, 1 H) 4.77 (d, J=2.5 Hz, 2 H) 5.14 (quin, J=6.6 Hz, 1 H) 5.36 (d, J=17.3 Hz, 1 H) 6.85 (s, 1 H) 7.21 (dd, J=10.0, 8.9 Hz, 1 H) 7.28 (ddd, J=7.5, 4.8, 1.1 Hz, 1 H) 7.37 (d, J=7.8 Hz, 1 H) 7.44 (ddd, J=8.9, 4.0, 0.8 Hz, 1 H) 7.76 (td, J=7.7, 1.8 Hz, 1 H) 8.53 (ddd, J=4.8, 1.8, 0.9 Hz, 1 H) 11.89 (br s, 1 H) |
| **C103** | 1H NMR (400 MHz, DMSO-*d6*, 150°C) δ ppm 1.21 (d, J=7.0 Hz, 3 H) 1.68 (dd, J=7.3, 3.3 Hz, 3 H) 2.60 (d, J=16.1 Hz, 1 H) 2.97 (dd, J=16.0, 5.8 Hz, 1 H) 3.54 - 3.64 (m, 1 H) 3.84 (ddt, J=12.9, 7.8, 5.1, 5.1 Hz, 1 H) 4.22 - 4.39 (m, 3 H) 4.62 - 4.77 (m, 1 H) 5.09 (dd, J=17.3, 8.9 Hz, 1 H) 5.77 (qd, J=7.1, 4.2 Hz, 1 H) 7.40 (dq, J=8.2, 1.1 Hz, 1 H) 7.60 - 7.72 (m, 2 H) 8.81 (d, J=2.4 Hz, 2 H) 9.09 (s, 1 H) |
| **C104** | 1H NMR (400 MHz, DMSO-*d6*, 80°C) δ ppm 1.19 (d, J=6.9 Hz, 3 H) 2.58 (d, J=16.0 Hz, 1 H) 2.94 - 3.03 (m, 1 H) 3.78 - 3.91 (m, 2 H) 4.24 - 4.41 (m, 3 H) 4.74 (s, 3 H) 4.98 - 5.39 (m, 1 H) 7.08 (dd, J=7.0, 1.6 Hz, 1 H) 7.23 - 7.29 (m, 1 H) 7.29 - 7.40 (m, 1 H) 7.70 - 7.79 (m, 2 H) 8.50 (dd, J=4.8, 1.8 Hz, 1 H) 8.54 (s, 1 H) 8.65 (dd, J=7.0, 0.9 Hz, 1 H) |
| **C105*R** | 1H NMR (400 MHz, DMSO-*d6*, 150°C) δ ppm 1.21 (d, J=6.8 Hz, 3 H) 1.64 (d, J=7.0 Hz, 3 H) 2.60 (d, J=15.8 Hz, 1 H) 2.97 (dd, J=16.1, 5.9 Hz, 1 H) 3.43 (ddd, J=13.2, 6.8, 4.8 Hz, 1 H) 3.77 (ddd, J=13.1, 8.0, 4.6 Hz, 1 H) 4.23 (dd, J=7.9, 4.8 Hz, 1 H) 4.27 - 4.37 (m, 2 H) 4.69 (br t, J=6.4 Hz, 1 H) 5.11 (d, J=17.2 Hz, 1 H) 5.86 (q, J=7.1 Hz, 1 H) 7.40 (dd, J=8.1, 2.0 Hz, 1 H) 7.57 - 7.65 (m, 3 H) 7.66 - 7.73 (m, 3 H) |
| **C105*S** | 1H NMR (400 MHz, DMSO-*d6*, 150°C) δ ppm 1.21 (d, J=6.8 Hz, 3 H) 1.64 (d, J=7.3 Hz, 3 H) 2.61 (d, J=16.3 Hz, 1 H) 2.98 (dd, J=16.0, 6.1 Hz, 1 H) 3.46 (ddd, J=13.2, 6.6, 4.8 Hz, 1 H) 3.77 (ddd, J=13.1, 8.1, 5.0 Hz, 1 H) 4.20 - 4.39 (m, 3 H) 4.71 (br t, J=6.4 Hz, 1 H) 5.09 (br d, J=17.2 Hz, 1 H) 5.86 (q, J=7.2 Hz, 1 H) 7.41 (dd, J=8.3, 1.9 Hz, 1 H) 7.59 - 7.66 (m, 3 H) 7.66 - 7.73 (m, 3 H) |
| **C106** | 1H NMR (400 MHz, DMSO-*d6*, 80°C) δ ppm 1.19 (d, J=6.9 Hz, 3 H) 2.59 (br d, J=16.0 Hz, 1 H) 2.96 (dd, J=16.0, 5.9 Hz, 1 H) 3.74 (t, J=6.2 Hz, 2 H) 3.88 (s, 3 H) 4.26 - 4.35 (m, 3 H) 4.53 - 4.71 (m, 3 H) 4.95 - 5.23 (m, 1 H) 6.80 (d, J=8.5 Hz, 1 H) 7.44 (dd, J=8.2, 1.3 Hz, 1 H) 7.66 - 7.71 (m, 2 H) 7.73 (d, J=8.3 Hz, 1 H) 8.17 (s, 1 H) |
| **C107** | 1H NMR (400 MHz, DMSO-*d6*, 80°C) δ ppm 1.23 (d, J=6.9 Hz, 3 H) 2.40 (d, J=1.9 Hz, 3 H) 2.66 (d, J=16.0 Hz, 1 H) 3.07 (s, 1 H) 3.77 - 3.93 (m, 2 H) 4.33 (td, J=6.1, 2.3 Hz, 2 H) 4.43 (br d, J=17.4 Hz, 1 H) 4.75 (s, 2 H) 5.15 (quin, J=6.6 Hz, 1 H) 5.35 (d, J=17.3 Hz, 1 H) 6.84 (s, 1 H) 6.97 (dd, J=10.2, 8.8 Hz, 1 H) 7.20 - 7.29 (m, 2 H) 7.35 (d, J=7.8 Hz, 1 H) 7.74 (td, J=7.7, 1.8 Hz, 1 H) 8.47 - 8.54 (m, 1 H) 11.42 (br s, 1 H) |
| **C108** | 1H NMR (400 MHz, DMSO-*d6*, 80°C) δ ppm 1.15 - 1.29 (m, 3 H) 2.65 (br d, J=15.9 Hz, 1 H) 3.06 (br d, J=1.8 Hz, 1 H) 3.77 - 3.95 (m, 2 H) 4.24 - 4.38 (m, 2 H) 4.43 (br d, J=17.9 Hz, 1 H) 4.75 (s, 2 H) 4.95 - 5.10 (m, 1 H) 5.27 (br d, J=17.3 Hz, 1 H) 6.70 - 7.06 (m, 3 H) 7.21 - 7.30 (m, 1 H) 7.31 - 7.41 (m, 1 H) 7.68 - 7.80 (m, 1 H) 8.46 - 8.58 (m, 1 H) 12.19 (br s, 1 H) |
| **C109** | 1H NMR (400 MHz, DMSO-*d6*, 80°C) δ ppm 1.22 (d, J=6.9 Hz, 3 H) 2.66 (d, J=15.9 Hz, 1 H) 3.00 - 3.09 (m, 4 H) 3.77 - 3.92 (m, 2 H) 4.24 - 4.39 (m, 2 H) 4.44 (br d, J=17.4 Hz, 1 H) 4.75 (s, 2 H) 5.18 (quin, J=6.6 Hz, 1 H) 5.37 (d, J=17.3 Hz, 1 H) 6.72 (ddd, J=10.6, 2.3, 1.1 Hz, 1 H) 6.85 (s, 1 H) 6.96 (dd, J=9.9, 2.3 Hz, 1 H) 7.26 (ddd, J=7.5, 4.9, 1.2 Hz, 1 H) 7.35 (d, J=7.7 Hz, 1 H) 7.74 (td, J=7.7, 1.8 Hz, 1 H) 8.51 (ddd, J=4.8, 1.8, 0.9 Hz, 1 H) 11.41 (s, 1 H) |
| **C110** | 1H NMR (400 MHz, DMSO-*d6*, 80°C) δ ppm 1.19 - 1.30 (m, 3 H) 2.68 (br d, J=15.8 Hz, 1 H) 2.99 - 3.15 (m, 1 H) 3.81 - 3.92 (m, 2 H) 4.26 - 4.41 (m, 2 H) 4.42 - 4.57 (m, 1 H) 4.69 - 4.87 (m, 2 H) 5.10 - 5.26 (m, 1 H) 5.36 (br d, J=17.2 Hz, 1 H) 6.76 - 6.95 (m, 2 H) 7.01 - 7.13 (m, 1 H) 7.23 - 7.33 (m, 1 H) 7.34 - 7.44 (m, 1 H) 7.72 - 7.82 (m, 1 H) 8.47 - 8.61 (m, 1 H) 11.84 (br s, 1 H) |
| **C111** | 1H NMR (400 MHz, DMSO-*d6*, 80°C) δ ppm 1.15 (d, J=6.9 Hz, 3 H) 2.37 (s, 3 H) 2.56 (d, J=16.0 Hz, 1 H) 2.93 (dd, J=15.9, 6.0 Hz, 1 H) 3.83 (dd, J=6.9, 5.4 Hz, 2 H) 4.24 (br d, J=17.6 Hz, 1 H) 4.32 (td, J=6.2, 1.6 Hz, 2 H) 4.74 (s, 4 H) 7.21 - 7.29 (m, 2 H) 7.34 (d, J=7.8 Hz, 1 H) 7.38 (d, J=2.1 Hz, 1 H) 7.46 (d, J=8.1 Hz, 1 H) 7.75 (td, J=7.7, 1.8 Hz, 1 H) 8.51 (ddd, J=4.8, 1.7, 0.9 Hz, 1 H) |
| **C112** | 1H NMR (400 MHz, DMSO-*d6*, 80°C) δ ppm 1.17 (d, J=6.8 Hz, 3 H) 2.57 (br d, J=16.0 Hz, 1 H) 2.95 (dd, J=15.9, 5.9 Hz, 1 H) 3.76 - 3.92 (m, 2 H) 4.16 - 4.40 (m, 3 H) 4.41 - 5.45 (m, 4 H) 7.23 - 7.30 (m, 1 H) 7.34 (d, J=7.8 Hz, 1 H) 7.70 - 7.82 (m, 3 H) 7.85 (d, J=1.5 Hz, 1 H) 8.51 (dd, J=4.8, 1.8 Hz, 1 H) |
| **C113** | 1H NMR (400 MHz, DMSO-*d6*, 80°C) δ ppm 1.16 (d, J=6.9 Hz, 3 H) 2.58 (d, J=16.0 Hz, 1 H) 2.95 (dd, J=15.9, 6.0 Hz, 1 H) 3.84 (dd, J=6.8, 5.5 Hz, 2 H) 4.16 - 4.41 (m, 3 H) 4.42 - 5.28 (m, 4 H) 7.07 - 7.50 (m, 5 H) 7.64 (d, J=1.9 Hz, 1 H) 7.75 (td, J=7.7, 1.8 Hz, 1 H) 8.51 (ddd, J=4.8, 1.8, 0.9 Hz, 1 H) |
| **C114** | 1H NMR (400 MHz, DMSO-*d6*, 80°C) δ ppm 1.22 (d, J=6.9 Hz, 3 H) 2.64 (br d, J=16.2 Hz, 1 H) 2.96 - 3.17 (m, 4 H) 3.77 - 3.99 (m, 2 H) 4.25 - 4.54 (m, 3 H) 4.66 - 4.89 (m, 2 H) 5.00 - 5.19 (m, 1 H) 5.23 - 5.42 (m, 1 H) 6.71 - 7.00 (m, 3 H) 7.21 - 7.31 (m, 1 H) 7.31 - 7.48 (m, 1 H) 7.67 - 7.90 (m, 1 H) 8.46 - 8.63 (m, 1 H) 11.76 (br s, 1 H) |
| **C115** | 1H NMR (400 MHz, DMSO-*d6*, 80°C) δ ppm 1.16 (d, J=6.9 Hz, 3 H) 2.57 (br d, J=16.2 Hz, 1 H) 2.94 (dd, J=16.0, 6.1 Hz, 1 H) 3.84 (dd, J=7.0, 5.3 Hz, 2 H) 4.07 - 5.60 (m, 7 H) 7.27 (dd, J=7.5, 4.8 Hz, 1 H) 7.35 (d, J=7.9 Hz, 1 H) 7.63 (d, J=7.9 Hz, 2 H) 7.75 (td, J=7.7, 1.9 Hz, 1 H) 7.80 (d, J=8.0 Hz, 2 H) 8.48 - 8.55 (m, 1 H) |
| **C116** | 1H NMR (400 MHz, DMSO-*d6*, 80°C) δ ppm 1.23 (d, J=6.8 Hz, 3 H) 2.67 (d, J=15.9 Hz, 1 H) 2.98 - 3.06 (m, 1 H) 3.80 - 3.89 (m, 2 H) 4.28 - 4.39 (m, 2 H) 4.47 (br d, J=17.3 Hz, 1 H) 4.75 (d, J=2.4 Hz, 2 H) 5.14 (quin, J=6.6 Hz, 1 H) 5.34 (d, J=17.3 Hz, 1 H) 6.81 (d, J=0.9 Hz, 1 H) 7.09 (dd, J=9.6, 2.1 Hz, 1 H) 7.17 (ddd, J=9.5, 2.1, 0.8 Hz, 1 H) 7.26 (ddd, J=7.5, 4.8, 1.2 Hz, 1 H) 7.32 - 7.39 (m, 1 H) 7.75 (td, J=7.7, 1.8 Hz, 1 H) 8.51 (ddd, J=4.8, 1.8, 0.9 Hz, 1 H) 11.67 - 12.09 (m, 1 H) |
| **C117** | 1H NMR (400 MHz, DMSO-*d6*, 80°C) δ ppm 1.22 (d, J=6.9 Hz, 3 H) 2.65 (d, J=15.9 Hz, 1 H) 2.96 - 3.05 (m, 1 H) 3.76 - 3.93 (m, 2 H) 4.26 - 4.39 (m, 2 H) 4.43 (br d, J=17.4 Hz, 1 H) 4.66 - 4.84 (m, 2 H) 4.92 - 5.11 (m, 1 H) 5.26 (br d, J=17.3 Hz, 1 H) 6.79 (d, J=3.0 Hz, 1 H) 6.97 - 7.05 (m, 1 H) 7.06 - 7.11 (m, 1 H) 7.26 (ddd, J=7.5, 4.8, 1.2 Hz, 1 H) 7.35 (d, J=7.8 Hz, 1 H) 7.74 (td, J=7.7, 1.8 Hz, 1 H) 8.50 (ddd, J=4.8, 1.8, 0.9 Hz, 1 H) 12.27 (br s, 1 H) |
| **C118** | 1H NMR (400 MHz, DMSO-*d6*, 100°C) δ ppm 1.16 (d, J=7.0 Hz, 3 H) 1.60 (dd, J=7.2, 4.5 Hz, 3 H) 2.56 (d, J=15.6 Hz, 1 H) 2.91 (m, J=5.9 Hz, 1 H) 3.38 (tdd, J=13.4, 13.4, 6.9, 4.8 Hz, 1 H) 3.70 - 3.78 (m, 1 H) 4.12 - 4.22 (m, 1 H) 4.23 - 4.28 (m, 1 H) 4.28 - 4.33 (m, 1 H) 4.63 (br s, 1 H) 5.06 (br s, 1 H) 5.80 - 5.87 (m, 1 H) 7.37 - 7.42 (m, 1 H) 7.56 - 7.70 (m, 6 H) |
| **C119*R** | 1H NMR (400 MHz, DMSO-*d6*, 100°C) δ ppm 1.17 (d, J=6.8 Hz, 3 H) 1.55 (d, J=7.1 Hz, 3 H) 2.50 - 2.65 (m, 1 H) 2.95 (dd, J=15.9, 6.0 Hz, 2 H) 3.40 - 3.52 (m, 1 H) 3.70 (ddd, J=13.1, 8.1, 4.9 Hz, 1 H) 4.15 - 4.36 (m, 3 H) 4.60 (s, 1 H) 4.92 - 5.17 (m, 1 H) 5.89 (q, J=7.1 Hz, 1 H) 7.73 - 7.78 (m, 2 H) 7.78 - 7.82 (m, 1 H) 8.00 (d, J=1.9 Hz, 1 H) |
| **C119*S** | 1H NMR (400 MHz, DMSO-*d6*, 100°C) δ ppm 1.16 (d, J=6.8 Hz, 3 H) 1.56 (d, J=7.1 Hz, 3 H) 2.55 (d, J=16.1 Hz, 1 H) 2.85 - 3.03 (m, 2 H) 3.38 - 3.50 (m, 1 H) 3.68 - 3.81 (m, 1 H) 4.13 - 4.37 (m, 3 H) 4.60 (s, 1 H) 5.07 (br d, J=11.1 Hz, 1 H) 5.89 (q, J=7.1 Hz, 1 H) 7.73 - 7.77 (m, 2 H) 7.77 - 7.80 (m, 1 H) 7.98 - 8.02 (m, 1 H) |
| **C120** | 1H NMR (400 MHz, DMSO-*d6*, 80°C) δ ppm 1.19 (d, J=6.9 Hz, 3 H) 2.58 (d, J=16.1 Hz, 1 H) 2.92 - 2.99 (m, 1 H) 3.59 - 3.66 (m, 2 H) 3.81 (s, 3 H) 4.15 - 4.27 (m, 2 H) 4.34 (br d, J=17.3 Hz, 1 H) 4.51 - 4.79 (m, 1 H) 4.88 - 4.97 (m, 2 H) 5.02 - 5.25 (m, 1 H) 6.53 (d, J=2.9 Hz, 1 H) 7.03 (d, J=7.1 Hz, 1 H) 7.16 (t, J=7.7 Hz, 1 H) 7.29 (d, J=3.0 Hz, 1 H) 7.40 (d, J=8.1 Hz, 1 H) 7.45 (dd, J=8.2, 1.6 Hz, 1 H) 7.71 (d, J=4.6 Hz, 2 H) |
| **C121** | 1H NMR (400 MHz, DMSO-*d6*) δ ppm 1.11 - 1.27 (m, 3 H) 2.54 - 2.61 (m, 1 H) 2.93 - 3.05 (m, 1 H) 4.09 - 4.27 (m, 1 H) 4.41 - 4.57 (m, 4 H) 5.21 - 5.31 (m, 1 H) 5.41 - 5.50 (m, 1 H) 7.47 (dd, J=8.3, 1.9 Hz, 1 H) 7.75 (d, J=8.3 Hz, 1 H) 7.78 (d, J=1.8 Hz, 1 H) 7.82 - 7.90 (m, 1 H) 7.93 - 8.01 (m, 1 H) 8.49 (br s, 1 H) |
| **C122** | 1H NMR (400 MHz, DMSO-*d6*) δ ppm 1.09 - 1.26 (m, 3 H) 2.56 - 2.63 (m, 1 H) 2.87 - 3.01 (m, 1 H) 3.88 (br s, 2 H) 4.06 - 4.23 (m, 1 H) 4.37 (br s, 2 H) 4.43 - 4.56 (m, 1 H) 4.61 - 4.84 (m, 2 H) 5.15 - 5.51 (m, 1 H) 7.09 (br d, J=6.5 Hz, 1 H) 7.29 - 7.41 (m, 1 H) 7.45 (dd, J=8.1, 1.0 Hz, 1 H) 7.71 - 7.79 (m, 2 H) 7.91 - 8.05 (m, 1 H) |
| **C124** | 1H NMR (400 MHz, DMSO-*d6*, 80°C) δ ppm 1.23 (d, J=6.8 Hz, 3 H) 2.67 (d, J=15.9 Hz, 1 H) 2.98 - 3.05 (m, 1 H) 3.78 - 3.90 (m, 2 H) 4.27 - 4.39 (m, 2 H) 4.44 (br d, J=17.4 Hz, 1 H) 4.69 - 4.83 (m, 2 H) 5.09 (quin, J=6.6 Hz, 1 H) 5.32 (d, J=17.3 Hz, 1 H) 6.95 (br s, 1 H) 7.07 - 7.18 (m, 1 H) 7.22 - 7.56 (m, 3 H) 7.58 - 7.66 (m, 1 H) 7.74 (td, J=7.7, 1.8 Hz, 1 H) 8.51 (ddd, J=4.8, 1.8, 0.9 Hz, 1 H) 11.86 (br s, 1 H) |
| **C125** | 1H NMR (400 MHz, DMSO-*d6*, 80°C) δ ppm 1.23 (d, J=6.9 Hz, 3 H) 2.67 (d, J=15.9 Hz, 1 H) 2.97 - 3.05 (m, 1 H) 3.78 - 3.91 (m, 2 H) 4.27 - 4.39 (m, 2 H) 4.45 (br d, J=17.4 Hz, 1 H) 4.68 - 4.82 (m, 2 H) 4.99 - 5.15 (m, 1 H) 5.30 (d, J=17.3 Hz, 1 H) 6.79 - 6.89 (m, 1 H) 7.16 - 7.30 (m, 2 H) 7.35 (d, J=7.8 Hz, 1 H) 7.69 - 7.82 (m, 2 H) 8.51 (ddd, J=4.9, 1.8, 0.9 Hz, 1 H) 12.07 (br s, 1 H) |
| **C88*R** | 1H NMR (400 MHz, DMSO-*d6*, 100°C) δ ppm 1.18 (d, J=7.0 Hz, 3 H) 1.60 (d, J=7.3 Hz, 3 H) 2.58 (d, J=15.8 Hz, 1 H) 2.92 - 2.97 (m, 1 H) 3.38 - 3.46 (m, 1 H) 3.75 (ddd, J=13.2, 8.1, 4.8 Hz, 1 H) 4.20 - 4.34 (m, 3 H) 4.66 (br s, 1 H) 5.04 (br s, 1 H) 5.82 (q, J=7.0 Hz, 1 H) 7.41 (dd, J=8.1, 2.0 Hz, 1 H) 7.58 (d, J=8.1 Hz, 2 H) 7.67 (d, J=1.8 Hz, 1 H) 7.70 (d, J=8.1 Hz, 1 H) 7.76 - 7.81 (m, 2 H) |
| **C126** | 1H NMR (400 MHz, DMSO-*d6* +CC14) δ ppm 1.14 - 1.29 (m, 3 H) 1.54 - 1.69 (m, 3 H) 2.52 - 2.61 (m, 1 H) 2.88 - 2.97 (m, 1 H) 3.27 - 3.37 (m, 1 H) 3.66 - 3.78 (m, 1 H) 4.10 - 4.33 (m, 3 H) 4.44 - 4.67 (m, 1 H) 5.26 - 5.51 (m, 1 H) 5.81 - 5.96 (m, 1 H) 7.37 (br s, 1 H) 7.51 - 7.71 (m, 5 H) 7.75 - 7.82 (m, 1 H) |
| **C131** | 1H NMR (400 MHz, DMSO-*d6* +CC14) δ ppm 1.17 - 1.27 (m, 3 H) 1.49 - 1.62 (m, 3 H) 2.53 - 2.61 (m, 1 H) 2.95 (br d, J=17.0 Hz, 1 H) 3.58 - 3.74 (m, 2 H) 3.77 (br d, J=5.0 Hz, 3 H) 4.05 - 4.30 (m, 3 H) 4.38 - 4.67 (m, 1 H) 5.25 - 5.50 (m, 1 H) 5.76 - 5.93 (m, 1 H) 6.77 - 6.95 (m, 3 H) 7.20 - 7.28 (m, 1 H) 7.38 (br d, J=6.6 Hz, 1 H) 7.55 - 7.66 (m, 2 H) |
| **C140** | 1H NMR (400 MHz, DMSO-*d6* +CC14) δ ppm 1.23 (br d, J=7.0 Hz, 3 H) 2.53 - 2.61 (m, 1 H) 2.95 (br dd, J=16.2, 5.6 Hz, 1 H) 3.64 - 3.73 (m, 2 H) 3.84 (s, 3 H) 4.06 - 4.22 (m, 1 H) 4.23 - 4.30 (m, 2 H) 4.37 - 4.68 (m, 3 H) 5.12 - 5.51 (m, 1 H) 6.97 - 7.15 (m, 3 H) 7.38 (br d, J=7.4 Hz, 1 H) 7.55 - 7.65 (m, 2 H) |
| **C157** | 1H NMR (400 MHz, DMSO-*d6* +CC14) δ ppm 1.17 - 1.28 (m, 3 H) 2.23 (s, 3 H) 2.53 - 2.61 (m, 1 H) 2.94 (br dd, J=16.2, 5.4 Hz, 1 H) 3.64 - 3.76 (m, 5 H) 4.26 (br s, 3 H) 4.33 - 4.57 (m, 3 H) 5.18 - 5.46 (m, 1 H) 5.90 (br s, 1 H) 7.37 (br d, J=8.3 Hz, 1 H) 7.59 (s, 1 H) 7.63 (d, J=8.3 Hz, 1 H) |
| **C160** | 1H NMR (400 MHz, DMSO-*d6*, 121°C) δ ppm 7.69 (d, J=8.2 Hz, 1 H), 7.64 (d, J=1.9 Hz, 1 H), 7.57 (br s, 1 H), 7.40 (dd, J=8.2, 2.0 Hz, 1 H), 5.08 (br d, J=17.2 Hz, 1 H), 4.75 - 4.86 (m, 2 H), 4.59 - 4.72 (m, 1 H), 4.26 - 4.34 (m, 3 H), 3.74 - 3.80 (m, 2 H), 2.96 (dd, J=15.9, 5.9 Hz, 1 H), 2.55 - 2.63 (m, 4 H), 1.18 (d, J=6.9 Hz, 3 H) |
| **C166** | 1H NMR (400 MHz, DMSO-*d6* +CC14) δ ppm 1.13 - 1.24 (m, 3 H) 2.50 - 2.57 (m, 1 H) 2.92 (br dd, J=15.4, 5.8 Hz, 1 H) 3.55 - 3.67 (m, 2 H) 3.85 (br s, 3 H) 4.04 - 4.25 (m, 3 H) 4.44 - 4.77 (m, 3 H) 5.23 - 5.46 (m, 1 H) 6.70 (br t, J=8.5 Hz, 1 H) 6.79 (br d, J=8.7 Hz, 1 H) 7.27 (q, J=7.5 Hz, 1 H) 7.36 (br d, J=7.5 Hz, 1 H) 7.57 (br s, 1 H) 7.61 (br d, J=8.3 Hz, 1 H) |
| **C175** | 1H NMR (500 MHz, DMSO-*d6*) δ ppm 1.04 - 1.24 (m, 3 H) 2.55 - 2.61 (m, 1 H) 2.89 - 2.97 (m, 1 H) 3.73 - 3.87 (m, 2 H) 4.13 - 4.21 (m, 1 H) 4.35 (br s, 2 H) 4.43 - 4.52 (m, 1 H) 4.78 (br s, 2 H) 5.32 - 5.46 (m, 1 H) 7.44 (br d, J=8.1 Hz, 1 H) 7.50 - 7.75 (m, 4 H) 7.84 - 7.91 (m, 1 H) |
| **C204** | 1H NMR (400 MHz, DMSO-*d6* +CC14) δ ppm 1.22 (br s, 3 H) 2.52 (br s, 3 H) 2.54 - 2.62 (m, 1 H) 2.91 - 2.98 (m, 1 H) 3.72 (br s, 2 H) 4.30 (br s, 3 H) 4.71 (br s, 3 H) 5.26 - 5.49 (m, 1 H) 7.26 - 7.43 (m, 3 H) 7.56 - 7.66 (m, 3 H) |
| **C221** | 1H NMR (400 MHz, CHLOROFORM-d) δ ppm 1.28 (br s, 3 H) 1.65 (br s, 3 H) 2.32 (br s, 3 H) 2.65 - 2.75 (m, 1 H) 2.98 - 3.19 (m, 1 H) 3.58 - 3.70 (m, 1 H) 3.71 - 3.81 (m, 1 H) 4.33 (br s, 3 H) 4.44 - 4.64 (m, 1 H) 5.34 - 5.56 (m, 1 H) 5.95 - 6.08 (m, 1 H) 6.70 (br d, J=4.6 Hz, 1 H) 7.28 - 7.31 (m, 1 H) 7.48 - 7.59 (m, 2 H) |

Compounds of Formula (Ia) were prepared by the following methods:

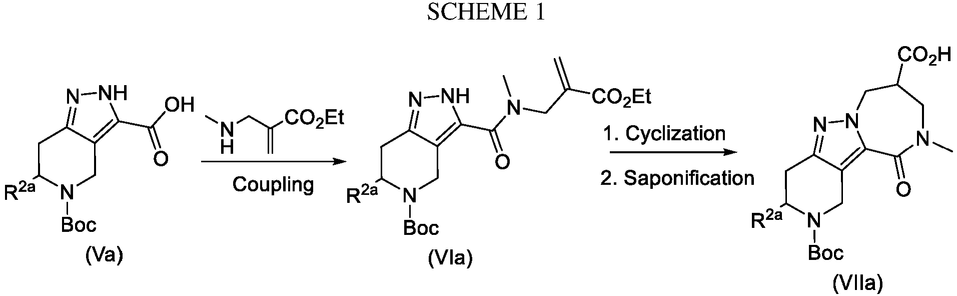

According to SCHEME 1, a commercially available or synthetically accessible compound of formula (Va), where R^{2a} is H or C₁₋₆alkyl, is coupled with commercially available or synthetically accessible ethyl 2-(methylaminomethyl) prop-2-enoate, under amide bond coupling conditions to provide a compound of formula (VIa). For example, an acid compound of formula (Va) is reacted with ethyl 2-(methylaminomethyl) prop-2-enoate, in the presence of a dehydrating agent such as hydroxybenzotriazole (HOBt)/1-ethyl-3-(3-dimethylaminopropyl)-carbodiimide (EDAC), 1,1'-carbonyldiimidazole (CDI), benzotriazol-1-yl-oxytripyrrolidino-phosphonium hexafluorophosphate (PyBOP), 1-[Bis(dimethylamino)methylene]-1H-1,2,3-triazolo[4,5-b]pyridinium 3-oxid hexafluorophosphate (HATU), 1-yydroxy-7-azabenzotriazole (HOAT), propylphosphonic anhydride (T₃P), a suitably selected base such as N,N-diisopropylethylamine (DIPEA), triethylamine (TEA), and the like, in a solvent such as toluene, acetonitrile (ACN), ethyl acetate (EtOAc), dimethylformamide (DMF), tetrahydrofuran (THF), dichloromethane (DCM), or a mixture thereof, to afford a compound of formula (VIa).

A compound of formula (VIa), where R^{2a} is H or C₁₋₆alkyl, is cyclized, employing a base such as 1,8-diazabicyclo[5.4.0]undec-7-ene (DBU), in a solvent such as ACN, at a temperature of about 40-60 °C for a period of 1-3 h. Subsequent saponification, employing a base such as sodium hydroxide, potassium hydroxide, or the like; in a polar solvent such as water, methanol (MeOH) or a mixture thereof; provides a compound of formula (VIIa), where R^{2a} is H or C₁₋₆alkyl.

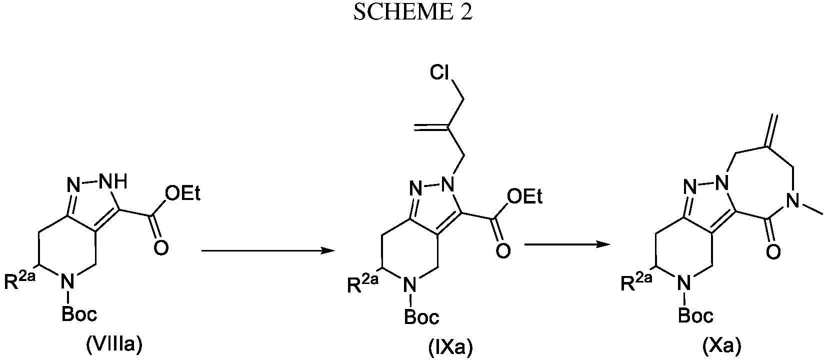

According to SCHEME 2, a commercially available or synthetically accessible compound of formula (IXa), where R^{2a} is H or C₁₋₆alkyl, is alkylated with 3-chloro-2-(chloromethyl)prop-1-ene, in a suitable solvent such as DMF, and the like, in the presence of a base such as K₂CO₃, and the like, at temperatures ranging from 25 °C to 75 °C, for a period of 10-24 h, to provide a compound of formula (IXa). A compound of formula (IXa), where R^{2a} is H or C₁₋₆alkyl, is reacted with methanamine, in a solvent such as ethanol (EtOH), at a temperature of about 80 °C for a period of 16 h, to provide a compound of formula (Xa) where R^{2a} is H or C₁₋₆alkyl.

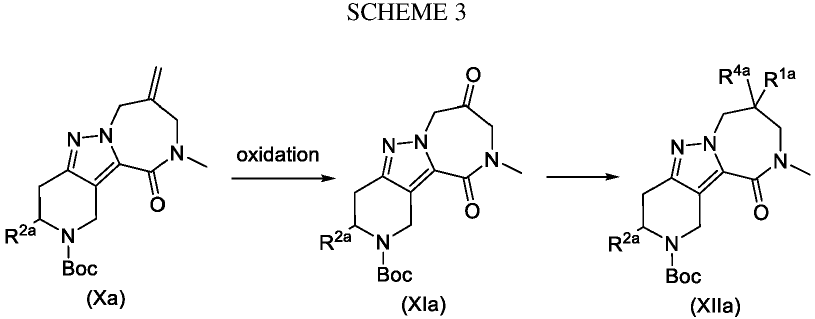

According to SCHEME 3, a compound of formula (Xa) where R^{2a} is H or C₁₋₆alkyl, is oxidized, employing oxidation conditions known to one skilled in the art, for example OsO₄ and NaIO₄, in a suitable solvent such as THF, and the like, at temperatures ranging from 0 °C to 15 °C, for a period of 10-20 h, provides a compound of formula (XIa). A compound of formula (XIa) is reacted with a Grignard reagent such as phenylmagnesium bromide, in a suitable solvent such as THF, and the like, to provide a compound of formula (XIIa), where R^{2a} is H or C₁₋₆alkyl, R^{1a} is OH, and R^{4a} is phenyl.

A compound of formula (XIIa), where R^{2a} is H or C₁₋₆alkyl, R^{1a} is OH, and R^{4a} is phenyl, is fluorinated with a fluorinating agent such as, diethylaminosulfur trifluoride (DAST), (Diethyl-amino)difluorosulfonium tetrafluoroborate (XtalFluor^{®}), bis(2-methoxyethyl)aminosulfur trifluoride (Deoxo-Fluor^{®}), and the like, in a suitable solvent such as DCM, and the like, at temperatures ranging from -78 °C to 50 °C, for a period of 2-16 h, to provide a compound of formula (XIIa), where R^{2a} is H or C₁₋₆alkyl, R^{1a} is F, and R^{4a} is phenyl.

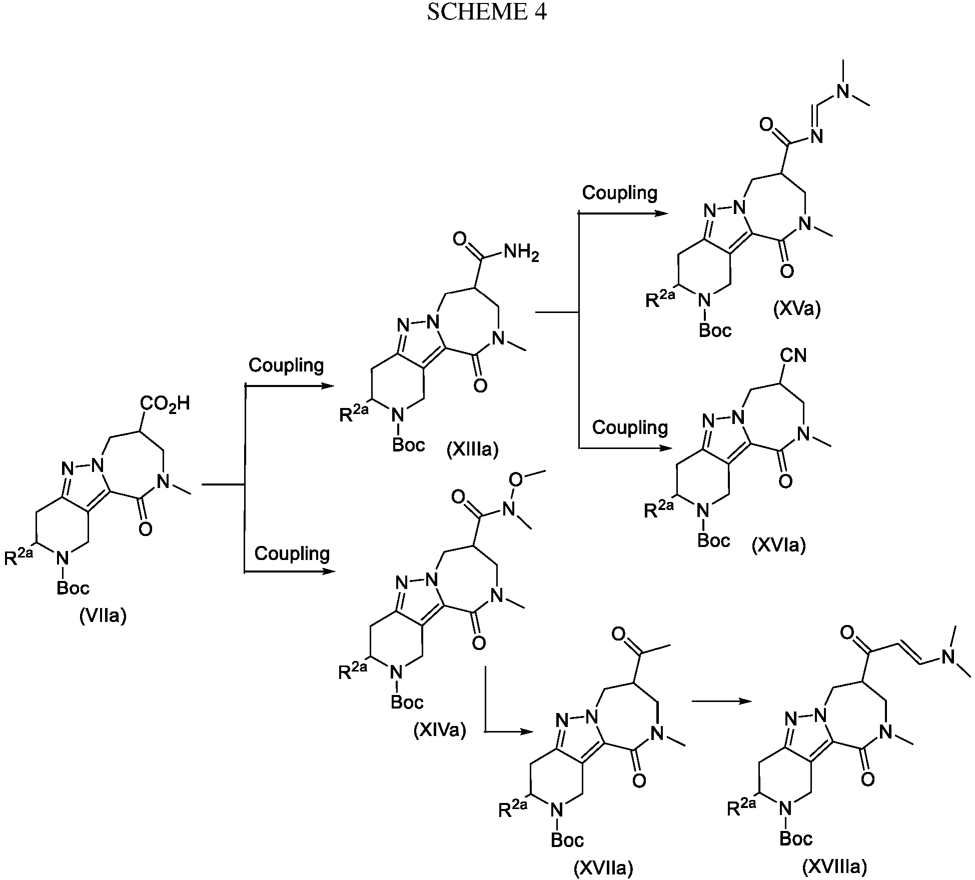

According to SCHEME 4, a compound of formula (VIIa) is converted to its corresponding amide employing amide bond forming conditions known to one skilled in the art, or as previously described. For example, reaction of a compound of formula (VIIa) with ammonium chloride; PyBOP; an activating agent such as HOBt; in the presence of a base such as DIPEA, and the like; in a suitable solvent, provides a compound of formula (XIIIa). In a similar fashion, a compound of formula (VIIa) is reacted with N-methoxymethanamine; in the presence of T₃P; and in the presence of a tertiary amino base such as triethylamine, and the like; in an aprotic organic solvent such as THF, and the like the like; to afford a compound of formula (XVIa), where R^{2a} is H or C₁₋₆alkyl.

Treatment of a compound of formula (XIIIa) with dimethylformamide (DMF)-dimethylacetamide (DMA) affords the dimethylaminomethylenecarbamoyl substituted compound of formula (XVa). Treatment of a compound of formula (XIIIa) with trifluoroacetic anhydride (TFAA), in the presence of an organic base such as triethylamine (TEA), in a polar aprotic solvent such as dichloromethane, affords the nitrile of formula (XVIa).

The compound of formula (XVIa) is converted to a ketone of formula (XVIIa) by the action of an organometallic halide such as methylmagnesium bromide, in a polar aprotic solvent such as THF. Subsequent treatment of a compound of formula (XVIIa) with DMF-DMA at a temperature of about 70 °C affords a compound of formula (XVIIIa).

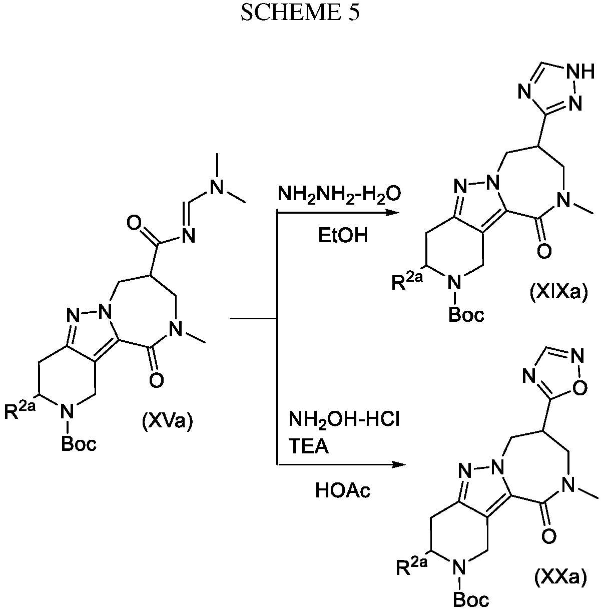

The triazolyl substituted compound of formula (XIXa) is prepared via the reaction of a compound of formula (XVa) with hydrazine hydrate in an alcoholic solvent.

Alternatively, a compound of formula (XVa) is reacted with hydroxylamine hydrochloride, in the presence of a base such as triethylamine, in the presence of acetic acid, to afford a oxadiazolyl substituted compound of formula (XXa).

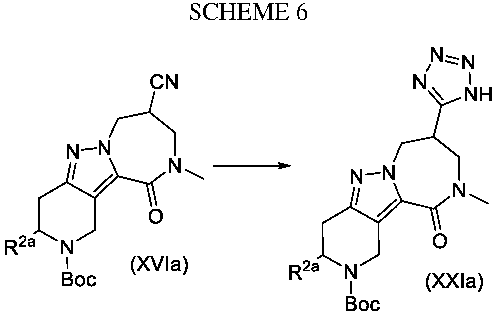

The compound of formula (XVIa) is reacted with sodium azide, in the presence of ACN, in a polar aprotic solvent such as DMF to afford a compound of formula (XXIa) wherein R^{4a} is tetrazolyl.

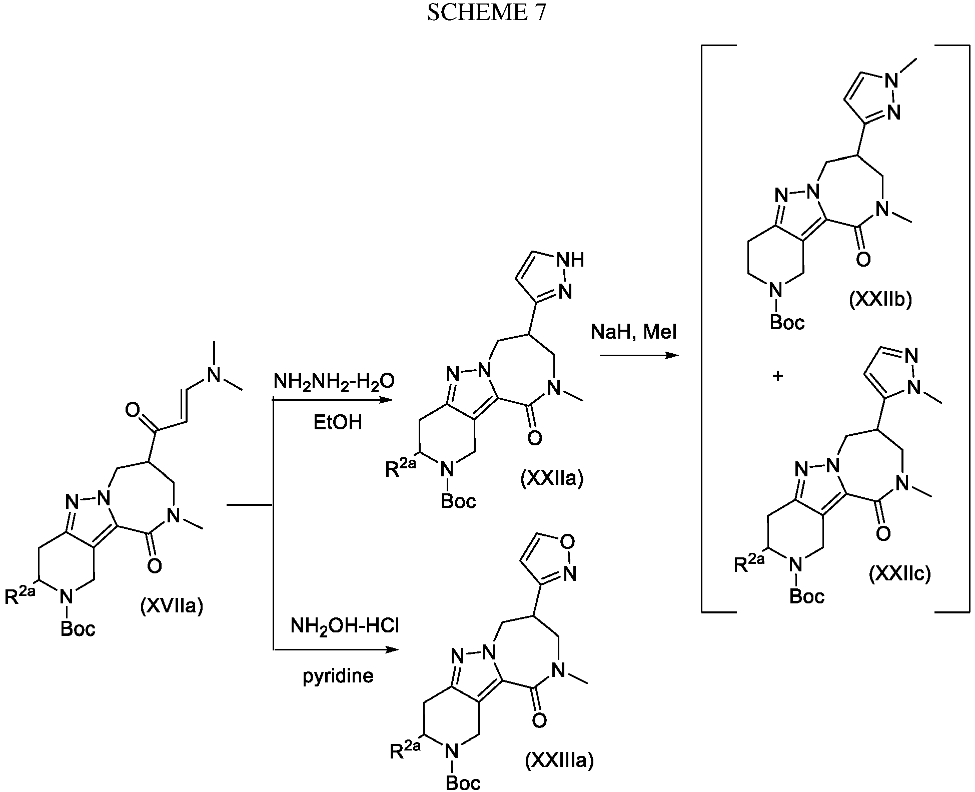

A compound of formula (XVIIIa), is cyclized in the presence of hydrazine hydrate to afford a compound of formula (XXIIa). A compound of formula (XXIIa) is alkylated with an alkylating agent such as a C₁₋₆alkylhalide, and the like, a base such as NaH, and the like, in a suitable solvent such as such as THF, DMF, and the like, to provide a compound of formula (XXIIb, and XXIIc).

Alternatively, a compound of formula (XVIIIa) with hydroxylamine hydrochloride, in the presence of a base such as triethylamine, in the presence of acetic acid, to afford a compound of formula (XXIIIa).

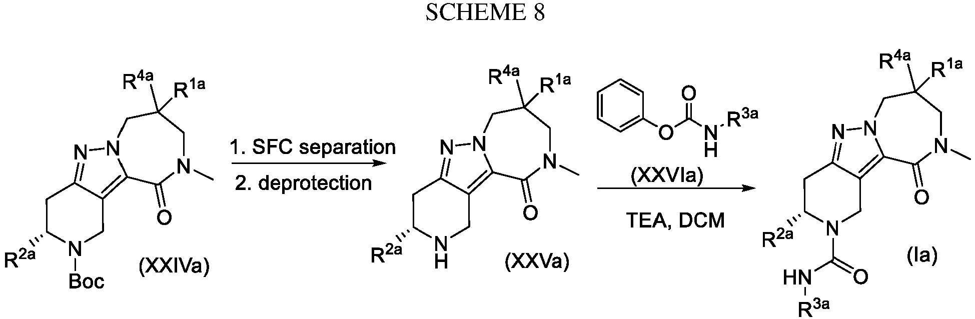

According to SCHEME 8, separation into stereoisomers of formula (XXIVa) (which encompasses compounds of formulas (XIIa), (XIXa), (XXa), (XXIa), (XXIIa), (XXIIb), (XXIIc) and (XXIIIa), is achieved via supercritical fluid chromatography. Cleavage of the BOC protecting group on a compound of formula (XXIVa) (which encompasses compounds of formulas (XIIa), (XIXa), (XXa), (XXIa), (XXIIa), (XXIIb), (XXIIc) and (XXIIIa), wherein R^{1a}, R^{2a}, and R^{4a} are as defined above; is achieved according to procedures known to one skilled in the art and employing established methodologies, such as those described in T. W. Greene and P. G. M. Wuts, "Protective Groups in Organic Synthesis," 3 ed., John Wiley & Sons, 1999. For example, under acidic conditions such as TFA/CH₂Cl₂, HCl/Dioxane, and the like, provides a compound of formula (XXVa).

Subsequent reaction with a commercially available or synthetically accessible compound of formula (XXVIa), where wherein R^{1a}, R^{2a}, and R^{4a} are as defined above; a suitable base such as TEA, and the like; in a suitable solvent such as DCM, and the like; provides a compound of Formula (Ia).

Compounds of Formula (Ia) may be converted to their corresponding salts using methods known to one of ordinary skill in the art. For example, an amine of Formula (Ia) is treated with trifluoroacetic acid, HCl, or citric acid in a solvent such as Et₂O, CH₂Cl₂, THF, MeOH, chloroform, or isopropanol to provide the corresponding salt form. Alternately, trifluoroacetic acid or formic acid salts are obtained as a result of reverse phase HPLC purification conditions. Cyrstalline forms of pharmaceutically acceptable salts of compounds of Formula (Ia) may be obtained in crystalline form by recrystallization from polar solvents (including mixtures of polar solvents and aqueous mixtures of polar solvents) or from non-polar solvents (including mixtures of non-polar solvents).

Where the compounds according to this present disclosure have at least one chiral center, they may accordingly exist as enantiomers. Where the compounds possess two or more chiral centers, they may additionally exist as diastereomers. It is to be understood that all such isomers and mixtures thereof are encompassed within the scope of the present disclosure.

Compounds of formulas represented in the SCHEMES above represented as "stereomeric mixture" (means a mixture of two or more stereoisomers and includes enantiomers, diastereomers and combinations thereof) are separated by SFC resolution.

### General Procedures

The following specific examples are provided to further illustrate the present disclosure and various preferred embodiments. In obtaining the compounds described in the examples below and the corresponding analytical data, the following experimental and analytical protocols were followed unless otherwise indicated.

Unless otherwise stated, reaction mixtures were magnetically stirred at room temperature (rt) under a nitrogen atmosphere. Where solutions were "dried," they were generally dried over a drying agent such as Na₂SO₄ or MgSO₄. Where mixtures, solutions, and extracts were "concentrated", they were typically concentrated on a rotary evaporator under reduced pressure.

Normal-phase silica gel chromatography (FCC) was performed on silica gel (SiO₂) using prepacked cartridges.

Preparative reverse-phase high performance liquid chromatography (RP HPLC) was performed on either:
METHOD A. A Gilson GX-281 semi-prep-HPLC with Phenomenex Synergi C18(10µm, 150 x 25mm), or Boston Green ODS C18(5µm, 150 x 30mm), and mobile phase of 5-99% ACN in water (with 0.225%FA) over 10 min and then hold at 100% ACN for 2 min, at a flow rate of 25 mL/min. or
METHOD B. A Gilson GX-281 semi-prep-HPLC with Phenomenex Synergi C18(10µm, 150 x 25mm), or Boston Green ODS C18(5µm, 150 x 30mm), and mobile phase of 5-99% ACN in water(0.1%TFA) over 10 min and then hold at 100% ACN for 2 min, at a flow rate of 25 mL/min. or
METHOD C. A Gilson GX-281 semi-prep-HPLC with Phenomenex Synergi C18(10µm, 150 x 25mm), or Boston Green ODS C18(5µm, 150 x 30mm), and mobile phase of 5-99% ACN in water(0.05%HCl) over 10 min and then hold at 100% ACN for 2 min, at a flow rate of 25 mL/min. or
METHOD D. A Gilson GX-281 semi-prep-HPLC with Phenomenex Gemini C18 (10µm, 150 x 25mm), AD(10µm, 250mm x 30mm), or Waters XBridge C18 column (5µm, 150 x 30mm), mobile phase of 0-99% ACN in water (with 0.05% ammonia hydroxide v/v) over 10 min and then hold at 100% ACN for 2 min, at a flow rate of 25 mL/min. or
METHOD E. A Gilson GX-281 semi-prep-HPLC with Phenomenex Gemini C18 (10µm, 150 x 25mm), or Waters XBridge C18 column (5µm, 150 x 30mm), mobile phase of 5-99% ACN in water(10mM NH4HCO3) over 10 min and then hold at 100% ACN for 2 min, at a flow rate of 25 mL/min.

Preparative supercritical fluid high performance liquid chromatography (SFC) was performed either on a Thar 80 Prep-SFC system, or Waters 80Q Prep-SFC system from Waters. The ABPR was set to 100bar to keep the CO₂ in SF conditions, and the flow rate may verify according to the compound characteristics, with a flow rate ranging from 50g/min to 70g/min. The column temperature was ambient temperature

Mass spectra (MS) were obtained on a SHIMADZU LCMS-2020 MSD or Agilent 1200\G6110A MSD using electrospray ionization (ESI) in positive mode unless otherwise indicated. Calculated (calcd.) mass corresponds to the exact mass.

Nuclear magnetic resonance (NMR) spectra were obtained on Bruker model AVIII 400 spectrometers. Definitions for multiplicity are as follows: s = singlet, d = doublet, t= triplet, q = quartet, m = multiplet, br = broad. It will be understood that for compounds comprising an exchangeable proton, said proton may or may not be visible on an NMR spectrum depending on the choice of solvent used for running the NMR spectrum and the concentration of the compound in the solution.

Chemical names were generated using ChemDraw Ultra 12.0, ChemDraw Ultra 14.0 (CambridgeSoft Corp., Cambridge, MA) or ACD/Name Version 10.01 (Advanced Chemistry).

Compounds designated as R* or S* are enantiopure compounds where the absolute configuration was not determined.

### Intermediate 1a. 2-tert-Butyl 8-ethyl 10-methyl-11-oxo-3,4,8,9,10,11-hexahydro-1H-pyrido[4',3':3,4]pyrazolo[1,5-a][1,4]diazepine-2,8(7H)-dicarboxylate.

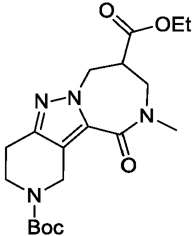

Step A. Ethyl 2-[[tert-butoxycarbonyl(methyl)amino]methyl]prop-2-enoate. A mixture of tert-butyl N-methylcarbamate (200.00 mg, 1.52 mmol, 1.00 *eq)* in THF (5.00 mL) was added NaH (91.20 mg, 2.28 mmol, 60% purity, 1.50 *eq)* at 0 °C for 0.5 hr under N₂, then ethyl 2-(bromomethyl)prop-2-enoate (352.10 mg, 1.82 mmol, 1.20 *eq)* was added to the mixture dropwise at 0 °C, and the mixture was stirred at 15 °C for 2 hr under N₂ atmosphere. The mixture was poured into ice-water (10 mL) and stirred for 5 min. The aqueous phase was extracted with ethyl acetate (5 mL x 3). The combined organic phase was washed with brine (10 mL), dried with anhydrous Na₂SO₄, filtered and concentrated in vacuum. The residue was purified by column chromatography (SiO₂, Petroleum ether/Ethyl acetate=100/1 to 5/1) to afford the title compound (112.00 mg, 460.34 µmol, 30.29% yield) as colorless oil. ¹H NMR (400MHz, CDCl₃) δ 6.28 (s, 1 H), 5.55 (s, 1 H), 4.23 (q, *J=* 7.1 Hz, 2 H), 4.07 (br s, 2 H), 2.88 (br s, 3 H), 1.45 (br s, 9 H), 1.31 (br s, 3 H).

Step B. Ethyl 2-(methylaminomethyl) prop-2-enoate. A mixture of ethyl 2-[[tert-butoxycarbonyl(methyl)amino]methyl]prop-2-enoate (112.00 mg, 460.34 µmol, 1.00 *eq)* in dioxane (1.00 mL) was added HCl/dioxane (4 M, 5.00 mL, 43.45 *eq),* and then the mixture was stirred at 15 °C for 0.5 hour. The mixture was concentrated in vacuum to afford the title compound (82.50 mg, 459.25 µmol, 99.76% yield, HCl) as a white solid, which was used directly for the next step.

Step C. tert-Butyl 3-[2-ethoxycarbonylallyl(methyl)carbamoyl]-2,4,6,7-tetrahydropyrazolo[4,3-clpyridine-5-carboxylate. A mixture of 5-tert-butoxycarbonyl-2,4,6,7-tetrahydropyrazolo[4,3-c]pyridine-3-carboxylic acid (80.00 mg, 299.31 µmol, 1.00 *eq),* ethyl 2-(methylaminomethyl) prop-2-enoate (59.14 mg, 329.24 µmol, 1.10 *eq,* HCl), T₃P (285.70 mg, 897.93 µmol, 267.01 µL, 3.00 *eq)* and TEA (151.44 mg, 1.50 mmol, 207.45 µL, 5.00 *eq)* in THF (3.00 mL) was degassed and purged with N₂ for 3 times, and then the mixture was stirred at 15 °C for 16 hours under N₂ atmosphere. The mixture was poured into water (10 mL) and stirred for 5 min. The aqueous phase was extracted with ethyl acetate (5 mL x 3). The combined organic phase was washed with brine (10 mL), dried with anhydrous Na₂SO₄, filtered and concentrated in vacuum. The residue was purified by prep-TLC (DCM/MeOH=10/1) to afford the title compound (46.00 mg, 105.49 µmol, 35.24% yield, 90% purity) as a white solid. LCMS: 393 [M+1]. ¹H NMR (400MHz, CDCl₃) δ 6.35 (s, 1 H), 5.67 (br s, 1 H), 4.63 (s, 4 H), 4.18 - 4.30 (m, 2 H), 3.71 (br s, 2 H), 2.91 - 3.47 (m, 3 H), 2.74 (br t, *J=* 5.4 Hz, 2 H), 1.48 (s, 9 H), 1.31 (t, *J* = 7.2 Hz, 3 H). Step D. 2-tert-Butyl 8-ethyl 10-methyl-11-oxo-3,4,8,9,10,11-hexahydro-1H -pyrido[4',3':3,4]-pyrazolo[1,5-a][1,4]diazepine-2,8(7H)-dicarboxylate. A mixture of tert-butyl 3-[2-ethoxy-carbonylallyl(methyl)carbamoyl]-2,4,6,7-tetrahyd ropyrazolo[4,3-c]pyridine-5-carboxylate (36.00 mg, 91.73 µmol, 1.00 *eq),* DBU (6.98 mg, 45.87 µmol, 6.91 µL, 0.50 *eq)* in MeCN (1.00 mL) was degassed and purged with N₂ for 3 times, and then the mixture was stirred at 50 °C for 2 hour under N₂ atmosphere. The mixture was poured into water (5 mL) and stirred for 5 min. The aqueous phase was extracted with ethyl acetate (3 mL x 3). The combined organic phase was washed with brine (5 mL), dried with anhydrous Na₂SO₄, filtered and concentrated in vacuum. The residue was purified by prep-TLC (DCM/MeOH=20/1) to afford the title compound (20.00 mg, 50.96 µmol, 55.56% yield) as a white solid. LCMS: 393 [M+1].

### Intermediate 2a: tert-Butyl 10-methyl-11-oxo-8-(1H-1,2,4-triazol-3-yl)-3,4,8,9,10,11-hexahydro-1H-pyrido[4',3':3,4]pyrazolo[1,5-a][1,4]diazepine-2(7H)-carboxylate.

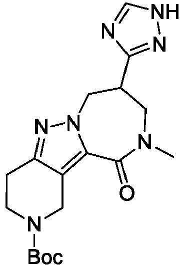

Step A. 2-(tert-Butoxycarbonyl)-10-methyl-11-o-2,3,4,7,8,9,10,11-octahydro-1H-pyrido-[4',3':3,4]pyrazolo[1,5-a][1,4]diazepine-8-carboxylic acid. To a solution of 2-tert-butyl 8-ethyl 10-methyl-11-oxo-3,4,8,9,10,11-hexahydro-1H -pyrido[4',3':3,4]pyrazolo[1,5-a][1,4]diazepine-2,8(7H)-dicarboxylate (Intermediate 1, 3.00 g, 7.64 mmol) in MeOH (30.00 mL) was added a solution of NaOH (458.40 mg, 11.46 mmol) in water (6.00 mL). The reaction mixture was stirred at 30 °C for 16 h. The reaction mixture was adjusted to about pH 6 by adding diluted hydrochloride acid (2 N, 3 mL). The mixture was diluted with water (20 mL), then extracted with ethyl acetate (100 mL×3). The combined organic phases were dried over anhydrous Na₂SO₄, filtered, and the filtrate concentrated under reduced pressure to give title compound (2.50 g, crude) as a white solid, used in the next step directly without further purification. MS (ESI): mass calcd. for C₁₇H₂₄N₄O₅, 364.1; m/z found, 365.1 [M+H]⁺.

Step B. tert-Butyl 8-carbamoyl-10-methyl-11-oxo-3,4,8,9,10,11-hexahydro-1H-pyrido-[4',3':3,4]pyrazolo[1,5-a][1,4]diazepine-2(7H)-carboxylate. To a mixture of 2-(tert-butoxycarbonyl)-10-methyl-11-oxo-2,3,4,7,8,9,10,11 -octahydro-1H-pyrido[4',3':3,4]pyrazolo[1,5-a][1,4]diazepine-8-carboxylic acid (200.00 mg, 548.85 µmol) and NH₄Cl (146.79 mg, 2.74 mmol, 95.94 µL) in DMF (5.00 mL) was added PyBOP (314.18 mg, 603.73µmol), HOBt (81.58 mg, 603.73µmol) and DIPEA (212.80 mg, 1.65 mmol, 287.57 µL). The reaction mixture was stirred at 15 °C for 2 h. The reaction mixture was diluted with ethyl acetate (100 mL), washed with diluted HCl (1N, 30 mL×2), and the organic phases were dried over anhydrous Na₂SO₄, filtered, and the filtrate concentrated under reduced pressure. The residue was purified by column chromatography over silica gel (Dichloromethane/Methanol=50/1 to 10/1) to give the title compound (173.00 mg, 476.05µmol, 86.74% yield) as a white solid. MS (ESI): mass calcd. for C₁₇H₂₅N₅O₄ 363.1; m/z found, 364.1 [M+H]⁺.

Step C. tert-Butyl 8-(((dimethylamino)methylene)carbamoyl)-10-methyl-11-oxo-3,4,8,9,10,11-hexahydro-1H-pyrido[4',3':3,4]pyrazolo[1,5-a][1,4]diazepine-2(7H)-carboxylate.Tert-butyl 8-carbamoyl-10-methyl-11-oxo-3,4,8,9,10,11-hexahydro-1H-pyrido [4',3':3,4]pyrazolo[1,5-a][1,4]diazepine-2(7H)-carboxylate (170.00 mg, 467.79µmol) was dissolved in DMF-DMA (1.80 g, 15.10 mmol, 2.00 mL). The reaction mixture was stirred at 70 °C for 16 h. The reaction mixture was diluted with ethyl acetate (100 mL), washed with water (50 mL×2), and the organic phases were dried over anhydrous Na₂SO₄, filtered, and the filtrate concentrated under reduced pressure to give title compound (170.00 mg, crude), which was used in the next step directly without further purification.

Step D. tert-Butyl 10-methyl-11-oxo-8-(1H-1,2,4-triazol-3-yl)-3,4,8,9,10,11-hexahydro-1H-pyrido[4',3':3,4]pyrazolo[1,5-a][1,4]diazepine-2(7H)-carboxylate. To a mixture of tert-butyl 8-(((dimethylamino)methylene)carbamoyl)-10-methyl-11-oxo-3,4,8,9,10,11-hexahydro-1H-pyrido[4',3':3,4]pyrazolo[1,5-a][1,4]diazepine-2(7H)-carboxylate (60 mg crude) in EtOH (5.00 mL) was added NH₂NH₂·H₂O (14.65 mg, 286.74 µmol, 14.22 µL, 98% purity), then the reaction mixture was stirred at 80 °C for 30 minutes. The reaction mixture was diluted with ethyl acetate (100 mL), washed with water (50 mL×2), and the organic phases were dried over anhydrous Na₂SO₄, filtered, and the filtrate concentrated under reduced pressure. The residue was purified by prep-TLC (DCM/MeOH=10/1) to give the title compound (30.00 mg, 77.43 µmol, 54.01% yield) as a yellow oil. MS (ESI): mass calcd. for C₁₈H₂₅N₇O, 387.2; m/z found, 388.2 [M+H]⁺.

### Intermediate 3a: tert-Butyl 10-methyl-8-(1,2,4-oxadiazol-5-yl)-11-oxo-3,4,8,9,10,11-hexahydro-1H-pyrido[4',3':3,4]pyrazolo[1,5-a][1,4]diazepine-2(7H)-carboxylate.

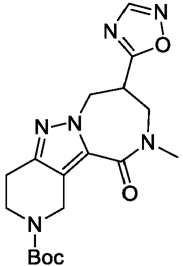

To a mixture tert-butyl 8-(((dimethylamino)methylene)carbamoyl)-10-methyl -11-oxo-3,4,8,9, 10,11-hexahydro-1H-pyrido[4',3':3,4]pyrazolo[1,5-a][1,4]diazepine-2(7H)-carboxylate (80 mg crude, prepared from Step C of Intermediate 2) in AcOH (2.00 mL) was added NH₂OH·HCl (106.27 mg, 1.53 mmol) and TEA (232.13 mg, 2.29 mmol, 317.99 µL). The reaction mixture was stirred at 50 °C for 3 h. The reaction mixture was diluted with ethyl acetate (100 mL), washed with water (50 mL×2), and the organic phases were dried over anhydrous Na₂SO₄, filtered, and the filtrate concentrated under reduced pressure. The residue was purified by prep-TLC (Rf=0.65, DCM/MeOH=10/1) to give the title compound (20.00 mg, 51.49 µmol, 26.94% yield) as a yellow oil. MS (ESI): mass calcd. For C₁₈H₂₄N₆O₄ 388.2; m/z found, 411.2 [M+Na]⁺.

### Intermediate 4a: tert-Butyl 10-methyl-11-oxo-8-(1H-tetrazol-5-yl)-3,4,8,9,10,11-hexahydro-1H-pyrido[4',3':3,4]pyrazolo[1,5-a][1,4]diazepine-2(7H)-carboxylate.

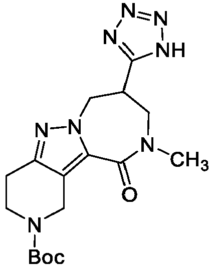

Step A. tert-Butyl 8-cyano-10-methyl-11-oxo-3,4,8,9,10,11-hexahydro-1H-pyrido[4',3':3,4]-pyrazolo[1,5-a][1,4]diazepine-2(7H)-carboxylate. To a mixture of tert-butyl 8-carbamoyl-10-methyl-11-oxo-3,4,8,9,10,11-hexahydro -1H-pyrido[4',3':3,4]pyrazolo[1,5-a][1,4]diazepine-2(7H)-carboxylate (Intermediate 2, Step B; 500.00 mg, 1.38 mmol) and TEA (696.11 mg, 6.88 mmol, 953.58 µL) in DCM (3.00 mL) was added TFAA (577.94 mg, 2.75 mmol, 382.74 µL) under N₂ at 0 °C. The reaction mixture was stirred at 15 °C for 3 h. The organic phases were dried over anhydrous Na₂SO₄, filtered, and the filtrate concentrated under reduced pressure. The residue was purified by column chromatography (SiO₂, Dichloromethane/Methanol=60/1 to 30/1) to give the title compound (320.00 mg, 926.46 µmol, 67.13% yield) as a white solid. MS (ESI): mass calcd. for C₁₇H₂₃N₅O₃ 345.2; m/z found, 368.2 [M+Na]⁺.

Step B. tert-Butyl 10-methyl-11-oxo-8-(1H-tetrazol-5-yl)-3,4,8,9,10,11-hexahydro-1H-pyrido[4',3':3,4]pyrazolo[1,5-a][1,4]diazepine-2(7H)-carboxylate. To a mixture of tert-butyl 8-cyano-10-methyl-11-oxo-3,4,8,9,10,11-hexahydro-1H -pyrido[4',3':3,4]pyrazolo[1,5-a][1,4]-diazepine-2(7H)-carboxylate (80.00 mg, 231.62 µmol) in DMF (2.00 mL) was added NaN₃ (22.59 mg, 347.43 µmol ) and ACN (12.70 mg, 23.16 µmol, 11.55 µL ) under N₂. The reaction mixture was stirred at 110 °C for 16 h. The reaction mixture was diluted with ethyl acetate (50 mL) and washed with diluted HCl (1N, 30 mL×2), and the organic phases were dried over anhydrous Na₂SO₄, filtered, and the filtrate concentrated under reduced pressure. The residue was purified by RP HPLC (Condition A) to give tert-butyl 10-methyl-11-oxo-8-(1H-tetrazol-5-yl)-3,4,8,9,10,11-hexahydro-1H- pyrido[4',3':3,4]pyrazolo[1,5-a][1,4]diazepine-2(7H)-carboxylate(43.00 mg, 110.70 µmol, 47.80% yield) as a white solid. MS (ESI): mass calcd. For C₁₇H₂₄N₈O₃ 388.2; m/z found, 389.2 [M+H]⁺. ¹H NMR (400 MHz, CDCl₃) δ 4.82 - 4.91 (m, 1H), 4.73 - 4.81 (m, 1H), 4.62 (br s, 2 H), 4.19 (br s, 1H), 3.78 - 3.94 (m, 2H), 3.65 (br s, 2H), 3.20 (br s, 3H), 2.71 - 2.85 (m, 2 H), 1.49 (s, 9 H).

### Intermediate 5a: tert-Butyl 10-methyl-11-oxo-8-(1H-pyrazol-3-yl)-3,4,8,9,10,11- hexahydro-1H-pyrido[4',3':3,4]pyrazolo[1,5-a][1,4]diazepine-2(7H)-carboxylate.

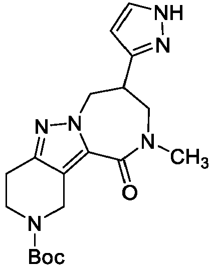

Step A. tert-Butyl 8-(methoxy(methyl)carbamoyl)-10-methyl-11-oxo-3,4,8,9,10,11-hexahydro-1H-pyrido[4',3':3,4]pyrazolo[1,5-a][1,4]diazepine-2(7H)-carboxylate. To a mixture of 2-(tert-butoxycarbonyl)-10-methyl-11-oxo-2,3,4,7,8,9,10,11-octahydro-1H-pyrido[4',3':3,4]pyrazolo-[1,5-a][1,4]diazepine-8-carboxylic acid (Intermediate 2, product from Step A; 7.00 g, 19.21 mmol) and N-methoxymethanamine hydrochloride (7.49 g, 76.84 mmol) in THF (100.00 mL) was added T₃P (24.45 g, 38.42 mmol, 22.85 mL, 50% purity) and TEA (29.16 g, 288.14 mmol,) in one portion under N₂. The mixture was stirred at 30 °C for 12 h. The mixture was poured into water (150 mL) and stirred for 15 min, and the aqueous phase was extracted with ethyl acetate (200 mL×2). The combined organic phases were washed with brine (50 mLx1), dried over anhydrous Na₂SO₄, filtered and the filtrate concentrated under reduced pressure. The residue was purified by column chromatography over silica gel (DCM/MeOH = 30/1) to afford the title compound (7.50 g, 18.41 mmol, 95.82% yield) as a yellow oil. MS (ESI): mass calcd. for C₁₉H₂₉N₅O₅ 407.2; m/z found, 408.1 [M+H]⁺. 1H NMR (400 MHz, CDCl₃) δ 4.69 -4.43 (m, 4 H), 3.79 - 3.73 (m, 1H), 7.56 - 7.64 (m, 1H), 3.65 -3.59 (m, 4H), 3.25 (s, 3 H), 3.20 (s, 3H), 2.79 - 2.70 (m, 2H), 1.48 (s, 9H).

Step B. tert-butyl 8-acetyl-10-methyl-11-oxo-3,4,8,9,10,11-hexahydro-1H-pyrido[4',3':3,4]-pyrazolo[1,5-a][1,4]diazepine-2(7H)-carboxylate.To a solution of tert-butyl 8-[methoxy-(methyl)carbamoyl]-10-methyl-11-oxo-1,3,4,7, 8,9-hexahydropyrido[2,3]pyrazolo[2,4-b] [1,4]-diazepine-2-carboxylate(300.00 mg, 736.27 µmol) in THF (5.00 mL) was added MeMgBr (3 M, 736.27 µL) dropwise at -30 °C under N₂. The mixture was heated to 0 °C and stirred for 1 h. The mixture was poured into HCl (1N aq, 80 mL) slowly and extracted with EtOAc (50 mL×3). The combined organic layers were dried over Na₂SO₄, filtered and the filtrate concentrated under reduced pressure to afford the title compound (170.00 mg, crude) as a brown oil. MS (ESI): mass calcd. for C₁₈H₂₆N₄O₄ 362.2; m/z found, 363.2 [M+H]⁺.

Step C. tert-Butyl 8-(3-(dimethylamino)acryloyl)-10-methyl-11-oxo-3,4,8,9,10,11 -hexahydro-1H-pyrido[4',3':3,4]pyrazolo[1,5-a][1,4]diazepine-2(7H)-carboxylate. Tert-butyl 8-acetyl-10-methyl-11-oxo-3,4,8,9,10,11-hexahydro-1H-pyrido[4',3':3,4] pyrazolo[1,5-a][1,4]diazepine-2(7H)-carboxylate (60.00 mg, 165.55 µmol) was dissolved in DMF-DMA (1.35 g, 11.33 mmol, 1.50 mL) and the mixture was stirred at 70 °C for 16 h. The mixture was extracted with EtOAc (20 mL×2) and water (20 mL). The combined organic layer was dried over Na₂SO₄, filtered and the filtrate concentrated under reduced pressure to afford the title compound (80.00 mg, crude) as a yellow oil. MS (ESI): mass calcd. for C₂₁H₃₁N₅O₄ 417.2; m/z found, 418.2 [M+H]⁺.

Step D. tert-Butyl 10-methyl-11-oxo-8-(1H-pyrazol-3-yl)-3,4,8,9,10,11-hexahydro -1H-pyrido[4',3':3,4]pyrazolo[1,5-a][1,4]diazepine-2(7H)-carboxylate.To a solution of tert-butyl 8-(3-(dimethylamino)acryloyl)-10-methyl-11-oxo-3,4,8,9,10,11-hexahydro-1H-pyrido[4',3':3,4]-pyrazolo[1,5-a][1,4]diazepine-2(7H)-carboxylate (90.00 mg, 215.57 µmol) in EtOH (500.00 µL) was added NH₂NH₂•H₂O (22.02 mg, 431.14 µmol, 21.38 µL, 98% purity). The mixture was stirred at 80 °C for 2 h. The mixture was diluted with water (10 mL) and extracted with EtOAc (20 mL×2). The combined organic layers were dried over anhydrous Na₂SO₄, filtered, and the filtrate concentrated under reduced pressure to afford the title compound (70.00 mg, crude) as a yellow oil. MS (ESI): mass calcd. for C₁₉H₂₆N₆O₃ 386.2; m/z found, 387.1 [M+H]⁺.

### Intermediate 6a: tert-Butyl 10-methyl-8-(1-methyl-1H-pyrazol-3-yl)-11-oxo-3,4,8,9,10,11-hexahydro-1H-pyrido[4',3':3,4]pyrazolo[1,5-a][1,4]diazepine-2(7H)-carboxylate.

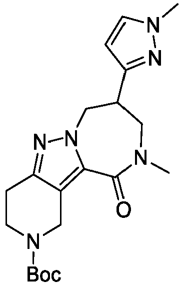

To a solution of tert-butyl 10-methyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate (Intermediate 5, 90.00 mg, 232.89 µmol) in THF (2.00 mL) was added NaH (27.95 mg, 698.67 µmol, 60% purity) at 0°C. The mixture was stirred at 0 °C for 30 min. Then CH₃I (99.17 mg, 698.67 µmol, 43.50 µL) was added. The mixture was stirred at 20°C for 2 h. The mixture was quenched by the addition of saturated NH₄Cl (20 mL) and then extracted with EA (30 mL×2). The combined organic layers were dried over anhydrous Na₂SO₄, filtered, and the filtrate concentrated under reduced pressure. The residue was purified by prep-TLC (EA/MeOH=10/1) to give the title compound (40.00 mg, 99.88 µmol, 42.89% yield, Rf=0.37 (EtOAc/MeOH=10/1)) as a colorless oil, MS (ESI): mass calcd. for C₂₀H₂₈N₆O₃ 400.2; m/z found, 401.2 [M+H]⁺. ¹H NMR (400MHz, CDCl₃) δ 7.25 (d, J=2.2 Hz, 1H), 6.07 (d, J=2.0 Hz, 1H), 5.23 (s, 1H), 4.58 - 4.50 (m, 3H), 4.48 - 4.41 (m, 1H), 3.80 (s, 3H), 3.75 - 3.44 (m, 5H), 3.06 (s, 3H), 2.69 (br, 2H), 1.41 (s, 9H). The regioisomer tert-butyl 10-methyl-8-(1-methyl-1H-pyrazol-5-yl)-11-oxo-3,4,8,9,10,11-hexahydro-1H-pyrido[4',3':3,4]pyrazolo[1,5-a][1,4]diazepine-2(7H)-carboxylate (8.00 mg, 17.48 µmol, 7.51% yield, 87.5% purity, Rf=0.30 (EtOAc/MeOH=10/1)) as a colorless oil.

### Intermediate 7a: tert-butyl 10-methyl-8-(1-methyl-1H-pyrazol-5-yl)-11-oxo-3,4,8,9,10,11-hexahydro-1H-pyrido[4',3':3,4]pyrazolo[1,5-a][1,4]diazepine-2(7H)-carboxylate

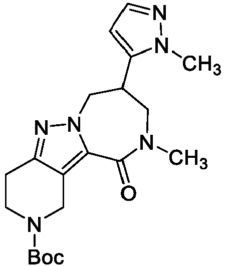

The title compound was isolated by prep-TLC from Intermediate 5 (8.00 mg, 17.48 µmol, 7.51% yield, 87.5% purity, Rf=0.30 (EtOAc/MeOH=10/1)). MS (ESI): mass calcd. for C₂₀H₂₈N₆O₃ 400.2; m/z found, 401.2 [M+H]⁺.

### Intermediate 8a: (3R,8S*)-tert-Butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate.

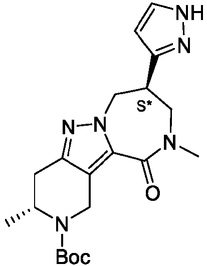

Step A. Ethyl 2-(((tert-butoxycarbonyl)(methyl)amino)methyl)acrylate. To a mixture of NaH (4.57 g, 114.36 mmol, 60% purity) in THF (80.00 mL) was added tert-butyl N-methylcarbamate (10.00 g, 76.24 mmol) dropwise with stirring at -30 °C under N₂, followed by ethyl 2-(bromomethyl)prop-2-enoate (19.13 g, 99.11 mmol) after 0.5 h. The resulting mixture was warmed to 20 °C with stirring for 16 h. The mixture was poured into ice water (200 mL) and stirred for 5 min. The aqueous phase was extracted with ethyl acetate (200 mL×3). The combined organic phases were washed with brine (200 mL), dried over anhydrous Na₂SO₄, filtered, and the filtrate concentrated under reduced pressure. The residue was purified by column chromatography (SiO₂, petroleum ether/ethyl acetate = 100/1 to 10/1) to afford a mixture of the title compound and ethyl 2-(hydroxymethyl)prop-2-enoate (21 g) as a light yellow liquid.

Step B. Ethyl 2-((methylamino)methyl)acrylate hydrochloride. To a mixture of ethyl ethyl 2-(((tert-butoxycarbonyl)(methyl)amino)methyl)acrylate and ethyl 2-(hydroxymethyl)prop-2-enoate (11.5 g, Step A) was added HCl/dioxane (4 M, 30.00 mL) with stirring at 20 °C for 2 h. The mixture was concentrated, and the residue was dissolved in water (100 mL). The resulting mixture was adjusted to pH 3 by the addition of HCl (1N), and the mixture was washed with DCM (100 mL×3). The resulting aqueous phase was concentrated to give the title compound (5.50 g, 30.62 mmol, 64.77% yield, HCl salt) as a colorless liquid.

Step C. (R)-tert-Butyl 3-((2-(ethoxycarbonyl)allyl)(methyl)carbamoyl)-6-methyl-6,7-dihydro-2H-pyrazolo[4,3-c]pyridine-5(4H)-carboxylate. To a solution of ethyl 2-((methylamino)methyl)-acrylate hydrochloride (5.50 g, 30.62 mmol) and (R)-5-(tert-butoxycarbonyl)-6-methyl-4,5,6,7-tetrahydro-2H-pyrazolo[4,3-c]pyridine-3-carboxylic acid (5.20 g, 18.48 mmol) in THF (80.00 mL) was added TEA (22.44 g, 221.76 mmol, 30.74 mL) and T₃P (58.82 g, 92.42 mmol, 54.97 mL, 50% purity) under N₂. The mixture was stirred at 30 °C for 32 h under a N₂ atmosphere. Additional T₃P (23.52 g, 36.96 mmol, 21.98 mL, 50% purity) and TEA (14.96 g, 147.84 mmol, 20.49 mL) were added into the mixture at 30 °C with stirring for another 16 h. The mixture was diluted with 80 mL of water and extracted with EtOAc (50 mL×4). The organic phase was washed sequentially with HCl (1N, 50 mL×2), saturated NaHCO₃ and brine (50 mL), then dried over anhydrous Na₂SO₄, filtered and the filtrated concentrated under reduced pressure. The residue was purified by flash column chromatography over silica gel (0-50% ethyl acetate/petroleum ether gradient) to obtain the title compound (5.52 g, 13.58 mmol, 73.49% yield) as a white solid. MS (ESI): mass calcd. for C₂₀H₃₀N₄O₅, 406.2; m/z found, 407.2 [M+H]⁺.

Step D. (3R)-2-tert-Butyl 8-ethyl 3,10-dimethyl-11-oxo-3,4,8,9,10,11-hexahydro-1H-pyrido-[4',3':3,4]pyrazolo[1,5-a][1,4]diazepine-2,8(7H)-dicarboxylate. To a solution of DBU (1.03 g, 6.79 mmol, 1.02 mL) in MeCN (600.00 mL) was added a solution of (R)-tert-butyl 3-((2-(ethoxycarbonyl)allyl)(methyl)carbamoyl)-6-methyl-6,7-dihydro-2H-pyrazolo[4,3-c]pyridine-5(4H)-carboxylate (5.52 g, 13.58 mmol) in MeCN (150.00 mL) dropwise with stirring at 50 °C for 16 h. The mixture was concentrated under reduced pressure and the resulting residue was dissolved in 100 mL of EtOAc. Then the resulting mixture was washed with HCl (1N, 100 mL) and brine (100 mL×1), dried over anhydrous Na₂SO₄, filtered and the filtrate concentrated under reduced pressure. The residue was purified via flash column chromatography over silica gel (50-100% ethyl acetate/petroleum ether gradient) to obtain the title compound (5.20 g, 12.79 mmol, 94.21% yield) as a colorless oil.

Step E. (3R)-2-(tert-Butoxycarbonyl)-3,10-dimethyl-11-oxo-2,3,4,7,8,9,10,11-octahydro-1H-pyrido[4',3':3,4]pyrazolo[1,5-a][1,4]diazepine-8-carboxylic acid. To a solution of (3R)-2-tert-butyl 8-ethyl 3,10-dimethyl-11-oxo-3,4,8,9,10,11-hexahydro-1H-pyrido[4',3':3,4]pyrazolo[1,5-a][1,4]diazepine-2,8(7H)-dicarboxylate (450.00 mg, 1.11 mmol) in THF (10.00 mL) and water (1.00 mL) was added NaOH (88.57 mg, 2.21 mmol), then the mixture was heated to 50 °C for 16 h. The mixture was diluted with 20 mL of water and the mixture pH was adjusted from 12 to 5 by the addition of HCl (1 N). The resulting mixture was extracted with EtOAc (20 mL×5). Then the combined organic phases were washed with brine (30 mL×1), dried over anhydrous Na₂SO₄, filtered, and the filtrate concentrated under reduced pressure to obtain the title compound (390.00 mg, 1.03 mmol, 92.85% yield) as a white solid.

Step F. (3R)-tert-Butyl 8-(methoxy(methyl)carbamoyl)-3,10-dimethyl-11-oxo-3,4,8,9,10,11-hexahydro-1H-pyrido[4',3':3,4]pyrazolo[1,5-a][1,4]diazepine-2(7H)-carboxylate. To a solution of (3R)-2-(tert-butoxycarbonyl)-3,10-dimethyl-11-oxo-2,3,4,7,8,9,10,11-octahydro-1H-pyrido-[4',3':3,4]pyrazolo[1,5-a][1,4]diazepine-8-carboxylic acid (390.00 mg, 1.03 mmol) and N-methoxymethanamine (130.61 mg, 1.34 mmol, HCl) in DMF (10.00 mL) was added PyBOP (589.60 mg, 1.13 mmol), HOBt (153.09 mg, 1.13 mmol) and DIEA (798.70 mg, 6.18 mmol, 1.08 mL) at 20 °C. The mixture was stirred at 20 °C for 3 h. The mixture was diluted with 50 mL of water and extracted with EtOAc (30 mL×4). The organic phases were washed with HCl (1N, 30 mL), saturated NaHCO₃ and brine (50 mL), dried over anhydrous Na₂SO₄, filtered and the filtrate concentrated under reduced pressure. The residue was purified by column chromatography (SiO₂, petroleum ether/ethyl acetate=5/1 to 1/5) to give the title compound (290.00 mg, 688.04 µmol, 66.80% yield) as a colorless oil.

Step G. (3R)-tert-Butyl 8-acetyl-3,10-dimethyl-11-oxo-3,4,8,9,10,11-hexahydro-1H-pyrido-[4',3':3,4]pyrazolo[1,5-a][1,4]diazepine-2(7H)-carboxylate. To a solution of (3R)-tert-butyl 8-(methoxy(methyl)carbamoyl)-3,10-dimethyl-11-oxo-3,4,8,9,10,11-hexahydro-1H-pyrido-[4',3':3,4]pyrazolo[1,5-a][1,4]diazepine-2(7H)-carboxylate (290.00 mg, 688.04 µmol) in THF (6.00 mL) was added dropwise MeMgBr (3 M, 1.15 mL) at - 78 °C with stirring, then the mixture was stirred at 0 °C for 2 h. The mixture was poured into HCl (1N, 10 mL) at 0 °C, then extracted with EtOAc (20 mL×3), and the combined organic phases were washed sequentially with saturated NaHCO₃ (20 mL) and brine (20 mL), then dried over anhydrous Na₂SO₄, filtered and the filtrate concentrated under reduced pressure to afford the title compound (260.00 mg, crude) as a white solid, used directly in the next step without purification. MS (ESI): mass calcd. for C₁₉H₂₈N₄O₄, 376.2; m/z found, 377.3 [M+H]⁺.

Step H. (3R)-tert-Butyl 8-(3-(dimethylamino)acryloyl)-3,10-dimethyl-11-oxo-3,4,8,9,10,11-hexahydro-1H-pyrido[4',3':3,4]pyrazolo[1,5-a][1,4]diazepine-2(7H)-carboxylate. (3R)-tert-Butyl 8-acetyl-3,10-dimethyl-11-oxo-3,4,8,9,10,11-hexahydro-1H-pyrido[4',3':3,4]pyrazolo[1,5-a][1,4]diazepine-2(7H)-carboxylate (260.00 mg, crude) was dissolved in N,N-dimethylformamide dimethyl acetal (DMF-DMA) (8.00 mL), and the resulting mixture was heated to 70 °C with stirring for 16 h. The mixture was heated to 80 °C with stirring for 16 h, then concentrated under reduced pressure. The residue was dissolved into 20 mL of EtOAc. The resulting organic phase was washed with water (10 mL) and brine (20 mL), dried over anhydrous Na₂SO₄, filtered and the filtrate concentrated under reduced pressure to obtain the title compound (300.00 mg, crude) as a yellow oil, which was directly used in the next step without purification. MS (ESI): mass calcd. for C₂₂H₃₃N₅O₄, 431.2; m/z found,432.2 [M+H]⁺.

Step I. (3R)-tert-Butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate. To a solution of (3R)-tert-butyl 8-(3-(dimethylamino)acryloyl)-3,10-dimethyl-11-oxo-3,4,8,9,10,11-hexahydro-1H-pyrido[4',3':3,4]pyrazolo[1,5-a][1,4]diazepine-2(7H)-carboxylate (300.00 mg, crude) in EtOH (6.00 mL) was added NH₂NH₂·H₂O (69.60 mg, 1.39 mmol, 67.57 µL), then the mixture was heated to 80 °C with stirring for 2 h. The mixture was concentrated under reduced pressure, and the resultant residue was purified by prep-TLC (EtOAc/MeOH=20/1) to obtain the title compound (200.00 mg, 494.42 µmol, 71.12% yield, 99% purity) as a colorless oil.

Step J. (3R,8S*)-tert-Butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate.(3R)-tert-Butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate (200.00 mg, 499.41 µmol) was separated by SFC (column: AD(250mm*30mm,10um); mobile phase: [0.1% NH₃•H₂O IPA]; B%: 25%-25%,1.6min; 50min) to obtain the title compound (90.00 mg, 224.74 µmol, Peak 1 on SFC (AD-3S_3_5_40_3ML Column: Chiralpak AD-3 100×4.6mm I.D., 3um Mobile phase: methanol (0.05% DEA) in CO₂ from 5% to 40% Flow rate: 3mL/min Wavelength: 220nm), retention time: 2.178 min) as a white solid and (3R,8R*)-tert-butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate (80.00 mg, 199.77 µmol, Peak 2 on SFC, retention time: 2.328 min) as a white solid.

### Intermediate 9a: (3R,8R*)-tert-Butyl 3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate.

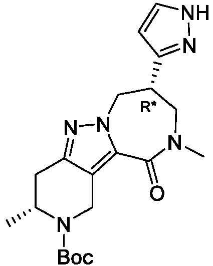

The title compound was separated by SFC from (3R)-tert-butyl 3,10-dimethyl-11-oxo-8-(1H-pyrazol-3-yl)-3,4,8,9, 10,11-hexahydro-1H-pyrido[4',3':3,4]pyrazolo[1,5-a][1,4]diazepine-2(7H)-carboxylate (product from Intermediate 9, Step I) in a manner analogous to Intermediate 7, Step J: (80.00 mg, 199.77 µmol, Peak 2 on SFC (AD-3S_3_5_40_3ML Column: Chiralpak AD-3 100×4.6mm I.D., 3um Mobile phase: methanol (0.05% DEA) in CO₂ from 5% to 40%; Flow rate: 3mL/min Wavelength: 220nm), retention time: 2.328 min). MS (ESI): mass calcd. for C₂₀H₂₇N₅O₄, 401.2; m/z found, 402.3 [M+H]⁺.

### Intermediate 10a: (3R,8S*)-tert-Butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate.

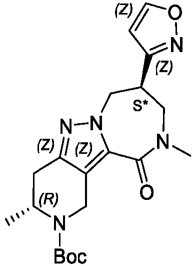

Step A. (3R)-2-(tert-Butoxycarbonyl)-3,10-dimethyl-11-oxo-2,3,4,7,8,9,10,11-octahydro-1H-pyrido[4',3':3,4]pyrazolo[1,5-a][1,4]diazepine-8-carboxylic acid. To a solution of (3R)-2-tert-butyl 8-ethyl 3,10-dimethyl-11-oxo-3,4,8,9,10,11-hexahydro-1H-pyrido[4',3':3,4]pyrazolo[1,5-a][1,4]diazepine-2,8(7H)-dicarboxylate (Intermediate 8, Step D; 2.50 g, 6.15 mmol) in THF (25.00 mL) and water (5.00 mL) was added NaOH (369.00 mg, 9.23 mmol). The mixture was stirred at 40 °C for 16 h, then diluted with water (30 mL) and adjusted to pH 5 by the addition of HCl (1N). The resulting mixture was extracted with ethyl acetate (50 mL×3), the combined organic phases washed with brine (40 mL), dried over anhydrous Na₂SO₄, filtered and the filtrate concentrated under reduced pressure to afford the title compound (2.00 g, 5.29 mmol, 85.94% yield) as a white solid. MS (ESI): mass calcd. for C₁₈H₂₆N₄O₅, 378.2; m/z found, 379.2 [M+H]⁺.

Step B. (3R)-tert-Butyl 8-(methoxy(methyl)carbamoyl)-3,10-dimethyl-11-oxo-3,4,8,9,10,11-hexahydro-1H-pyrido[4',3':3,4]pyrazolo[1,5-a][1,4]diazepine-2(7H)-carboxylate. To a solution of (3R)-2-(tert-butoxycarbonyl)-3,10-dimethyl-11-oxo-2,3,4,7,8,9,10,11-octahydro-1H-pyrido-[4',3':3,4]pyrazolo[1,5-a][1,4]diazepine-8-carboxylic acid (2.00 g, 5.28 mmol) and N-methoxymethanamine (669.51 mg, 6.86 mmol, HCl) in DMF (30.00 mL) was added PyBOP (3.02 g, 5.81 mmol), HOBt (784.78 mg, 5.81 mmol) and DIEA (4.09 g, 31.68 mmol, 5.52 mL). Then the mixture was stirred at 20 °C for 3 h. The mixture was diluted with water (50 mL) and extracted with ethyl acetate (100 mL×2). The combined organic layers were washed sequentially with water (100 mL×2), 1N HCl (50 mL), and sat.aq NaHCO₃ (50 mL). The organic portion was then dried over Na₂SO₄, filtered the filtrate concentrated under reduced pressure. The residue was purified by column chromatography over silica gel (petroleum ether/EtOAc=50%~100%) to obtain the title compound (2.06 g, 4.89 mmol, 92.56% yield) as a white solid. MS (ESI): mass calcd. for C₂₀H₃₁N₅O₅, 421.2; m/z found, 422.3 [M+H]⁺.

Step C. (3R)-tert-Butyl 8-acetyl-3,10-dimethyl-11-oxo-3,4,8,9,10,11-hexahydro-1H-pyrido[4',3':3,4]pyrazolo[1,5-a][1,4]diazepine-2(7H)-carboxylate. To a solution of (3R)-tert-butyl 8-(methoxy(methyl)carbamoyl)-3,10-dimethyl-11-oxo-3,4,8,9,10,11-hexahydro-1H-pyrido[4',3':3,4]pyrazolo[1,5-a][1,4]diazepine-2(7H)-carboxylate (2.06 g, 4.89 mmol) in THF (30 mL) was added MeMgBr (3 M, 4.89 mL) dropwise at -30 °C under N₂. The mixture was warmed to 0 °C with stirring for 1 h. The mixture was poured into 1N HCl (100 mL) slowly and extracted with ethyl acetate (80 mL×3). The combined organic layers were dried over Na₂SO₄, filtered, and the filtrate concentrated under reduced pressure to afford the title compound (1.67 g, crude) as a white solid. MS (ESI): mass calcd. for C₁₉H₂₈N₄O₄, 376.2; m/z found, 377.1 [M+H]⁺.

Step D. (3R)-tert-Butyl 8-((E)-3-(dimethylamino)acryloyl)-3,10-dimethyl-11-oxo-3,4,8,9,10,11-hexahydro-1H-pyrido[4',3':3,4]pyrazolo[1,5-a][1,4]diazepine-2(7H)-carboxylate. (3R)-tert-Butyl 8-acetyl-3,10-dimethyl-11-oxo-3,4,8,9,10,11-hexahydro-1H-pyrido[4',3':3,4]pyrazolo[1,5-a][1,4]diazepine-2(7H)-carboxylate (400 mg, 1.06 mmol) was dissolved in DMFDMA (1.79 g, 15.06 mmol, 2 mL). The mixture was stirred at 75 °C for 24 h. The mixture was extracted with ethyl acetate (40 mL × 2) and washed with water (20 mL). The combined organic layers were washed with water (30 mL×2). The organic portion was dried over anhydrous Na₂SO₄, filtered and the filtrate concentrated under reduced pressure to afford the title compound (400 mg, 926.94 µmol, 87.24% yield) as a brown solid.

Step E. (3R)-tert-Butyl 8-₍isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate.₂OH•HCl (193.24 mg, 2.78 mmol). The mixture was stirred at 115 °C for 16 h, then diluted with water (30 mL) and extracted with EtOAc (30 mL×3). The combined organic layers were washed with 1N HCl (20 mL×2), dried over Na₂SO₄, filtered and the filtrate concentrated under reduced pressure. The residue was purified by prep-TLC (EtOAc=1) to afford (3R)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (87 mg, 166.87 µmol, 36.00% yield, 77% purity) as a brown oil. MS (ESI): mass calcd. for C₂₀H₂₇N₅O₄, 401.2; m/z found,402.3 [M+H]⁺.

Step F. (3R,8S*)-tert-Butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate. (3R)-tert-Butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (92 mg) was purified by HPLC (column: Phenomenex Synergi C18 150*30mm*4um;mobile phase: [water (0.225%FA)-ACN]; B%: 33%-63%,10.5min) to afford the title compound (60 mg), which was separated by SFC (column: AD (250 mm*30 mm, 10 µm); mobile phase: [0.1% NH₃•H₂O MEOH]; B%: 25%-25%, 3.3 min; 70 min) to afford (3R,8S*)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (Peak 1 on SFC (AD-3S_3_5_40_3ML Column: Chiralpak AD-3 100×4.6mm I.D., 3um Mobile phase: methanol(0.05% DEA) in CO₂ from 5% to 40% Flow rate: 3mL/min Wavelength: 220nm), retention time=1.662 min, 17 mg, 42.35 µmol, 18.48% yield) as a white solid and diastereomer (3R,8R*)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (Peak 2, retention time =1.858 min, 17 mg, 42.35 µmol, 18.48% yield) as a white solid. MS (ESI): mass calcd. for C₂₀H₂₇N₅O₄, 401.2; m/z found, 402.3 [M+H]⁺.

### Intermediate 11a: (3R,8R*)-tert-Butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate

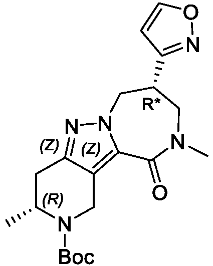

The title compound was separated by SFC from (3R)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (product from Intermediate 4, Step E) in a manner analogous to Intermediate 10, Step F. (17 mg, 42.35 µmol, Peak 2 on SFC (AD-3S_3_5_40_3ML Column: Chiralpak AD-3 100×4.6mm I.D., 3um Mobile phase: methanol (0.05% DEA) in CO₂ from 5% to 40% Flow rate: 3mL/min Wavelength: 220nm), retention time =1.858 min). MS (ESI): mass calcd. for C₂₀H₂₇N₅O₄, 401.2; m/z found, 402.3 [M+H]⁺.

### Intermediate 12a:(3R,8S*)-tert-Butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate.

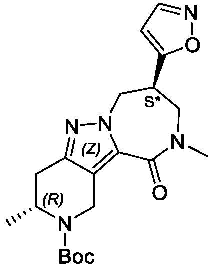

### Step A. (3R)-tert-Butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate.

To a solution of (3R)-tert-butyl 8-((E)-3-(dimethylamino)acryloyl)-3,10-dimethyl-11-oxo-3,4,8,9,10,11-hexahydro-1H-pyrido[4',3':3,4]pyrazolo[1,5-a] [1,4]diazepine-2(7H)-carboxylate (Intermediate 10, Step D; 200 mg, 463.47 µmol) in HOAc (3 mL) and water (6 mL) was added NH₂OH•HCl (193.24 mg, 2.78 mmol). The mixture was stirred at 10 °C for 16 h, and then the heating was continued for another 48 h at 70 °C. At that time, the mixture was concentrated under reduced pressure, and the resultant residue was diluted with THF (3 mL) and adjusted pH to 9~10 with aq.Na₂CO₃. Boc₂O (303.46 mg, 1.39 mmol, 319.43 µL) was added, and the mixture was stirred at 10 °C for 16 h. The mixture was diluted with water (50 mL) and extracted with EtOAc (30 mL×3). The combined organic layers were dried over Na₂SO₄, filtered and the filtrate concentrated under reduced pressure. The residue was purified by prep-TLC (EtOAc/MeOH=10/1) to afford a mixture (140 mg, 327.48 µmol, 70.66% yield, 93.8% purity) of the title compound (3R)-tert-butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate and (3R)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate as a colorless oil. MS (ESI): mass calcd. for C₂₀H₂₇N₅O₄, 401.2; m/z found, 402.3 [M+H]⁺.

### Step B. (3R,8S*)-tert-Butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate.

The mixture of (3R)-tert-butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate and (3R)-tert-butyl 8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxylate (140 mg) was separated via SFC (column: AD (250 mm*30 mm, 5 µm); mobile phase: [0.1% NH₃•H₂O IPA]; 2.6min; 120 min) to afford title compound (3R,8S*)-tert-butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate (Peak 1 on SFC (AD-3S_5_5_40_3ML Column: Chiralpak AD-3 100×4.6mm I.D., 3um Mobile phase: ethanol (0.05% DEA) in CO₂ from 5% to 40% Flow rate: 3mL/min Wavelength: 220nm), retention time: 1.753 min, 36 mg, 89.67 µmol, 27.41% yield) as a colorless oil and a mixture (Peak 2 and Peak 3, retention time: 1.866 and 1.907 min, 50 mg, including intermediate 7). MS (ESI): mass calcd. for C₂₀H₂₇N₅O₄, 401.2; m/z found, 402.3 [M+H]⁺.

### Intermediate 13a: (3R,8R*)-tert-butyl 8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxylate.

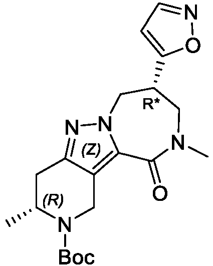

A mixture (Peak 2 and Peak 3, retention time: 1.866 and 1.907 min, 50 mg) from product of Intermediate 12, Step B, was further purified by SFC (column: OJ (250 mm*30 mm, 5 µm); mobile phase: [0.1% NH₃•H₂O IPA]; B%: 20%-20%, 2.2 min; 30 min) to afford the title compound (Peak 1 on SFC (OJ-3S_4_5_40_3ML Column: Chiralcel OJ-3 100×4.6mm I.D., 3um Mobile phase: iso-propanol (0.05% DEA) in CO₂ from 5% to 40% Flow rate: 3mL/min Wavelength: 220nm"), retention time=1.233 min, 11 mg, 27.40 µmol, 8.38% yield). MS(ESI): mass calcd. for C₂₀H₂₇N₅O₄, 401.2; m/z found, 402.3 [M+H]⁺.

### Intermediate 14a. tert-Butyl 10-methyl-8-methylene-11-oxo-3,4,8,9,10,11-hexahydro-1H-pyrido[4',3':3,4]pyrazolo[1,5-a][1,4]diazepine-2(7H)-carboxylate.

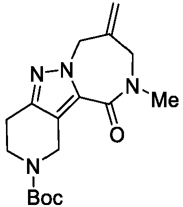

### Method A

Step A. 5-Methylene-1,3,2-dioxathiane 2-oxide. To a solution of 2-methylenepropane-1,3-diol (5.00 g, 56.75 mmol, 4.63 mL, 1.00 *eq)* in CCl₄ (50.00 mL) was added a solution of SOCl₂ (10.13 g, 85.13 mmol, 6.18 mL, 1.50 *eq)* in CCl₄ (10.00 mL) at 0 °C under N₂, and the mixture was stirred at 0 °C for 45 mins. The mixture was concentrated under reduced pressure to afford 5-methylene-1,3,2-dioxathiane 2-oxide (6.90 g, 51.43 mmol, 90.63% yield) as yellow oil, which was used directly for the next step. ¹H NMR (400 MHz, CDCl₃) δ 5.36 - 5.39 (m, 2 H), 5.16 (s, 2 H), 4.22 - 4.28 (m, 2H).

Step B. 2-((Methylamino)methyl)prop-2-en-1-ol. A solution of 5-methylene-1,3,2-dioxathiane 2-oxide (1.00 g, 7.45 mmol, 1.00 *eq)* and methanamine (2 M, 11.18 mL, 3.00 *eq)* in THF (2.00 mL) was heated to 70 °C for 16 h. The mixture was filtered and the filtrate was concentrated in vacuo to afford the title compound (750.00 mg, 7.41 mmol, 99.46% yield) as yellow oil, which was used directly for the next step.

Step C. tert-Butyl (2-(hydroxymethyl)allyl)(methyl)carbamate. To a solution of 2-(methylaminomethyl)prop-2-en-1-ol (750.00 mg, 7.41 mmol, 1.00 *eq)* in dioxane (5.00 mL)/H₂O (5.00 mL) was added Boc₂O (1.94 g, 8.89 mmol, 2.04 mL, 1.20 *eq)* and NaHCO₃ (622.40 mg, 7.41 mmol, , 1.00 *eq).* The mixture was stirred at 30 °C for 16 h. The mixture with was diluted with EA (50 mL) and washed with brine (50 mL). The organic phase was dried over Na₂SO₄, filtered and concentrated to give yellow oil, which was purified by silica gel column to afford the title compound (710.00 mg, 3.53 mmol, 47.61% yield) as yellow oil. ¹H NMR (400 MHz, CD₃OD) δ 5.10 (s, 1 H), 4.97 (s, 1 H), 3.91 - 4.10 (m, 4 H), 2.81 (s, 3 H), 1.50 (s, 9 H).

Step D. 2-((Methylamino)methyl)prop-2-en-1-ol hydrochloride. To a solution of tert-butyl N-[2-(hydroxymethyl)allyl]-N-methyl-carbamate (710.00 mg, 3.53 mmol, 1.00 *eq)* in dioxane (3.00 mL) was added HCl/dioxane (4 M, 5.00 mL, 5.67 *eq)* and the mixture was stirred at 15 °C for 1 h. The mixture was concentrated in vacuo to afford the title compound (480.00 mg, 3.49 mmol, 98.81% yield, HCl) as yellow oil, which was used directly for the next step. ¹H NMR (400 MHz, CD₃OD) δ 5.46 (s, 1 H), 5.32 (s, 1 H), 4.20 (s, 2 H), 3.71 (s, 2 H), 2.73 (s, 3 H).

Step E. tert-Butyl 3-((2-(hydroxymethyl)allyl)(methyl)carbamoyl)-6,7-dihydro-2H-pyrazolo[4,3-c]pyridine-5(4H)-carboxylate. A mixture of 5-tert-butoxycarbonyl-1,4,6,7- tetrahydropyrazolo [4,3-c]pyridine-3- carboxylic acid (550.00 mg, 2.06 mmol, 1.00 *eq),* DIPEA (798.70 mg, 6.18 mmol, 1.08 mL, 3.00 *eq),* HATU (939.93 mg, 2.47 mmol, 1.20 *eq)* and 2-(methylaminomethyl)prop-2-en-1-ol (425.21 mg, 3.09 mmol, 1.50 *eq,* HCl) in DMF (6.00 mL) was heated to 80 °C for 16 h. The mixture was diluted with EtOAc (80 mL) and washed with brine (80 mL *3). The organic phase was dried over Na₂SO₄, filtered and concentrated under reduced pressure to give yellow oil. The yellow oil was purified by silica gel column to afford the title compound (390.00 mg, 1.11 mmol, 54.03% yield) as yellow solid. LCMS: 351 [M+1].

Step F. tert-Butyl 10-methyl-8-methylene-11-oxo-3,4,8,9,10,11-hexahydro-1H-pyrido[4',3':3,4]-pyrazolo[1,5-a][1,4]diazepine-2(7H)-carboxylate. To a solution of tert-butyl 3-[2-(hydroxymethyl)allyl-methyl-carbamoyl]-2,4,6,7- tetrahydropyrazolo[4,3-c]pyridine-5-carboxylate (200.00 mg, 570.76 µmol, 1.00 *eq)* and triphenylphosphane (194.62 mg, 741.99 µmol, 1.30 *eq)* in THF (3.00 mL) was added DIAD (150.04 mg, 741.99 µmol, 144.27 mL, 1.30 *eq)* and the mixture was stirred at 30 °C for 4 h. The mixture was diluted with EtOAc (50 mL) and washed with HCl (1 M, 50 mL). The organic phase was dried over Na₂SO₄, filtered and concentrated in vacuo to give oil. The oil was purified by silica gel column to afford the title compound as impure product (320.00 mg, crude, containing Ph₃PO) as yellow oil. LCMS: 355 [M+23].

### Method B

Step A. 5-tert-Butyl 3-ethyl 2-(2-(chloromethyl)allyl)-6,7-dihydro-2H-pyrazolo[4,3-c]pyridine-3,5(4H)-dicarboxylate. To a solution of 3-chloro-2-(chloromethyl)prop-1-ene (7.62 g, 60.95 mmol, 7.05 mL, 3.00 *eq)* in DMF (100.00 mL) was added 5-tert-butyl 3-ethyl 2,4,6,7-tetrahydropyrazolo[4,3-c]pyridine-3,5-dicarboxylate (6.00 g, 20.32 mmol, 1.00 *eq)* and K₂CO₃ (3.65 g, 26.41 mmol, 1.30 *eq).* The mixture was stirred at 25 °C for 6 h and then heated to 75 °C for 16 h. The mixture was diluted with EtOAc (80 mL), washed with HCl (1M, 80 mL) and brine (80 mL*2). The organic phase was dried over Na₂SO₄, filtered and concentrated in vacuo to give yellow oil. The oil was purified by silica gel column to afford the title compound (2.90 g, 7.55 mmol, 37.18% yield) as colorless oil.

Step B. tert-Butyl 10-methyl-8-methylene-11-oxo-3,4,8,9,10,11-hexahydro-1H-pyrido[4',3':3,4]-pyrazolo[1,5-a][1,4]diazepine-2(7H)-carboxylate. A solution of 5-tert-butyl 3-ethyl 2-(2-(chloromethyl)allyl)-6,7-dihydro-2H-pyrazolo[4,3-c]pyridine-3,5(4H)-dicarboxylate (1.00 g, 2.61 mmol, 1.00 *eq)* and methanamine (7.5 M, 40.00 mL, 33% purity, 114.94 *eq)* in EtOH (30.00 mL) was heated to 80 °C in sealed tube for 16 h. The mixture was concentrated in vacuo to give yellow oil. The yellow oil was purified by silica gel column to afford the title compound (560.00 mg, 1.68 mmol, 64.37% yield) as yellow oil. LCMS: 333 [M+1].

### Intermediate 15a: tert-Butyl 8-hydroxy-10-methyl-11-oxo-8-phenyl-3,4,8,9,10,11-hexahydro-1H-pyrido[4',3':3,4]pyrazolo[1,5-a][1,4]diazepine-2(7H)-carboxylate.

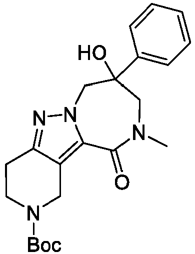

Step A. tert-Butyl 10-methyl-8,11-dioxo-3,4,8,9,10,11-hexahydro-1H-pyrido[4',3':3,4]pyrazolo-[1,5-a][1,4]diazepine-2(7H)-carboxylate. To a solution of tert-butyl 10-methyl-8-methylene-11-oxo-3,4,8,9,10,11-hexahydro-1H-pyrido[4',3':3,4]pyrazolo[1,5-a][1,4]diazepine-2(7H)-carboxylate (Intermediate 14a, 1.4 g, 4.21 mmol) in THF (50 mL) was added OsO₄ (107.08 mg, 421.18 µmol, 21.85 µL) and a solution of NaIO₄ (2.70 g, 12.64 mmol, 700.16 µL) in H₂O (25 mL) at 0 °C, the mixture was stirred at 15 °C for 16 h. The reaction mixture was quenched with saturated aqueous NaHCO₃ (30 mL) at 0 °C, then diluted with water (50 mL) and extracted with ethyl acetate (50 mL×2). The combined organic layers were washed with brine (50 mL), dried over Na₂SO₄, filtered and the filtrate concentrated under reduced pressure. The residue was purified by column chromatography (SiO₂) to afford the title compound (0.9 g, 2.69 mmol, 63.91% yield) as a yellow solid. MS (ESI): mass calcd. for C₁₆H₂₂N₄O₄, 334.4; m/z found, 279 [M+H-56]⁺. ¹H NMR (400 MHz, CDCl₃) δ 5.01(s, 2 H), 4.66(s, 2 H), 4.04(s, 2 H), 3.73(t, J=5.1 Hz, 2 H), 3.20(s, 3 H), 2.76(s, 2 H), 1.49(s, 9 H).

Step B. tert-Butyl 8-hydroxy-10-methyl-11-oxo-8-phenyl-3,4,8,9,10,11-hexahydro-1H-pyrido[4',3':3,4]pyrazolo[1,5-a][1,4]diazepine-2(7H)-carboxylate.To a solution of phenylmagnesium bromide (3 M, 2.09 mL) in THF (2 mL) was added a solution of tert-butyl 10-methyl-8,11-dioxo-3,4,8,9,10,11-hexahydro-1H-pyrido[4',3':3,4]pyrazolo[1,5-a][1,4]diazepine-2(7H)-carboxylate(0.7 g, 2.09 mmol) in THF (10 mL) at -30 °C, the mixture was stirred at 15 °C for 2 h under a N₂ atmosphere. The reaction mixture was quenched with saturated NH₄Cl (5mL) solution and extracted with EtOAc (3 mL×2). The combined organic layers were washed with brine (10 mL) and dried over Na₂SO₄, filtered and the filtrate concentrated under reduced pressure. The residue was purified by prep-TLC (SiO₂) to afford title compound (0.320 g, 721.49 µmol, 34.46% yield, 93% purity) as a white solid. MS (ESI): mass calcd. for C₂₂H₂₈N₄O₄, 412.5; m/z found, 413.1 [M+H]⁺. ¹H NMR (400 MHz, CDCl₃) δ 7.60 (d, *J* = 7.5 Hz, 2H), 7.47 - 7.31 (m, 3 H), 4.90 (d, *J* =14.4 Hz, 1 H), 4.63 (s, 2H), 4.46 (d, *J* = 14.4 Hz, 1 H), 3.82 (d, J=5.5, 13.4 Hz, 1 H), 3.65 - 3.36 (m, 3 H), 3.19 (s, 3 H), 2.79 - 2.65 (m, 2 H), 1.49 (s, 9 H).

### Intermediate 16a: tert-Butyl 8-fluoro-10-methyl-11-oxo-8-phenyl-3,4,8,9,10,11-hexahydro-1H-pyrido[4',3":3,4]pyrazolo[1,5-a][1,4]diazepine-2(7H)-carboxylate.

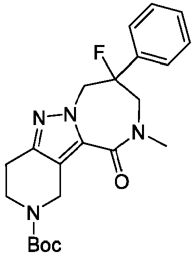

To a solution of tert-butyl 8-hydroxy-10-methyl-11-oxo-8-phenyl-3,4,8,9,10,11-hexahydro-1H-pyrido[4',3':3,4]pyrazolo[1,5-a][1,4]diazepine-2(7H)-carboxylate (Intermediate 15a, 0.08 g, 180.37 µmol) in DCM (1 mL) was added a solution of DAST (0.0625 g, 387.74 µmol, 51.23 µL) in DCM (1 mL) at 0 °C under a N₂ atmosphere. The mixture was stirred at 15 °C for 2 h. The reaction mixture was quenched with H₂O (3 mL) at 0 °C, then extracted with EtOAc (5 mL×2). The combined organic layers were washed with brine (5 mL), dried over Na₂SO₄, filtered and the filtrate concentrated under reduced pressure. The residue was purified via prep-TLC (SiO₂) to afford the title compound (0.065 g, 156.83 µmol, 86.95% yield) as a white solid. MS (ESI): mass calcd. for C₂₂H₂₇N₄O₃F, 414.5.4. ¹H NMR (400 MHz, CDCl₃) δ 7.51 - 7.33 (m, 5 H), 4.90 (m, 1 H), 4.73 - 4.52 (m, 3 H), 3.98 - 3.83 (m, 1 H), 3.77 - 3.48 (m, 3 H), 3.23 (s, 3 H), 2.80 (s, 2 H), 1.54 - 1.39 (m, 9H).

### Example 1a: N-(3-Cyano-4-fluorophenyl)-10-methyl-11-oxo-8-(1H- 1,2,4-triazol-3-yl)-3,4,8,9,10,11-hexahydro-1H-pyrido[4',3':3,4]pyrazolo[1,5-a][1,4]diazepine-2(7H)-carboxamide.

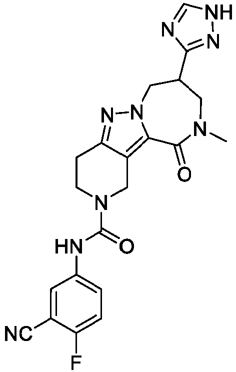

### Step A. 10-Methyl-8-(1H-1,2,4-triazol-3-yl)-3,4,7,8,9,10-hexahydro-1H-pyrido[4',3':3,4]-pyrazolo[1,5-a][1,4]diazepin-11(2H)-one.

To a solution of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2, 40.00 mg, 103.24 µmol) in DCM (3.00 mL) was added TFA (770.00 mg, 6.75 mmol, 500.00 µL). The reaction mixture was stirred at 15 °C for 30 min. The mixture was concentrated under reduced pressure to afford the title compound (40.00 mg, crude, TFA salt) as a yellow oil, which was used in the next step directly without purification.

### Step B. N-(3-Cyano-4-fluorophenyl)-10-methyl-11-oxo-8-(1H-1,2,4-triazol-3-yl)-3,4,8,9,10,11-hexahydro-1H-pyrido[4',3':3,4]pyrazolo[1,5-a][1,4]diazepine-2(7H)-carboxamide.

To a mixture of 10-methyl-8-(1H-1,2,4-triazol-3-yl)-2,3,4,7,8,9-hexahydro-1H-pyrido [2,3]pyrazolo[2,4-b][1,4]diazepin-11-one (40.00 mg, 99.67 µmol, TFA) in DCM (2.00 mL) was added TEA (40.34 mg, 398.66 µmol, 55.26 µL), followed by phenyl N-(3-cyano-4-fluorophenyl)carbamate (25.54 mg, 99.67 µmol). The reaction mixture was stirred at 15 °C for 16 h. The reaction mixture was concentrated under reduced pressure, and the resultant residue was purified by RP HPLC (Condition A) twice to afford the title compound (28.00 mg, 61.68 µmol, 61.88% yield, 99% purity) as a white solid. MS (ESI): mass calcd. for C₂₁H₂₀FN₉O₂ 449.2; m/z found, 450 [M+H]⁺. ¹H NMR (400 MHz, CDCl₃) δ 8.25 (s, 1 H), 7.78 (dd, *J* = 2.8, 5.40 Hz, 1H), 7.56 - 7.64 (m, 1H), 7.13 (t, *J* = 8.7 Hz, 1H), 6.88 (s, 1 H), 4.75 - 4.85 (m, 2H), 4.65 - 4.74 (m, 2H), 3.90 - 4.02 (m, 2H), 3.76 - 3.87 (m, 3 H), 3.15 (s, 3H), 2.87 (t, *J* = 5.7 Hz, 2 H).

### Example 2a: N-(3-Cyano-4-fluorophenyl)-10-methyl-8-(1,2,4-oxadiazol-5-yl)-11-oxo-3,4,8,9,10,11-hexahydro-1H-pyrido[4',3':3,4]pyrazolo[1,5-a][1,4]diazepine-2(7H)-carboxamide.

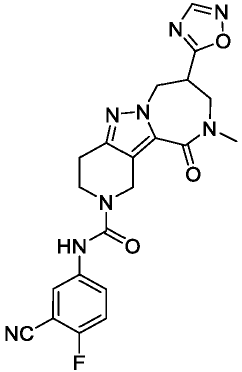

The title compound was prepared in a manner analogous to Example 1a, using tert-butyl 10-methyl-8-(1,2,4-oxadiazol-5-yl)-11-oxo-3,4,8,9,10,11 -hexahydro-1H-pyrido[4',3':3,4]-pyrazolo[1,5-a][1,4]diazepine-2(7H)-carboxylate (Intermediate 3a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A. MS (ESI): mass calcd. For C₂₁H₁₉FN₈O₃ 450.2; m/z found, 451.2 [M+H]⁺. 1H NMR (400 MHz, DMSO-d6) δ 9.02 (s, 1H), 8.43 (s, 1H), 7.93 (dd, *J* = 2.8, 5.77 Hz, 1H), 7.79 (ddd, *J* = 2.8, 4.9, 9.3 Hz, 1H), 7.42 (t, *J* = 9.2 Hz, 1H), 4.57 (d, *J* = 3.1 Hz, 2H), 4.38 - 4.45 (m, 1H), 4.28 - 4.35 (m, 1H), 3.63 - 3.80 (m, 3H), 3.58 (br dd, *J* = 5.4, 14.8 Hz, 2H), 3.02 (s, 3H), 2.91 - 3.01 (m, 2H).

### Example 3a: N-(3-Cyano-4-fluorophenyl)-10-methyl-11-oxo-8-(1H-tetrazol-5-yl)-3,4,8,9,10,11-hexahydro-1H-pyrido[4',3':3,4]pyrazolo[1,5-a][1,4]diazepine-2(7H)-carboxamide.

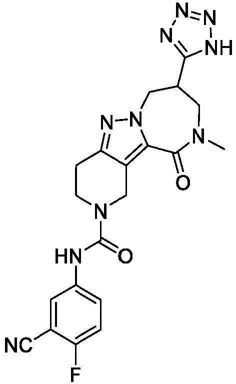

The title compound was prepared in a manner analogous to Example 1a, using tert-butyl 10-methyl-11-oxo-8-(1H-tetrazol-5-yl)-3,4,8,9,10,11-hexahydro-1H-pyrido[4',3':3,4]pyrazolo[1,5-a][1,4]diazepine-2(7H)-carboxylate (Intermediate 4a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A. MS (ESI): mass calcd. For C₂₀H₁₉FN₁₀O₂ 450.2; m/z found, 451.2 [M+H]⁺. 1H NMR (400 MHz, DMSO-d6) δ 9.05 (s, 1H), 7.93 (dd, *J* = 2.8, 5.8 Hz, 1H), 7.79 (ddd, *J* = 2.8, 4.9, 9.3 Hz, 1H), 7.42 (t, *J* = 9.2 Hz, 1H), 4.74 (dd, *J* = 7.3, 14.3 Hz, 1H), 4.61 (s, 2H), 4.53 (dd, *J* = 6.7, 14.2 Hz, 1H), 4.09 - 4.18 (m, 1H), 3.62 - 3.83 (m, 4H), 3.01 (s, 3 H), 2.67 - 2.73 (m, 2H).

### Example 4a: N-(3-Chloro-4-fluorophenyl)-10-methyl-11-oxo-8-(1H-pyrazol-3-yl)-3,4,8, 9,10,11-hexahydro-1H-pyrido[4',3':3,4]pyrazolo[1,5-a][1,4]diazepine-2(7H)-carboxamide.

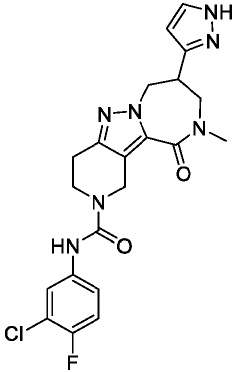

The title compound was prepared in a manner analogous to Example 1a, using tert-butyl 10-methyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxylate (Intermediate 5a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A, and phenyl (3-chloro-4-fluorophenyl)carbamate instead of phenyl (3-cyano-4-fluorophenyl)carbamate in Step B. MS (ESI): mass calcd. for C₂₁H₂₁N₇O₂ClF, 457.1; m/z found, 458.1 [M+H]+. ¹H NMR (400MHz, CDCl₃) δ 7.62 (dd, *J*= 2.6, 6.5 Hz, 1H), 7.56 (d, *J* = 2.2 Hz, 1H), 7.28 - 7.22 (m, 1H), 7.10 - 7.03 (m, 2H), 6.28 (d, *J*= 2.3 Hz, 1H), 4.89 - 4.80 (m, 1 H), 4.75 - 4.64 (m, 3H), 4.10 - 3.99 (m, 1H), 3.96 - 3.87 (m, 1H), 3.79 - 3.769 (m, 2H), 3.57 (m, 1H), 3.09 (s, 3H), 3.08 - 3.07 (m, 1H), 2.88 (br t, *J* = 5.4 Hz, 2H).

### Example 5a: N-(3-Cyano-4-fluorophenyl)-10-methyl-8-(1-methyl-1H-pyrazol-3-yl)-11-oxo-3,4,8,9,10,11-hexahydro-1H-pyrido[4',3':3,4]pyrazolo[1,5-a][1,4]diazepine-2(7H)-carboxamide

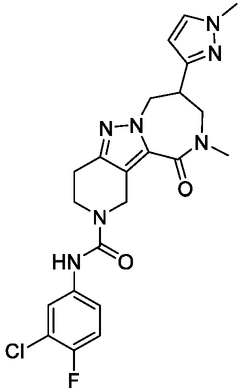

The title compound was prepared in a manner analogous to Example 1a, using tert-butyl 10-methyl-8-(1-methyl-1H-pyrazol-3-yl)-11-oxo-3,4,8,9,10,11-hexahydro-1H-pyrido[4',3':3,4]-pyrazolo[1,5-a][1,4]diazepine-2(7H)-carboxylate (Intermediate 6a) instead of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2a) in Step A. MS (ESI): mass calcd. for C₂₃H₂₃N₈O₂F, 462.4; m/z found, 463.1 [M+H]+; ¹H NMR (400MHz, CDCl₃) δ 7.80 (dd, *J* = 2.8, 5.4 Hz, 1H), 7.60 (ddd, *J* = 2.8, 4.6, 9.1 Hz, 1H), 7.35 (d, *J* = 2.2 Hz, 1H), 7.14 (t, *J* = 8.7 Hz, 1H), 6.87 (s, 1H), 6.17 (d, *J* = 2.3 Hz, 1H), 5.32 (s, 1H), 4.78 - 4.55 (m, 4H), 3.99 - 3.91 (m, 1H), 3.90 (s, 3H), 3.88 - 3.79 (m, 2H), 3.75 - 3.68 (m, 1H), 3.65 - 3.57 (m, 1H), 3.17 (s, 3H), 2.88 (t, *J* = 5.7 Hz, 2H).

### Example 6a: N-(3-Cyano-4-fluorophenyl)-10-methyl-8-(1-methyl-1H-pyrazol-5-yl)-11-oxo-3,4,8,9,10,11-hexahydro-1H-pyrido[4',3':3,4]pyrazolo[1,5-a][1,4]diazepine-2(7H)-carboxamide.

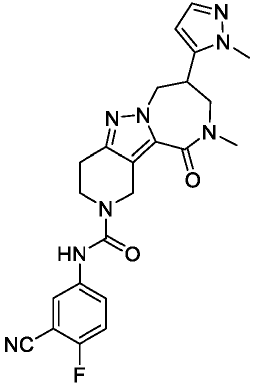

The title compound was prepared in a manner analogous to Example 1a, using tert-butyl 10-methyl-8-(1-methyl-1H-pyrazol-5-yl)-11-oxo-3,4,8,9,10,11-hexahydro-1H-pyrido[4',3':3,4]-pyrazolo[1,5-a][1,4]diazepine-2(7H)-carboxylate (Intermediate 7a) instead of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2a) in Step A. MS (ESI): mass calcd for C₂₃H₂₃N₈O₂F, 462.2; m/z found, 463.2 [M+H]⁺. ¹H NMR (400MHz, CDCl₃) δ=7.79 (dd, *J* = 2.8, 5.4 Hz, 1H), 7.61 (ddd, *J* = 2.9, 4.6, 9.1 Hz, 1H), 7.49 (d, *J* = 2.0 Hz, 1H), 7.15 (t, *J* = 8.7 Hz, 1H), 6.80 (s, 1H), 6.25 (d, *J* = 2.0 Hz, 1H), 4.73 (s, 2H), 4.67 (m, 1H), 4.51 (m, 1H), 3.94 - 3.79 (m, 6H), 3.67 (m, 1H), 3.44 (m, 1H), 3.16 (s, 3H), 2.94 - 2.84 (m, 2H).

### Example 7a: (3R,8S*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][ 1,4]diazepine-2(7H)-carboxamide.

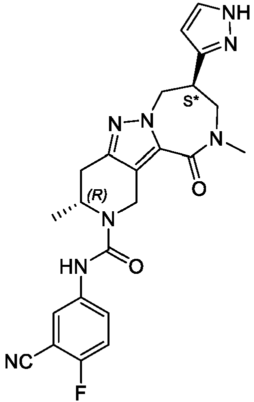

### Step A. (3R,8S*)-3,10-Dimethyl-8-(1H-pyrazol-3-yl)-3,4,7,8,9,10-hexahydro-1H-pyrido-[4',3':3,4]pyrazolo[1,5-a][1,4]diazepin-11(2H)-one.

To a solution of tert-butyl (3R,8S*)-3,10-dimethyl-11-oxo-8-(1H-pyrazol-3-yl)-1,3,4,7,8,9-hexahydropyrido[2,3]pyrazolo[2,4-b][1,4]diazepine-2-carboxylate (Intermediate 7a, 90.00 mg, 224.74 µmol) in DCM (3.00 mL) was added TFA (462.00 mg, 4.05 mmol, 300.00 µL) at 15 °C with stirring for 1 h. The mixture was concentrated under reduced pressure to afford the title compound (100.00 mg, crude, TFA) as a yellow oil, which was directly used in the next step. Step B. (3R,8S*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][ 1,4]diazepine-2(7H)-carboxamide. To a solution of (3R,8S*)-3,10-dimethyl-8-(1H-pyrazol-3-yl)-2,3,4,7,8,9-hexahydro-1H-pyrido[2,3]pyrazolo[2,4-b][1,4]diazepin-11-one (50.00 mg, 120.66 µmol TFA) and phenyl N-(3-cyano-4-fluoro-phenyl)carbamate (30.92 mg, 120.66 µmol) in DCM (3.00 mL) was added TEA (73.26 mg, 723.96 µmol, 100.36 µL) at 15 °C with stirring for 16 h. The mixture was concentrated under reduced pressure and the resultant residue was purified by RP HPLC

(Method A) to obtain the title compound (24.00 mg, 51.89 µmol, 43.01% yield) as a white solid. MS (ESI): mass calcd. for C₂₃H₂₃FN₈O₂, 462.2; m/z found, 463.2 [M+H]⁺. ¹H NMR (400MHz, CDCl₃) δ 7.80 (dd, *J* = 2.8, 5.4 Hz, 1H), 7.63 - 7.55 (m, 2H), 7.14 (t, *J =* 8.7 Hz, 1H), 6.77 (s, 1H), 6.27 (d, *J =* 2.4 Hz, 1H), 5.20 - 5.09 (m, 1H), 4.84 (d, *J =* 15.3 Hz, 1H), 4.75 - 4.60 (m, 2H), 4.51 (d, *J* = 15.2 Hz, 1H), 3.88 (m, 1H), 3.79 - 3.71 (m, 1H), 3.67 - 3.59 (m, 1H), 3.15 (s, 3H), 3.04 (dd, *J* = 6.0, 15.9 Hz, 1H), 2.71 (d, *J =* 15.9 Hz, 1H), 1.20 (d, *J =* 6.90 Hz, 3H).

### Example 8a: (3R,8S*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide.

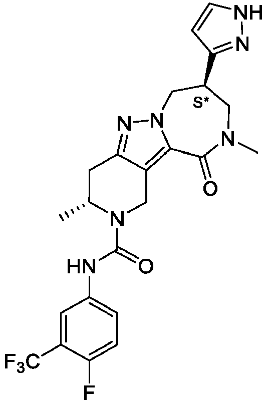

The title compound was prepared in a manner analogous to Step B in Example 7a, using phenyl (4-fluoro-3-(trifluoromethyl)phenyl) carbamate instead of phenyl (3-cyano-4-fluorophenyl)-carbamate. MS (ESI): mass calcd. for C₂₃H₂₃FN₈O₂, 505.2; m/z found, 506.2 [M+H]⁺. ¹H NMR (400MHz, CDCl₃) δ 7.70 (dd, *J* = 2.7, 6.1 Hz, 1H), 7.63 - 7.56 (m, 2 H), 7.13 (t, *J =* 9.5 Hz, 1H), 6.68 (s, 1H), 6.27 (d, *J =* 2.4 Hz, 1H), 5.22 - 5.11 (m, 1H), 4.84 (d, *J =* 15.4 Hz, 1H), 4.75 - 4.59 (m, 2H), 4.52 (d, *J =* 15.3 Hz, 1H), 3.93 - 3.83 (m, 1H), 3.79 - 3.71 (m, 1H), 3.67 - 3.58 (m, 1H), 3.15 (s, 3H), 3.09 - 3.01 (m, 1H), 2.71 (d, *J =* 16.1 Hz, 1H), 1.20 (d, *J =* 6.9 Hz, 3H).

### Example 9a:. (3R,8R*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide.

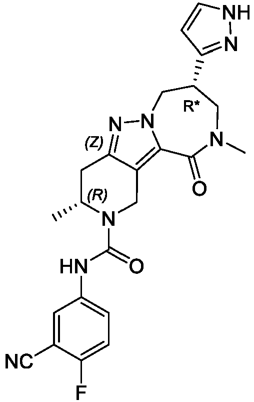

The title compound was prepared in a manner analogous to Example 7a, using (3R,8S)-tert-butyl3,10-dimethyl-11-oxo-8-(1H-pyrazol-3-yl)-3,4,8,9,10,11-hexahydro-1H-pyrido-[4',3':3,4]pyrazolo[1,5-a][1,4]diazepine-2(7H)-carboxylate (Intermediate 9a) instead of (3R,8R)-3,10-dimethyl-8-(1H-pyrazol-3-yl)-3,4,7,8,9,10-hexahydro-1H-pyrido[4',3':3,4]pyrazolo[1,5-a][1,4]diazepin-11(2H)-one (Intermediate 8a). MS (ESI): mass calcd. for C₂₃H₂₃FN₈O₂, 462.19; m/z found, 463.1 [M+H]⁺. ¹H NMR (400MHz, CDCl₃) δ 7.80 (dd, *J =* 2.76, 5.52 Hz, 1H), 7.54 - 7.64 (m, 2H), 7.13 (t, *J =* 8.7 Hz, 1H), 6.80 (s, 1H), 6.27 (d, *J =* 2.38 Hz, 1H), 5.15 (m, 1 H), 4.86 (d, *J =* 15.2 Hz, 1H), 4.61 - 4.75 (m, 2H), 4.49 (d, *J =* 15.4 Hz, 1H), 3.90 (m, 1H), 3.59 - 3.76 (m, 2H), 3.16 (s, 3H), 3.05 (dd, *J* = 5.7, 15.9 Hz, 1H), 2.69 (d, *J =* 15.9 Hz, 1 H), 1.20 (d, *J* = 6.9 Hz, 3H).

### Example 10a: (3R,8R*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide.

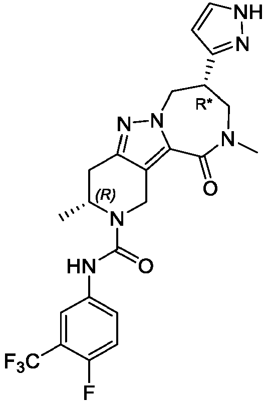

The title compound was prepared in a manner analogous to Example 7a, using (3R,8R*)-tert-butyl 3,10-dimethyl-11-oxo-8-(1H-pyrazol-3-yl)-3,4,8,9,10,11-hexahydro-1H-pyrido[4',3':3,4]-pyrazolo[1,5-a][1,4]diazepine-2(7H)-carboxylate (Intermediate 9a) instead of (3R,8S*)-3,10-dimethyl-8-(1H-pyrazol-3-yl)-3,4,7,8,9,10-hexahydro-1H-pyrido[4',3':3,4]pyrazolo[1,5-a][1,4]-diazepin-11(2H)-one (Intermediate 8) in step A, and using phenyl (4-fluoro-3-(trifluoromethyl)-phenyl) carbamate instead of phenyl (3-cyano-4-fluorophenyl)carbamate in step B. MS (ESI): mass calcd. for C₂₃H₂₃F₄N₇O₂, 505.2; m/z found, 506.1 [M+H]⁺. ¹H NMR (400MHz, CDCl₃) δ 7.70 (dd, *J* = 2.64, 6.02 Hz, 1H), 7.63 - 7.56 (m, 2H), 7.13 (t, *J =* 9.4 Hz, 1H), 6.73 (s, 1H), 6.27 (d, *J* = 2.3 Hz, 1H), 5.16 (m, 1H), 4.86 (d, *J =* 15.18 Hz, 1H), 4.73 - 4.61 (m, 2H), 4.50 (d, *J* = 15.3 Hz, 1H), 3.90 (m, *J* = 6.4 Hz, 1H), 3.75 - 3.60 (m, 2 H), 3.17 (s, 3H), 3.09 - 3.01 (m, 1H), 2.69 (d, *J =* 15.9 Hz, 1H), 1.20 (d, *J =* 6.9 Hz, 3H).

### Example 11a: (3R,8S*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide

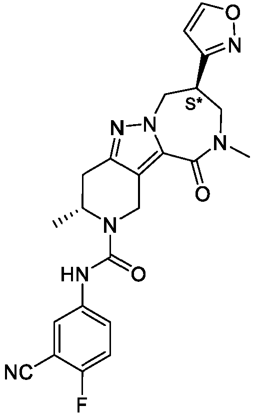

The title compound was prepared in a manner analogous to Example 1a, using (3R,8S*)-tert-butyl 8-(isoxazol-3-yl)-3,10-dimethyl-11-oxo-3,4,8,9,10,11-hexahydro-1H-pyrido[4',3':3,4]-pyrazolo[1,5-a][1,4]diazepine-2(7H)-carboxylate (Intermediate 10a) instead of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2a) in Step A. MS (ESI): mass calcd. for C₂₃H₂₂FN₇O₃, 463.2; m/z found, 464.2 [M+H]⁺. ¹H NMR (400MHz, CDCl₃) δ 8.48 (d, *J* =1.5 Hz, 1H), 7.83-7.77 (m, 1H), 7.61-7.53 (m, 1H), 7.20-7.11 (m, 1H), 6.58 (s, 1H), 6.36 (d, *J* = 1.6 Hz, 1H), 5.24-5.06 (m, 1H), 4.82 (d, *J* =15.0 Hz, 1H), 4.78 - 4.71 (m, 1H), 4.60-4.47 (m, 2H), 3.99-3.89 (m, 1H), 3.77 (d, *J* =5.4 Hz, 1H), 3.70 (d, *J* =5.9 Hz, 1H), 3.19 (s, 3H), 3.08-2.99 (m, 1H), 2.71 (d, *J* =16.0 Hz, 1H), 1.24-1.14 (m, 3H).

### Example 12a: (3R,8R*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide.

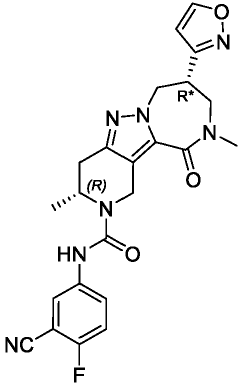

The title compound was prepared in a manner analogous to Example 1a, using (3R,8R*)-tert-butyl 8-(isoxazol-3-yl)-3,10-dimethyl-11-oxo-3,4,8,9,10,11-hexahydro-1H-pyrido[4',3':3,4]-pyrazolo[1,5-a][1,4]diazepine-2(7H)-carboxylate (Intermediate 11a) instead of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2a) in Step A. MS (ESI): mass calcd. for C₂₃H₂₂FN₇O₃, 463.2; m/z found, 464.2 [M+H]⁺. ¹H NMR (400 MHz, CDCl₃) δ 8.47 (d, *J* = 1.5 Hz, 1H), 7.81 (dd, *J* = 2.8, 5.44 Hz, 1H), 7.58 (ddd, *J* =2.8, 4.5, 9.1 Hz, 1H), 7.16 (t, *J =* 8.7 Hz, 1H), 6.64 (s, 1H), 6.38 (d, *J* =1.6 Hz, 1H), 5.16 (m, *J* = 6.11 Hz, 1H), 4.85 (d, *J* =15.6 Hz, 1H), 4.75 (dd, *J* = 7.0, 14.37 Hz, 1H), 4.59 (dd, *J* =5.6, 14.43 Hz, 1H), 4.51 (d, *J =* 15.4 Hz, 1H), 3.99 (m, *J* = 6.4 Hz, 1H), 3.80 - 3.65 (m, 2H), 3.22 (s, 3H), 3.07 (dd, *J* =5.9, 15.8 Hz, 1H), 2.71 (d, *J* =16.0 Hz, 1H), 1.22 (d, *J* =6.8 Hz, 3H).

### Example 13a: (3R,8S*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide.

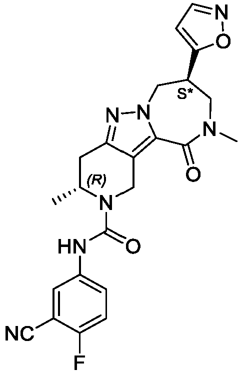

The title compound was prepared in a manner analogous to Example 1a, using (3R,8S*)-tert-butyl 8-(isoxazol-5-yl)-3,10-dimethyl-11-oxo-3,4,8,9,10,11-hexahydro-1H-pyrido[4',3':3,4]-pyrazolo[1,5-a][1,4]diazepine-2(7H)-carboxylate (Intermediate 12a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A. MS (ESI): mass calcd. for C₂₃H₂₂FN₇O₃, 463.2; m/z found,464.2 [M+H]⁺. ¹H NMR (400 MHz, CDCl₃) δ 8.30 (d, *J* = 1.7 Hz, 1H), 7.80 (dd, *J* =2.7, 5.32 Hz, 1H), 7.61-7.55 (m, 1H), 7.16 (t, *J* =8.7 Hz, 1H), 6.59 (s, 1H), 6.29 (d, *J* = 1.2 Hz, 1H), 5.19-5.10 (m, 1H), 4.83 (d, *J* =15.4 Hz, 1H), 4.76 (dd, *J* =7.3, 14.1 Hz, 1H), 4.65-4.56 (m, 1H), 4.51 (d, *J* =15.2 Hz, 1H), 4.01 (m, *J* =5.5 Hz, 1H), 3.86 (dd, *J* =5.3, 14.98 Hz, 1H), 3.64 (dd, *J* =5.6, 14.98 Hz, 1H), 3.17 (s, 3H), 3.05 (dd, *J* =5.7, 15.96 Hz, 1H), 2.72 (d, *J* =16.5 Hz, 1H), 1.22 (d, *J* =7.0 Hz, 3H).

### Example 14a: (3R,8R*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide.

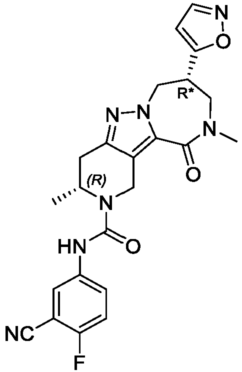

The title compound was prepared in a manner analogous to Example 1a, using (3R,8R*)-tert-butyl 8-(isoxazol-5-yl)-3,10-dimethyl-11-oxo-3,4,8,9,10,11-hexahydro-1H-pyrido[4',3':3,4]-pyrazolo[1,5-a][1,4]diazepine-2(7H)-carboxylate (Intermediate 13a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A. MS (ESI): mass calcd. for C₂₃H₂₂FN₇O₃, 463.2; m/z found, 464.2 [M+H]⁺. ¹H NMR (400 MHz, CDCl₃) δ 8.28 (d, *J* =1.6 Hz, 1H), 7.80 (dd, *J* =2.7, 5.3 Hz, 1H), 7.58 (m, 1H), 7.16 (t, *J* =8.7 Hz, 1H), 6.63 (s, 1H), 6.29 (d, J=1.22 Hz, 1H), 5.15 (m, *J* = 6.97 Hz, 1H), 4.85 (d, *J* =15.3 Hz, 1H), 4.79-4.70 (m, 1H), 4.66-4.58 (m, 1H), 4.49 (d, *J* =15.3 Hz, 1H), 4.07-3.98 (m, 1H), 3.83 (dd, *J* =5.7, 15.1 Hz, 1H), 3.68-3.58 (m, 1H), 3.19 (s, 3H), 3.06 (dd, *J* =5.8, 16.0 Hz, 1H), 2.70 (d, *J* = 15.4 Hz, 1H), 1.22 (d, *J* =7.0 Hz, 3H).

### Example 15a: N-(3-Cyano-4-fluoro-phenyl)-11-hydroxy-13-methyl-14-oxo-11-phenyl-4,8,9,13-tetrazatricyclo[7.5.0.0^{2,7}]tetradeca-1,7-diene-4-carboxamide.

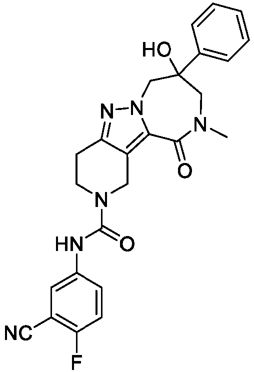

The title compound was prepared in a manner analogous to Example 1a, using tert-butyl 8-hydroxy-10-methyl-11-oxo-8-phenyl-3,4,8,9,10,11-hexahydro-1H-pyrido[4',3':3,4]pyrazolo-[1,5-a][1,4]diazepine-2(7H)-carboxylate (Intermediate 15a) instead of tert-butyl 10-methyl-11-oxo-8-(1H-1,2,4-triazol-3-yl)-1,3,4,7,8,9-hexahydropyrido[2,3]pyrazolo[2,4-b][1,4]diazepine-2-carboxylate (Intermediate 2a) in Step A. MS (ESI): mass calcd. for C₂₅H₂₃N₆O₃F, 474.2; m/z found, 475.2 [M+H]⁺. ¹H NMR (400MHz, CDCl₃) δ 7.78 -7.76 (m, 1 H), 7.65 - 7.53 (m, 3 H), 7.48 - 7.35 (m, 3 H), 7.14 (t, *J* = 8.7 Hz, 1 H), 6.68 (s, 1 H), 4.96 (d, *J =* 14.3 Hz, 1 H), 4.81 - 4.62 (m, 2 H), 4.49 (d, *J =* 14.4 Hz, 1 H), 3.97 - 3.81 (m, 2 H), 3.63 - 3.39 (m, 2 H), 3.24 (s, 3 H), 2.90 (t, *J* = 5.7 Hz, 2H), 2.49 (s, 1 H).

### Example 16a: N-(3-Cyano-4-fluorophenyl)-8-fluoro-10-methyl-11-oxo-8-phenyl-3,4,8,9,10,11-hexahydro-1H-pyrido[4',3':3,4]pyrazolo[1,5-a][1,4]diazepine-2(7H)-carboxamide.

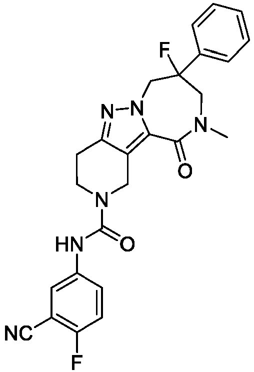

The title compound was prepared in a manner analogous to Example 1a, using tert-butyl 8-fluoro-10-methyl-11-oxo-8-phenyl-3,4,8,9,10,11-hexahydro-1H-pyrido[4',3':3,4]pyrazolo[1,5-a][1,4]diazepine-2(7H)-carboxylate (Intermediate 16a) instead of tert-butyl 10-methyl-11-oxo-8-(1H-1,2,4-triazol-3-yl)-1,3,4,7,8,9- hexahydropyrido[2,3]pyrazolo[2,4-b][1,4]diazepine-2-carboxylate (Intermediate 2a) in Step A. MS (ESI): mass calcd. for C₂₅H₂₂F₂N₆O₂, 476.5; m/z found, 477.2 [M+H]⁺. ¹H NMR (400MHz, CDCl₃) δ 7.78 (d, *J =* 2.8, 5.5 Hz, 1 H), 7.60 (m, 1 H), 7.50 - 7.34 (m, 5 H), 7.14 (t, *J =* 8.7 Hz, 1 H), 6.81 (s, 1 H), 4.97 (d, *J* = 6.8, 14.7 Hz, 1 H), 4.85 - 4.60 (m, 3 H), 3.95 - 3.84 (m, 2 H), 3.78 - 3.53 (m, 2 H), 3.26 (s, 3 H), 2.92 (t, *J* = 5.8 Hz, 2 H).

### 4. ANTI-HBV ACTIVITY OF COMPOUNDS OF FORMULA (I)

### Procedure

The anti HBV activity was measured using the HepG2.117 cell line, a stable, inducibly HBV producing cell line, which replicates HBV in the absence of doxicycline (Tet-off system). The HepG2 cell line is available from ATCC^{®} under number HB-8065. Transfection of the HepG2 cell line can be as described in Sun and Nassal 2006 Journal of Hepatology 45 (2006) 636-645 "Stable HepG2- and Huh7-based human hepatoma cell lines for efficient regulated expression of infectious hepatitis B virus"*.*

For the antiviral assay, HBV replication was induced, followed by a treatment with serially diluted compound in 96-well plates. After 3 days of treatment, the antiviral activity was determined by quantification of intracellular HBV DNA using real-time PCR and an HBV specific primer set and probe.

Cytotoxicity of the compounds was tested using HepG2 or HepG2.117 cells, incubated for 3 days in the presence of compounds. The viability of the cells was assessed using the PERKIN ELMER ATPlite Luminescence Assay System."

### Results:

**Table 8**

| Compound number | | HBV-AVE-HepG2.117 EC₅₀ (µM, mean value) | | TOX-HepG2.117 CC₅₀ (µM, mean value) | |
|---|---|---|---|---|---|
| **1** | | 9.628 | | >50 | |
| **2** | | 1.146 | | >50 | |
| **3** | | 0.188 | | 25.26 | |
| **4** | | 0.091 | | 17.94 | |
| **5** | | 0.213 | | >50 | |
| **6** | | 0.287 | | 27.98 | |
| **7** | | 0.399 | | >50 | |
| **8** | | <0.039 | | 10.82 | |
| **9** | | 0.054 | | 16.36 | |
| **10** | | 0.062 | | 7.26 | |
| **11** | | 0.054 | | 8.43 | |
| **12** | | 0.066 | | 25.28 | |
| **13** | | 0.055 | | NA | |
| **14** | | 0.085 | | 6.96 | |
| **15** | | 0.055 | | >50 | |
| **16** | | 0.079 | | >50 | |
| **17** | | 0.436 | | >50 | |
| **18** | | 0.252 | | >50 | |
| **19** | | 0.079 | | >50 | |
| **20** | | 0.108 | | >50 | |
| **21** | | 0.268 | | >50 | |
| **22** | | 0.129 | | >50 | |
| **23** | | 0.022 | | >50 | |
| **24** | | 0.036 | | 10.41 | |
| **25** | | 0.016 | | >25 | |
| **26** | | 0.028 | | 26.23 | |
| **27** | | 0.103 | | >50 | |
| **28** | | 0.057 | | 16.69 | |
| **29** | | 0.741 | | >50 | |
| **30** | | 1.504 | | >50 | |
| **31** | | 0.075 | | >50 | |
| **32** | | 0.332 | | >50 | |
| **33** | | 9.612 | | >50 | |
| **34** | | 1.381 | | >50 | |
| **36** | | 0.077 | | 24.55 | |
| **37** | | 0.345 | | >50 | |
| **38** | | 1.747 | | >50 | |
| **39** | | 2.487 | | >50 | |
| **40** | | 0.022 | | 20.93 | |
| **41** | | 0.328 | | >50 | |
| **42** | | 0.308 | | 1.44 | |
| **43** | | 0.210 | | >50 | |
| **44** | | 0.079 | | >50 | |
| **45** | | 0.192 | | >50 | |
| **46** | | 0.505 | | >50 | |
| **47** | | 0.249 | | 3.63 | |
| **48** | | 1.228 | | >50 | |
| **C49** | | 0.274 | | 20.55 | |
| **C50** | | NA | | | |
| **C51** | | 1.025 | | 39.03 | |
| **C52** | | 0.995 | | >50 | |
| **C53** | | 1.081 | | 20.41 | |
| **C54** | | 1.994 | | 13.61 | |
| **C55*R** | | >2.5 | | 3.22 | |
| **C55*S** | | 0.069 | | >50 | |
| **C56** | | 0.962 | | 28.4 | |
| **C57** | | 0.04 | | 18.85 | |
| **C58** | | 0.531 | | 18 | |
| **C59** | | 0.576 | | >50 | |
| **C60** | | 0.565 | | >50 | |
| **C61*S** | | 0.153 | | >50 | |
| **C62** | | 0.13 | | 9.31 | |
| **C63** | | 0.824 | | 8.21 | |
| **C64** | | 0.089 | | >49 | |
| **C65** | | 0.299 | | >50 | |
| **C61*R** | | >2.5 | | 8.11 | |
| **C66** | | 0.042 | | 9.18 | |
| **C67** | | 0.07 | | 22.53 | |
| **C68** | | 0.321 | | 10.55 | |
| **C69** | | 0.063 | | 14.5 | |
| **C70** | | 0.232 | | >50 | |
| **C71** | | >2.5 | | >50 | |
| **C72** | | >2.5 | | >50 | |
| **C73** | | >2.5 | | >50 | |
| **C74** | | >2.5 | | >50 | |
| **C75** | | 1.428 | | >50 | |
| **C76** | | 0.983 | | >25 | |
| **C77** | | 0.038 | | >50 | |
| **C78** | | 0.707 | | >50 | |
| **C79** | | > 1.6 | | >50 | |
| **C80** | | 0.05 | | 26.11 | |
| **C81** | | 0.058 | | 46.22 | |
| **C82** | | 0.019 | | 32.52 | |
| **C83** | | 0.229 | | >50 | |
| **C84** | | 0.311 | | 9.16 | |
| **C85*R** | | 0.088 | | >50 | |
| **C85*S** | | 0.035 | | >50 | |
| **C86** | | 0.046 | | >50 | |
| **C87** | | 0.066 | | >50 | |
| **C88** | | 0.038 | | >48 | |
| **C89** | | 0.067 | | 22.81 | |
| **C90** | | 0.667 | | 21.94 | |
| **C91** | | 0.083 | | 36.74 | |
| **C92** | | 0.025 | | 24.45 | |
| **C93** | | 0.055 | | 30 | |
| **C94** | | 0.162 | | 4.77 | |
| **C95** | | 0.2 | | >47 | |
| **C96** | | 0.025 | | 10.65 | |
| **C97** | | 0.011 | | 9.79 | |
| **C98** | | 0.073 | | 9.83 | |
| **C99** | | 0.072 | | >50 | |
| **C100** | | 0.085 | | 15.5 | |
| **C101** | | 0.036 | | 8.95 | |
| **C102** | | 0.019 | | 7.14 | |
| **C103** | | 0.39 | | >50 | |
| **C104** | | 0.632 | | >50 | |
| **C105** | | | | | |
| **C105*R** | | 0.059 | | 14.56 | |
| **C105*S** | | 0.297 | | >50 | |
| **C106** | | 0.031 | | 33.79 | |
| **C107** | | 0.015 | | 13.22 | |
| **C108** | | 0.085 | | 20.02 | |
| **C109** | | 0.013 | | 18.91 | |
| **C110** | | 0.017 | | 16.03 | |
| **C111** | | 0.086 | | >50 | |
| **C112** | | 0.025 | | >50 | |
| **C113** | | 0.257 | | >50 | |
| **C114** | | 0.048 | | 30.85 | |
| **C115** | | 0.265 | | >50 | |
| **C116** | | 0.015 | | 12.36 | |
| **C117** | | 0.119 | | 18.17 | |
| **C118** | | 0.057 | | 24.1 | |
| **C119*R** | | 0.269 | | >50 | |
| **C119*S** | | 0.757 | | >50 | |
| **C120** | | 0.047 | | | |
| **C121** | | >2.5 | | >50 | |
| **C122** | | 0.214 | | >50 | |
| **C123*R** | | 0.174 | | 19.98 | |
| **C123*S** | | 0.517 | | 19.21 | |
| **C124** | | 0.011 | | 26.7 | |
| **C125** | | 0.037 | | >50 | |
| **C88*R** | | 0.076 | | >50 | |
| **C88*S** | | 0.023 | | >50 | |
| **C126** | | 0.024 | | >50 | |
| **C127** | | 0.133 | | 15.73 | |
| **C128** | | 0.081 | | >50 | |
| **C129** | | 0.143 | | >50 | |
| **C130** | | 0.082 | | 33.66 | |
| **C131** | | 0.038 | | 19.57 | |
| **C132** | | 0.061 | | >50 | |
| **C133** | | 0.332 | | >50 | |
| **C134** | | >2.5 | | >50 | |
| **C135** | | 0.1 | | >50 | |
| **C136** | | >2.5 | | >50 | |
| **C137** | | 0.056 | | >50 | |
| **C138** | | 0.061 | | >50 | |
| **C139** | | 0.04 | | >50 | |
| **C140** | | 0.032 | | | |
| **C141** | | 0.056 | | >50 | |
| **C142** | | 0.268 | | >50 | |
| **C143** | | 0.16 | | >50 | |
| **C144** | | >2.5 | | >50 | |
| **C145** | | 0.106 | | >50 | |
| **C146** | | 1.794 | | >50 | |
| **C147** | | 0.054 | | >50 | |
| **C148** | | 0.385 | | >50 | |
| **C149** | | 0.059 | | >50 | |
| **C150** | | 0.075 | | >50 | |
| **C151** | | 0.42 | | >50 | |
| **C152** | | 0.145 | | >50 | |
| **C153** | | 0.082 | | >50 | |
| **C154** | | 0.071 | | 9.53 | |
| **C155** | | 0.083 | | >50 | |
| **C156** | | 0.174 | | >50 | |
| **C157** | | 0.02 | | >50 | |
| **C158** | | 2.381 | | >50 | |
| **C159** | | 0.979 | | 36.03 | |
| **C160** | | 0.029 | | >50 | |
| **C161** | | 0.051 | | >50 | |
| **C162** | | 0.082 | | >50 | |
| **C163** | | 0.098 | | >50 | |
| **C164** | | 0.039 | | 4.29 | |
| **C165** | | 0.676 | | >50 | |
| **C166** | | 0.034 | | 29.1 | |
| **C167** | | 0.277 | | >50 | |
| **C168** | | 0.046 | | 6.11 | |
| **C169** | | 0.831 | | >50 | |
| **C170** | | 0.432 | | >50 | |
| **C171** | | 0.082 | | 15.33 | |
| **C172** | | 0.19 | | >50 | |
| **C173** | | 0.41 | | >50 | |
| **C174** | | 0.18 | | 15.34 | |
| **C175** | | 0.038 | | 35.5 | |
| **C176** | | 0.274 | | 14.99 | |
| **C177** | | 0.136 | | >50 | |
| **C178** | | 0.207 | | >50 | |
| **C179** | | 0.219 | | >50 | |
| **C180** | | 0.123 | | >50 | |
| **C181** | | 0.152 | | >50 | |
| **C182** | | 0.075 | | >50 | |
| **C183** | | 0.108 | | >50 | |
| **C184** | | >2.5 | | >50 | |
| **C185** | | 0.114 | | >50 | |
| **C186** | | 0.049 | | 21.64 | |
| **C187** | | 0.155 | | >50 | |
| **C188** | | 0.04 | | 49.63 | |
| **C189** | | 0.043 | | 41.81 | |
| **C190** | | 0.115 | | 33.14 | |
| **C191** | | 0.097 | | 32.95 | |
| **C192** | | 0.076 | | 39.72 | |
| **C193** | | 0.197 | | >50 | |
| **C194** | | 0.075 | | 26.44 | |
| **C195** | | 0.566 | | >50 | |
| **C196** | | 0.048 | | 38.03 | |
| **C197** | | 0.24 | | >50 | |
| **C198** | | 0.053 | | >50 | |
| **C199** | | 0.33 | | >50 | |
| **C200** | | 0.25 | | >50 | |
| **C201** | | 0.229 | | >50 | |
| **C202** | | 0.071 | | >50 | |
| **C203** | | 0.09 | | >50 | |
| **C204** | | 0.028 | | 19.2 | |
| **C205** | | 0.04 | | -1 | |
| **C206** | | 0.038 | | -1 | |
| **C207** | | 0.053 | | 25.55 | |
| **C208** | | 0.13 | | >50 | |
| **C209** | | >2.5 | | >50 | |
| **C210** | | 0.062 | | >50 | |
| **C211** | | 0.285 | | >50 | |
| **C212** | | 0.05 | | >50 | |
| **C213** | | 0.382 | | 37.52 | |
| **C214** | | 0.074 | | >50 | |
| **C215** | | 0.106 | | >50 | |
| **C216** | | 0.09 | | >50 | |
| **C217** | | 0.183 | | 39.54 | |
| **C218** | | 0.039 | | >50 | |
| **C219** | | 0.068 | | >50 | |
| **C220** | | 0.077 | | >50 | |
| **C221** | | 0.022 | | >50 | |
| **C222** | | 0.124 | | >50 | |
| **C223** | | 0.219 | | >50 | |
| **C224** | | 0.326 | | >50 | |
| **C225** | | 0.263 | | >50 | |
| **C226** | | 0.194 | | >50 | |
| **C227** | | 0.129 | | 37.33 | |
| **C228** | | 0.062 | | 37.51 | |
| **C229** | | 0.438 | | >25 | |
| **C230** | | 0.045 | | >12.5 | |
| **C231** | | 0.09 | | >50 | |
| **C232** | | 0.066 | | 24.44 | |
| **C233** | | 0.423 | | 29.76 | |
| **C234** | | 0.036 | | >50 | |
| **C235** | | 1.934 | | >50 | |
| **C236** | | 0.325 | | >51 | |
| **C237** | | 0.107 | | 18.79 | |
| **C238** | | 0.029 | | 39.9 | |
| **C239** | | 0.154 | | >50 | |
| **C240** | | 0.017 | | 35.23 | |
| **C241** | | 0.19 | | >50 | |
| **C242** | | 0.589 | | >50 | |
| **C243** | | >2.5 | | >50 | |
| **C244** | | 0.048 | | 24.52 | |
| **C245** | | 0.072 | | >50 | |
| **C246** | | 0.171 | | >50 | |
| **C247** | | >2.5 | | >50 | |
| **C248** | | 0.013 | | 24.97 | |
| **C249** | | >2.5 | | >50 | |
| **C250** | | 0.033 | | >12.5 | |

Induction or non-induction of HBc speckling HepG2.117 cells were cultured in the presence of DMSO or test compound in absence of doxycycline. After formaldehyde fixation and Triton-X-100 permeabilization, Hepatitis B virus core protein (HBc) was immunolabeled with a primary anti-HBc antibody. ALEXA 488-conjugated secondary antibody was used for fluorescent detection of the primary HBV Core signal. CELLMASK Deep Red and HOECHST 33258 were used for the detection of cytoplasm and nucleus respectively, which allowed the segmentation of cellular compartments.
An image analysis software that allows to detect different morphological phenotypes was used to determine the level of HBV core in the cytoplasm or nucleus (high content imaging assay).

### HBV Replication Inhibition Assay

HBV replication inhibition by the disclosed compounds were determined in cells infected or transfected with HBV or cells with stably integrated HBV, such as HepG2.2.15 cells (Sells et al. 1987). In this example, HepG2.2.15 cells were maintained in cell culture medium containing 10% fetal bovine serum (FBS), Geneticin, L-glutamine, penicillin and streptomycin. HepG2.2.15 cells were seeded in 96-well plates at a density of 40,000 cells/well and were treated with serially diluted compounds at a final DMSO concentration of 0.5% either alone or in combination by adding drugs in a checker box format. Cells were incubated with compounds for three days, after which medium was removed and fresh medium containing compounds was added to cells and incubated for another three days. At day 6, supernatant was removed and treated with DNase at 37 °C for 60 minutes, followed by enzyme inactivation at 75 °C for 15 minutes. Encapsidated HBV DNA was released from the virions and covalently linked HBV polymerase by incubating in lysis buffer (Affymetrix QS0010) containing 2.5 µg proteinase K at 50 °C for 40 minutes. HBV DNA was denatured by addition of 0.2 M NaOH and detected using a branched DNA (BDNA) QuantiGene assay kit according to manufacturer recommendation (Affymetrix). HBV DNA levels were also quantified using qPCR, based on amplification of encapsidated HBV DNA extraction with QuickExtraction Solution (Epicentre Biotechnologies) and amplification of HBV DNA using HBV specific PCR probes that can hybridize to HBV DNA and a fluorescently labeled probe for quantitation. In addition, cell viability of HepG2.2.15 cells incubated with test compounds alone or in combination was determined by using CellTitre-Glo reagent according to the manufacturer protocol (Promega). The mean background signal from wells containing only culture medium was subtracted from all other samples, and percent inhibition at each compound concentration was calculated by normalizing to signals from HepG2.2.15 cells treated with 0.5% DMSO using equation E1.$E 1 : %inhibition = \left(DMSOave-Xi\right) / DMSOave \times 100 %$ where DMSOave is the mean signal calculated from the wells that were treated with DMSO control (0% inhibition control) and Xi is the signal measured from the individual wells. EC₅₀ values, effective concentrations that achieved 50% inhibitory effect, were determined by nonlinear fitting using Graphpad Prism software (San Diego, CA) and equation E2. $E 2 : Ymin + \left(Ymax-Ymin\right) / \left(1+10\left(LogEC50-X\right)\times HillSlope\right)$ where Y represents percent inhibition values and X represents the logarithm of compound concentrations.

Selected disclosed compounds were assayed in the HBV replication assay (BDNA assay), as described above, and a representative group of these active compounds is shown in Table 3. Table 9 shows EC₅₀ values obtained by the BDNA assay for a group of select compounds.

**Table 9.**

| **Example #** | **Compound name** | **HepG2.2.15 EC₅₀ HBV DNA (uM)** |
|---|---|---|
| **1a** | N-(3-Cyano-4-fluorophenyl)-10-methyl-11-oxo-8-(1H-1,2,4-triazol-3-yl)-3,4,8,9,10,11-hexahydro-1H-pyrido[4',3':3,4]pyrazolo[1,5-a] [1,4]diazepine-2(7H)-carboxamide; | > 4000 |
| **2a** | N-(3-Cyano-4-fluorophenyl)-10-methyl-8-(1,2,4-oxadiazol-5-yl)-11-oxo-3,4,8,9,10,11-hexahydro-1H-pyrido[4',3':3,4]pyrazolo[1,5-a] [1,4]diazepine-2(7H)-carboxamide; | > 4000 |
| **3a** | N-(3-Cyano-4-fluorophenyl)-10-methyl-11-oxo-8-(1H-tetrazol-5-yl)-3,4,8,9,10,11-hexahydro-1H-pyrido[4',3':3,4]pyrazolo[1,5-a] [1,4]diazepine-2(7H)-carboxamide; | > 4000 |
| **4a** | N-(3-Chloro-4-fluorophenyl)-10-methyl-11-oxo-8-(1H-pyrazol-3-yl)-3,4,8, 9,10,11-hexahydro-1H-pyrido[4',3':3,4]pyrazolo[1,5-a][1,4]diazepine-2(7H)-carboxamide; | 93 |
| **5a** | N-(3-Cyano-4-fluorophenyl)-10-methyl-8-(1-methyl-1H-pyrazol-3-yl)-11-oxo-3,4,8,9,10,11-hexahydro-1H-pyrido[4',3':3,4]pyrazolo[1,5-a][1,4]diazepine-2(7H)-carboxamide; | 160 |
| **6a** | N-(3-Cyano-4-fluorophenyl)-10-methyl-8-(1-methyl-1H-pyrazol-5-yl)-11-oxo-3,4,8,9,10,11-hexahydro-1H-pyrido[4',3':3,4]pyrazolo[1,5-a][1,4]diazepine-2(7H)-carboxamide; | 83 |
| **7a** | (3R,8S*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; | 37 |
| **8a** | (3R,8S*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; | 20 |
| **9a** | (3R,8R*)-N-(3-Cyano-4-fluorophenyl)-3,10-dimethyl-11-oxo-8-(1H-pyrazol-3-yl)-3,4,8,9,10,11-hexahydro-1H-pyrido[4',3':3,4]pyrazolo[1,5-a][1,4]diazepine-2(7H)-carboxamide; | 260 |
| **10a** | (3R,8R*)-N-(4-Fluoro-3-(trifluoromethyl)phenyl)-3,10-dimethyl-11-oxo-8-(1H-pyrazol-3-yl)-3,4,8,9,10,11-hexahydro-1H-pyrido[4',3':3,4]pyrazolo[ 1,5-a][1,4]diazepine-2(7H)-carboxamide | 280 |
| **11a** | (3R,8S*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide; | 27 |
| **12a** | (3R,8R*)-N-(3-Cyano-4-fluorophenyl)-8-(isoxazol-3-yl)-3,10-dimethyl-11-oxo-3,4,8,9,10,11-hexahydro-1H-pyrido[4',3':3,4]pyrazolo[1,5-a][1,4]diazepine-2(7H)-carboxamide; | 240 |
| **13a** | (3R,8S*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide; | 22 |
| **14a** | (3R,8R*)-N-(3-Cyano-4-fluorophenyl)-8-(isoxazol-5-yl)-3,10-dimethyl-11-oxo-3,4,8,9,10,11-hexahydro-1H-pyrido[4',3':3,4]pyrazolo[1,5-a][1,4]diazepine-2(7H)-carboxamide; | 150 |
| **15a** | N-(3-Cyano-4-fluoro-phenyl)-11-hydroxy-13-methyl-14-oxo-11-phenyl-4,8,9,13-tetrazatricyclo[7.5.0.02,7]tetradeca-1,7-diene-4-carboxamide; | 140 |
| **16a** | N-(3-Cyano-4-fluorophenyl)-8-fluoro-10-methyl-11-oxo-8-phenyl-3,4,8,9,10,11-hexahydro-1H-pyrido[4',3':3,4]pyrazolo[1,5-a][1,4]diazepine-2(7H)-carboxamide; | 176 |

The disclosed subject matter is not to be limited in scope by the specific embodiments and examples described herein. Indeed, various modifications of the disclosure in addition to those described will become apparent to those skilled in the art from the foregoing description and accompanying figures. Such modifications are intended to fall within the scope of the appended claims. . Other embodiments are within the following claims.

## Claims

1. A compound of Formula (I), 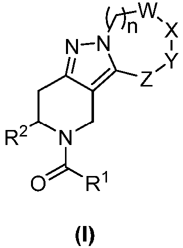
or a stereoisomer or tautomer thereof,
wherein
R¹ is a 5- to 10-membered monocyclic or bicyclic ring, more particularly a 5- to 9-membered monocyclic or bicyclic ring, wherein the 5- to 10-membered monocyclic or bicyclic ring, more particularly the 5- to 9-membered monocyclic or bicyclic ring :
- optionally contains 1 to 3 heteroatoms, the heteroatoms each independently being selected from N, O and S; and/or
- is optionally substituted with one or more substituents each independently selected from hydrogen, halogens, CN, CF₃, CF₂H, CFH₂, CF₂CH₃, C₁₋₆alkyl, OC₁₋₆alkyl, OCF₃, OCF₂H and C₃₋₄cycloalkyl;
more particularly R¹ is phenyl substituted with one or more substituents each independently selected from the group consisting of Cl, F, CN, CH₃, CF₃, and CF₂H;
R² is selected from the group consisting of H, C₁₋₄alkyl and C₁₋₄alkyl substituted with one or more F;
n is an integer of 0 or 1;
W is CHR³ or C=CH₂;
R³ is selected from the group consisting of H, F, OH, C₁₋₄alkyl optionally substituted with OH or F, CONHC₁₋₄alkyl, CONHC₃₋₆cycloalkyl, NHSO₂C₁₋₄alkyl and NHSO₂C₃₋₆cycloalkyl;
X is CH₂;
Y is NR⁵
R⁵ is selected from the group consisting of
H,
C₁₋₄alkyl,
C₁₋₄alkyl substituted with one of more F,
C₁₋₄alkyl substituted with a 5- to 10-membered monocyclic or bicyclic aromatic ring, wherein the 5- to 10-membered monocyclic or bicyclic aromatic ring optionally contains one, two, three or four heteroatoms, the heteroatoms independently being selected from N, O and S, and
wherein the said 5- to 10-membered monocyclic or bicyclic aromatic ring is optionally substituted with one or more substituents each independently selected from the group consisting of hydrogen, halogens, CN, CF₃, CF₂H, CFH₂, CF₂CH₃, CH₂CF₃, C₁₋₄alkyl, OH, OC₁₋₄alkyl, OCF₃, OCF₂H, SO₂N(CH₃)₂ and C₃₋₄cycloalkyl; and
Z is selected from the group consisting of C(=O) and C(=S),
or a pharmaceutically acceptable salt thereof.

2. The compound of claim 1, wherein R¹ is phenyl substituted with one or more chlorine substituents.

3. The compound of claim 1 or 2, wherein R² is H or methyl.

4. The compound of any one of claims 1 to 3, wherein Z is C=O.

5. The compound of any one of claims 1 to 4, wherein R¹ is dichlorophenyl.

6. The compound of any one of claims 1 to 5, wherein W is CH₂ or CHF.

7. The compound of any one of claims 1 to 6, wherein R⁵ is selected from the group consisting of C₁₋₄alkyl and C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms independently being selected from N, O and S, and wherein the said 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

8. The compound of any one of claims 1 to 6, wherein R⁵ is C₁₋₄alkyl substituted with a 5- to 10-membered monocyclic or bicyclic aromatic ring, wherein the 5- to 10-membered monocyclic or bicyclic aromatic ring optionally contains one, two or three heteroatoms, the heteroatoms being N, and wherein the said 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

9. The compound of claim 8, wherein the 5- to 10-membered monocyclic or bicyclic aromatic ring, is phenyl, pyridyl, pyrazolyl or indolyl, and wherein the said 5- to 10-membered monocyclic or bicyclic aromatic ring is optionally substituted with one or more substituents selected from the group consisting of halogens, CN, CF₃, CF₂H, CFH₂, CF₂CH₃, C₁₋₄alkyl, OC₁₋₄alkyl, OCF₃, OCF₂H and C₃₋₄cycloalkyl.

10. The compound of claim 9, wherein the 5- to 10-membered monocyclic or bicyclic aromatic ring, is phenyl, pyridyl, pyrazolyl or indolyl, and wherein the said 5- to 10-membered monocyclic or bicyclic aromatic ring is optionally substituted with one or more substituents selected from the group consisting of halogens, C₁₋₄alkyl, OC₁₋₄alkyl, OCF₃ and OCF₂H.

11. The compound of claim 1 wherein the compound is:

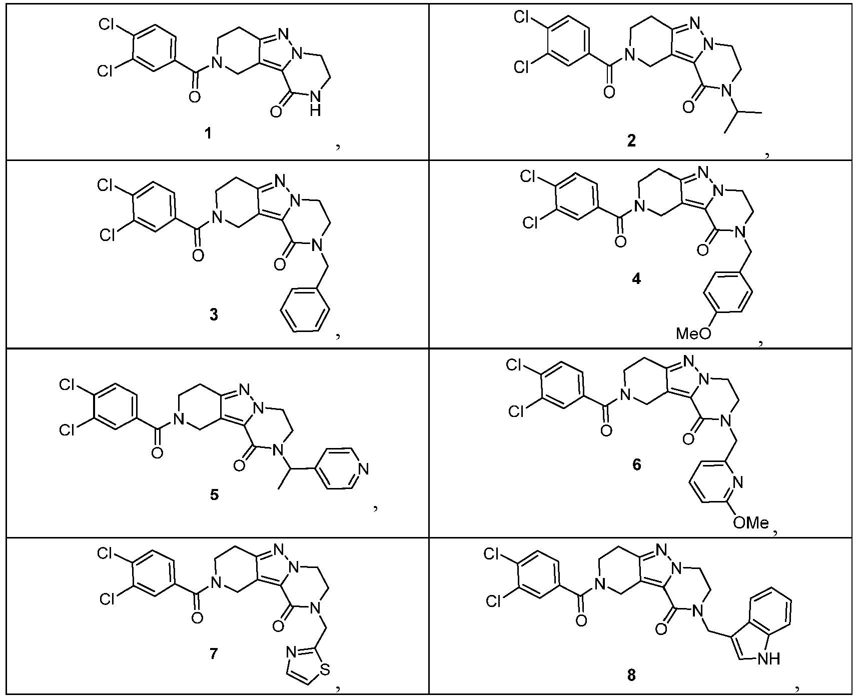

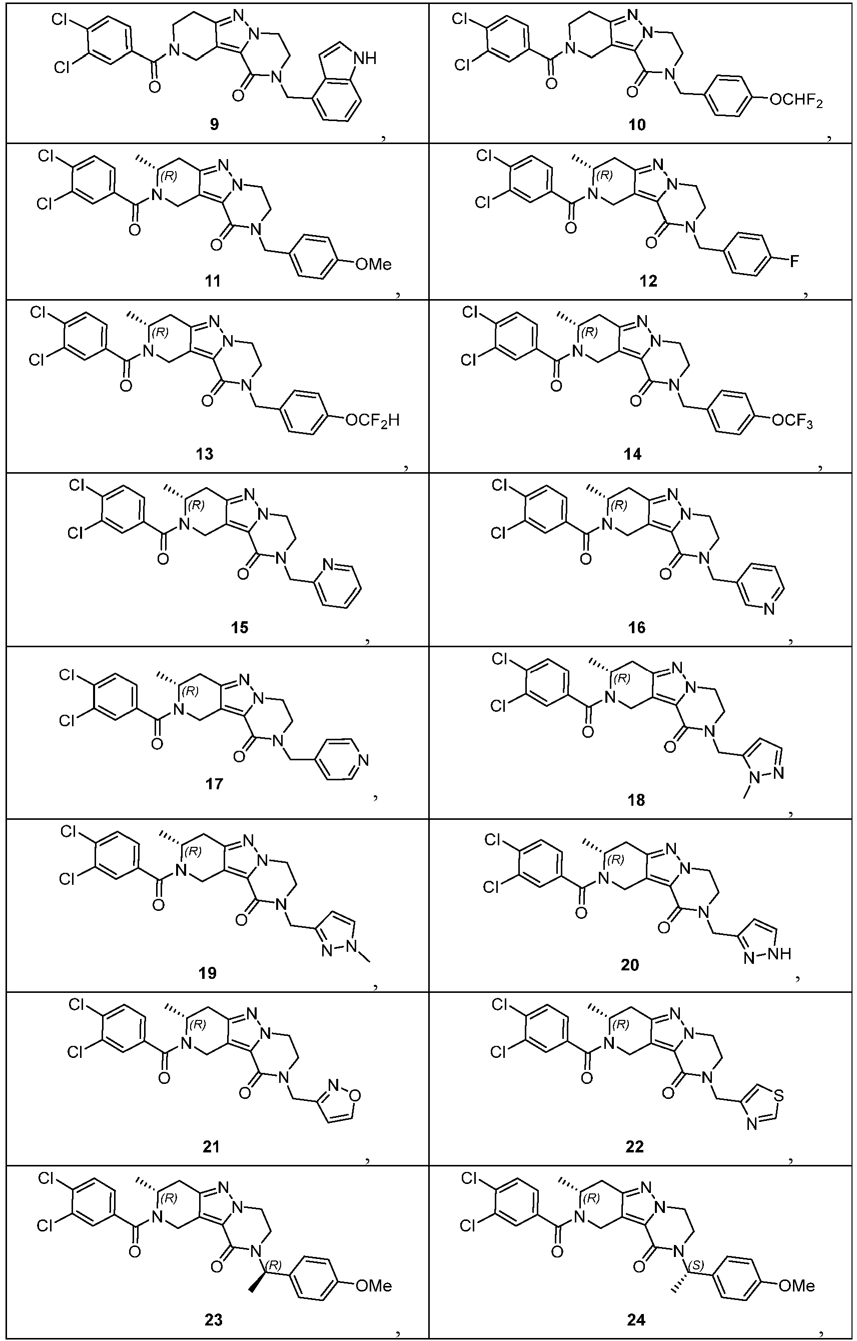

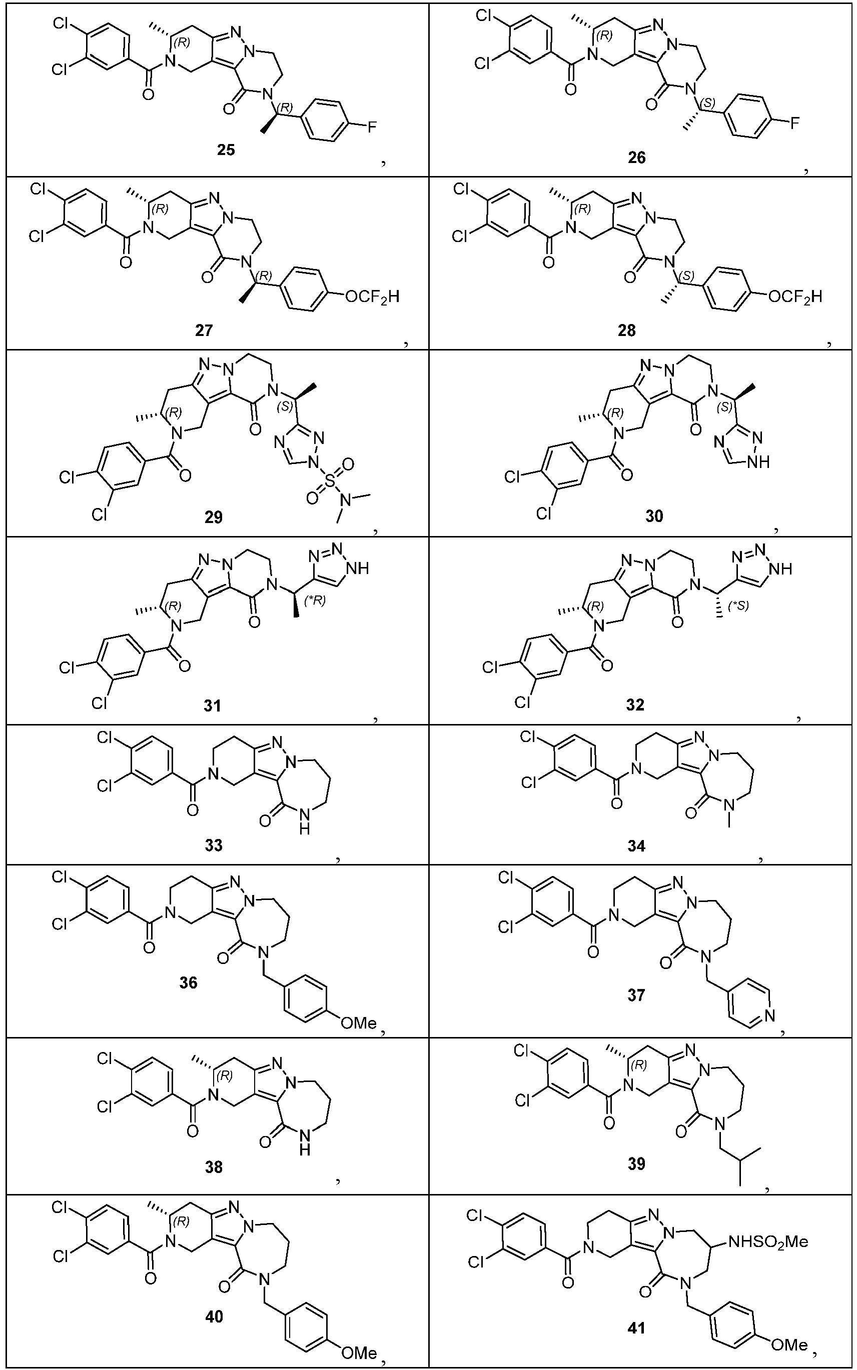

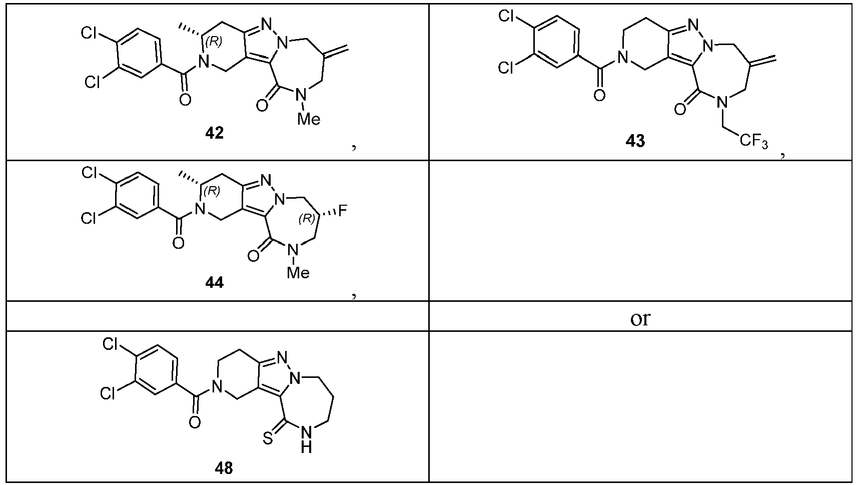

12. A pharmaceutical composition, which comprises the compound or pharmaceutically acceptable salt of any one of claims 1 to 11, and which further comprises at least one pharmaceutically acceptable excipient.

13. The compound or pharmaceutically acceptable salt of any one of claims 1 to 11, or the pharmaceutical composition of claim 12, for use as a medicament.

14. The compound or pharmaceutically acceptable salt of any one of claims 1 to 11, or the pharmaceutical composition of claim 12, for use in the prevention or treatment of an HBV infection or of an HBV-induced disease in mammal in need thereof.

15. The compound or pharmaceutically acceptable salt of any one of claims 1 to 11, or the pharmaceutical composition of claim 12, for use in the prevention or treatment of chronic hepatitis B.

16. The compound or pharmaceutically acceptable salt of any one of claims 1 to 11, or the pharmaceutical composition of claim 12, for use in the prevention or treatment of an HBV infection or of an HBV-induced disease in mammal in need thereof, in combination with another HBV inhibitor.

17. A product comprising a first compound and a second compound as a combined preparation for simultaneous, separate or sequential use in the prevention or treatment of an HBV infection or of an HBV-induced disease in mammal in need thereof, wherein said first compound is different from said second compound, wherein said first compound is the compound or pharmaceutically acceptable salt of any one of claims 1 to 11 or the pharmaceutical composition of claim 12, and wherein said second compound is another HBV inhibitor.

## Patentansprüche

1. Verbindung der Formel (I), 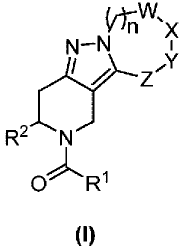
oder ein Stereoisomer oder Tautomer davon,
wobei
R¹ ein 5- bis 10-gliedriger monocyclischer oder bicyclischer Ring, insbesondere ein 5- bis 9-gliedriger monocyclischer oder bicyclischer Ring ist, wobei der 5- bis 10-gliedrige monocyclische oder bicyclische Ring, insbesondere der 5- bis 9-gliedrige monocyclische oder bicyclische Ring:
- optional 1 bis 3 Heteroatome enthält, wobei die Heteroatome jeweils unabhängig voneinander ausgewählt sind aus N, O und S; und/oder
- optional mit einem oder mehreren Substituenten substituiert ist, die jeweils unabhängig voneinander ausgewählt sind aus Wasserstoff, Halogenen, CN, CF₃, CF₂H, CFH₂, CF₂CH₃, C₁₋₆-Alkyl, OC₁₋₆-Alkyl, OCF₃, OCF₂H und C₃₋₄-Cycloalkyl;
insbesondere R¹ Phenyl ist, das mit einem oder mehreren Substituenten substituiert ist, die jeweils unabhängig ausgewählt sind aus der Gruppe, bestehend aus Cl, F, CN, CH₃, CF₃, und CF₂H;
R² aus der Gruppe ausgewählt ist, bestehend aus H, C₁₋₄-Alkyl und C₁₋₄-Alkyl, das mit einem oder mehreren F substituiert ist;
n eine ganze Zahl von 0 oder 1 ist;
W ist CHR³ oder C=CH₂ ist;
R³ aus der Gruppe ausgewählt ist, bestehend aus H, F, OH, C₁₋₄-Alkyl, das optional mit OH oder F substituiert ist, CONHC₁₋₄-Alkyl, CONHC₃₋₆-Cycloalkyl, NHSO₂C₁₋₄-Alkyl und NHSO₂C₃₋₆-Cycloalkyl;
X CH₂ ist;
Y NR⁵ ist;
R⁵ aus der Gruppe ausgewählt ist, bestehend aus
H,
C₁₋₄-Alkyl,
C₁₋₄-Alkyl, das mit einem oder mehreren F substituiert ist,
C₁₋₄-Alkyl, das mit einem 5- bis 10-gliedrigen monocyclischen oder bicyclischen aromatischen Ring substituiert ist, wobei der 5- bis 10-gliedrige monocyclische oder bicyclische aromatische Ring optional ein, zwei, drei oder vier Heteroatome enthält, wobei die Heteroatome unabhängig voneinander ausgewählt sind aus N, O und S, und
wobei der 5- bis 10-gliedrige monocyclische oder bicyclische aromatische Ring optional mit einem oder mehreren Substituenten substituiert ist, die jeweils unabhängig ausgewählt sind aus der Gruppe, bestehend aus Wasserstoff, Halogenen, CN, CF₃, CF₂H, CFH₂, CF₂CH₃, CH₂CF3, C₁₋₄-Alkyl, OH, OC₁₋₄-Alkyl, OCF₃, OCF₂H, SO₂N(CH₃)₂ und C₃₋₄-Cycloalkyl; und Z aus der Gruppe ausgewählt ist, bestehend aus C(=O) und C(=S), oder ein pharmazeutisch verträgliches Salz davon.

2. Verbindung nach Anspruch 1, wobei R¹ Phenyl ist, das mit einem oder mehreren Chlorsubstituenten substituiert ist.

3. Verbindung nach Anspruch 1 oder 2, wobei R² H oder Methyl ist.

4. Verbindung nach einem der Ansprüche 1 bis 3, wobei Z C=O ist.

5. Verfahren nach einem der Ansprüche 1 bis 4, wobei R¹ Dichlorphenyl ist.

6. Verbindung nach einem der Ansprüche 1 bis 5, wobei W CH₂ oder CHF ist.

7. Verbindung nach einem der Ansprüche 1 bis 6, wobei R⁵ aus der Gruppe ausgewählt ist, bestehend aus C₁₋₄-Alkyl und C₁₋₄-Alkyl, das mit einem 5- bis 10-gliedrigen monocyclischen oder bicyclischen aromatischen Ring substituiert ist, wobei der 5- bis 10-gliedrige monocyclische oder bicyclische aromatische Ring optional ein, zwei oder drei Heteroatome enthält, wobei die Heteroatome unabhängig voneinander ausgewählt sind aus N, O und S, und wobei der 5- bis 10-gliedrige monocyclische oder bicyclische aromatische Ring optional mit einem oder mehreren Substituenten substituiert ist, die aus der Gruppe ausgewählt sind, bestehend aus Halogenen, CN, CF₃, CF₂H, CFH₂, CF₂CH₃, CH₂CF3, C₁₋₄-Alkyl, OC₁₋₄-Alkyl, OCF₃, OCF₂H und C₃₋₄-Cycloalkyl.

8. Verbindung nach einem der Ansprüche 1 bis 6, wobei R⁵ C₁₋₄-Alkyl das, das mit einem 5- bis 10-gliedrigen monocyclischen oder bicyclischen aromatischen Ring substituiert ist, wobei der 5- bis 10-gliedrige monocyclische oder bicyclische aromatische Ring optional ein, zwei oder drei Heteroatome enthält, wobei die Heteroatome N sind, und wobei der 5- bis 10-gliedrige monocyclische oder bicyclische aromatische Ring optional mit einem oder mehreren Substituenten substituiert ist, die aus der Gruppe ausgewählt sind, bestehend aus Halogenen, CN, CF₃, CF₂H, CFH₂, CF₂CH₃, C₁₋₄-Alkyl, OC₁₋₄-Alkyl, OCF₃, OCF₂H und C₃₋₄-Cycloalkyl.

9. Verbindung nach Anspruch 8, wobei der 5- bis 10-gliedrige monocyclische oder bicyclische aromatische Ring Phenyl, Pyridyl, Pyrazolyl oder Indolyl ist und wobei der 5- bis 10-gliedrige monocyclische oder bicyclische aromatische Ring optional mit einem oder mehreren Substituenten substituiert ist, die aus der Gruppe ausgewählt sind, bestehend aus Halogenen, CN, CF₃, CF₂H, CFH₂, CF₂CH₃, C₁₋₄-Alkyl, OC₁₋₄-Alkyl, OCF₃, OCF₂H und C₃₋₄-Cycloalkyl.

10. Verbindung nach Anspruch 9, wobei der 5- bis 10-gliedrige monocyclische oder bicyclische aromatische Ring Phenyl, Pyridyl, Pyrazolyl oder Indolyl ist, und wobei der 5- bis 10-gliedrige monocyclische oder bicyclische aromatische Ring optional mit einem oder mehreren Substituenten substituiert ist, die aus der Gruppe ausgewählt sind, bestehend aus Halogenen, C₁₋₄-Alkyl, OC₁₋₄-Alkyl, OCF₃, OCF₂H.

11. Verbindung nach Anspruch 1, wobei die Verbindung ist:

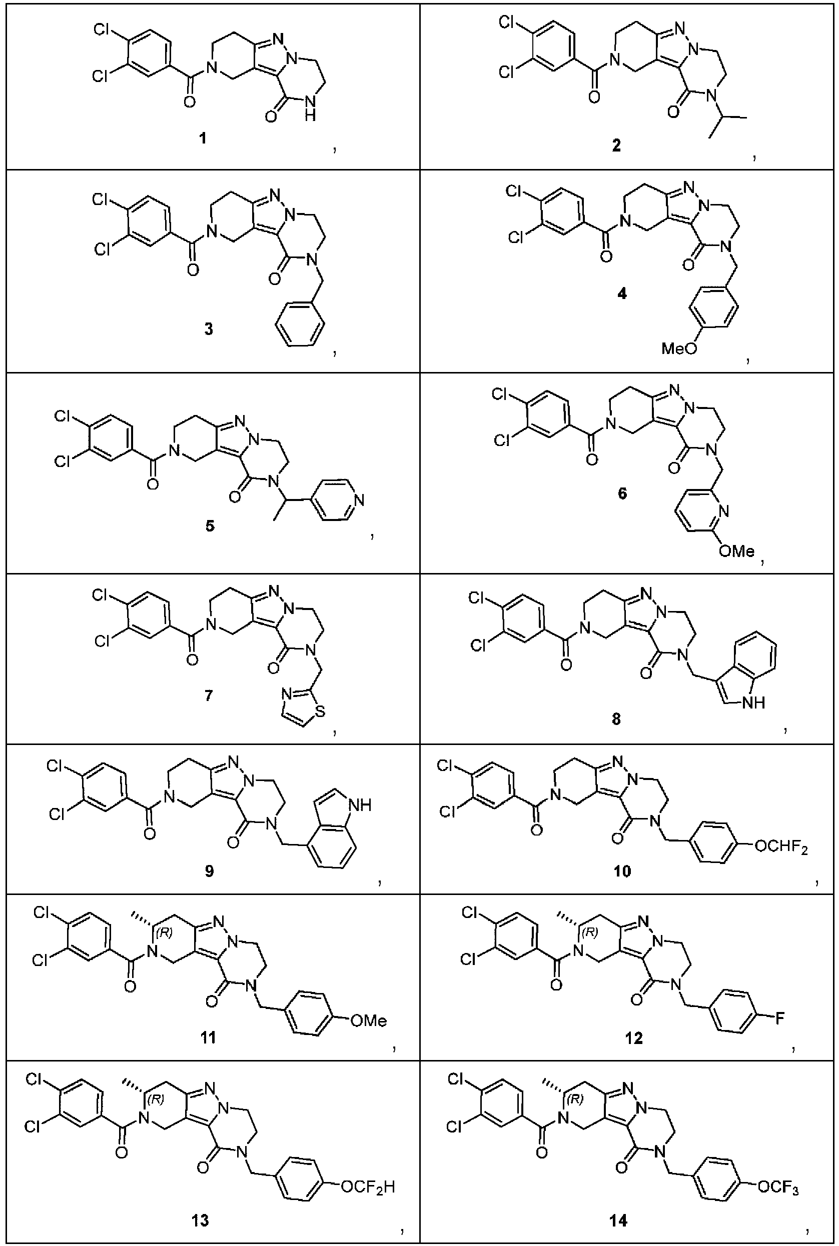

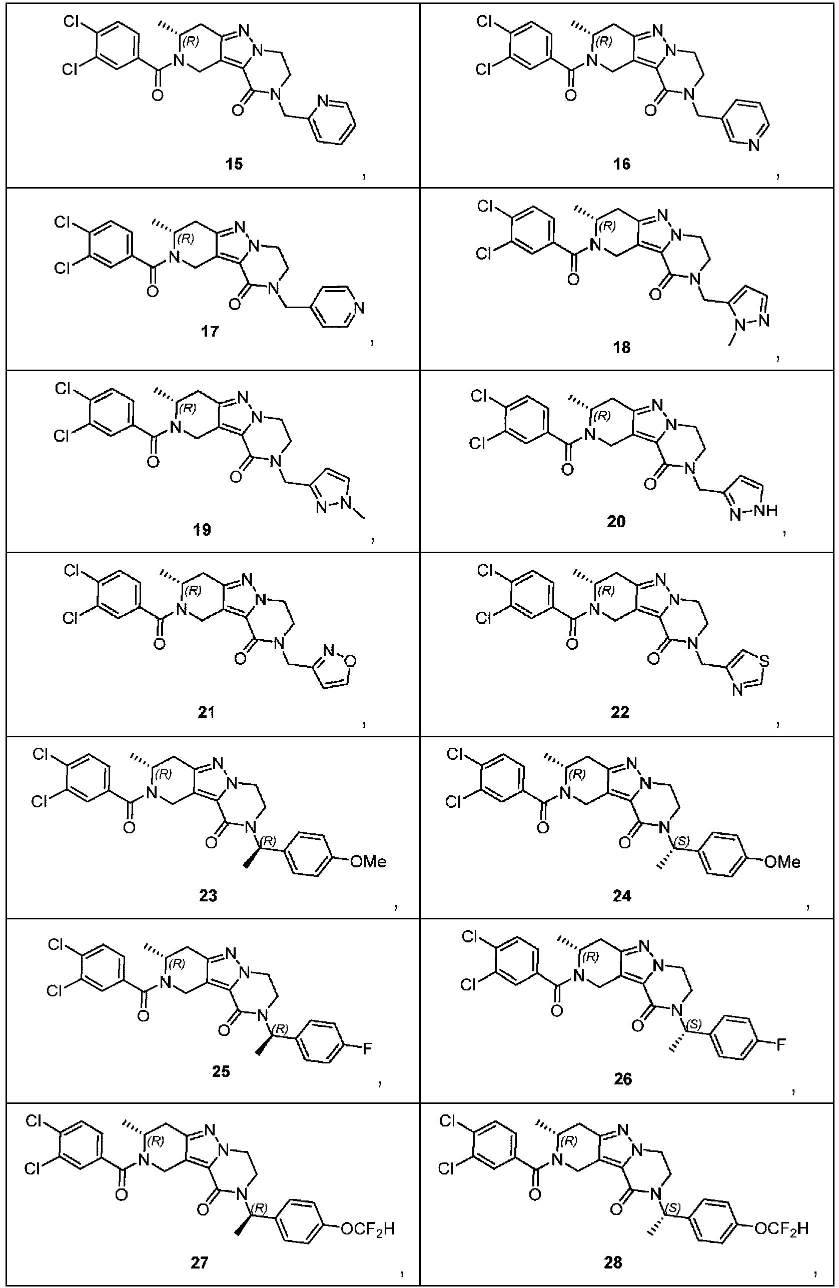

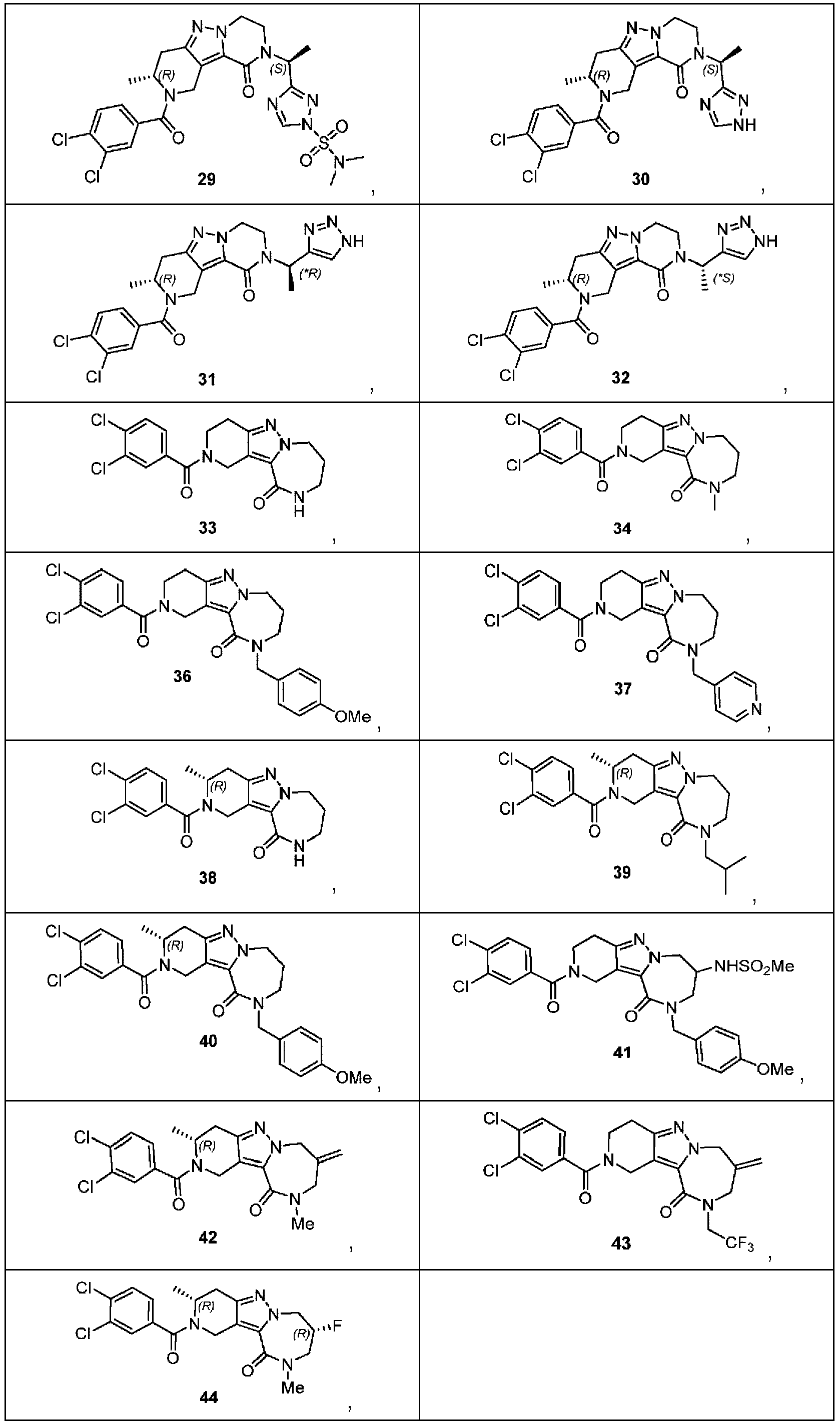

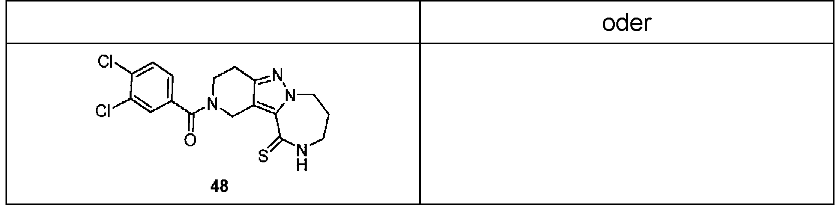

12. Pharmazeutische Zusammensetzung, die die Verbindung oder das pharmazeutisch verträgliche Salz nach einem der Ansprüche 1 bis 11 umfasst und ferner mindestens ein pharmazeutisch verträgliches Hilfsmittel umfasst.

13. Verbindung oder pharmazeutisch verträgliches Salz nach einem der Ansprüche 1 bis 11 oder pharmazeutische Zusammensetzung nach Anspruch 12 zur Verwendung als ein Medikament.

14. Verbindung oder pharmazeutisch verträgliches Salz nach einem der Ansprüche 1 bis 11 oder pharmazeutische Zusammensetzung nach Anspruch 12 zur Verwendung bei der Vorbeugung oder Behandlung einer HBV-Infektion oder einer durch HBV hervorgerufenen Krankheit bei einem Säugetier, das dieser bedarf.

15. Verbindung oder pharmazeutisch verträgliches Salz nach einem der Ansprüche 1 bis 11 oder pharmazeutische Zusammensetzung nach Anspruch 12 zur Verwendung bei der Vorbeugung oder Behandlung von chronischer Hepatitis B.

16. Verbindung oder pharmazeutisch verträgliches Salz nach einem der Ansprüche 1 bis 11 oder pharmazeutische Zusammensetzung nach Anspruch 12 zur Verwendung bei der Vorbeugung oder Behandlung einer HBV-Infektion oder einer durch HBV hervorgerufenen Krankheit bei einem Säugetier, das dieser bedarf, in Kombination mit einem weiteren HBV-Hemmer.

17. Produkt, umfassend eine erste Verbindung und eine zweite Verbindung als eine kombinierte Zubereitung zur gleichzeitigen, separaten oder sequentiellen Verwendung bei der Vorbeugung oder Behandlung einer HBV-Infektion oder einer durch HBV hervorgerufenen Krankheit bei einem Säugetier, das dieser bedarf, wobei sich die erste Verbindung von der zweiten Verbindung unterscheidet, wobei die erste Verbindung die Verbindung oder das pharmazeutisch verträgliche Salz nach einem der Ansprüche 1 bis 11 oder die pharmazeutische Zusammensetzung nach Anspruch 12 ist, wobei die zweite Verbindung ein weiterer HBV-Hemmer ist.

## Revendications

1. Composé de formule (I), 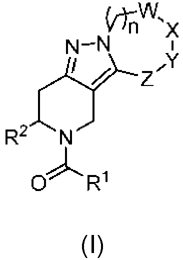
ou stéréoisomère ou tautomère de celui-ci,
dans lequel
R¹ est un cycle monocyclique ou bicyclique de 5 à 10 chaînons, plus particulièrement un cycle monocyclique ou bicyclique de 5 à 9 chaînons, dans lequel le cycle monocyclique ou bicyclique de 5 à 10 chaînons, plus particulièrement le cycle monocyclique ou bicyclique de 5 à 9 chaînons :
- contient éventuellement de 1 à 3 hétéroatomes, les hétéroatomes étant choisis chacun indépendamment parmi N, O et S ; et/ou
- est éventuellement substitué par un ou plusieurs substituants choisis chacun indépendamment parmi hydrogène, halogènes, CN, CF₃, CF₂H, CFH₂, CF₂CH₃, C₁₋₆alkyle, OC₁₋₆alkyle, OCF₃, OCF₂H et C₃₋₄cycloalkyle ;
plus particulièrement, R¹ est un phényle substitué par un ou plusieurs substituants choisis chacun indépendamment dans le groupe constitué de Cl, F, CN, CH₃, CF₃ et CF₂H ;
R² est choisi dans le groupe constitué de H, C₁₋₄alkyle, et C₁₋₄alkyle substitué par un ou plusieurs F ;
n est un nombre entier égal à 0 ou 1 ;
W est CHR³ ou C=CH₂ ;
R³ est choisi dans le groupe constitué de H, F, OH, C₁₋₄alkyle éventuellement substitué par OH ou F, CONHC₁₋₄alkyle, CONHC₃₋₆cycloalkyle, NHSO₂C₁₋₄alkyle et NHSO₂C₃₋₆cycloalkyle ;
X est CH₂ ;
Y est NR⁵ ;
R⁵ est choisi dans le groupe constitué de
H,
C₁₋₄alkyle,
C₁₋₄alkyle substitué par un ou plusieurs F,
C₁₋₄alkyle substitué par un cycle aromatique monocyclique ou bicyclique de 5 à 10 chaînons, dans lequel le cycle aromatique monocyclique ou bicyclique de 5 à 10 chaînons contient éventuellement un, deux, trois ou quatre hétéroatomes, les hétéroatomes étant choisis indépendamment parmi N, O et S, et
dans lequel ledit cycle aromatique monocyclique ou bicyclique de 5 à 10 chaînons est éventuellement substitué par un ou plusieurs substituants choisis chacun indépendamment dans le groupe constitué d'hydrogène, halogènes, CN, CF₃, CF₂H, CFH₂, CF₂CH₃, CH₂CF3, C₁₋₄alkyle, OH, OC₁₋₄alkyle, OCF₃, OCF₂H, SO₂N(CH₃)₂ et C₃₋₄cycloalkyle ; et Z est choisi dans le groupe constitué de C(=O) et C(=S), ou un sel pharmaceutiquement acceptable de celui-ci.

2. Composé selon la revendication 1, dans lequel R¹ est un phényle substitué par un ou plusieurs substituants de chlore.

3. Composé selon la revendication 1 ou 2, dans lequel R² est H ou méthyle.

4. Composé selon l'une quelconque des revendications 1 à 3, dans lequel Z est C=O.

5. Procédé selon l'une quelconque des revendications 1 à 4, dans lequel R¹ est dichlorophényle.

6. Composé selon l'une quelconque des revendications 1 à 5, dans lequel W est CH₂ ou CHF.

7. Composé selon l'une quelconque des revendications 1 à 6, dans lequel R⁵ est choisi dans le groupe constitué de C₁₋₄alkyle et C₁₋₄alkyle substitué par un cycle aromatique monocyclique ou bicyclique de 5 à 10 chaînons, dans lequel le cycle aromatique monocyclique ou bicyclique de 5 à 10 chaînons contient éventuellement un, deux ou trois hétéroatomes, les hétéroatomes étant indépendamment choisis parmi N, O et S, et dans lequel ledit cycle aromatique monocyclique ou bicyclique de 5 à 10 chaînons est éventuellement substitué par un ou plusieurs substituants choisis dans le groupe constitué de halogènes, CN, CF₃, CF₂H, CFH₂, CF₂CH₃, C₁₋₄alkyle, OC₁₋₄alkyle, OCF₃, OCF₂H et C₃₋₄cycloalkyle.

8. Composé selon l'une quelconque des revendications 1 à 6, dans lequel R⁵ est un C₁₋₄alkyle substitué par un cycle aromatique monocyclique ou bicyclique de 5 à 10 chaînons, dans lequel le cycle aromatique monocyclique ou bicyclique de 5 à 10 chaînons contient éventuellement un, deux ou trois hétéroatomes, les hétéroatomes étant N, et dans lequel ledit cycle aromatique monocyclique ou bicyclique de 5 à 10 chaînons est éventuellement substitué par un ou plusieurs substituants choisis dans le groupe constitué de halogènes, CN, CF₃, CF₂H, CFH₂, CF₂CH₃, C₁₋₄alkyle, OC₁₋₄alkyle, OCF₃, OCF₂H et C₃₋₄cycloalkyle.

9. Composé selon la revendication 8, dans lequel le cycle aromatique monocyclique ou bicyclique de 5 à 10 chaînons est phényle, pyridyle, pyrazolyle ou indolyle, et dans lequel ledit cycle aromatique monocyclique ou bicyclique de 5 à 10 chaînons est éventuellement substitué par un ou plusieurs substituants choisis dans le groupe constitué de halogènes, CN, CF₃, CF₂H, CFH₂, CF₂CH₃, C₁₋₄alkyle, OC₁₋₄alkyle, OCF₃, OCF₂H et C₃₋₄cycloalkyle.

10. Composé selon la revendication 9, dans lequel le cycle aromatique monocyclique ou bicyclique de 5 à 10 chaînons est phényle, pyridyle, pyrazolyle ou indolyle, et dans lequel ledit cycle aromatique monocyclique ou bicyclique de 5 à 10 chaînons est éventuellement substitué par un ou plusieurs substituants choisis dans le groupe constitué de halogènes, C₁₋₄alkyle, OC₁₋₄alkyle, OCF₃ et OCF₂H.

11. Composé selon la revendication 1, dans lequel le composé est :

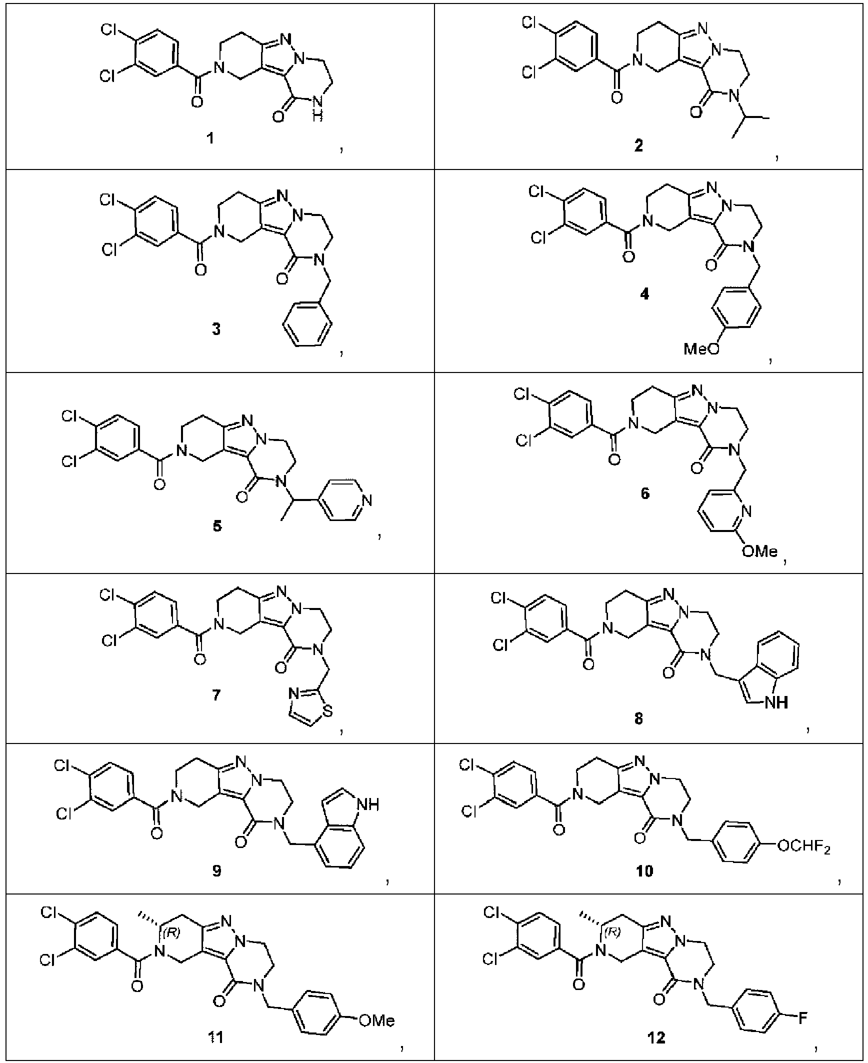

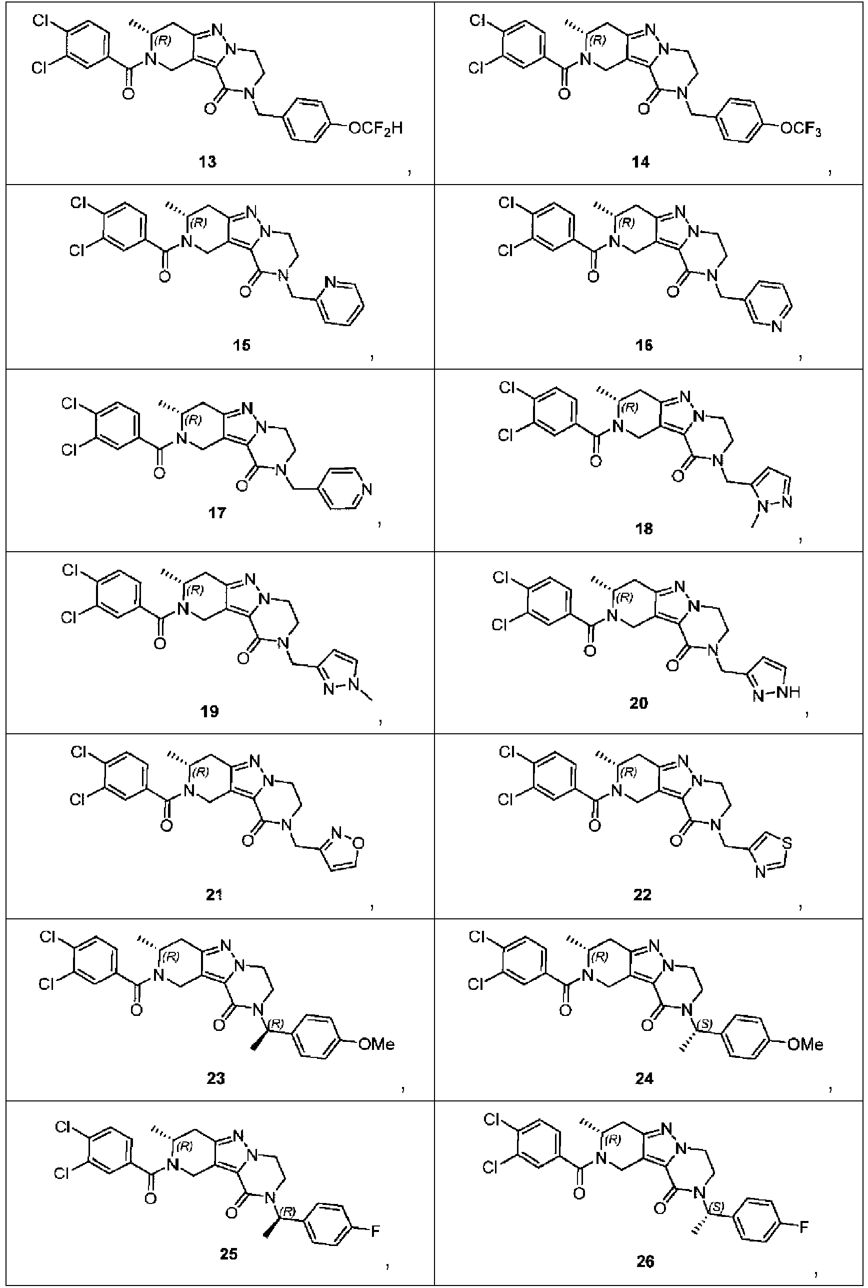

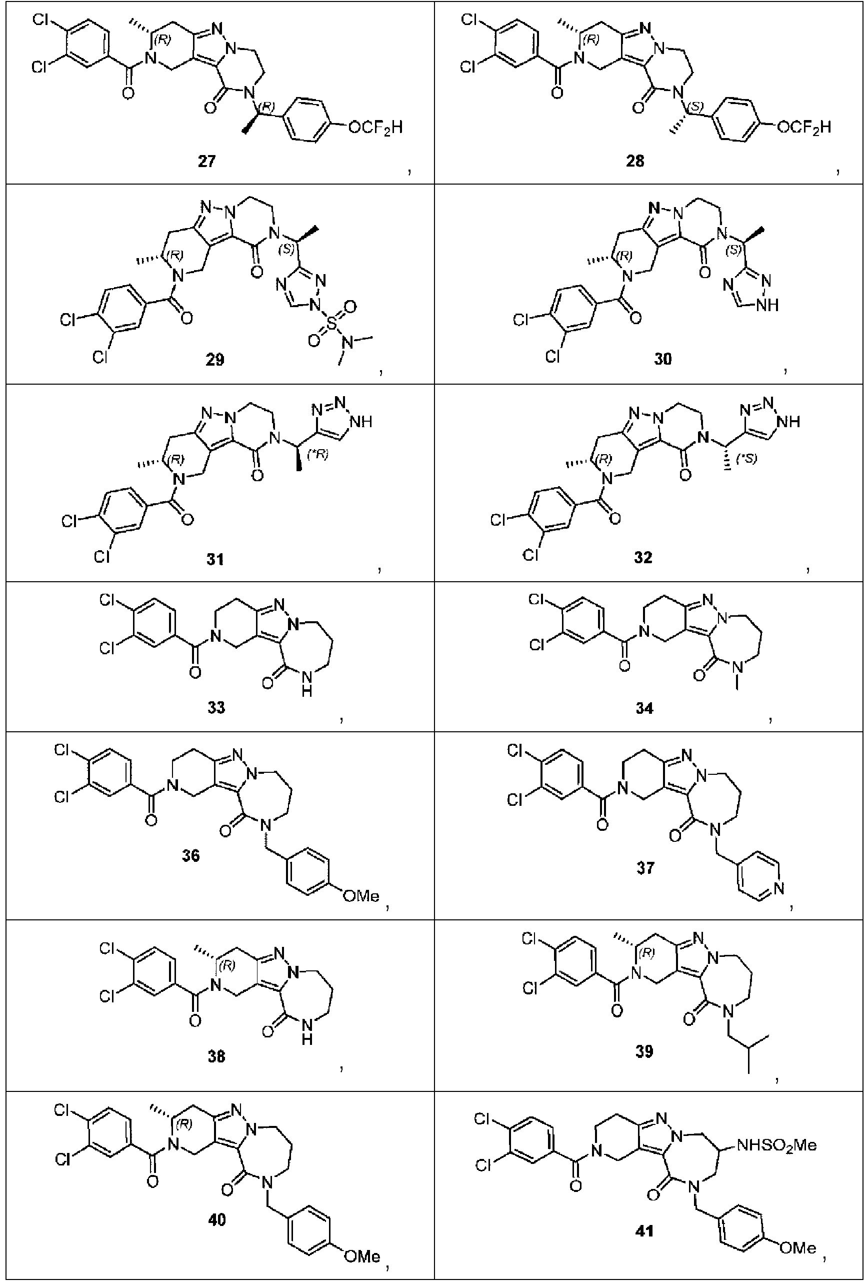

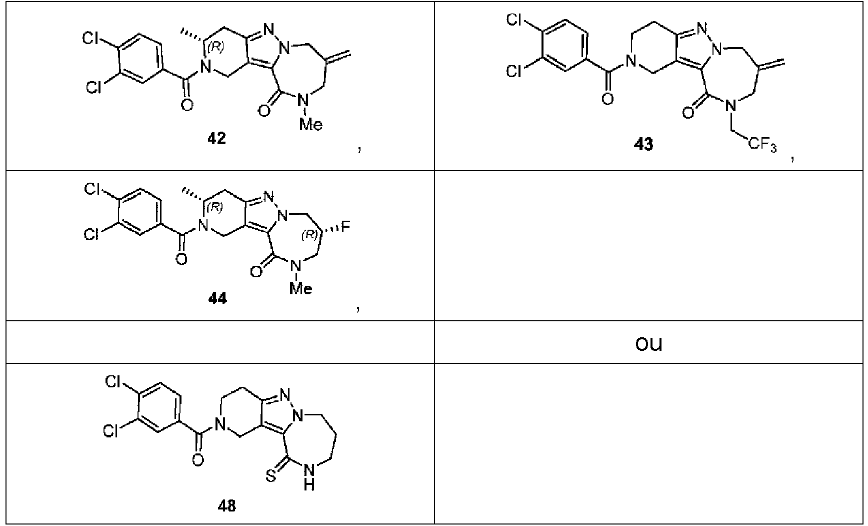

12. Composition pharmaceutique, qui comprend le composé ou sel pharmaceutiquement acceptable selon l'une quelconque des revendications 1 à 11, et qui comprend en outre au moins un excipient pharmaceutiquement acceptable.

13. Composé ou sel pharmaceutiquement acceptable selon l'une quelconque des revendications 1 à 11, ou composition pharmaceutique selon la revendication 12, pour une utilisation en tant que médicament.

14. Composé ou sel pharmaceutiquement acceptable selon l'une quelconque des revendications 1 à 11, ou composition pharmaceutique selon la revendication 12, pour une utilisation dans la prévention ou le traitement d'une infection par le VHB ou d'une maladie induite par le VHB chez un mammifère qui en a besoin.

15. Composé ou sel pharmaceutiquement acceptable selon l'une quelconque des revendications 1 à 11, ou composition pharmaceutique selon la revendication 12, pour une utilisation dans la prévention ou le traitement de l'hépatite B chronique.

16. Composé ou sel pharmaceutiquement acceptable selon l'une quelconque des revendications 1 à 11, ou composition pharmaceutique selon la revendication 12, pour une utilisation dans la prévention ou le traitement d'une infection par le VHB ou d'une maladie induite par le VHB chez un mammifère qui en a besoin, en association avec un autre inhibiteur du VHB.

17. Produit comprenant un premier composé et un second composé en tant que préparation combinée pour une utilisation simultanée, séparée ou séquentielle dans la prévention ou le traitement d'une infection par le VHB ou dune maladie induite par le VHB chez un mammifère qui en a besoin, dans laquelle ledit premier composé est différent dudit second composé, dans lequel ledit premier composé est le composé ou sel pharmaceutiquement acceptable selon l'une quelconque des revendications 1 à 11 ou la composition pharmaceutique selon la revendication 12, et dans lequel ledit second composé est un autre inhibiteur du VHB.
